# EUROPEAN PATENT APPLICATION

(11) **EP 1 477 806 A1**
(43) Date of publication of application: **17.11.2004**
(21) Application number: 03706989.5
(22) Date of filing: 21.02.2003
(51) Int. Cl.: G01N 33/50, G01N 33/15, C12N 15/09

(54) **METHOD OF DETERMINING LIGAND**

(30) Priority: 22.02.2002 JP 2002045728; 23.07.2002 JP 2002213949; 11.10.2002 JP 2002298237
(71) Applicant: Takeda Chemical Industries, Ltd., Osaka-shi, Osaka 541-0045 (JP)
(72) Inventor: HINUMA, Shuji, Tsukuba-shi, Ibaraki 305-0821 (JP); FUJII, Ryo, Tsukuba-shi, Ibaraki 305-0821 (JP); OGI, Kazuhiro, Tsukuba-shi, Ibaraki 305-0045 (JP); KOMATSU, Hidetoshi, Ibaraki 300-4117 (JP); KAWAMATA, Yuji, Tsukuba-shi, Ibaraki 305-0035 (JP); HOSOYA, Masaki, Tsuchiura-shi, Ibaraki 300-0007 (JP)
(74) Representative: Lewin, John Harvey
(86) International application number: PCT/JP2003/001901
(87) International publication number: WO 2003/071272

(57) **Abstract**

The present invention aims at providing a method of determining a ligand to an orphan receptor. Specifically, the present invention provides a method of determining a ligand to a receptor protein, to which no ligand has been determined, which comprises using a fusion protein of the receptor protein and a fluorescent protein.

## Description

### FIELD OF THE INVENTION

The present invention relates to a method of determining a ligand to a receptor protein present on cell membrane and in particular, relates to a method of determining a ligand to a so-called orphan receptor, of which the ligand is totally unknown.

### BACKGROUND ART

Physiologically active substances such as various hormones and neurotransmitters regulate the biological function through specific receptor proteins present on cell membranes. Many of these receptor proteins are coupled with guanine nucleotide-binding protein (hereinafter sometimes simply referred to as G protein) and mediate the intracellular signal transduction through activation of G protein. These receptor proteins have the common structure containing seven transmembrane domains and are thus collectively referred to as G protein-coupled receptor proteins or seven-transmembrane receptor proteins (7TMR).

G protein-coupled receptor proteins are present on the cell surface of each functional cell and organ in an organism, and play important physiological roles as the target of the molecules that regulate the functions of the cells and organs, e.g., hormones, neurotransmitters, physiologically active substances or the like. Receptors transmit signals to cells through binding with physiologically active substances, and the signals induce various reactions such as activation or suppression of the cells.

To clarify the relationship between substances that regulate the functions of cells and organs in various organisms, and their specific receptor proteins, in particular, G protein-coupled receptor proteins would elucidate the functions of cells and organs in various organisms to provide a very important means for development of pharmaceuticals closely associated with the functions.

Substances that inhibit binding of G protein-coupled receptor proteins to physiologically active substances (i.e., ligands) or substances that bind and induce signal transduction similar to that induced by physiologically active substances (i.e., ligands) have been hitherto used for pharmaceuticals, as antagonists and agonists specific to these receptors that regulate the biological functions.

However, even at the present moment, there are a large number of so-called orphan receptors in which the corresponding ligands are yet unidentified. Thus, it has been earnestly desired to identify the ligands and elucidate their functions.

G protein-coupled receptors are useful in searching for a novel physiological active substance (i.e., ligand) using the signal transduction activity as the index and in search for agonists and antagonists to the receptor. Ligands, agonists, antagonists, or the like to these receptors are expected to be used as prophylactic and/or therapeutic and diagnostic agents for diseases associated with dysfunction of the G protein-coupled receptors.

GFP (Green Fluorescent Protein) is one of fluorescent proteins derived from the jellyfish Aequorea Victoria of mesozoan animals (WO 96/23810, WO 96/27675, WO 97/26333, WO 97/28261, WO 97/42320).

A method of determining a ligand to Gi-, Gs- or Gq-coupled orphan receptor using multiple response elements (MRE) and a cAMP response element (CRE) in combination with a reporter activity is reported (Analytical Biochemistry, 275, 54-61, 1999).

A method of screening molecules which interact on Gs- or Gq-coupled orphan receptors using a cAMP response element (CRE) in combination with a reporter activity is reported (Analytical Biochemistry, 226, 349-354, 1995).

A method of characterization for Gi- or Gs-coupled receptors with known ligands using a cAMP response element (CRE) in combination with a reporter activity is reported (Current Opinion in Biotechnology, 1995, 6: 574-581).

A method of detecting the response of a G protein-coupled receptor using a cell in which chimera G protein α subunits with increased promiscuity in coupling specificity for the G protein-coupled receptor, chimera G protein has been expressed is reported (WO 01/36481).

A method of identifying a ligand for an orphan G protein-coupled receptor using a recombinant yeast expression library is reported (USP 6,255,059).

A method of identifying a receptor activity modifying substance composed of a cell containing both a receptor and a test peptide, which cell is useful for identifying or screening an orphan receptor is reported (US 2001/0026926).

An expression system for identifying a ligand to an orphan receptor utilizing the gamogenesis system of Saccharomyces is reported (WO 00/031261).

In conventional methods for searching/identifying a ligand, for example, when a ligand is screened using an eukaryotic cell, a so-called stable cell line capable of stably expressing its receptor should be established, and a special cell was required for establishing the cell line. Moreover, it was necessary for the screening to use a plurality of assays in combination. Thus, when a plurality of test compounds existed, it took a long time, which made it difficult to assay them. That is, conventional methods for searching/identifying ligands, etc. encounter problems that (1) a usable cell line is restricted, (2) it takes time to establish the cell line, (3) because of using a plurality of assays in combination, the number of specimens increases so that it becomes difficult to implement the methods, and so on. A method of determining a ligand that solves these problems and can use various cell lines and implement in a short period of time is desired.

### DISCLOSURE OF THE INVENTION

The present inventors made extensive investigations. As a result, by preparing a cell wherein a fusion protein of a receptor protein, to which no ligand has been determined, and a fluorescent protein such as GFP was stably or transiently expressed, the inventors have found (1) to (5) below, using immunostaining assay, western blotting assay, etc. utilizing fluorescence from a fluorescent protein such as GFP or a fluorescent protein antibody such as a GFP antibody that:
(1) expression of the receptor protein could be confirmed on a protein level;
(2) expression of the receptor protein on a cell membrane could be confirmed;
(3) an expression level of the receptor protein could be approximated;
(4) a cell with the receptor protein being highly expressed could be screened; and,
(5) a specific reaction of the receptor with the ligand could be detected as intracellular internalization of the fusion protein of the receptor and the fluorescent protein; etc. By utilizing these characteristics, the inventors found that a ligand to the receptor protein for which a ligand has not been identified (hereinafter sometimes simply referred to as an orphan receptor) could efficiently be determined. Based on these findings, the present inventors have made further investigations and come to accomplish the present invention.

That is, the present invention relates to the following features:
[1] A method of determining a ligand to a receptor protein for which a ligand has not been identified, which comprises using a fusion protein of the receptor protein with a fluorescent protein;
[2] The ligand determination method according to [1], wherein the fusion protein of a receptor protein for which a ligand has not been identified and GFP is used;
[3] The ligand determination method according to [1], wherein a cell expressed with the fusion protein of a receptor protein for which a ligand has not been identified and GFP or a membrane fraction of the cell ;
[4] The ligand determination method according to [1], which comprises assaying (1) an activity that accelerates or suppresses arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos or pH reduction, (2) MAP kinase activation, (3) transcription factor activation, (4) diacylglycerol production, (5) opening or closing of ion channels on a cell membrane, (6) apoptosis induction, (7) morphological changes in cells, (8) transport of the fusion protein from cell membrane to cytoplasm, (9) low molecular weight G protein activation, (10) cell division-promoting activity or (11) DNA synthesis-promoting activity;
[5] The ligand determination method according to [1], wherein transport of the fusion protein from cell membrane to cytoplasm is assayed;
[6] The ligand determination method according to [5], wherein transport of the fusion protein from cell membrane to cytoplasm is assayed by observing the fluorescence of GFP;
[7] The ligand determination method according to [1], which comprises bringing a cell capable of expressing the fusion protein of a receptor protein for which a ligand has not been identified the receptor protein and a fluorescent protein and containing a plasmid ligated with a DNA encoding a reporter protein at the downstream of cAMP response element/promoter in contact with a test compound and assaying the activity of the reporter protein;
[8] The method according to [7], which comprises culturing a cell containing (1) a plasmid containing a DNA encoding the fusion protein of a receptor protein for which a ligand has not been identified and a fluorescent protein and (2) a plasmid ligated with a DNA encoding a reporter protein at the downstream of cAMP response element/promoter, bringing the cell in contact with a test compound and assaying the activity of the reporter protein;
[9] The ligand determination method according to [2], wherein a cell capable of expressing the fusion protein of a receptor protein for which a ligand has not been identified and GFP and containing a plasmid ligated with a DNA encoding a reporter protein at the downstream of cAMP response element/promoter and assaying the activity of the reporter protein;
[10] The ligand determination method according to [9], which comprises culturing a cell containing (1) a plasmid containing a DNA encoding the fusion protein of a receptor protein for which a ligand has not been identified and GFP and (2) a plasmid ligated with a DNA encoding a reporter protein at the downstream of cAMP response element/promoter, bringing the cell in contact with a test compound and assaying the activity of the reporter protein;
[11] The method according to [1], wherein the receptor protein is a G protein-coupled receptor protein;
[12] The method according to [1], wherein GFP is a protein containing the same or substantially the same amino acid sequence as the amino acid sequence represented by SEQ ID NO: 1, SEQ ID NO: 3, SEQ ID NO: 5 or SEQ ID NO: 7;
[13] The method according to [7], wherein the promoter is a TATA-like sequence;
[14] The method according to [7], wherein the reporter protein is luciferase;
[15] The method according to [7], wherein the plasmid is a plasmid ligated with a TATA-like promoter and a gene encoding the reporter protein at the downstream of cAMP response element;
[16] The method according to [7], wherein the cell has expressed at least two receptor proteins for which ligand s have not been identified;
[17] The method according to [7], wherein the cell further contains a plasmid containing a gene encoding an inhibitory G protein α subunit Gi;
[18] The method according to [7], wherein forskolin is further added;
[19] The method according to [16], wherein the at least two receptor proteins have similar characteristics;
[20] The method according to [19], wherein the similar characteristics are the basic expression level of the reporter protein and/or the expression level of the reporter protein when forskolin is added;
[21] The method according to [16], which comprises measuring the basic expression level of the reporter protein when the at least two receptor proteins are previously expressed individually and/or the expression level of the reporter protein by the addition of forskolin and being expressed in combination the at least two receptor proteins having an equivalent expression level of the reporter protein ;
[22] A fusion protein of a receptor protein for which a ligand has not been identified and a fluorescent protein, or a salt thereof;
[23] The fluorescent protein or a salt thereof according to [22], wherein the fluorescent protein is GFP;
[24] A DNA containing a DNA encoding the fusion protein according to [22];
[25] A recombinant vector containing the DNA according to [22];
[26] A transformant transformed with the recombinant vector according to [25];
[27] Use of a fluorescent protein to determine a ligand to a receptor protein for which a ligand has not been identified; and,
[28] Use of GFP to determine a ligand to a receptor protein for which a ligand has not been identified.

### BRIEF DESCRIPTION OF THE DRAWINGS

FIG. 1 shows the results (n=2) of detection of reporter activation by cholesterol metabolism-related substances in HEK293 cells transfected with TGR5 expression vector (A) and HEK293 cells transfected with intact vector alone (B).
FIG. 2 shows the results of induced expression of reporter genes by ligand stimulation in HEK293 cells.
FIG. 3 shows the results of TGR5 response to lithocholic acid in the co-presence of Gi.
FIG. 4 shows localization of the TGR5-GFP fusion protein expressed in CHO cells in the absence of ligand. In the figure, white line shows the length of 4 µm.
FIG. 5 shows localization of the fusion protein 30 minutes after the addition of TLCA to TGR5-GFP-expressed CHO cells. In the figure, white line shows the length of 4 µm.

### BEST MODE FOR CARRYING OUT THE INVENTION

The ligand determination method of the present invention is characterized by using (1) a fusion protein of a receptor protein for which a ligand has not been identified and a fluorescent protein, more specifically, is a method which comprises bringing a cell wherein the fusion protein of a receptor protein for which a ligand has not been identified and a fluorescent protein has been expressed or a membrane fraction of the cell in contact with a test compound (ligand determination method A).

Furthermore, the ligand determination method of the present invention includes (1) a method which involves bringing a cell capable of expressing a fusion protein of a receptor protein for which a ligand has not been identified and a fluorescent protein and containing a plasmid ligated with DNA encoding a reporter protein at the downstream of enhancer/promoter in contact with a test compound and assaying the activity of the expression-induced reporter protein, and (2) a method which involves culturing a cell containing (i) a plasmid containing DNA encoding a fusion protein of a receptor protein for which a ligand has not been identified and a fluorescent protein and (ii) a plasmid ligated with DNA encoding a reporter protein at the downstream of enhancer/promoter, and bringing the cell in contact with a test compound and assaying the activity of the expression-induced reporter protein (ligand determination method B).

As the orphan receptors used in the present invention, for example, G protein-coupled receptor proteins, etc. are employed. Specific examples include G protein-coupled receptor proteins containing the same or substantially the same amino acid sequence as the amino acid sequence represented by SEQ ID NO: 9, G protein-coupled receptor proteins described in WO96/05302, EP-A-711831, EP-A-789076, EP-A-1103563, EP-A-1103562, Published Japanese Patent Application KOKAI Nos. 8-154682, 8-283295, 8-196278, 8-245697, 8-266280, 9-51795, 9-121865, 9-2388686 and 10-146192, and the like.

The fluorescent proteins are not particularly limited as far as it is visually recognizable, and include, e.g., GFP (Green Fluorescent Protein), Tag sequence, EGFP (enhanced green fluorescent protein), ECFP (enhanced cyan fluorescent protein), EYFP (enhanced yellow fluorescent protein), DsRED (Discosoma sp. red fluorescent protein), EBFP (enhanced blue fluorescent protein), etc.

GFP is one of fluorescent proteins derived from the jellyfish Aequorea Victoria of mesozoan animals, and includes proteins containing the same or substantially the same amino acid sequence as the amino acid sequence represented by SEQ ID NO: 1, SEQ ID NO: 3, SEQ ID NO: 5 or SEQ ID NO: 7, etc.

The amino acid sequence which is substantially the same amino acid sequence as the amino acid sequence represented by SEQ ID NO: 1, SEQ ID NO: 3, SEQ ID NO: 5 or SEQ ID NO: 7 includes an amino acid sequence having at least about 70% homology, preferably at least about 80% homology, more preferably at least about 90% homology and most preferably at least about 95% homology, to the amino acid sequence represented by SEQ ID NO: 1, SEQ ID NO: 3, SEQ ID NO: 5 or SEQ ID NO: 7; or the like.

Preferred examples of the protein of the present invention which contains substantially the same amino acid sequence as the amino acid sequence represented by SEQ ID NO: 1, SEQ ID NO: 3, SEQ ID NO: 5 or SEQ ID NO: 7 include a protein having substantially the same amino acid sequence as the amino acid sequence represented by SEQ ID NO: 1, SEQ ID NO: 3, SEQ ID NO: 5 or SEQ ID NO: 7 and having the activity substantially equivalent to proteins comprising the amino acid sequence represented by SEQ ID NO: 1, SEQ ID NO: 3, SEQ ID NO: 5 or SEQ ID NO: 7; and the like.

The activities of GFP include, e.g., fluorescence by excitation light irradiation, etc. The term substantially equivalent activities are used to mean that these activities are equivalent in nature. Thus, though it is preferred that these activities are equivalent (e.g., about 0.01- to about 100-fold, preferably about 0.5- to about 20-fold, more preferably about 0.5- to about 2-fold), quantitative factors such as an activity level, a molecular weight of the protein, etc. may be different.

The activities in GFP such as fluorescence by excitation light irradiation, etc. can be assayed by publicly known methods with modifications.

Proteins containing the following amino acid sequences are used as GFP: (1) the amino acid sequences wherein at least 1 or 2 amino acids (preferably approximately 1 to 30 amino acids, more preferably approximately 1 to 10 amino acids, most preferably several (1 to 5) amino acids) are deleted in the amino acid sequence represented by SEQ ID NO: 1, SEQ ID NO: 3, SEQ ID NO: 5 or SEQ ID NO: 7; (2) the amino acid sequences wherein at least 1 or 2 amino acids (preferably approximately 1 to 30 amino acids, more preferably approximately 1 to 10 amino acids, and most preferably several (1 to 5) amino acids) are added to or inserted into the amino acid sequence represented by SEQ ID NO: 1, SEQ ID NO: 3, SEQ ID NO: 5 or SEQ ID NO: 7; (3) the amino acid sequences wherein at least 1 or 2 amino acids (preferably approximately 1 to 30 amino acids, more preferably approximately 1 to 10 amino acids, and most preferably several (1 to 5) amino acids) are substituted by other amino acids in the amino acid sequence represented by SEQ ID NO: 1, SEQ ID NO: 3, SEQ ID NO: 5 or SEQ ID NO: 7; or (4) the amino acid sequence wherein these deletion, addition and substitution are combined; etc. Among others, preferably used are proteins containing (i) the amino acid sequence wherein the N-terminal methionine residue is deleted in the amino acid sequence represented by SEQ ID NO: 1, SEQ ID NO: 3, SEQ ID NO: 5 or SEQ ID NO: 7, (ii) the amino acid sequence wherein the N-terminal methionine residue is deleted and the following alanine residue is substituted with a threonine residue or serine residue in the amino acid sequence represented by SEQ ID NO: 1, SEQ ID NO: 3, SEQ ID NO: 5 or SEQ ID NO: 7; etc.

Specifically, there are used, for example, (i) GFP consisting of amino acid sequences represented by SEQ ID NO: 1, SEQ ID NO: 3, SEQ ID NO: 5 or SEQ ID NO: 7, (ii) GFP consisting of amino acid sequences represented by SEQ ID NO: 1, SEQ ID NO: 3, SEQ ID NO: 5 or SEQ ID NO: 7, wherein the N-terminal methionine residue is deleted, (iii) GFP consisting of amino acid sequences represented by SEQ ID NO: 1, wherein the N-terminal methionine residue is deleted and the following alanine residue is substituted with a threonine residue or serine residue; etc.

As the Tag sequence, for example, known sequences below are employed.
(1) His-tag (PCDNA3.1/His A) His His His His His His (SEQ ID NO: 17)
(2) V5-tag (PCDNA3.1/V5-His A) 
Gly Lys Pro Ile Pro Asn Pro Leu Leu Gly Leu Asp Ser Thr (SEQ ID NO:
18)
(3) myc-tag (pCDNA3.1/myc-His A) Glu Gln Lys Leu Ile Ser Glu Glu Asp Leu (SEQ ID NO: 19)
(4) Xpress-tag (PCDNA3.1/His A) Asp Leu Tyr Asp Asp Asp Asp Lys (SEQ ID NO: 20)
(5) HA-tag (PCluzHA Expression vector)

Met Gly Ser Tyr Pro Tyr Asp Val Pro Asp Tyr Ala Ser Leu Glu Phe (SEQ
ID NO: 21)

As EGFP, there is used a protein consisting of the amino acid sequence represented by SEQ ID NO: 7.

As ECFP, there is used a protein consisting of the amino acid sequence represented by SEQ ID NO: 22.

As EYFP, there is used a protein consisting of the amino acid sequence represented by SEQ ID NO: 24.

As DsRED, there is used a protein consisting of the amino acid sequence represented by SEQ ID NO: 26.

As EBFP, there is used a protein consisting of the amino acid sequence represented by SEQ ID NO: 28.

These EGFP, ECFP, EYFP, DsRED and EBFP may be proteins having (1) the amino acid sequence wherein at least 1 or 2 amino acids (preferably approximately 1 to 30 amino acids, more preferably approximately 1 to 10 amino acids, most preferably several (1 to 5) amino acids) are deleted in the amino acid sequences described above; (2) the amino acid sequence wherein at least 1 or 2 amino acids (preferably approximately 1 to 30 amino acids, more preferably approximately 1 to 10 amino acids, and most preferably several (1 to 5) amino acids) are added to or inserted into the amino acid sequences described above; (3) the amino acid sequence wherein at least 1 or 2 amino acids (preferably approximately 1 to 30 amino acids, more preferably approximately 1 to 10 amino acids, and most preferably several (1 to 5) amino acids) are substituted by other amino acids in the amino acid sequences described above; or (4) the amino acid sequence wherein these deletion, addition and substitution are combined; etc., so long as they have the activities such as fluorescence by excitation light irradiation, etc.

Specifically, the DNA encoding GFP may be any DNA, so long as it is, for example, DNA containing the base sequence represented by SEQ ID NO: 2, SEQ ID NO: 4, SEQ ID NO: 6 or SEQ ID NO: 8, or DNA having a base sequence hybridizable to a complementary base sequence to the base sequence represented by SEQ ID NO: 2, SEQ ID NO: 4, SEQ ID NO: 6 or SEQ ID NO: 8 under high stringent conditions and encoding a protein which has the activities (e.g., fluorescence by excitement light irradiation, etc.) substantially equivalent to those of GFP consisting of the amino acid sequence represented by SEQ ID NO: 1, SEQ ID NO: 3, SEQ ID NO: 5 or SEQ ID NO: 7.

The DNA hybridizable to the base sequence represented by SEQ ID NO: 2, SEQ ID NO: 4, SEQ ID NO: 6 or SEQ ID NO: 8 includes DNA containing a base sequence having at least about 70% homology, preferably at least about 80% homology, more preferably at least about 90% homology and most preferably at least about 95% homology, to the base sequence represented by SEQ ID NO: 2, SEQ ID NO: 4, SEQ ID NO: 6 or SEQ ID NO: 8; etc.

The hybridization can be carried out by publicly known methods or by modifications of these methods, for example, according to the method described in Molecular Cloning, 2nd (J. Sambrook et al., Cold Spring Harbor Lab. Press, 1989), etc. A commercially available library may also be used according to the instructions of the attached manufacturer's protocol. More preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, the DNA encoding GFP consisting of the amino acid sequence represented by SEQ ID NO: 1 includes DNA containing the base sequence represented by SEQ ID NO: 2 (WO 97/42320), etc.; the DNA encoding GFP consisting of the amino acid sequence represented by SEQ ID NO: 3 includes DNA containing the base sequence represented by SEQ ID NO: 4 (WO 96/23810), etc.; the DNA encoding GFP (GFPuv) consisting of the amino acid sequence represented by SEQ ID NO: 5 includes DNA containing the base sequence represented SEQ ID NO: 6, etc.; the DNA encoding GFP (GFPuv) consisting of the amino acid sequence represented by SEQ ID NO: 5 includes DNA containing the base sequence represented SEQ ID NO: 6, etc. (WO 97/26333), etc.; the DNA encoding GFP (EGFP) consisting of the amino acid sequence represented by SEQ ID NO: 7 includes DNA containing the base sequence represented SEQ ID NO: 8, etc. (NCBI Accession No. AAB0572), etc.

For the DNA encoding EGFP consisting of the amino acid sequence represented by SEQ ID NO: 7, there may be employed DNA consisting of the base sequence represented by SEQ ID NO: 8, etc.

For the DNA encoding ECFP consisting of the amino acid sequence represented by SEQ ID NO: 22, there may be employed DNA consisting of the base sequence represented by SEQ ID NO: 23, etc.

For the DNA encoding EYFP consisting of the amino acid sequence represented by SEQ ID NO: 24, there may be employed DNA consisting of the base sequence represented by SEQ ID NO: 25, etc.

For the DNA encoding DsRED consisting of the amino acid sequence represented by SEQ ID NO: 26, there may be employed DNA consisting of the base sequence represented by SEQ ID NO: 27, etc.

For the DNA encoding EBFP consisting of the amino acid sequence represented by SEQ ID NO: 28, there may be employed DNA consisting of the base sequence represented by SEQ ID NO: 29, etc.

The fusion protein of an orphan receptor and a fluorescent protein can be manufactured using DNA ligated with DNA encoding the orphan receptor protein and DNA encoding GFP.

The ligated DNA is constructed in frame by ligating the 5' end of DNA encoding GFP with the 3' end of the base sequence of DNA encoding the orphan receptor.

Also, DNA encoding an amino acid sequence composed of approximately 1 to 5 amino acid residues with a small molecular weight such as Ala, Gly, Ser, etc., which is called a linker, may be inserted between the both DNAs.

The expression vector for the fusion protein of an orphan receptor and a fluorescent protein (hereinafter sometimes simply referred to as the fusion protein) can be manufactured, for example, by (1) preparing a DNA fragment encoding the fusion protein and (2) ligating the DNA fragment at the downstream of a promoter in an appropriate expression vector.

As the fusion protein of a receptor protein for which a ligand has not been identified and a fluorescent protein, preferably used are, for example, fusion proteins of 102 kinds of receptor proteins for which ligands have not been identified, each receptor protein having the amino acid sequence represented by any one of SEQ ID NO: 30 through SEQ ID NO: 131, and GFP consisting of the amino acid sequence represented by SEQ ID NO: 1. In these amino acid sequences represented by any of SEQ ID NO: 30 through SEQ ID NO: 131, the N-terminal methionine residue is deleted in the amino acid sequence represented by SEQ ID NO: 1 and as the case may be, the following alanine residue is further replaced by a threonine residue or a serine residue.

The DNA encoding this fusion protein can be prepared by ligating DNA (SEQ ID NO: 2) encoding GFP consisting of the amino acid sequence represented by SEQ ID NO: 1 at the downstream of the region encoding each of the 102 kinds of receptor proteins for which ligands have not been identified. Specifically, a DNA having the base sequence represented by either one of SEQ ID NO: 132 through SEQ ID NO: 233 is used. In these base sequences represented by any of SEQ ID NO: 132 through SEQ ID NO: 233, the 5' end methionine residue codon is deleted in the base sequence represented by SEQ ID NO: 2 and as the case may be, the following alanine residue codon is further replaced by a threonine residue codon or a serine residue codon.

Examples of the expression vector include plasmids derived form Escherichia coli (e.g., pCR4, pCR2.1, pBR322, pBR325, pUC12, pUCl3), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc., as well as pA1-11, pXT1, pRc/CMV, pRc/RSV, pcDNAI/Neo, etc.

The promoter used in the vector may be any promoter if it is suitable to function well with a host used for gene expression. For example, in the case of using animal cells as the host, SRα promoter, SV40 promoter, LTR promoter, CMV promoter, HSV-TK promoter, etc. are used.

Further in the case of expressing in various tissues, there are employed insulin II promoter (pancreas), Glycoprotein-α subunit promoter (pituitary), transthyretin promoter (liver), renin promoter (kidney), PSE promoter (prostate), CD2 promoter (T cell), IgG-heavy chain promoter (B cell), scavenger-receptor A promoter (macrophage), etc.

Where the host is bacteria of the genus Escherichia, preferred examples of the promoter include trp promoter, lac promoter, recA promoter, λP_{L} promoter, lpp promoter, etc. Where the host is bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in CHO (dhfr⁻) cells, selection can also be made on thymidine free media.

If necessary, a signal sequence that matches with a host is added to the N-terminus of the receptor protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the expression vector containing the DNA encoding the fusion protein of the receptor protein and the fluorescent protein thus constructed, transformants can be manufactured.

Examples of the host, which may be used, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), DH5α (Inoue, H., Nojima, H., Gene, 96, 23-28 (1990)), DH10B (Proc. Natl. Acad. Sci. USA, 87, 4645-4649 (1990)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc. Where the virus is BmNPV, Bombyx mori N cells (BmN cells), etc. are used. Examples of the Sf cell which can be used are Sf9 cells (ATCC CRL1711), Sf21 cells (both cells are described in Vaughn, J. L. et al., In Vivo, 13, 213-217 (1977), etc.

As the insect, for example, a larva of Bombyx mori is used (Maeda, et al., Nature, 315, 592 (1985)).

Examples of animal cells, which are used, are monkey cells COS-7, Vero, Chinese hamster cells CHO (hereinafter referred to as CHO cells), dhfr gene deficient Chinese hamster cells CHO (hereinafter simply referred to as CHO (dhfr⁻) cell), mouse L cells, mouse AtT-20, mouse myeloma cells, rat GH3, human FL cells, pancreas-derived cells (RINm5F, HIT-T15, etc.), pituitary-derived cells (GH3, GH1, RC4/BC, etc.), placenta-derived cells (BeWo, JAR, JEG-3, etc.), liver-derived cells (HepG2, etc.), kidney-derived cells (ACHN, etc.), hematocyte-derived cells (H9, THP-1, U-937, etc.), HeLa cells, etc.

Bacteria belonging to the genus Escherichia can be transformed, for example, by the method described in Proc. Natl. Acad. Sci. U.S.A., 69, 2110 (1972), Gene, 17, 107 (1982) or the like.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the fusion protein described above can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York (1972)]. If necessary, a chemical such as 3β-indolylacrylic acid can be added to the medium thereby to activate the promoter efficiently.

Where the bacteria belonging to the genus Escherichia are used as the host, the transformant is usually cultured at about 15°C to about 43°C for about 3 hours to about 24 hours. If necessary, the culture may be aerated or stirred.

Where the bacteria belonging to the genus Bacillus are used as the host, the transformant is cultured generally at about 30°C to about 40°C for about 6 hours to about 24 hours. If necessary, the culture can be aerated or stirred.

Where yeast is used as the host, the transformant is cultured, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultured at about 20°C to about 35°C for about 24 hours to about 72 hours. If necessary, the culture can be aerated or stirred.

Where insect cells or insects are used as the host, the transformant is cultured in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultured at about 27°C for about 3 days to about 5 days and, if necessary, the culture can be aerated or stirred.

Where animal cells are employed as the host, the transformant is cultured in, for example, MEM medium containing about 5% to about 20% fetal calf serum [Science, 122, 501 (1952)), DMEM medium (Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultured at about 30°C to about 40°C for about 15 hours to about 60 hours and, if necessary, the culture can be aerated or stirred.

Suitable media, conditions for culturing, etc. on the respective hosts are publicly known (especially see WO 00/14227, page 24, line 24 to page 26, line 8, EP1111047A2, paragraphs [0090] to [0096]).

As above, the fusion protein can be expressed in the cell membrane of the transformant.

The fusion protein can be separated and purified from the culture described above, for example, by the following procedures.

When the fusion protein is extracted from the culture or cells, after culturing the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the fusion protein can be obtained. The buffer used for the procedures may contain a protein modifier such as urea or guanidine hydrochloride, or a surfactant such as Triton X-100™, etc. When the receptor protein is secreted in the culture, after completion of the cultivation the supernatant can be separated from the transformants or cells to collect the supernatant by a publicly known method.

The fusion protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the fusion protein thus obtained is in a free form, it can be converted into the salt by publicly known methods or modifications thereof. On the other hand, when the fusion protein is obtained in the form of a salt, it can be converted into the free form or in the form of a different salt by publicly known methods or modifications thereof.

The fusion protein produced by the recombinant can be treated, prior to or after the purification, with an appropriate protein modifying enzyme so that the fusion protein can be appropriately modified to partially remove a polypeptide. Examples of the protein-modifying enzyme include trypsin, chymotrypsin, arginyl endopeptidase, protein kinase, glycosidase or the like.

The activity of the thus produced fusion protein or salts thereof can be determined by a test binding to a labeled ligand, by an enzyme immunoassay using a specific antibody, or the like.

Examples of test compounds which are used for the ligand determination method of the present invention include publicly known ligands (for example, angiotensin, bombesin, canavinoid, cholecystokinin, glutamine, serotonin, melatonin, neuropeptide Y, opioids, purines, vasopressin, oxytocin, PACAP (e.g., PACAP27, PACAP38), secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotriens, pancreastatin, prostaglandins, thromboxane, adenosine, adrenaline, the chemokine superfamily (e.g., the CXC chemokine subfamily such as IL-8, GROα, GROβ, GROγ, NAP-2, ENA-78, GCP-2, PF4, IP-10, Mig, PBSF/SDF-1, etc.; the CC chemokine subfamily such as MCAF/MCP-1, MCP-2, MCP-3, MCP-4, eotaxin, RANTES, MIP-1α, MIP-1β, HCC-1, MIP-3α/LARC, MIP-3β/ELC, I-309, TARC, MIPF-1, MIPF-2/eotaxin-2, MDC, DC-CK1/PARC, SLC, etc.; the C chemokine subfamily such as lymphotactin, etc.; the CX3C chemokine subfamily such as fractalkine, etc.), endothelin, enterogastrin, histamine, neurotensin, TRH, pancreatic polypeptide, galanin, lysophosphatidic acid (LPA), sphingosine 1 -phosphate, lysophosphatidylserine, sphingosylphosphorylcholine, lyzophosphatidylcholine, steroids, bile acids, isoprenoids, arachidonic acid metabolites, amines, amino acids, nucleotides, nucleosides, saturated or saturated fatty acids, etc.) as well as other substances, for example, tissue extracts, cell culture supernatants from humans or mammals (e.g., mice, rats, swine, bovine, sheep, monkeys, etc.). For example, the tissue extract, cell culture supernatant, etc. is added to the cell wherein the fusion protein of the orphan receptor and GFP has been expressed, and screening is made while assaying the cell-stimulating activities, etc. to finally determine a single ligand.

Specifically, the ligand determination method A of the present invention is a method which involves constructing the fusion protein-expressed cell and assaying cell stimulating activities or perform a receptor binding assay by using the expressed cell or its cell membrane fraction, thus determining compounds that bind to the orphan receptors to have the cell stimulating activities (e.g., activities that accelerate or suppress arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos, pH reduction, etc.), MAP kinase activation, activation of transcription factors (e.g., CRE, AP1, NFκB, etc.), diacylglycerol production, opening or closing of ion channels (e.g., K⁺, Ca²⁺, Na⁺, Cl⁻, etc.) on cell membranes, apoptosis induction, morphological changes in cells, transport of the receptor (fusion protein) from cell membrane to cytoplasm, activation of low molecular weight G proteins (e.g., Ras, Rap, Rho, Rab, etc.), cell division-promoting activity, DNA synthesis-promoting activity, etc., that is, ligands (e.g., peptides, proteins, non-peptide compounds, synthetic compounds, fermentation products, etc.).

The ligand determination methods of the present invention are characterized by assaying, e.g., the binding amount of a test compound to the orphan receptor or the cell stimulating activities, etc., when a cell wherein the fusion protein has been expressed or a membrane fraction of the cell is brought in contact with the test compound.

More specifically, the present invention provides the following ligand determination methods:
(1) a method of determining a ligand to the orphan receptor, which comprises assaying the amount of a labeled test compound bound to a cell wherein the fusion protein has been expressed, when the labeled test compound is brought in contact with the cell or the cell membrane fraction;
(2) a method of determining a ligand to the orphan receptor, which comprises assaying the amount of a labeled test compound bound to a cell wherein the fusion protein has been expressed on a cell membrane by culturing a transformant containing DNA encoding the fusion protein, when the labeled test compound is brought in contact with the fusion protein;
(3) a method of determining a ligand to the orphan receptor, which comprises assaying the aforesaid cell stimulating activities, MAP kinase activation, activation of transcription factors (e.g., CRE, AP1, NFκB, etc.), diacylglycerol production, opening or closing of ion channels (e.g., K⁺, Ca²⁺, Na⁺, Cl⁻, etc.) on cell membranes, apoptosis induction, morphological changes in cells, transport of the receptor (fusion protein) from cell membrane to cytoplasm, activation of low molecular weight G proteins (e.g., Ras, Rap, Rho, Rab, etc.), cell division-promoting activity, DNA synthesis-promoting activity, etc., which are mediated by the orphan receptor, when a test compound is brought in contact with a cell wherein the fusion protein has been expressed; and,
(4) a method of determining a ligand to the orphan receptor, which comprises assaying the aforesaid mediated the cell stimulating activities, MAP kinase activation, activation of transcription factors (e.g., CRE, AP1, NFκB, etc.), diacylglycerol production, opening or closing of ion channels (e.g., K⁺, Ca²⁺, Na⁺, Cl⁻, etc.) on cell membranes, apoptosis induction, morphological changes in cells, transport of the receptor (fusion protein) from cell membrane to cytoplasm, activation of low molecular weight G proteins (e.g., Ras, Rap, Rho, Rab, etc.), cell division-promoting activity, DNA synthesis-promoting activity, etc., which are mediated by the orphan receptor, when a test compound is brought in contact with the fusion protein expressed on a cell membrane by culturing a transformant containing DNA encoding the fusion protein.

In particular, it is preferred to perform the tests (1) and (2) described above, thereby to confirm that the test compound can bind to the orphan receptor, followed by the tests (3) and (4) described above.

As the case may be, the fusion protein can be isolated and purified from the expression cell described above and the receptor binding assay, etc. may be carried out using the same. For the fusion protein used in the ligand determination method, fusion proteins abundantly expressed using the cells described above are appropriate.

The fusion protein can be manufactured by the method for expression described above, preferably by expressing DNA encoding the fusion protein in mammalian or insect cells, etc. For introducing a DNA fragment encoding the fusion protein into host animal cells and efficiently expressing the same, it is preferred to insert the DNA fragment downstream a polyhedrin promoter of nuclear polyhedrosis virus (NPV) belonging to a baculovirus, an SV40-derived promoter, a retrovirus promoter, a metallothionein promoter, a human heat shock promoter, a cytomegalovirus promoter, an SRα promoter or the like. The amount and quality of the fusion protein expressed can be determined by a publicly known method. For example, this determination can be made by the method described in the literature [Nambi, P., et al., J. Biol. Chem., 267, 19555-19559 (1992)].

In the ligand determination methods of the present invention, it is preferred to use the fusion protein-expressed cell or the cell membrane fraction.

Where the fusion protein-expressed cells are used in the ligand determination methods of the present invention, the cells may be fixed using glutaraldehyde, formalin, etc. The fixation can be made by publicly known methods.

The cells wherein the fusion protein has been expressed refer to host cells wherein the fusion protein has been expressed, which host cells include Escherichia coli, Bacillus subtilis, yeast, insect cells, animal cells, and the like.

The cell membrane fraction means a fraction abundant in cell membrane obtained by cell disruption and subsequent fractionation by publicly known methods. Useful cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the fusion protein expressed and membrane components such as cell-derived phospholipids, membrane proteins, etc.

The amount of the fusion protein in the fusion protein-expressed cells or the cell membrane fraction is preferably 10³ to 10⁸ molecules per cell, more preferably 10⁵ to 10⁷ molecules per cell. As the amount of expression increases, the ligand binding activity per unit of membrane fraction (specific activity) increases so that not only the highly sensitive screening system can be constructed but also large quantities of samples can be assayed with the same lot. The amount of this fusion protein expressed can be roughly estimated from the fluorescence level of GFP in the cells or cell membranes, using a fluorescence microscope or a fluorophotometer.

To perform the methods (1) and (2) described above for determining a ligand to the orphan receptor, appropriate cells or cell membrane fractions containing the fusion protein and a labeled test compound are required. The fusion protein fraction is desirably a fraction containing a recombinant fusion receptor having an activity equivalent to a naturally occurring fusion protein. Herein, the term "equivalent activity" means a ligand binding activity, a signal transduction activity, etc., which is equivalent.

As the labeled test compounds, there are used compounds selected from the aforesaid group of ligand compounds, which are labeled with [³H], [¹²⁵I], [¹⁴C], [³⁵S], etc.

Specifically, the ligand determination methods of the present invention can be performed by the following procedures. First, a standard fusion protein preparation is prepared by suspending cells wherein the fusion protein has been expressed or the cell membrane fraction in a buffer appropriate for use in the determination method. Any buffer can be used so long as it does not inhibit the ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, and various proteins such as bovine serum albumin or gelatin, may optionally be added to the buffer. Further for the purpose of suppressing the degradation of the receptors or ligands by proteases, a protease inhibitor such as PMSF, leupeptin, E-64 (manufactured by Peptide Institute, Inc.) and pepstatin may also be added. A given amount (5,000 to 500,000 cpm) of the test compound labeled with [³H], [¹²⁵I], [¹⁴C], [³⁵S] or the like is added to 0.01 ml to 10 ml of the receptor preparation. To determine the amount of non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. A test compound exceeding 0 cpm in count obtained by subtracting nonspecific binding (NSB) from the total binding (B) (B minus NSB) may be selected as a ligand (including an agonist) to the orphan receptor.

The method (3) or (4) of the present invention described above for determining a ligand can be performed as follows. The aforesaid orphan receptor-mediated cell stimulating activities, for example, MAP kinase activation, activation of transcription factors (e.g., CRE, AP1, NFκB, etc.), diacylglycerol production, opening or closing of ion channels (e.g., K⁺, Ca²⁺, Na⁺, Cl⁻, etc.) on cell membranes, apoptosis induction, morphological changes in cells, transport of the receptor (fusion protein) from cell membrane into cytoplasm, activation of low molecular weight G proteins (e.g., Ras, Rap, Rho, Rab, etc.), cell division-promoting activity, DNA synthesis-promoting activity, etc. can be assayed by publicly known methods or using assay kits commercially available. Specifically, cells wherein the fusion protein has expressed are first cultured on a multi-well plate, etc. Prior to the ligand determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the indicator substance (e.g., arachidonic acid, etc.) for the cell-stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

In the cell stimulating activities described above, "transport of the receptor to cytoplasm" is assayed by measuring the fluorescence of a fluorescent protein such as GFP, etc., whereby movement of the fusion protein from the cell membrane to the cytoplasm can be observed. In order to observe the movement of the fusion protein from the cell membrane to the cytoplasm, it is suited to use animal cells which have expressed the aforesaid fusion protein stably or transiently. The cells are incubated in an appropriate incubator using an ordinary medium and a test compound diluted to a suitable concentration is added to the cells. In this case, a diluted solution of the test compound may be added directly to the medium, or after the cells are washed with Hanks' balanced salt solution (HBSS, to which BSA may be added in 0 to 10%, preferably 0.1 to 1%), HBSS containing the test compound may be added to the cells. The cells are allowed to stand at 4°C to 37°C, preferably at 20°C to 37°C for 1 minute to 6 hours, preferably for 10 minutes to 2 hours, followed by observing movement of the fusion protein from the cell membrane to the cytoplasm. Though the cells can be observed as they are, the cells may also be fixed with glutaraldehyde, formalin, etc. The cells can be fixed by publicly known methods.

Observation may be made using an ordinary fluorescence microscope or confocal laser scanning microscope. Also, a plate reader having the function to irradiate excitement light and the function to take fluorescent images in can be used. In this case, after excitement with ultraviolet light, preferably at 395 nm, the fusion protein of the receptor and wild type GFP or GFPuv represented by SEQ ID NO: 3 or SEQ ID NO: 5 can be detected using fluorescein isothianate (FITC) or filters normally commercially available for detecting GFP. In order to detect the fusion protein with GFP represented by SEQ ID NO: 1 or SEQ ID NO: 7, excitement may be effected with excitement light at 460 - 500nm, preferably at 488 nm and observation may be made using FITC or filters normally commercially available for detecting GFP.

In addition to the transport of the receptor (fusion protein) into cytoplasm, morphological changes of the receptor including receptor aggregation, receptor localization, decreased or increased expression of the receptor, etc., which can be microscopically observed, may also be observed.

In practicing the ligand determination method B of the present invention, it is necessary to incorporate a plasmid containing a DNA encoding the reporter protein at the downstream of a particular enhancer/promoter into cells, preferably eukaryote-derived cells, in addition to the expression vector for the fusion protein. This plasmid may contain a promoter capable of expressing the reporter protein in cells, preferably in eukaryote-derived cells, may further contain a chemical resistant gene (e.g., ampicillin-resistant gene), etc. as a selection marker in the case of proliferating in eukaryotes.

The plasmid containing a DNA encoding the reporter protein at the downstream of the enhancer/promoter may be any plasmid such as commercially available plasmid, etc., so far as it is capable of expressing the reporter protein in cells under control of the enhancer and is capable of introducing into cells.

As the enhancer, there is used, for example, a virus-derived enhancer such as papilloma virus, etc., LTR of a retrovirus, cAMP response element (CRE), TPA response element (TRE) or the like, preferably cAMP response element. An enhancer which can be activated by the aforesaid cell stimulating activities mediated by the orphan receptor the cells express is appropriate.

As the promoter, there is used, for example, SV40 promoter, a TATA-like promoter of HSV thymidine kinase gene, etc., with a TATA-like promoter being preferred.

As the reporter protein gene, there is used, for example, luciferase gene, β-galactosidase gene, GFP gene, alkaline phosphatase gene, etc. Any enzyme gene is usable as the reporter gene so long as its enzyme activity can be detected by publicly known methods.

Specific examples of such plasmids include a plasmid ligated with a TATA-like promoter and a reporter protein (e.g., luciferase gene) at the downstream of cAMP response element, e.g., pCRE-Luc (Clontech, Inc.), etc.

The cells used in the ligand determination method B are the host cells described above, preferably animal cells (e.g., monkey cells COS-7, Vero, CHO cells, CHO (dhfr⁻) cells, mouse L cells, mouse AtT-20, mouse myeloma cells, rat GH3, human FL cells, human HEK293 cells, etc.) and the like.

The cells may express 2 or more (preferably 2 or 3) fusion proteins.

Where 2 or more fusion proteins are expressed, it is preferred to use 2 or more orphan receptors having similar biological characteristics.

Examples of the similar biological characteristics include expression levels of the reporter protein when 2 or more orphan receptors used are expressed independently; etc. Specifically, the characteristics of the respective receptor proteins can be distinguished, using as an indicator (1) the basic expression level of the reporter protein and/or (2) the expression level of the reporter protein in the presence of forskolin, when 2 or more orphan receptors are expressed individually.

Accordingly, when 2 or more orphan receptors are expressed to determine the ligand, it is desired to previously sort the reporter proteins into those having low, medium and obviously high basic expression levels, etc., or to clarify the receptor protein difficult to increase an expression level of the reporter protein by the addition of forskolin. This is because where two receptor protein, for example, a receptor protein having a high basic expression level of the reporter protein and a receptor having a low basic expression level of the reporter protein are expressed, it becomes difficult to detect an increase in the expression level of the reporter protein when a ligand binds to the latter. That is:
(1) it is preferred to avoid the mixture of an orphan receptor that the basic expression level of the reporter protein is high and a receptor protein that the basic expression level is low;
(2) it is preferred not to mix a receptor protein that the expression level of the reporter protein increased by the addition of forskolin is remarkably high with an orphan receptor that the expression level increased is not; and,
(3) it is preferred that orphan receptors having almost equal basic expression levels of the reporter protein are used and expressed in combination.

As such, the combination of orphan receptors having similar characteristics includes, for example, the combination of APJ (apelin receptor; Gene, 136, 355 (1993)) and TGR-1 (Japanese Patent Application KOKAI No. 2002-078492).

The ligand determination method B is specifically described below.

Cells are plated on a 96-well plate and incubated overnight in DMEM containing, e.g., 10% fetal calf serum. At this stage, an expression plasmid and a reporter plasmid in the fluorescent protein are co-transfected to the cells, using a transfection kit commercially available. The cells are further incubated overnight to express the orphan receptor transiently in the cells. After the cells are washed and the medium is made serum-free, a test compound is added. Where the enhancer is CRE, forskolin may be added simultaneously with the test compound. After incubation is carried out for a given period of time, the cells are lysed and the activities of the reporter protein are assayed.

In the determination method described above, when the base line for the activities of the reporter protein is high so that a change in the activities is detected by a test compound only with difficulties, it is preferred to take measures for reducing the base line. For example in the case that the orphan receptor is a G protein-coupled receptor protein (GPCR), Gi protein, among α subunits of G protein, showing a cAMP inhibitory effect is added, which makes the detection of a change in activities easy. To express Gi protein, a plasmid capable of expressing a DNA encoding Gi protein can be transfected to cells together with the orphan receptor plasmid and the reporter plasmid. In this case, a mixing ratio of these three plasmids (the orphan receptor plasmid, the reporter plasmid and the Gi plasmid) are preferably in about 5 - 15 : 1 : 1 to 6, more preferably in about 7 : 1 : 3.

In the ligand determination method B of the present invention, where the activities of the reporter protein increase or decrease by about 20% or more, preferably by about 50% or more when a test compound is added, the test compound can be identified to be a ligand.

The test compound used in the ligand determination method B of the present invention is a compound selected from the test compounds described above.

Furthermore, the kit of the present invention for determination of the ligand contains cells capable of expressing the fusion protein of the present invention or its cell membrane fraction, etc.

Examples of the ligand determination kit of the present invention are given below.
1. Reagents for determining ligands
   (1) Assay buffer and wash buffer
      Hanks' Balanced Salt Solution (manufactured by Gibco Co.) supplemented with 0.05% bovine serum albumin (Sigma Co.).
      The solution is sterilized by filtration through a 0.45 µm filter and stored at 4°C. Alternatively, the solution may be prepared at use.
   (2) Fusion protein preparation
      CHO cells wherein the fusion protein has been expressed are passaged in a 12-well plate in a density of 5 x 10⁵ cells/well followed by culturing at 37°C under 5% CO₂ and 95% air for 2 days.
   (3) Labeled test compound
      Compounds labeled with [³H], [¹²⁵I], [¹⁴C], [³⁵S], etc., which are commercially available labels, or compounds labeled by appropriate methods.
      An aqueous solution of the compound is stored at 4°C or -20°C. The solution is diluted to 1 µM with an assay buffer at use. A sparingly water-soluble test compound is dissolved in dimethylformamide, DMSO, methanol, etc.
   (4) Non-labeled test compound
      A non-labeled form of the same compound as the labeled compound is prepared in a concentration of 100 to 1,000-fold higher than that of the labeled compound.
2. Assay method
   (1) CHO cells wherein the fusion protein has been expressed are cultured in a 12-well culture plate. After washing twice with 1 ml of an assay buffer, 490 µl of the assay buffer is added to each well.
   (2) After 5 µl of the labeled test compound is added, the resulting mixture is incubated at room temperature for an hour. To determine the non-specific binding, 5 µl of the non-labeled compound is added to the system.
   (3) The reaction mixture is removed and the wells are washed 3 times with 1 ml of washing buffer. The labeled test compound bound to the cells is dissolved in 0.2N NaOH-1% SDS and then mixed with 4 ml of liquid scintillator A (manufactured by Wako Pure Chemical Industries, Ltd.).
   (4) The radioactivity is measured using a liquid scintillation counter (manufactured by Beckman Co.).

As such, according to the ligand determination methods (A and B) of the present invention, immunostaining or western blot assay utilizing the fluorescence from the fluorescent protein such as GFP, etc. or a fluorescent protein antibody such as a GFP antibody, etc. is used to effect (1) to (5) below:
(1) it can be confirmed that the receptor protein has been expressed on a protein level;
(2) it can be confirmed that the receptor protein has been expressed on a cell membrane;
(3) an expression level of the receptor protein can be estimated;
(4) a cell wherein the receptor protein has been expressed abundantly can be selected; and,
(5) a specific reaction of the receptor by a ligand can be detected as internalization of the fusion protein of the receptor and the fluorescent protein into cells. By utilizing these features, a ligand to the receptor protein for which a ligand has not been identified (orphan receptor) can be determined efficiently.

The ligand thus determined binds to its receptor protein to regulate its physiological functions and thus can be used as an agent for the prevention and/or treatment of diseases associated with the functions of the receptor protein. Further using the ligand and its receptor protein, an agonist/antagonist to the receptor can be screened.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

The sequence identification numbers in the sequence listing of the specification indicates the following sequence, respectively.
[SEQ ID NO: 1]
   This shows the amino acid sequence of GFP used in EXAMPLE 1 (hereinafter merely referred to as GFP-1).
SEQ ID NO: 2
   This shows the base sequence of DNA encoding GFP used in EXAMPLE 1.
SEQ ID NO: 3
   This shows the amino acid sequence of wild type GFP.
SEQ ID NO: 4
   This shows the base sequence of DNA encoding wild type GFP.
SEQ ID NO: 5
   This shows the amino acid sequence of GFPuv.
SEQ ID NO: 6
   This shows the base sequence of DNA encoding GFPuv.
SEQ ID NO: 7
   This shows the amino acid sequence of EGFP.
SEQ ID NO: 8
   This shows the base sequence of cDNA encoding EGFP.
SEQ ID NO: 9
   This shows the amino acid sequence of human-derived G protein-coupled receptor protein TGR5 used in EXAMPLE 1.
SEQ ID NO: 10
   This shows the base sequence of cDNA encoding human-derived G protein-coupled receptor protein TGR5 used in EXAMPLE 1.
SEQ ID NO: 11
   This shows the base sequence of primer 1 used for PCR in REFERENCE EXAMPLE 1.
SEQ ID NO: 12
   This shows the base sequence of primer 2 used for PCR in REFERENCE EXAMPLE 1.
SEQ ID NO: 13
   This shows the amino acid sequence of human-derived parathyroid hormone receptor (PTH-R).
SEQ ID NO: 14
   This shows the base sequence of cDNA encoding human-derived parathyroid hormone receptor (PTH-R).
SEQ ID NO: 15
   This shows the amino acid sequence of human-derived GPR40.
SEQ ID NO: 16
   This shows the base sequence of cDNA encoding human-derived GPR40.
SEQ ID NO: 17
   This shows the amino acid sequence of His-Tag.
SEQ ID NO: 18
   This shows the amino acid sequence of V5-tag.
SEQ ID NO: 19
   This shows the amino acid sequence of myc-tag.
SEQ ID NO: 20
   This shows the amino acid sequence of Xpress-tag.
SEQ ID NO: 21
   This shows the amino acid sequence of HA-tag.
SEQ ID NO: 22
   This shows the amino acid sequence of ECFP.
SEQ ID NO: 23
   This shows the base sequence of cDNA encoding ECFP.
SEQ ID NO: 24
   This shows the amino acid sequence of EYFP.
SEQ ID NO: 25
   This shows the base sequence of cDNA encoding EYFP.
SEQ ID NO: 26
   This shows the amino acid sequence of DsRED.
SEQ ID NO: 27
   This shows the base sequence of cDNA encoding DsRED.
SEQ ID NO: 28
   This shows the amino acid sequence of EBFP.
SEQ ID NO: 29
   This shows the base sequence of cDNA encoding EBFP.
SEQ ID NO: 30
   This shows the amino acid sequence of the fusion protein of orphan receptor hBL5 and GFP-1.
SEQ ID NO: 31
   This shows the amino acid sequence of the fusion protein of orphan receptor h7TBA62 and GFP-1.
SEQ ID NO: 32
   This shows the amino acid sequence of the fusion protein of orphan receptor 14273 and GFP-1.
SEQ ID NO: 33
   This shows the amino acid sequence of the fusion protein of orphan receptor EMR3 and GFP-1.
SEQ ID NO: 34
   This shows the amino acid sequence of the fusion protein of orphan receptor GPR15 and GFP-1.
SEQ ID NO: 35
   This shows the amino acid sequence of the fusion protein of orphan receptor GPR31 and GFP-1.
SEQ ID NO: 36
   This shows the amino acid sequence of the fusion protein of orphan receptor GPRC5B and GFP-1.
SEQ ID NO: 37
   This shows the amino acid sequence of the fusion protein of orphan receptor PSEC0142 and GFP-1.
SEQ ID NO: 38
   This shows the amino acid sequence of the fusion protein of orphan receptor HE6 and GFP-1.
SEQ ID NO: 39
   This shows the amino acid sequence of the fusion protein of orphan receptor GPR61 and GFP-1.
SEQ ID NO: 40
   This shows the amino acid sequence of the fusion protein of orphan receptor TGR9 and GFP-1.
SEQ ID NO: 41
   This shows the amino acid sequence of the fusion protein of orphan receptor TGR24 and GFP-1.
SEQ ID NO: 42
   This shows the amino acid sequence of the fusion protein of orphan receptor ZGPR1 and GFP-1.
SEQ ID NO: 43
   This shows the amino acid sequence of the fusion protein of orphan receptor EMR1 and GFP-1.
SEQ ID NO: 44
   This shows the amino acid sequence of the fusion protein of orphan receptor GPR25 and GFP-1.
SEQ ID NO: 45
   This shows the amino acid sequence of the fusion protein of orphan receptor GPR55 and GFP-1.
SEQ ID NO: 46
   This shows the amino acid sequence of the fusion protein of orphan receptor AXOR14 and GFP-1.
SEQ ID NO: 47
   This shows the amino acid sequence of the fusion protein of orphan receptor TM7SF1 and GFP-1.
SEQ ID NO: 48
   This shows the amino acid sequence of the fusion protein of orphan receptor PSP24B and GFP-1.
SEQ ID NO: 49
   This shows the amino acid sequence of the fusion protein of orphan receptor SREB3 and GFP-1.
SEQ ID NO: 50
   This shows the amino acid sequence of the fusion protein of orphan receptor TGR37 and GFP-1.
SEQ ID NO: 51
   This shows the amino acid sequence of the fusion protein of orphan receptor H963 and GFP-1.
SEQ ID NO: 52
   This shows the amino acid sequence of the fusion protein of orphan receptor GPR87 and GFP-1.
SEQ ID NO: 53
   This shows the amino acid sequence of the fusion protein of orphan receptor GPR91 and GFP-1.
SEQ ID NO: 54
   This shows the amino acid sequence of the fusion protein of orphan receptor PNR and GFP-1.
SEQ ID NO: 55
   This shows the amino acid sequence of the fusion protein of orphan receptor TGR29 and GFP-1.
SEQ ID NO: 56
   This shows the amino acid sequence of the fusion protein of orphan receptor TGR36 and GFP-1.
SEQ ID NO: 57
   This shows the amino acid sequence of the fusion protein of orphan receptor H9 and GFP-1.
SEQ ID NO: 58
   This shows the amino acid sequence of the fusion protein of orphan receptor TGR18 and GFP-1.
SEQ ID NO: 59
   This shows the amino acid sequence of the fusion protein of orphan receptor TGR19 and GFP-1.
SEQ ID NO: 60
   This shows the amino acid sequence of the fusion protein of orphan receptor AM-R and GFP-1.
SEQ ID NO: 61
   This shows the amino acid sequence of the fusion protein of orphan receptor GPR19 and GFP-1.
SEQ ID NO: 62
   This shows the amino acid sequence of the fusion protein of orphan receptor GPR45 and GFP-1.
SEQ ID NO: 63
   This shows the amino acid sequence of the fusion protein of orphan receptor GPRC5D and GFP-1.
SEQ ID NO: 64
   This shows the amino acid sequence of the fusion protein of orphan receptor LGR6 and GFP-1.
SEQ ID NO: 65
   This shows the amino acid sequence of the fusion protein of orphan receptor RUP3 and GFP-1.
SEQ ID NO: 66
   This shows the amino acid sequence of the fusion protein of orphan receptor TGR14 and GFP-1.
SEQ ID NO: 67
   This shows the amino acid sequence of the fusion protein of orphan receptor TPRA40 and GFP-1.
SEQ ID NO: 68
   This shows the amino acid sequence of the fusion protein of orphan receptor GPR22 and GFP-1.
SEQ ID NO: 69
   This shows the amino acid sequence of the fusion protein of orphan receptor GPR52 and GFP-1.
SEQ ID NO: 70
   This shows the amino acid sequence of the fusion protein of orphan receptor FLH2882 and GFP-1.
SEQ ID NO: 71
   This shows the amino acid sequence of the fusion protein of orphan receptor SNORF36 and GFP-1.
SEQ ID NO: 72
   This shows the amino acid sequence of the fusion protein of orphan receptor MRG and GFP-1.
SEQ ID NO: 73
   This shows the amino acid sequence of the fusion protein of orphan receptor SREB2 and GFP-1.
SEQ ID NO: 74
   This shows the amino acid sequence of the fusion protein of orphan receptor GPR12 and GFP-1.
SEQ ID NO: 75
   This shows the amino acid sequence of the fusion protein of orphan receptor GPR30 and GFP-1.
SEQ ID NO: 76
   This shows the amino acid sequence of the fusion protein of orphan receptor GPR82 and GFP-1.
SEQ ID NO: 77
   This shows the amino acid sequence of the fusion protein of orphan receptor RECAP and GFP-1.
SEQ ID NO: 78
   This shows the amino acid sequence of the fusion protein of orphan receptor HB954 and GFP-1.
SEQ ID NO: 79
   This shows the amino acid sequence of the fusion protein of orphan receptor RDC1 and GFP-1.
SEQ ID NO: 80
   This shows the amino acid sequence of the fusion protein of orphan receptor TGR6 and GFP-1.
SEQ ID NO: 81
   This shows the amino acid sequence of the fusion protein of orphan receptor A-2 and GFP-1.
SEQ ID NO: 82
   This shows the amino acid sequence of the fusion protein of orphan receptor JEG18 and GFP-1.
SEQ ID NO: 83
   This shows the amino acid sequence of the fusion protein of orphan receptor GPR17 and GFP-1.
SEQ ID NO: 84
   This shows the amino acid sequence of the fusion protein of orphan receptor GPR35 and GFP-1.
SEQ ID NO: 85
   This shows the amino acid sequence of the fusion protein of orphan receptor GPRC5C and GFP-1.
SEQ ID NO: 86
   This shows the amino acid sequence of the fusion protein of orphan receptor HM74 and GFP-1.
SEQ ID NO: 87
   This shows the amino acid sequence of the fusion protein of orphan receptor RPE and GFP-1.
SEQ ID NO: 88
   This shows the amino acid sequence of the fusion protein of orphan receptor TGR13 and GFP-1.
SEQ ID NO: 89
   This shows the amino acid sequence of the fusion protein of orphan receptor TGR27 and GFP-1.
SEQ ID NO: 90
   This shows the amino acid sequence of the fusion protein of orphan receptor DEZ and GFP-1.
SEQ ID NO: 91
   This shows the amino acid sequence of the fusion protein of orphan receptor ratGPR1 and GFP-1.
SEQ ID NO: 92
   This shows the amino acid sequence of the fusion protein of orphan receptor GPR3 and GFP-1.
SEQ ID NO: 93
   This shows the amino acid sequence of the fusion protein of orphan receptor GPR6 and GFP-1.
SEQ ID NO: 94
   This shows the amino acid sequence of the fusion protein of orphan receptor RAIG1 and GFP-1.
SEQ ID NO: 95
   This shows the amino acid sequence of the fusion protein of orphan receptor TGR2-1 and GFP-1.
SEQ ID NO: 96
   This shows the amino acid sequence of the fusion protein of orphan receptor TGR2-2 and GFP-1.
SEQ ID NO: 97
   This shows the amino acid sequence of the fusion protein of orphan receptor TGR21 and GFP-1.
SEQ ID NO: 98
   This shows the amino acid sequence of the fusion protein of orphan receptor GPR56 and GFP-1.
SEQ ID NO: 99
   This shows the amino acid sequence of the fusion protein of orphan receptor KIAA0758 and GFP-1.
SEQ ID NO: 100
   This shows the amino acid sequence of the fusion protein of orphan receptor RE2 and GFP-1.
SEQ ID NO: 101
   This shows the amino acid sequence of the fusion protein of orphan receptor P40 and GFP-1.
SEQ ID NO: 102
   This shows the amino acid sequence of the fusion protein of orphan receptor GPR27 and GFP-1.
SEQ ID NO: 103
   This shows the amino acid sequence of the fusion protein of orphan receptor HG38 and GFP-1.
SEQ ID NO: 104
   This shows the amino acid sequence of the fusion protein of orphan receptor DRR1 and GFP-1.
SEQ ID NO: 105
   This shows the amino acid sequence of the fusion protein of orphan receptor TGR12 and GFP-1.
SEQ ID NO: 106
   This shows the amino acid sequence of the fusion protein of orphan receptor TGR11 and GFP-1.
SEQ ID NO: 107
   This shows the amino acid sequence of the fusion protein of orphan receptor TGR15 and GFP-1.
SEQ ID NO: 108
   This shows the amino acid sequence of the fusion protein of orphan receptor TGR8 and GFP-1.
SEQ ID NO: 109
   This shows the amino acid sequence of the fusion protein of orphan receptor GPR20 and GFP-1.
SEQ ID NO: 110
   This shows the amino acid sequence of the fusion protein of orphan receptor TGR10 and GFP-1.
SEQ ID NO: 111
   This shows the amino acid sequence of the fusion protein of orphan receptor TGR30 and GFP-1.
SEQ ID NO: 112
   This shows the amino acid sequence of the fusion protein of orphan receptor GPR18 and GFP-1.
SEQ ID NO: 113
   This shows the amino acid sequence of the fusion protein of orphan receptor TGR25 and GFP-1.
SEQ ID NO: 114
   This shows the amino acid sequence of the fusion protein of orphan receptor GPR23 and GFP-1.
SEQ ID NO: 115
   This shows the amino acid sequence of the fusion protein of orphan receptor P2Y10 and GFP-1.
SEQ ID NO: 116
   This shows the amino acid sequence of the fusion protein of orphan receptor GPR37 and GFP-1.
SEQ ID NO: 117
   This shows the amino acid sequence of the fusion protein of orphan receptor ET(B)R-LP-2 and GFP-1.
SEQ ID NO: 118
   This shows the amino acid sequence of the fusion protein of orphan receptor FPRL2 and GFP-1.
SEQ ID NO: 119
   This shows the amino acid sequence of the fusion protein of orphan receptor GPR32 and GFP-1.
SEQ ID NO: 120
   This shows the amino acid sequence of the fusion protein of orphan receptor dj287G14.2 and GFP-1.
SEQ ID NO: 121
   This shows the amino acid sequence of the fusion protein of orphan receptor BRS-3 and GFP-1.
SEQ ID NO: 122
   This shows the amino acid sequence of the fusion protein of orphan receptor GPR39 and GFP-1.
SEQ ID NO: 123
   This shows the amino acid sequence of the fusion protein of orphan receptor 63A2 and GFP-1.
SEQ ID NO: 124
   This shows the amino acid sequence of the fusion protein of orphan receptor GPR84 and GFP-1.
SEQ ID NO: 125
   This shows the amino acid sequence of the fusion protein of orphan receptor GPR21 and GFP-1.
SEQ ID NO: 126
   This shows the amino acid sequence of the fusion protein of orphan receptor GPR48 and GFP-1.
SEQ ID NO: 127
   This shows the amino acid sequence of the fusion protein of orphan receptor SNORF1 and GFP-1.
SEQ ID NO: 128
   This shows the amino acid sequence of the fusion protein of orphan receptor BA12 and GFP-1.
SEQ ID NO: 129
   This shows the amino acid sequence of the fusion protein of orphan receptor MAS and GFP-1.
SEQ ID NO: 130
   This shows the amino acid sequence of the fusion protein of orphan receptor OT7T009 and GFP-1.
SEQ ID NO: 131
   This shows the amino acid sequence of the fusion protein of orphan receptor TGR34 and GFP-1.
SEQ ID NO: 132
   This shows the base sequence of DNA encoding the fusion protein of orphan receptor hBL5 and GFP-1.
SEQ ID NO: 133
   This shows the base sequence of DNA encoding the fusion protein of orphan receptor h7TBA62 and GFP-1.
SEQ ID NO: 134
   This shows the base sequence of DNA encoding the fusion protein of orphan receptor 14273 and GFP-1.
SEQ ID NO: 135
   This shows the base sequence of DNA encoding the fusion protein of orphan receptor EMR3 and GFP-1.
SEQ ID NO: 136
   This shows the base sequence of DNA encoding the fusion protein of orphan receptor GPR15 and GFP-1.
SEQ ID NO: 137
   This shows the base sequence of DNA encoding the fusion protein of orphan receptor GPR31 and GFP-1.
SEQ ID NO: 138
   This shows the base sequence of DNA encoding the fusion protein of orphan receptor GPRC5B and GFP-1.
SEQ ID NO: 139
   This shows the base sequence of DNA encoding the fusion protein of orphan receptor PSEC0142 and GFP-1.
SEQ ID NO: 140
   This shows the base sequence of DNA encoding the fusion protein of orphan receptor HE6 and GFP-1.
SEQ ID NO: 141
   This shows the base sequence of DNA encoding the fusion protein of orphan receptor GPR61 and GFP-1.
SEQ ID NO: 142
   This shows the base sequence of DNA encoding the fusion protein of orphan receptor TGR9 and GFP-1.
SEQ ID NO: 143
   This shows the base sequence of DNA encoding the fusion protein of orphan receptor TGR24 and GFP-1.
SEQ ID NO: 144
   This shows the base sequence of DNA encoding the fusion protein of orphan receptor ZGPR1 and GFP-1.
SEQ ID NO: 145
   This shows the base sequence of DNA encoding the fusion protein of orphan receptor EMR1 and GFP-1.
SEQ ID NO: 146
   This shows the base sequence of DNA encoding the fusion protein of orphan receptor GPR25 and GFP-1.
SEQ ID NO: 147
   This shows the base sequence of DNA encoding the fusion protein of orphan receptor GPR55 and GFP-1.
SEQ ID NO: 148
   This shows the base sequence of DNA encoding the fusion protein of orphan receptor AXOR14 and GFP-1.
SEQ ID NO: 149
   This shows the base sequence of DNA encoding the fusion protein of orphan receptor TM7SF1 1 and GFP-1.
SEQ ID NO: 150
   This shows the base sequence of DNA encoding the fusion protein of orphan receptor PSP24B and GFP-1.
SEQ ID NO: 151
   This shows the base sequence of DNA encoding the fusion protein of orphan receptor SREB3 and GFP-1.
SEQ ID NO: 152
   This shows the base sequence of DNA encoding the fusion protein of orphan receptor TGR37 and GFP-1.
SEQ ID NO: 153
   This shows the base sequence of DNA encoding the fusion protein of orphan receptor H963 and GFP-1.
SEQ ID NO: 154
   This shows the base sequence of DNA encoding the fusion protein of orphan receptor GPR87 and GFP-1.
SEQ ID NO: 155
   This shows the base sequence of DNA encoding the fusion protein of orphan receptor GPR91 and GFP-1.
SEQ ID NO: 156
   This shows the base sequence of DNA encoding the fusion protein of orphan receptor PNR and GFP-1.
SEQ ID NO: 157
   This shows the base sequence of DNA encoding the fusion protein of orphan receptor TGR29 and GFP-1.
SEQ ID NO: 158
   This shows the base sequence of DNA encoding the fusion protein of orphan receptor TGR36 and GFP-1.
SEQ ID NO: 159
   This shows the base sequence of DNA encoding the fusion protein of orphan receptor H9 and GFP-1.
SEQ ID NO: 160
   This shows the base sequence of DNA encoding the fusion protein of orphan receptor TGR18 and GFP-1.
SEQ ID NO: 161
   This shows the base sequence of DNA encoding the fusion protein of orphan receptor TGR19 and GFP-1.
SEQ ID NO: 162
   This shows the base sequence of DNA encoding the fusion protein of orphan receptor AM-R and GFP-1.
SEQ ID NO: 163
   This shows the base sequence of DNA encoding the fusion protein of orphan receptor GPR19 and GFP-1.
SEQ ID NO: 164
   This shows the base sequence of DNA encoding the fusion protein of orphan receptor GPR45 and GFP-1.
SEQ ID NO: 165
   This shows the base sequence of DNA encoding the fusion protein of orphan receptor GPRC5D and GFP-1.
SEQ ID NO: 166
   This shows the base sequence of DNA encoding the fusion protein of orphan receptor LGR6 and GFP-1.
SEQ ID NO: 167
   This shows the base sequence of DNA encoding the fusion protein of orphan receptor RUP3 and GFP-1.
SEQ ID NO: 168
   This shows the base sequence of DNA encoding the fusion protein of orphan receptor TGR14 and GFP-1.
SEQ ID NO: 169
   This shows the base sequence of DNA encoding the fusion protein of orphan receptor TPRA40 and GFP-1.
SEQ ID NO: 170
   This shows the base sequence of DNA encoding the fusion protein of orphan receptor GPR22 and GFP-1.
SEQ ID NO: 171
   This shows the base sequence of DNA encoding the fusion protein of orphan receptor GPR52 and GFP-1.
SEQ ID NO: 172
   This shows the base sequence of DNA encoding the fusion protein of orphan receptor FLH2882 and GFP-1.
SEQ ID NO: 173
   This shows the base sequence of DNA encoding the fusion protein of orphan receptor SNORF36 and GFP-1.
SEQ ID NO: 174
   This shows the base sequence of DNA encoding the fusion protein of orphan receptor MRG and GFP-1.
SEQ ID NO: 175
   This shows the base sequence of DNA encoding the fusion protein of orphan receptor SREB2 and GFP-1.
SEQ ID NO: 176
   This shows the base sequence of DNA encoding the fusion protein of orphan receptor GPR12 and GFP-1.
SEQ ID NO: 177
   This shows the base sequence of DNA encoding the fusion protein of orphan receptor GPR30 and GFP-1.
SEQ ID NO: 178
   This shows the base sequence of DNA encoding the fusion protein of orphan receptor GPR82 and GFP-1.
SEQ ID NO: 179
   This shows the base sequence of DNA encoding the fusion protein of orphan receptor RECAP and GFP-1.
SEQ ID NO: 180
   This shows the base sequence of DNA encoding the fusion protein of orphan receptor HB954 and GFP-1.
SEQ ID NO: 181
   This shows the base sequence of DNA encoding the fusion protein of orphan receptor RDC1 and GFP-1.
SEQ ID NO: 182
   This shows the base sequence of DNA encoding the fusion protein of orphan receptor TGR6 and GFP-1.
SEQ ID NO: 183
   This shows the base sequence of DNA encoding the fusion protein of orphan receptor A-2 and GFP-1.
SEQ ID NO: 184
   This shows the base sequence of DNA encoding the fusion protein of orphan receptor JEG18 and GFP-1.
SEQ ID NO: 185
   This shows the base sequence of DNA encoding the fusion protein of orphan receptor GPR17 and GFP-1.
SEQ ID NO: 186
   This shows the base sequence of DNA encoding the fusion protein of orphan receptor GPR35 and GFP-1.
SEQ ID NO: 187
   This shows the base sequence of DNA encoding the fusion protein of orphan receptor GPRC5C and GFP-1.
SEQ ID NO: 188
   This shows the base sequence of DNA encoding the fusion protein of orphan receptor HM74 and GFP-1.
SEQ ID NO: 189
   This shows the base sequence of DNA encoding the fusion protein of orphan receptor RPE and GFP-1.
SEQ ID NO: 190
   This shows the base sequence of DNA encoding the fusion protein of orphan receptor TGR13 and GFP-1.
SEQ ID NO: 191
   This shows the base sequence of DNA encoding the fusion protein of orphan receptor TGR27 and GFP-1.
SEQ ID NO: 192
   This shows the base sequence of DNA encoding the fusion protein of orphan receptor DEZ and GFP-1.
SEQ ID NO: 193
   This shows the base sequence of DNA encoding the fusion protein of orphan receptor ratGPR1 and GFP-1.
SEQ ID NO: 194
   This shows the base sequence of DNA encoding the fusion protein of orphan receptor GPR3 and GFP-1.
SEQ ID NO: 195
   This shows the base sequence of DNA encoding the fusion protein of orphan receptor GPR6 and GFP-1.
SEQ ID NO: 196
   This shows the base sequence of DNA encoding the fusion protein of orphan receptor RAIG 1 and GFP-1.
SEQ ID NO: 197
   This shows the base sequence of DNA encoding the fusion protein of orphan receptor TGR2-1 and GFP-1.
SEQ ID NO: 198
   This shows the base sequence of DNA encoding the fusion protein of orphan receptor TGR2-2 and GFP-1.
SEQ ID NO: 199
   This shows the base sequence of DNA encoding the fusion protein of orphan receptor TGR21 and GFP-1.
SEQ ID NO: 200
   This shows the base sequence of DNA encoding the fusion protein of orphan receptor GPR56 and GFP-1.
SEQ ID NO: 201
   This shows the base sequence of DNA encoding the fusion protein of orphan receptor KIAA0758 and GFP-1.
SEQ ID NO: 202
   This shows the base sequence of DNA encoding the fusion protein of orphan receptor RE2 and GFP-1.
SEQ ID NO: 203
   This shows the base sequence of DNA encoding the fusion protein of orphan receptor P40 and GFP-1.
SEQ ID NO: 204
   This shows the base sequence of DNA encoding the fusion protein of orphan receptor GPR27 and GFP-1.
SEQ ID NO: 205
   This shows the base sequence of DNA encoding the fusion protein of orphan receptor HG38 and GFP-1.
SEQ ID NO: 206
   This shows the base sequence of DNA encoding the fusion protein of orphan receptor DRR1 and GFP-1.
SEQ ID NO: 207
   This shows the base sequence of DNA encoding the fusion protein of orphan receptor TGR12 and GFP-1.
SEQ ID NO: 208
   This shows the base sequence of DNA encoding the fusion protein of orphan receptor TGR11 and GFP-1.
SEQ ID NO: 209
   This shows the base sequence of DNA encoding the fusion protein of orphan receptor TGR15 and GFP-1.
SEQ ID NO: 210
   This shows the base sequence of DNA encoding the fusion protein of orphan receptor TGR8 and GFP-1.
SEQ ID NO: 211
   This shows the base sequence of DNA encoding the fusion protein of orphan receptor GPR20 and GFP-1.
SEQ ID NO: 212
   This shows the base sequence of DNA encoding the fusion protein of orphan receptor TGR10 and GFP-1.
SEQ ID NO: 213
   This shows the base sequence of DNA encoding the fusion protein of orphan receptor TGR30 and GFP-1.
SEQ ID NO: 214
   This shows the base sequence of DNA encoding the fusion protein of orphan receptor GPR18 and GFP-1.
SEQ ID NO: 215
   This shows the base sequence of DNA encoding the fusion protein of orphan receptor TGR25 and GFP-1.
SEQ ID NO: 216
   This shows the base sequence of DNA encoding the fusion protein of orphan receptor GPR23 and GFP-1.
SEQ ID NO: 217
   This shows the base sequence of DNA encoding the fusion protein of orphan receptor P2Y10 and GFP-1.
SEQ ID NO: 218
   This shows the base sequence of DNA encoding the fusion protein of orphan receptor GPR37 and GFP-1.
SEQ ID NO: 219
   This shows the base sequence of DNA encoding the fusion protein of orphan receptor ET(B)R-LP-2 and GFP-1.
SEQ ID NO: 220
   This shows the base sequence of DNA encoding the fusion protein of orphan receptor FPRL2 and GFP-1.
SEQ ID NO: 221
   This shows the base sequence of DNA encoding the fusion protein of orphan receptor GPR32 and GFP-1.
SEQ ID NO: 222
   This shows the base sequence of DNA encoding the fusion protein of orphan receptor dj287G14.2 and GFP-1.
SEQ ID NO: 223
   This shows the base sequence of DNA encoding the fusion protein of orphan receptor BRS-3 and GFP-1.
SEQ ID NO: 224
   This shows the base sequence of DNA encoding the fusion protein of orphan receptor GPR39 and GFP-1.
SEQ ID NO: 225
   This shows the base sequence of DNA encoding the fusion protein of orphan receptor 63A2 and GFP-1.
SEQ ID NO: 226
   This shows the base sequence of DNA encoding the fusion protein of orphan receptor GPR84 and GFP-1.
SEQ ID NO: 227
   This shows the base sequence of DNA encoding the fusion protein of orphan receptor GPR21 and GFP-1.
SEQ ID NO: 228
   This shows the base sequence of DNA encoding the fusion protein of orphan receptor GPR48 and GFP-1.
SEQ ID NO: 229
   This shows the base sequence of DNA encoding the fusion protein of orphan receptor SNORF1 and GFP-1.
SEQ ID NO: 230
   This shows the base sequence of DNA encoding the fusion protein of orphan receptor BA12 and GFP-1.
SEQ ID NO: 231
   This shows the base sequence of DNA encoding the fusion protein of orphan receptor MAS and GFP-1.
SEQ ID NO: 232
   This shows the base sequence of DNA encoding the fusion protein of orphan receptor OT7T009 and GFP-1.
SEQ ID NO: 233
   This shows the base sequence of DNA encoding the fusion protein of orphan receptor TGR34 and GFP-1.

The transformant Escherichia coli JM109/pCR4-hTGR5 obtained in REFERENCE EXAMPLE 1 has been on deposit with International Patent Organisms Depository, National Institute of Advanced Industrial Science and Technology (formerly, National Institute of Bioscience and Human-Technology (NIBH), Ministry of Economics, Trade and Industry) located at Central 6, 1-1-1 Higashi, Tsukuba, Ibaraki (post code: 305-8566), as the Accession Number FERM BP-7114 since April 3, 2000, and with Institute for Fermentation (IFO), located at 2-17-85 Juso-honmachi, Yodogawa-ku, Osaka-shi, Osaka (post code: 532-8686), as the Accession Number IFO 16410 since March 23, 2000.

### EXAMPLES

The present invention is described in detail below with reference to EXAMPLES and REFERENCE EXAMPLES, but is not deemed to limit the scope of the present invention thereto. The gene manipulation procedures using Escherichia coli were performed according to the methods described in the Molecular Cloning.

### REFERENCE EXAMPLE 1

### Cloning of cDNA encoding G protein-coupled receptor protein of human spleen and determination of the base sequence

Using human spleen-derived cDNA (Clontech) as a template and two primers, namely, primer 1 (SEQ ID NO: 11) and primer 2 (SEQ ID NO: 12), PCR was carried out. The reaction solution in the above reaction comprised of 1/10 volume of the cDNA described above as a template, 1/50 volume of Advantage-GC2 Polymerase Mix (Clontech), 0.5 µM each of primer 1 (SEQ ID NO: 11) and primer 2 (SEQ ID NO: 12), 200 µM of dNTPs, 1/5 volume of a buffer attached to the enzyme product and 1/5 volume of GC Melt to make the total volume 20 µl. The PCR reaction was carried out by reacting at 94°C for 5 minutes, then repeating 30 times a cycle set to include 94°C for 30 seconds, 60°C for 30 seconds and 68°C for 2 minutes, and finally conducting extension at 68°C for 5 minutes. The PCR product was subcloned to plasmid vector pCR4 (Invitrogen) following the instructions attached to the TA Cloning Kit (Invitrogen). The plasmid was then transfected to Escherichia coli JM109, and the clones containing the cDNA of the PCR product were selected on LB agar plates containing ampicillin. As a result of analysis for the sequence of each clone, cDNA sequence encoding a novel G protein-coupled receptor protein was obtained (SEQ ID NO: 10). The novel G protein-coupled receptor protein containing the amino acid sequence (SEQ ID NO: 9) deduced from this cDNA sequence was named TGR5. Also, the transformant containing DNA represented by SEQ ID NO: 10 was named Escherichia coli JM109/pCR4-hTGR5.

### REFERENCE EXAMPLE 2

### Detection of reporter activation with cholesterol metabolism-related substance in human HEK293 cells wherein TGR5 was expressed transiently

The TGRS-specific stimulation activity by cholesterol metabolism-related substance was detected using as an indicator the expression level of a reporter gene product (luciferase) produced by expression induction of CRE promoter.

Human-derived HEK293 cells were suspended in growth medium (DMEM (Dulbecco's Modified Eagle Medium) (Gibco BRL) supplemented with 10% fetal cow serum (Gibco BRL)). The suspension adjusted to a concentration of 1 x 10⁵ cells/well was plated on a collagen-coated black well 96-well plate (Becton Dickinson, Inc.).

After culturing overnight at 37°C under the 5% CO₂ condition, the TGR5 gene was inserted into an expression vector for animal cell pAKKO-111H (the same plasmid vector as pAKKO-1.111H described in Biochem. Biophys. Acta, Hinuma, S. et al., 1219, 251-259, 1994) by publicly known methods, together with a reporter gene-containing plasmid pCRE-Luc (Clontech). The thus prepared TGR5 expression vector plasmid or intact pAKKO-111H containing no TGR5 gene was transfected to the cells as described below.

By mixing OPTI-MEM-I (Gibco BRL, Inc.) and Lipofectamine™ 2000 reagent (Gibco BRL, Inc.) in 24:1, a Lipofectamine dilution was prepared. Also, OPTI-MEM-I, TGR5 expression vector plasmid or the intact vector plasmid (240 ng/µl) and pCRE-Luc (240 ng/µl) were mixed in 24 : 0.9 : 0.1 to prepare a DNA dilution. The Lipofectamine dilution and the DNA dilution were mixed in equal volumes and the mixture was allowed to stand for 20 minutes at room temperature to produce the DNA-Lipofectamine complex. Then, 25 µl of the solution was added to the HEK293 cell-incubated plate described above, followed by further culturing overnight at 37°C under the 5% CO₂ condition.

The transfected HEK293 cells were washed with an assay medium (DMEM supplemented with 0.1% bovine serum albumin) and lithocholic acid (Wako Pure Chemical Industries, Inc.) and progesterone (Wako Pure Chemical Industries, Inc.) diluted with the assay medium to 2 x 10⁻⁵ M were added to the plate, which was then incubated for 4 hours at 37°C under the 5% CO₂ condition. The culture supernatant was discarded and 50 µl of a substrate for assaying the luciferase activity or PicaGene LT 2.0 (Toyo Ink Mfg. Co., Ltd.) was added. The fluorescence level of luciferase was measured by using a plate reader (ARVO sx multi-label counter, Wallac, Inc.).

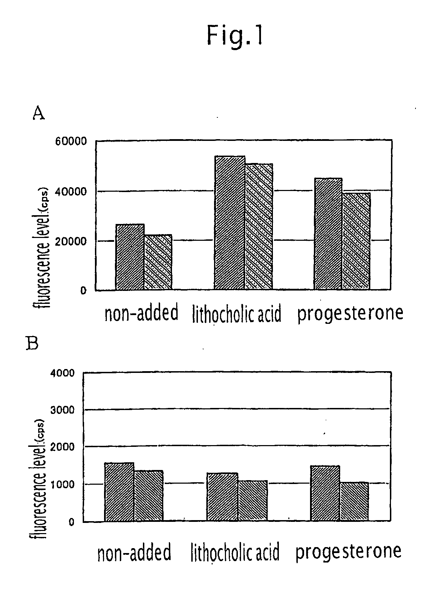

As a result, an increased luciferase activity by lithocholic acid and progesterone was noted specifically to the HEK293 cells transfected with TGR5 gene having the base sequence represented by SEQ ID NO: 10 (FIG. 1).

### REFERENCE EXAMPLE 3

### Transfection of G protein-coupled receptor protein-expressed plasmid and reporter plasmid to host cells

Using expression plasmids for animal cells inserted with various G protein-coupled receptor protein cDNAs prepared by publicly known methods, i.e., thyrotropin releasing hormone receptor (TRHR), neuromedin U receptor (FM-3 and TGR-1), prolactin releasing factor receptor (hGR3), apelin receptor (APJ), etc., Escherichia coli JM109 was transfected and the colonies obtained were isolated and cultured. Thereafter, plasmid was prepared using QIAGEN Plasmid Maxi Kit (Qiagen). Furthermore, the reporter plasmid of pCRE-Luc (Clontech) ligated with a luciferase gene as a reporter at the downstream of cAMP response element (CRE) was prepared in a similar manner.

Human HEK293 cells as host cells for transfecting the G protein-coupled receptor protein and the reporter plasmid thereto were plated on a type I collagen-coated 96-well black plate (Becton Dickinson, Inc.) in 100,000 cells/well in a culture solution volume of 100 µl, followed by culturing overnight. In the same way, CHO (dhfr-) cells were transformed by pAKKO-111H and the resulting CHO-mock cells were plated on a 96-well black plate of Costar, Inc. in 40,000 cells/well in a culture solution volume of 100 µl, followed by culturing overnight. In the both cells, DMEM (Gibco BRL, Inc.) supplemented with 10% fetal calf serum alone was used as a medium for plate culture.

Each of the plasmids above was diluted to a concentration of 240 ng/µl, and the dilution was added to 240 µl of Opti-MEM-I (Gibco BRL, Inc.) in a proportion of 9 µl of the G protein-coupled receptor protein expression plasmid and 1 µl of the reporter plasmid. The mixture was mixed with 240 µl of Opti-MEM-I added with 10 µl of Lipofectamine 2000 (Gibco BRL, Inc.) in equal volumes to form the liposome and plasmid complex, in accordance with the procedures in the instruction manuals attached to Lipofectamine 2000. Also, for carrying out efficient screening, 5 µl each of the 3 receptor expression plasmids was added in a concentration of 240 ng/µl and the mixtures having the same proportion of the other reagents as described above were prepared. The mixtures were added to the culture solution of HEK293 or CHO-mock cells in 25 µl each/well and incubated at 37°C overnight to introduce the plasmids. In the CHO-mock cells, the culture solution was replaced by an assay buffer (DMEM supplemented with 0.1% bovine serum albumin) 4 hours after the plasmid addition to make serum-free.

### REFERENCE EXAMPLE 4

### Detection of ligand activity by reporter assay

In the HEK293 cells, the culture solution was replaced by the assay buffer of REFERENCE EXAMPLE 3 an hour before the assaying and pre-incubated. A solution of a ligand or a ligand candidate compound in the assay buffer was prepared and added to the HEK293 cells or CHO-mock cells prepared in REFERENCE EXAMPLE 3. Also, an assay was conducted in a similar manner under the conditions where forskolin was added to the assay buffer in the final concentration of 2 µM. After the ligand or the test compound was added, incubation was carried out for 4 hours to induce promotion or suppression of the transcription/translation of the reporter gene derived from intracellular signal transduction caused by the agonist activity of ligand mediated by the receptor. After the incubation was completed, the assay buffer in each well was removed and 50 µl each of luminescent substrate PicaGene LT 2.0 (Toyo Ink Mfg. Co., Ltd.) was added to the well. After the cells were lysed and thoroughly mixed with the substrate, the luminescent level corresponding to the expression induction level of the reporter gene in each well was measured on the plate reader described in REFERENCE EXAMPLE 2.

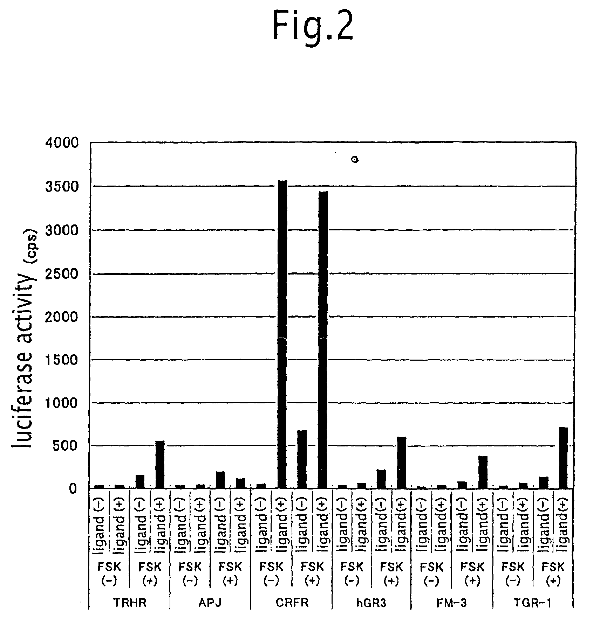

Using expression plasmids in which various G protein-coupled receptor protein cDNAs were inserted in accordance with the procedures described in REFERENCE EXAMPLES 3 and 4, the expression induction of the reporter gene by ligand stimulation was assayed in HEK293 cells. In CRFR coupled to GS as α subunit of G protein for transduction of signals into cells, activation of the reporter gene due to the ligand addition was detected under both conditions in the absence of and in the presence of forskolin. In APJ coupled to inhibitory Gi, suppressed expression of the reporter gene due to the ligand addition was detected under the condition that forskolin was added. Further in the receptors TRHR, FM-3 and TGR-1 coupled to Gq, promoted expression of the reporter gene was detected under the condition that forskolin was added. Also in the receptor hGR3 coupled to both Gq and Gi, promoted expression of the reporter gene was detected as well, under the condition that forskolin was added (FIG. 2).

### REFERENCE EXAMPLE 5

### Reporter assay using inhibitory G protein α subunit Gi-expressed plasmid

Inhibitory G protein α subunit (Gi)-expressed plasmid was prepared in a similar manner to the G protein receptor-expressed plasmid shown in REFERENCE EXAMPLE 3 (herein Gi may be any kind irrespective of any animal species). This plasmid, the receptor-expressed plasmid and the reporter plasmid were added to 240 µl of Opti-MEM-I in the proportion of 3 µl, 7 µl and 1 µl and DNA was introduced into HEK293 or CHO-mock cells otherwise under the same conditions as in EXAMPLE 2. The mixing ratio of these 3 plasmids is from 1 to 6 µl of Gi, preferably from 1 to 3 µl, when the total amount is made 11 µl. These plasmids were assayed in accordance with the procedures of EXAMPLE 3 and the ligand activities were detected.

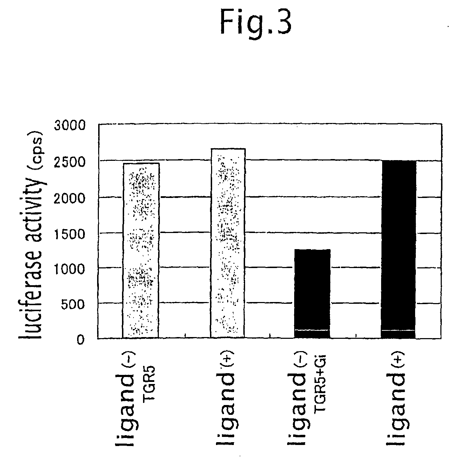

That is, the response of TGR5 to lithocholic acid in the co-presence of Gi was detected. As a result, Gi was co-expressed together with TGR5 in the assay for G protein receptor TGR5 using the CHO-mock cells, whereby the luciferase activity added with no ligand (ligand (-)) could be markedly reduced, which enabled to detect the increase in activity by the ligand (lithocholic acid, 2 x 10⁻⁵ M, ligand (+)) (FIG. 3).

### EXAMPLE 1

### Internalization of the TGR5-GFP fusion protein expressed on CHO cells by the addition of taurolithocholic acid

An expression plasmid was constructed to express the fusion protein in which TGR5 was fused at the C terminus with Green Fluorescent Protein (GFP) isolated from jelly fish Aequorea victoria. In this case, a fragment excised from expression vector pQBI25 (TaKaRa Shuzo) for GFP was used as cDNA (SEQ ID NO: 2) of GFP. In the cDNA of TGR5, its termination codon was corrected by PCR to the recognition sequence of restriction enzyme NheI, and the cDNA fragment of GFP was ligated thereto, which was inserted into expression vector pAKKO-111H described in EXAMPLE 1. The expression vector plasmid for the thus obtained fusion protein of TGR5 and GFP (hereinafter TGR5-GFP) was transfected to CHO-mock cells by the following procedures. The CHO-mock cells were suspended in growth medium [DMEM (Dulbecco's Modified Eagle Medium) (GIBCO BRL, Inc.) supplemented with 10% fetal calf serum (GIBCO BRL, Inc.)] and plated on a Lab-TekII coverglass chamber (Nalgen Nunc, Inc.) with 4 chamber compartments in a concentration of 0.6 x 10⁵ cells/chamber. After culturing overnight at 37°C under the 5% CO₂ condition, transfection was effected. For the transfection, Lipofectamine™ 2000 reagent (GIBCO BRL, Inc.) was used. First, 2 µl of Lipofectamine™ 2000 reagent was mixed with 50 µl of OPTI-MEM-I (GIBCO BRL, Inc.). After allowing to stand for 5 minutes, the mixture was mixed with a solution mixture of 0.48 µg of DNA and 50 µl of OPTI-MEM-I. The mixture was left to stand at room temperature for 20 minutes, thereby to form the DNA-lipofectamine complex. After 100 µl of the mixture was added to the chamber above where the CHO cells were incubated, incubation was conducted overnight at 37°C under the 5% CO₂ condition. The medium was replaced by medium for confocal laser scanning microscopic observation [suspension of 0.1% bovine albumin (Essentially Fatty Acid Free (GIBCO BRL, Inc.) in Hanks' Balanced Salt Solution (GIBCO BRL, Inc.))] and the fluorescent image of GFP was observed on a confocal laser scanning microscope (Leica). In this case, GFP was excited at 488 nm.

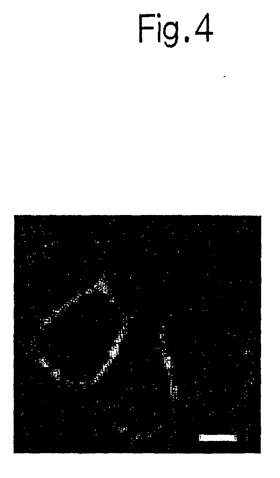

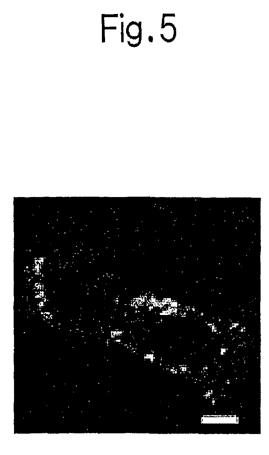

As a result, the TGR5-GFP fluorescent protein was observed in the cell membrane (FIG. 4). Taurolithocholic acid was added to the cells to have 10⁻⁵ M in the medium. It was found that 30 minutes after, the fluorescence of GFP was moved to the cytoplasm, not in the cell membrane (FIG. 5). This indicates that TGR5 is a G protein-coupled receptor and at the same time, TGR5 moves to the cytoplasm, namely, internalized, in response to taurolithocholic acid.

### EXAMPLE 2

### Expression of the fusion protein of parathyroid hormone receptor (PTH-R) and GFP in pancreas β cell line RINm5F by transient expression

Expression vector was prepared in a manner similar to EXAMPLE 1 by inserting an expression plasmid for expressing the fusion protein of human PTH-R (SEQ ID NO: 13) and GFP at the C terminus into expression vector pAKKO-111H described in REFERENCE EXAMPLE 2. The expression vector plasmid for the thus obtained fusion protein of PTH-R and GFP (hereinafter PTH-GFP) was transfected to RINm5F cells by the following procedures. The RINm5F cells were suspended in growth medium [RPMI1640 (GIBCO BRL, Inc.) supplemented with 10% fetal calf serum (GIBCO BRL, Inc.) treated by Charcoal/Dextran] and plated on a Lab-TekII coverglass chamber (Nalgen Nunc, Inc.) with 8 chamber compartments in a concentration of 0.3 x 10⁵ cells/chamber. After culturing overnight at 37°C under the 5% CO₂ condition, transfection was effected. For the transfection, Lipofectamine™ 2000 reagent (GIBCO BRL, Inc.) was used. First, 3.3 µl of Lipofectamine™ 2000 reagent was mixed with 80 µl of OPTI-MEM-I (GIBCO BRL, Inc.). After allowing to stand for 5 minutes, the mixture was mixed with a solution mixture of 0.72 µg of DNA and 80 µl of OPTI-MEM-I. The mixture was left to stand at room temperature for 20 minutes, thereby to form the DNA-lipofectamine complex. After 160 µl of the solution mixture was added to the chamber above where the RINm5F cells were incubated, the cells were incubated overnight at 37°C under the 5% CO₂ condition. Confocal laser scanning microscopic observation was performed in a manner similar to EXAMPLE 1.

As a result, it was observed that the PTH-GFP fusion protein was expressed in the cell membrane.

### EXAMPLE 3

### Expression of the GPCR and GFP fusion protein using insulin II promoter

In a manner similar to EXAMPLE 1, a fragment of human GPR40 (SEQ ID NO: 15) ligated with GFP at the C terminus was inserted at the downstream of mouse insulin II promoter cloned from mouse genome to prepare an expression vector for expressing the fusion protein of GPR40 and GFP (hereinafter GPR40-GFP). The expression vector plasmid for the thus obtained GPR40-GFP was transfected to MIN6 cells by the following procedures. The MIN6 cells were suspended in growth medium [DMEM (containing 4.5 g/l Glucose) (Invitrogen, Inc.) supplemented with 15% fetal calf serum (Trace, Inc.) in the final concentration, 55 µM of 2-mercaptoethanol (Invitrogen, Inc.) and 20 mM HEPES (Dainippon Pharmaceutical Co., Ltd.)] and plated on a Lab-TekII coverglass chamber (Nalgen Nunc, Inc.) with 4 chamber compartments in a concentration of 1.2 x 10⁵ cells/chamber. After culturing for 2 nights at 37°C under the 5% CO₂ condition, transfection was effected. For the transfection, Lipofectamine™ 2000 reagent (GIBCO BRL, Inc.) was used. First, 4 µl of Lipofectamine™ 2000 reagent was mixed with 100 µl of Opti-MEM (Invitrogen, Inc.). After allowing to stand for 5 minutes, the mixture was mixed with a solution mixture of 2 µg of DNA and 100 µl of Opti-MEM medium. The mixture was left to stand at room temperature for 20 minutes, thereby to form the DNA-lipofectamine complex. After 100 µl of the solution mixture was added to the chamber above where the MIN6 cells were incubated, the cells were incubated for 4 hours at 37°C under the 5% CO₂ condition. Then, the medium was replaced by 400 µl of a fresh growth medium followed by further incubation overnight. Confocal laser scanning microscopic observation was made as in EXAMPLE 1.

As a result, the GPR40-GFP fluorescent protein was observed in the cell membrane of MIN6 cells.

### EXAMPLE 4

### Preparation of transformants capable of individually expressing 102 kinds of fusion proteins

Expression vector plasmids of 102 kinds containing the respective DNAs (SEQ ID NO: 132 through SEQ ID NO: 233) encoding the fusion proteins of 102 kinds of the respective receptor proteins for which ligands have not been identified and GFP consisting of the amino acid sequence represented by SEQ ID NO: 1 or its modified amino acid sequences were prepared, and transfected into CHO-mock cells. These CHO cells were incubated as in EXAMPLE 1. It was observed that the fusion proteins were expressed in the CHO-mock cells.

### INDUSTRIAL APPLICABILITY

Since various cell lines can be used, the methods of the present invention for determining ligands to receptor proteins for which ligands have not been identified are simple and can be performed in a short period of time.

## Claims

1. A method of determining a ligand to a receptor protein for which a ligand has not been identified, which comprises using a fusion protein of the receptor protein and a fluorescent protein.

2. The ligand determination method according to claim 1, wherein the fusion protein of a receptor protein for which a ligand has not been identified and GFP is used.

3. The ligand determination method according to claim 1, wherein a cell expressed with the fusion protein of a receptor protein for which a ligand has not been identified and GFP or a membrane fraction of the cell is brought in contact with a test compound.

4. The ligand determination method according to claim 1, which comprises assaying (1) an activity that accelerates or suppresses arachidonic acid release, acetylcholine release, intracellular Ca²⁺ release, intracellular cAMP production, intracellular cGMP production, inositol phosphate production, changes in cell membrane potential, phosphorylation of intracellular proteins, activation of c-fos or pH reduction, (2) MAP kinase activation, (3) transcription factor activation, (4) diacylglycerol production, (5) opening or closing of ion channels on a cell membrane, (6) apoptosis induction, (7) morphological changes in cells, (8) transport of the fusion protein from cell membrane to cytoplasm, (9) low molecular weight G protein activation, (10) cell division-promoting activity or (11) DNA synthesis-promoting activity.

5. The ligand determination method according to claim 1, wherein transport of the fusion protein from cell membrane to cytoplasm is assayed.

6. The ligand determination method according to claim 5, wherein transport of the fusion protein from cell membrane to cytoplasm is assayed by observing the fluorescence of GFP.

7. The ligand determination method according to claim 1, which comprises bringing a cell capable of expressing the fusion protein of a receptor protein for which a ligand has not been identified the receptor protein and a fluorescent protein and containing a plasmid ligated with a DNA encoding a reporter protein at the downstream of cAMP response element/promoter in contact with a test compound and assaying the activity of the reporter protein.

8. The method according to claim 7, which comprises culturing a cell containing (1) a plasmid containing a DNA encoding the fusion protein of a receptor protein for which a ligand has not been identified and a fluorescent protein and (2) a plasmid ligated with a DNA encoding a reporter protein at the downstream of cAMP response element/promoter, and bringing the cell in contact with a test compound and assaying the activity of the reporter protein.

9. The ligand determination method according to claim 2, wherein a cell capable of expressing the fusion protein of a receptor protein for which a ligand has not been identified and GFP and containing a plasmid ligated with a DNA encoding a reporter protein at the downstream of cAMP response element/promoter is brought in contact with a test compound and assaying the activity of the reporter protein.

10. The ligand determination method according to claim 9, which comprises culturing a cell containing (1) a plasmid containing a DNA encoding the fusion protein of a receptor protein for which a ligand has not been identified and GFP and (2) a plasmid ligated with a DNA encoding a reporter protein at the downstream of cAMP response element/promoter, bringing the cell in contact with a test compound and assaying the activity of the reporter protein.

11. The method according to claim 1, wherein the receptor protein is a G protein-coupled receptor protein.

12. The method according to claim 1, wherein GFP is a protein containing the same or substantially the same amino acid sequence as the amino acid sequence represented by SEQ ID NO: 1, SEQ ID NO: 3, SEQ ID NO: 5 or SEQ ID NO: 7.

13. The method according to claim 7, wherein the promoter is a TATA-like sequence.

14. The method according to claim 7, wherein the reporter protein is luciferase.

15. The method according to claim 7, wherein the plasmid is a plasmid ligated with a TATA-like promoter and a gene encoding the reporter protein at the downstream of cAMP response element.

16. The method according to claim 7, wherein the cell has expressed at least two receptor proteins for which ligands have not been identified.

17. The method according to claim 7, wherein the cell further contains a plasmid containing a gene encoding an inhibitory G protein α subunit Gi.

18. The method according to claim 7, wherein forskolin is further added.

19. The method according to claim 16, wherein the at least two receptor proteins have similar characteristics.

20. The method according to claim 19, wherein the similar characteristics are the basic expression level of the reporter protein and/or the expression level of the reporter protein when forskolin is added.

21. The method according to claim 16, which comprises measuring the basic expression level of the reporter protein when the at least two receptor proteins are expressed individually and/or the expression level of the reporter protein by the addition of forskolin, and being expressed in combination the at least two receptor proteins having an equivalent expression level of the reporter protein.

22. A fusion protein of a receptor protein for which a ligand has not been identified and a fluorescent protein, or a salt thereof.

23. The fluorescent protein or a salt thereof according to claim 22, wherein the fluorescent protein is GFP.

24. A DNA containing a DNA encoding the fusion protein according to claim 22.

25. A recombinant vector containing the DNA according to claim 22.

26. A transformant transformed with the recombinant vector according to claim 25.

27. Use of a fluorescent protein to determine a ligand to a receptor protein for which a ligand has not been identified.

28. Use of GFP to determine a ligand to a receptor protein for which a ligand has not been identified.


SEQUENCE LISTING

<110> Takeda Chemical Industries, Ltd.

<120> Determination of a ligand

<130> P03-0006PCT

<150> JP 2002-45728
<151> 2002-02-22

<150> JP 2002-213949
<151> 2002-07-23

<150> JP 2002-298237
<151> 2002-10-11

<160> 233

<210> 1
<211> 239
<212> PRT
<213> Artificial Sequence

<220>
<223> Variant of wild-type GFP

<400> 1
Met Ala Ser Lys Gly Glu Glu Leu Phe Thr Gly Val Val Pro Ile Leu
                5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Cys Tyr Gly Val Gln Cys Phe Ser Arg Tyr Pro Asp His Met Lys
65                  70                  75                  80
Arg His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Thr Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr His Gly Met Asp Glu Leu Tyr Asn
225                 230                 235

<210> 2
<211> 717
<212> DNA
<213> Artificial Sequence

<220>
<223> Variant of wild-type GFP cDNA

<400> 2
atggctagca aaggagaaga actcttcact ggagttgtcc caattcttgt tgaattagat     60
ggtgatgtta acggccacaa gttctctgtc agtggagagg gtgaaggtga tgcaacatac    120
ggaaaactta ccctgaagtt catctgcact actggcaaac tgcctgttcc atggccaaca    180
ctagtcacta ctctgtgcta tggtgttcaa tgcttttcaa gatacccgga tcatatgaaa    240
cggcatgact ttttcaagag tgccatgccc gaaggttatg tacaggaaag gaccatcttc    300
ttcaaagatg acggcaacta caagacacgt gctgaagtca agtttgaagg tgataccctt    360
gttaatagaa tcgagttaaa aggtattgac ttcaaggaag atggcaacat tctgggacac    420

aaattggaat acaactataa ctcacacaat gtatacatca tggcagacaa acaaaagaat 480
ggaatcaaag tgaacttcaa gacccgccac aacattgaag atggaagcgt tcaactagca 540
gaccattatc aacaaaatac tccaattggc gatggccctg tccttttacc agacaaccat 600
tacctgtcca cacaatctgc cctttcgaaa gatcccaacg aaaagagaga ccacatggtc 660
cttcttgagt ttgtaacagc tgctgggatt acacatggca tggatgaact gtacaac 717

<210> 3
<211> 238
<212> PRT
<213> Aequorea coerulescens

<400> 3
Met Ser Lys Gly Glu Glu Leu Phe Thr Gly Val Val Pro Ile Leu Val
                5                   10                  15
Glu Leu Asp Gly Asp Val Asn Gly His Lys Phe Ser Val Ser Gly Glu
            20                  25                  30
Gly Glu Gly Asp Ala Thr Tyr Gly Lys Leu Thr Leu Lys Phe Ile Cys
        35                  40                  45
Thr Thr Gly Lys Leu Pro Val Pro Trp Pro Thr Leu Val Thr Thr Phe
    50                  55                  60
Ser Tyr Gly Val Gln Cys Phe Ser Arg Tyr Pro Asp His Met Lys Arg
65                  70                  75                  80
His Asp Phe Phe Lys Ser Ala Met Pro Glu Gly Tyr Val Gln Glu Arg
                85                  90                  95
Thr Ile Phe Phe Lys Asp Asp Gly Asn Tyr Lys Thr Arg Ala Glu Val
            100                 105                 110
Lys Phe Glu Gly Asp Thr Leu Val Asn Arg Ile Glu Leu Lys Gly Ile
        115                 120                 125
Asp Phe Lys Glu Asp Gly Asn Ile Leu Gly His Lys Leu Glu Tyr Asn
    130                 135                 140
Tyr Asn Ser His Asn Val Tyr Ile Met Ala Asp Lys Gln Lys Asn Gly
145                 150                 155                 160
Ile Lys Val Asn Phe Lys Ile Arg His Asn Ile Glu Asp Gly Ser Val
                165                 170                 175
Gln Leu Ala Asp His Tyr Gln Gln Asn Thr Pro Ile Gly Asp Gly Pro
            180                 185                 190
Val Leu Leu Pro Asp Asn His Tyr Leu Ser Thr Gln Ser Ala Leu Ser
        195                 200                 205
Lys Asp Pro Asn Glu Lys Arg Asp His Met Val Leu Leu Glu Phe Val
    210                 215                 220
Thr Ala Ala Gly Ile Thr His Gly Met Asp Glu Leu Tyr Lys
225                 230                 235

<210> 4
<211> 714
<212> DNA
<213> Aequorea coerulescens

<400> 4
   atgagtaaag gagaagaact tttcactgga gttgtcccaa ttcttgttga attagatggt 60
   gatgttaatg ggcacaaatt ttctgtcagt ggagagggtg aaggtgatgc aacatacgga 120
   aaacttaccc ttaaatttat ttgcactact ggaaaactac ctgttccatg gccaacactt 180
   gtcactactt tctcttatgg tgttcaatgc ttttcaagat acccagatca tatgaaacgg 240
   catgactttt tcaagagtgc catgcccgaa ggttatgtac aggaaagaac tatatttttc 300
   aaagatgacg ggaactacaa gacacgtgct gaagtcaagt ttgaaggtga tacccttgtt 360
   aatagaatcg agttaaaagg tattgatttt aaagaagatg gaaacattct tggacacaaa 420
   ttggaataca actataactc acacaatgta tacatcatgg cagacaaaca aaagaatgga 480
   atcaaagtta acttcaaaat tagacacaac attgaagatg gaagcgttca actagcagac 540
   cattatcaac aaaatactcc aattggcgat ggccctgtcc ttttaccaga caaccattac 600
   ctgtccacac aatctgccct ttcgaaagat cccaacgaaa agagagacca catggtcctt 660
   cttgagtttg taacagctgc tgggattaca catggcatgg atgaactata caaa 714

<210> 5
<211> 238
<212> PRT
<213> Artificial Sequence

<220>
<223> Variant of wild-type GFP

<400> 5
Met Ser Lys Gly Glu Glu Leu Phe Thr Gly Val Val Pro Ile Leu Val
                5                   10                  15
Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45
Thr Thr Gly Lys Leu Pro Val Pro Trp Pro Thr Leu Val Thr Thr Phe
    50                  55                  60
Ser Tyr Gly Val Gln Cys Phe Ser Arg Tyr Pro Asp His Met Lys Arg
65                  70                  75                  80
His Asp Phe Phe Lys Ser Ala Met Pro Glu Gly Tyr Val Gln Glu Arg
                85                  90                  95
Thr Ile Ser Phe Lys Asp Asp Gly Asn Tyr Lys Thr Arg Ala Glu Val
            100                 105                 110
Lys Phe Glu Gly Asp Thr Leu Val Asn Arg Ile Glu Leu Lys Gly Ile
        115                 120                 125
Asp Phe Lys Glu Asp Gly Asn Ile Leu Gly His Lys Leu Glu Tyr Asn
    130                 135                 140
Tyr Asn Ser His Asn Val Tyr Ile Thr Ala Asp Lys Gln Lys Asn Gly
145                 150                 155                 160
Ile Lys Ala Asn Phe Lys Ile Arg His Asn Ile Glu Asp Gly Ser Val
                165                 170                 175
Gln Leu Ala Asp His Tyr Gln Gln Asn Thr Pro Ile Gly Asp Gly Pro
            180                 185                 190
Val Leu Leu Pro Asp Asn His Tyr Leu Ser Thr Gln Ser Ala Leu Ser
        195                 200                 205
Lys Asp Pro Asn Glu Lys Arg Asp His Met Val Leu Leu Glu Phe Val
    210                 215                 220
Thr Ala Ala Gly Ile Thr His Gly Met Asp Glu Leu Tyr Lys
225                 230                 235

<210> 6
<211> 714
<212> DNA
<213> Artificial Sequence

<220>
<223> Variant of wild-type GFP cDNA

<400> 6
atgagtaaag gagaagaact tttcactgga gttgtcccaa ttcttgttga attagatggt      60
gatgttaatg ggcacaaatt ttctgtcagt ggagagggtg aaggtgatgc aacatacgga     120
aaacttaccc ttaaatttat ttgcactact ggaaaactac ctgttccatg gccaacactt     180
gtcactactt tctcttatgg tgttcaatgc ttttcccgtt atccggatca tatgaaacgg     240
catgactttt tcaagagtgc catgcccgaa ggttatgtac aggaacgcac tatatctttc     300
aaagatgacg ggaactacaa gacgcgtgct gaagtcaagt ttgaaggtga tacccttgtt     360
aatcgtatcg agttaaaagg tattgatttt aaagaagatg gaaacattct cggacacaaa     420
ctcgagtaca actataactc acacaatgta tacatcacgg cagacaaaca aaagaatgga     480
atcaaagcta acttcaaaat tcgccacaac attgaagatg gatccgttca actagcagac     540
cattatcaac aaaatactcc aattggcgat ggccctgtcc ttttaccaga caaccattac     600
ctgtcgacac aatctgccct ttcgaaagat cccaacgaaa agcgtgacca catggtcctt     660
cttgagtttg taactgctgc tgggattaca catggcatgg atgagctcta caaa           714

<210> 7
<211> 239
<212> PRT
<213> Artificial Sequence

<220>
<223> Variant of wild-type GFP

<400> 7
Met Val Ser Lys Gly Glu Glu Leu Phe Thr Gly Val Val Pro Ile Leu
                5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Tyr Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr

130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 8
<211> 717
<212> DNA
<213> Artificial Sequence

<220>
<223> Variant of wild-type GFP cDNA

<400> 8
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgaccta cggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacaa cagccacaac gtctatatca tggccgacaa gcagaagaac     480
ggcatcaagg tgaacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgccgggatc actctcggca tggacgagct gtacaag        717

<210> 9
<211> 330
<212> PRT
<213> Human

<400> 9
Met Thr Pro Asn Ser Thr Gly Glu Val Pro Ser Pro Ile Pro Lys Gly
                5                   10                  15
Ala Leu Gly Leu Ser Leu Ala Leu Ala Ser Leu Ile Ile Thr Ala Asn
            20                  25                  30
Leu Leu Leu Ala Leu Gly Ile Ala Trp Asp Arg Arg Leu Arg Ser Pro
        35                  40                  45
Pro Ala Gly Cys Phe Phe Leu Ser Leu Leu Leu Ala Gly Leu Leu Thr
    50                  55                  60
Gly Leu Ala Leu Pro Thr Leu Pro Gly Leu Trp Asn Gln Ser Arg Arg
65                  70                  75                  80
Gly Tyr Trp Ser Cys Leu Leu Val Tyr Leu Ala Pro Asn Phe Ser Phe
                85                  90                  95
Leu Ser Leu Leu Ala Asn Leu Leu Leu Val His Gly Glu Arg Tyr Met
            100                 105                 110
Ala Val Leu Arg Pro Leu Gln Pro Pro Gly Ser Ile Arg Leu Ala Leu
        115                 120                 125
Leu Leu Thr Trp Ala Gly Pro Leu Leu Phe Ala Ser Leu Pro Ala Leu
    130                 135                 140
Gly Trp Asn His Trp Thr Pro Gly Ala Asn Cys Ser Ser Gln Ala Ile
145                 150                 155                 160
Phe Pro Ala Pro Tyr Leu Tyr Leu Glu Val Tyr Gly Leu Leu Leu Pro
                165                 170                 175
Ala Val Gly Ala Ala Ala Phe Leu Ser Val Arg Val Leu Ala Thr Ala
            180                 185                 190
His Arg Gln Leu Gln Asp Ile Cys Arg Leu Glu Arg Ala Val Cys Arg
        195                 200                 205
Asp Glu Pro Ser Ala Leu Ala Arg Ala Leu Thr Trp Arg Gln Ala Arg
    210                 215                 220
Ala Gln Ala Gly Ala Met Leu Leu Phe Gly Leu Cys Trp Gly Pro Tyr
225                 230                 235                 240
Val Ala Thr Leu Leu Leu Ser Val Leu Ala Tyr Glu Gln Arg Pro Pro
                245                 250                 255
Leu Gly Pro Gly Thr Leu Leu Ser Leu Leu Ser Leu Gly Ser Ala Ser

            260                 265                 270
Ala Ala Ala Val Pro Val Ala Met Gly Leu Gly Asp Gln Arg Tyr Thr
        275                 280                 285
Ala Pro Tyr Arg Ala Ala Ala Gln Arg Cys Leu Gln Gly Leu Trp Gly
    290                 295                 300
Arg Ala Ser Arg Asp Ser Pro Gly Pro Ser Ile Ala Tyr His Pro Ser
305                 310                 315                 320
Ser Gln Ser Ser Val Asp Leu Asp Leu Asn
                325                 330

<210> 10
<211> 990
<212> DNA
<213> Human

<400> 10
atgacgccca acagcactgg cgaggtgccc agccccattc ccaagggggc tttggggctc 60
tccctggccc tggcaagcct catcatcacc gcgaacctgc tcctagccct gggcatcgcc 120
tgggaccgcc gcctgcgcag cccacctgct ggctgcttct tcctgagcct actgctggct 180
gggctgctca cgggtctggc attgcccaca ttgccagggc tgtggaacca gagtcgccgg 240
ggttactggt cctgcctcct cgtctacttg gctcccaact tctccttcct ctccctgctt 300
gccaacctct tgctggtgca cggggagcgc tacatggcag tcctgaggcc actccagccc 360
cctgggagca ttcggctggc cctgctcctc acctgggctg gtcccctgct ctttgccagt 420
ctgcccgctc tggggtggaa ccactggacc cctggtgcca actgcagctc ccaggctatc 480
ttcccagccc cctacctgta cctcgaagtc tatgggctcc tgctgcccgc cgtgggtgct 540
gctgccttcc tctctgtccg cgtgctggcc actgcccacc gccagctgca ggacatctgc 600
cggctggagc gggcagtgtg ccgcgatgag ccctccgccc tggcccgggc ccttacctgg 660
aggcaggcaa gggcacaggc tggagccatg ctgctcttcg ggctgtgctg ggggccctac 720
gtggccacac tgctcctctc agtcctggcc tatgagcagc gcccgccact ggggcctggg 780
acactgttgt ccctcctctc cctaggaagt gccagtgcag cggcagtgcc cgtagccatg 840
gggctgggcg atcagcgcta cacagccccc tggagggcag ccgcccaaag gtgcctgcag 900
gggctgtggg gaagagcctc ccgggacagt cccggcccca gcattgccta ccacccaagc 960
agccaaagca gtgtcgacct ggacttgaac 990

<210> 11
<211> 30
<212> DNA
<213> Artificial Sequence

<220>
<223> Designed oligonucleotide primer to amplify DNA encoding TGR5

<400> 11
gatgacgccc aacagcactg gcgaggtgcc 30

<210> 12
<211> 31
<212> DNA
<213> Artificial Sequence

<220>
<223> Designed oligonucleotide primer to amplify DNA encoding TGR5

<400> 12
ttagttcaag tccaggtcga cactgctttg g 31

<210> 13
<211> 593
<212> PRT
<213> Human

<400> 13

<210> 14
<211> 1779
<212> DNA
<213> Human

<400> 14
atggggaccg cccggatcgc acccggcctg gcgctcctgc tctgctgccc cgtgctcagc    60
tccgcgtacg cgctggtgga tgcagatgac gtcatgacta aagaggaaca gatcttcctg    120
ctgcaccgtg ctcaggccca gtgcgaaaaa cggctcaagg aggtcctgca gaggccagcc    180
agcataatgg aatcagacaa gggatggaca tctgcgtcca catcagggaa gcccaggaaa    240
gataaggcat ctgggaagct ctaccctgag tctgaggagg acaaggaggc acccactggc    300
agcaggtacc gagggcgccc ctgtctgccg gaatgggacc acatcctgtg ctggccgctg    360

ggggcaccag gtgaggtggt ggctgtgccc tgtccggact acatttatga cttcaatcac 420
aaaggccatg cctaccgacg ctgtgaccgc aatggcagct gggagctggt gcctgggcac 480
aacaggacgt gggccaacta cagcgagtgt gtcaaatttc tcaccaatga gactcgtgaa 540
cgggaggtgt ttgaccgcct gggcatgatt tacaccgtgg gctactccgt gtccctggcg 600
tccctcaccg tagctgtgct catcctggcc tactttaggc ggctgcactg cacgcgcaac 660
tacatccaca tgcacctgtt cctgtccttc atgctgcgcg ccgtgagcat cttcgtcaag 720
gacgctgtgc tctactctgg cgccacgctt gatgaggctg agcgcctcac cgaggaggag 780
ctgcgcgcca tcgcccaggc gcccccgccg cctgccaccg ccgctgccgg ctacgcgggc 840
tgcagggtgg ctgtgacctt cttcctttac ttcctggcca ccaactacta ctggattctg 900
gtggagggcc tgtacctgca cagcctcatc ttcatggcct tcttctcaga gaagaagtac 960
ctgtggggct tcacagtctt cggctggggt ctgcccgctg tcttcgtggc tgtgtgggtc 1020
agtgtcagag ctaccctggc caacaccggg tgctgggact tgagctccgg gaacaaaaag 1080
tggatcatcc aggtgcccat cctggcctcc attgtgctca acttcatcct cttcatcaat 1140
atcgtccggg tgctcgccac caagctgcgg gagaccaacg ccggccggtg tgacacacgg 1200
cagcagtacc ggaagctgct caaatccacg ctggtgctca tgcccctctt tggcgtccac 1260
tacattgtct tcatggccac accatacacc gaggtctcag ggacgctctg gcaagtccag 1320
atgcactatg agatgctctt caactccttc cagggatttt ttgtcgcaat catatactgt 1380
ttctgcaatg gcgaggtaca agctgagatc aagaaatctt ggagccgctg gacactggca 1440
ctggacttca agcgaaaggc acgcagcggg agcagcagct atagctacgg ccccatggtg 1500
tcccacacaa gtgtgaccaa tgtcggcccc cgtgtgggac tcggcctgcc cctcagcccc 1560
cgcctactgc ccactgccac caccaacggc caccctcagc tgcctggcca tgccaagcca 1620
gggaccccag ccctggagac cctcgagacc acaccacctg ccatggctgc tcccaaggac 1680
gatgggttcc tcaacggctc ctgctcaggc ctggacgagg aggcctctgg gcctgagcgg 1740
ccacctgccc tgctacagga agagtgggag acagtcatg 1779

<210> 15
<211> 300
<212> PRT
<213> Human

<400> 15
Met Asp Leu Pro Pro Gln Leu Ser Phe Gly Leu Tyr Val Ala Ala Phe
                5                   10                  15
Ala Leu Gly Phe Pro Leu Asn Val Leu Ala Ile Arg Gly Ala Thr Ala
            20                  25                  30
His Ala Arg Leu Arg Leu Thr Pro Ser Leu Val Tyr Ala Leu Asn Leu
        35                  40                  45
Gly Cys Ser Asp Leu Leu Leu Thr Val Ser Leu Pro Leu Lys Ala Val
    50                  55                  60
Glu Ala Leu Ala Ser Gly Ala Trp Pro Leu Pro Ala Ser Leu Cys Pro
65                  70                  75                  80
Val Phe Ala Val Ala His Phe Phe Pro Leu Tyr Ala Gly Gly Gly Phe
                85                  90                  95
Leu Ala Ala Leu Ser Ala Gly Arg Tyr Leu Gly Ala Ala Phe Pro Leu
            100                 105                 110
Gly Tyr Gln Ala Phe Arg Arg Pro Cys Tyr Ser Trp Gly Val Cys Ala
        115                 120                 125
Ala Ile Trp Ala Leu Val Leu Cys His Leu Gly Leu Val Phe Gly Leu
    130                 135                 140
Glu Ala Pro Gly Gly Trp Leu Asp His Ser Asn Thr Ser Leu Gly Ile
145                 150                 155                 160
Asn Thr Pro Val Asn Gly Ser Pro Val Cys Leu Glu Ala Trp Asp Pro
                165                 170                 175
Ala Ser Ala Gly Pro Ala Arg Phe Ser Leu Ser Leu Leu Leu Phe Phe
            180                 185                 190
Leu Pro Leu Ala Ile Thr Ala Phe Cys Tyr Val Gly Cys Leu Arg Ala
        195                 200                 205
Leu Ala Arg Ser Gly Leu Thr His Arg Arg Lys Leu Arg Ala Ala Trp
    210                 215                 220
Val Ala Gly Gly Ala Leu Leu Thr Leu Leu Leu Cys Val Gly Pro Tyr
225                 230                 235                 240
Asn Ala Ser Asn Val Ala Ser Phe Leu Tyr Pro Asn Leu Gly Gly Ser
                245                 250                 255
Trp Arg Lys Leu Gly Leu Ile Thr Gly Ala Trp Ser Val Val Leu Asn
            260                 265                 270
Pro Leu Val Thr Gly Tyr Leu Gly Arg Gly Pro Gly Leu Lys Thr Val
        275                 280                 285
Cys Ala Ala Arg Thr Gln Gly Gly Lys Ser Gln Lys
    290                 295                 300

<210> 16
<211> 900
<212> DNA
<213> Human

<400> 16
atggacctgc ccccgcagct ctccttcggc ctctatgtgg ccgcctttgc gctgggcttc 60
ccgctcaacg tcctggccat ccgaggcgcg acggcccacg cccggctccg tctcacccct 120
agcctggtct acgccctgaa cctgggctgc tccgacctgc tgctgacagt ctctctgccc 180
ctgaaggcgg tggaggcgct agcctccggg gcctggcctc tgccggcctc gctgtgcccc 240
gtcttcgcgg tggcccactt cttcccactc tatgccggcg ggggcttcct ggccgccctg 300
agtgcaggcc gctacctggg agcagccttc cccttgggct accaagcctt ccggaggccg 360
tgctattcct ggggggtgtg cgcggccatc tgggccctcg tcctgtgtca cctgggtctg 420
gtctttgggt tggaggctcc aggaggctgg ctggaccaca gcaacacctc cctgggcatc 480
aacacaccgg tcaacggctc tccggtctgc ctggaggcct gggacccggc ctctgccggc 540
ccggcccgct tcagcctctc tctcctgctc ttttttctgc ccttggccat cacagccttc 600
tgctacgtgg gctgcctccg ggcactggcc cgctccggcc tgacgcacag gcggaagctg 660
cgggccgcct gggtggccgg cggggccctc ctcacgctgc tgctctgcgt aggaccctac 720
aacgcctcca acgtggccag cttcctgtac cccaatctag gaggctcctg gcggaagctg 780
gggctcatca cgggtgcctg gagtgtggtg cttaatccgc tggtgaccgg ttacttggga 840
aggggtcctg gcctgaagac agtgtgtgcg gcaagaacgc aagggggcaa gtcccagaag 900

<210> 17
<211> 6
<212> PRT
<213> Artificial sequence

<220>
<223> His-tag

<400> 17
His His His His His His
1               5

<210> 18
<211> 14
<212> PRT
<213> Artificial sequence

<220>
<223> V5-tag

<400> 18
Gly Lys Pro Ile Pro Asn Pro Leu Leu Gly Leu Asp Ser Thr
1               5                   10

<210> 19
<211> 10
<212> PRT
<213> Artificial sequence

<220>
<223> myc-tag

<400> 19
Glu Gln Lys Leu Ile Ser Glu Glu Asp Leu
1               5                   10

<210> 20
<211> 8
<212> PRT
<213> Artificial sequence

<220>
<223> Xpress-tag

<400> 20
Asp Leu Tyr Asp Asp Asp Asp Lys
1               5

<210> 21
<211> 16
<212> PRT
<213> Artificial sequence

<220>
<223> HA-tag

<400> 21
Met Gly Ser Tyr Pro Tyr Asp Val Pro Asp Tyr Ala Ser Leu Glu Phe

1                 5                   10                  15

<210> 22
<211> 239
<212> PRT
<213> Artificial sequence

<220>
<223> Variant of wild-type GFP

<400> 22
Met Val Ser Lys Gly Glu Glu Leu Phe Thr Gly Val Val Pro Ile Leu
                5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr Trp Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Ile Ser His Asn Val Tyr Ile Thr Ala Asp Lys Gln Lys Asn
145                 150                 155                 160
Gly Ile Lys Ala Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 23
<211> 720
<212> DNA
<213> Artificial sequence

<220>
<223> Variant of wild-type GFP cDNA

<400> 23
atggtgagca agggcgagga gctgttcacc ggggtggtgc ccatcctggt cgagctggac      60
ggcgacgtaa acggccacaa gttcagcgtg tccggcgagg gcgagggcga tgccacctac     120
ggcaagctga ccctgaagtt catctgcacc accggcaagc tgcccgtgcc ctggcccacc     180
ctcgtgacca ccctgacctg gggcgtgcag tgcttcagcc gctaccccga ccacatgaag     240
cagcacgact tcttcaagtc cgccatgccc gaaggctacg tccaggagcg caccatcttc     300
ttcaaggacg acggcaacta caagacccgc gccgaggtga agttcgaggg cgacaccctg     360
gtgaaccgca tcgagctgaa gggcatcgac ttcaaggagg acggcaacat cctggggcac     420
aagctggagt acaactacat cagccacaac gtctatatca ccgccgacaa gcagaagaac     480
ggcatcaagg ccaacttcaa gatccgccac aacatcgagg acggcagcgt gcagctcgcc     540
gaccactacc agcagaacac ccccatcggc gacggccccg tgctgctgcc cgacaaccac     600
tacctgagca cccagtccgc cctgagcaaa gaccccaacg agaagcgcga tcacatggtc     660
ctgctggagt tcgtgaccgc cgccgggatc actctcggca tggacgagct gtacaagtaa     720

<210> 24
<211> 239
<212> PRT
<213> Artificial sequence

<220>
<223> Variant of wild-type GFP

<400> 24
Met Val Ser Lys Gly Glu Glu Leu Phe Thr Gly Val Val Pro Ile Leu
                5                   10                  15

Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Phe Gly Tyr Gly Leu Gln Cys Phe Ala Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Tyr Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Tyr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 25
<211> 720
<212> DNA
<213> Artificial sequence

<220>
<223> Variant of wild-type GFP cDNA

<400> 25
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccttcggcta cggcctgcag tgcttcgccc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagct accagtccgc cctgagcaaa gaccccaacg agaagcgcga tcacatggtc 660
ctgctggagt tcgtgaccgc cgccgggatc actctcggca tggacgagct gtacaagtaa 720

<210> 26
<211> 225
<212> PRT
<213> Discosoma sp.

<400> 26
Met Arg Ser Ser Lys Asn Val Ile Lys Glu Phe Met Arg Phe Lys Val
                5                   10                  15
Arg Met Glu Gly Thr Val Asn Gly His Glu Phe Glu Ile Glu Gly Glu
            20                  25                  30
Gly Glu Gly Arg Pro Tyr Glu Gly His Asn Thr Val Lys Leu Lys Val
        35                  40                  45
Thr Lys Gly Gly Pro Leu Pro Phe Ala Trp Asp Ile Leu Ser Pro Gln
    50                  55                  60
Phe Gln Tyr Gly Ser Lys Val Tyr Val Lys His Pro Ala Asp Ile Pro
65                  70                  75                  80
Asp Tyr Lys Lys Leu Ser Phe Pro Glu Gly Phe Lys Trp Glu Arg Val
                85                  90                  95
Met Asn Phe Glu Asp Gly Gly Val Val Thr Val Thr Gln Asp Ser Ser
            100                 105                 110
Leu Gln Asp Gly Cys Phe Ile Tyr Lys Val Lys Phe Ile Gly Val Asn
        115                 120                 125
Phe Pro Ser Asp Gly Pro Val Met Gln Lys Lys Thr Met Gly Trp Glu
    130                 135                 140

Ala Ser Thr Glu Arg Leu Tyr Pro Arg Asp Gly Val Leu Lys Gly Glu
145                 150                 155                 160
Ile His Lys Ala Leu Lys Leu Lys Asp Gly Gly His Tyr Leu Val Glu
                165                 170                 175
Phe Lys Ser Ile Tyr Met Ala Lys Lys Pro Val Gln Leu Pro Gly Tyr
            180                 185                 190
Tyr Tyr Val Asp Ser Lys Leu Asp Ile Thr Ser His Asn Glu Asp Tyr
        195                 200                 205
Thr Ile Val Glu Gln Tyr Glu Arg Thr Glu Gly Arg His His Leu Phe
    210                 215                 220
Leu
225

<210> 27
<211> 678
<212> DNA
<213> Discosoma sp.

<400> 27
atgaggtctt ccaagaatgt tatcaaggag ttcatgaggt ttaaggttcg catggaagga      60
acggtcaatg ggcacgagtt tgaaatagaa ggcgaaggag aggggaggcc atacgaaggc     120
cacaataccg taaagcttaa ggtaaccaag gggggacctt tgccatttgc ttgggatatt     180
ttgtcaccac aatttcagta tggaagcaag gtatatgtca agcaccctgc cgacatacca     240
gactataaaa agctgtcatt tcctgaagga tttaaatggg aaagggtcat gaactttgaa     300
gacggtggcg tcgttactgt aacccaggat tccagtttgc aggatggctg tttcatctac     360
aaggtcaagt tcattggcgt gaactttcct tccgatggac ctgttatgca aaagaagaca     420
atgggctggg aagccagcac tgagcgtttg tatcctcgtg atggcgtgtt gaaaggagag     480
attcataagg ctctgaagct gaaagacggt ggtcattacc tagttgaatt caaaagtatt     540
tacatggcaa agaagcctgt gcagctacca gggtactact atgttgactc caaactggat     600
ataacaagcc acaacgaaga ctatacaatc gttgagcagt atgaaagaac cgagggacgc     660
caccatctgt tcctttag                                                   678

<210> 28
<211> 239
<212> PRT
<213> Artificial sequence

<220>
<223> Variant of wild-type GFP

<400> 28
Met Val Ser Lys Gly Glu Glu Leu Phe Thr Gly Val Val Pro Ile Leu
                5                   10                  15
Val Glu Leu Asp Gly Asp Val Asn Gly His Lys Phe Ser Val Ser Gly
            20                  25                  30
Glu Gly Glu Gly Asp Ala Thr Tyr Gly Lys Leu Thr Leu Lys Phe Ile
        35                  40                  45
Cys Thr Thr Gly Lys Leu Pro Val Pro Trp Pro Thr Leu Val Thr Thr
    50                  55                  60
Leu Thr His Gly Val Gln Cys Phe Ser Arg Tyr Pro Asp His Met Lys
65                  70                  75                  80
Gln His Asp Phe Phe Lys Ser Ala Met Pro Glu Gly Tyr Val Gln Glu
                85                  90                  95
Arg Thr Ile Phe Phe Lys Asp Asp Gly Asn Tyr Lys Thr Arg Ala Glu
            100                 105                 110
Val Lys Phe Glu Gly Asp Thr Leu Val Asn Arg Ile Glu Leu Lys Gly
        115                 120                 125
Ile Asp Phe Lys Glu Asp Gly Asn Ile Leu Gly His Lys Leu Glu Tyr
    130                 135                 140
Asn Phe Asn Ser His Asn Val Tyr Ile Met Ala Asp Lys Gln Lys Asn
145                 150                 155                 160
Gly Ile Lys Val Asn Phe Lys Ile Arg His Asn Ile Glu Asp Gly Ser
                165                 170                 175
Val Gln Leu Ala Asp His Tyr Gln Gln Asn Thr Pro Ile Gly Asp Gly
            180                 185                 190
Pro Val Leu Leu Pro Asp Asn His Tyr Leu Ser Thr Gln Ser Ala Leu
        195                 200                 205
Ser Lys Asp Pro Asn Glu Lys Arg Asp His Met Val Leu Leu Glu Phe
    210                 215                 220
Val Thr Ala Ala Gly Ile Thr Leu Gly Met Asp Glu Leu Tyr Lys
225                 230                 235

<210> 29
<211> 720
<212> DNA

<213> Artificial sequence

<220>
<223> Variant of wild-type GFP cDNA

<400> 29
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgaccca cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaacttcaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagtaa 720

<210> 30
<211> 544
<212> PRT
<213> Human

<400> 30

<210> 31
<211> 612
<212> PRT
<213> Human

<400> 31

<210> 32
<211> 599
<212> PRT
<213> Human

<400> 32

<210> 33
<211> 890
<212> PRT
<213> Human

<400> 33

<210> 34
<211> 598
<212> PRT
<213> Human

<400> 34

<210> 35
<211> 557
<212> PRT
<213> Human

<400> 35

<210> 36
<211> 641
<212> PRT
<213> Human

<400> 36

<210> 37
<211> 581
<212> PRT
<213> Human

<400> 37

<210> 38
<211> 1252
<212> PRT
<213> Human

<400> 38

<210> 39
<211> 655
<212> PRT
<213> Human

<400> 39

<210> 40
<211> 573
<212> PRT
<213> Human

<400> 40

<210> 41
<211> 920
<212> PRT
<213> Human

<400> 41

<210> 42
<211> 766
<212> PRT
<213> Human

<400> 42

<210> 43
<211> 1124
<212> PRT
<213> Human

<400> 43

<210> 44
<211> 599
<212> PRT
<213> Human

<400> 44

<210> 45
<211> 557
<212> PRT
<213> Human

<400> 45

<210> 46
<211> 708
<212> PRT
<213> Human

<400> 46

<210> 47
<211> 637
<212> PRT
<213> Human

<400> 47

<210> 48
<211> 657
<212> PRT
<213> Human

<400> 48

<210> 49
<211> 611
<212> PRT
<213> Human

<400> 49
Met Ala Asn Thr Thr Gly Glu Pro Glu Glu Val Ser Gly Ala Leu Ser
                5                   10                  15
Pro Pro Ser Ala Ser Ala Tyr Val Lys Leu Val Leu Leu Gly Leu Ile
            20                  25                  30
Met Cys Val Ser Leu Ala Gly Asn Ala Ile Leu Ser Leu Leu Val Leu
        35                  40                  45
Lys Glu Arg Ala Leu His Lys Ala Pro Tyr Tyr Phe Leu Leu Asp Leu
    50                  55                  60
Cys Leu Ala Asp Gly Ile Arg Ser Ala Val Cys Phe Pro Phe Val Leu
65                  70                  75                  80
Ala Ser Val Arg His Gly Ser Ser Trp Thr Phe Ser Ala Leu Ser Cys
                85                  90                  95
Lys Ile Val Ala Phe Met Ala Val Leu Phe Cys Phe His Ala Ala Phe
            100                 105                 110
Met Leu Phe Cys Ile Ser Val Thr Arg Tyr Met Ala Ile Ala His His
        115                 120                 125
Arg Phe Tyr Ala Lys Arg Met Thr Leu Trp Thr Cys Ala Ala Val Ile
    130                 135                 140

Cys Met Ala Trp Thr Leu Ser Val Ala Met Ala Phe Pro Pro Val Phe
145                 150                 155                 160
Asp Val Gly Thr Tyr Lys Phe Ile Arg Glu Glu Asp Gln Cys Ile Phe
                165                 170                 175
Glu His Arg Tyr Phe Lys Ala Asn Asp Thr Leu Gly Phe Met Leu Met
            180                 185                 190
Leu Ala Val Leu Met Ala Ala Thr His Ala Val Tyr Gly Lys Leu Leu
        195                 200                 205
Leu Phe Glu Tyr Arg His Arg Lys Met Lys Pro Val Gln Met Val Pro
    210                 215                 220
Ala Ile Ser Gln Asn Trp Thr Phe His Gly Pro Gly Ala Thr Gly Gln
225                 230                 235                 240
Ala Ala Ala Asn Trp Ile Ala Gly Phe Gly Arg Gly Pro Met Pro Pro
                245                 250                 255
Thr Leu Leu Gly Ile Arg Gln Asn Gly His Ala Ala Ser Arg Arg Leu
            260                 265                 270
Leu Gly Met Asp Glu Val Lys Gly Glu Lys Gln Leu Gly Arg Met Phe
        275                 280                 285
Tyr Ala Ile Thr Leu Leu Phe Leu Leu Leu Trp Ser Pro Tyr Ile Val
    290                 295                 300
Ala Cys Tyr Trp Arg Val Phe Val Lys Ala Cys Ala Val Pro His Arg
305                 310                 315                 320
Tyr Leu Ala Thr Ala Val Trp Met Ser Phe Ala Gln Ala Ala Val Asn
                325                 330                 335
Pro Ile Val Cys Phe Leu Leu Asn Lys Asp Leu Lys Lys Cys Leu Arg
            340                 345                 350
Thr His Ala Pro Cys Trp Gly Thr Gly Gly Ala Pro Ala Pro Arg Glu
        355                 360                 365
Pro Tyr Cys Val Met Ala Ser Lys Gly Glu Glu Leu Phe Thr Gly Val
    370                 375                 380
Val Pro Ile Leu Val Glu Leu Asp Gly Asp Val Asn Gly His Lys Phe
385                 390                 395                 400
Ser Val Ser Gly Glu Gly Glu Gly Asp Ala Thr Tyr Gly Lys Leu Thr
                405                 410                 415
Leu Lys Phe Ile Cys Thr Thr Gly Lys Leu Pro Val Pro Trp Pro Thr
            420                 425                 430
Leu Val Thr Thr Leu Cys Tyr Gly Val Gln Cys Phe Ser Arg Tyr Pro
        435                 440                 445
Asp His Met Lys Arg His Asp Phe Phe Lys Ser Ala Met Pro Glu Gly
    450                 455                 460
Tyr Val Gln Glu Arg Thr Ile Phe Phe Lys Asp Asp Gly Asn Tyr Lys
465                 470                 475                 480
Thr Arg Ala Glu Val Lys Phe Glu Gly Asp Thr Leu Val Asn Arg Ile
                485                 490                 495
Glu Leu Lys Gly Ile Asp Phe Lys Glu Asp Gly Asn Ile Leu Gly His
            500                 505                 510
Lys Leu Glu Tyr Asn Tyr Asn Ser His Asn Val Tyr Ile Met Ala Asp
        515                 520                 525
Lys Gln Lys Asn Gly Ile Lys Val Asn Phe Lys Thr Arg His Asn Ile
    530                 535                 540
Glu Asp Gly Ser Val Gln Leu Ala Asp His Tyr Gln Gln Asn Thr Pro
545                 550                 555                 560
Ile Gly Asp Gly Pro Val Leu Leu Pro Asp Asn His Tyr Leu Ser Thr
                565                 570                 575
Gln Ser Ala Leu Ser Lys Asp Pro Asn Glu Lys Arg Asp His Met Val
            580                 585                 590
Leu Leu Glu Phe Val Thr Ala Ala Gly Ile Thr His Gly Met Asp Glu
        595                 600                 605
Leu Tyr Asn
    610

<210> 50
<211> 798
<212> PRT
<213> Human

<400> 50

<210> 51
<211> 557
<212> PRT
<213> Human

<400> 51

<210> 52
<211> 596
<212> PRT
<213> Human

<400> 52

<210> 53
<211> 572
<212> PRT
<213> Human

<400> 53

<210> 54
<211> 575
<212> PRT
<213> Human

<400> 54

<210> 55
<211> 580
<212> PRT
<213> Human

<400> 55

<210> 56
<211> 583
<212> PRT
<213> Human

<400> 56

<210> 57
<211> 851
<212> PRT
<213> Human

<400> 57

<210> 58
<211> 895
<212> PRT
<213> Human

<400> 58

<210> 59
<211> 1148
<212> PRT
<213> Human

<400> 59

<210> 60
<211> 642
<212> PRT
<213> Human

<400> 60

<210> 61
<211> 653
<212> PRT
<213> Human

<400> 61

<210> 62
<211> 610
<212> PRT
<213> Human

<400> 62

<210> 63
<211> 583
<212> PRT
<213> Human

<400> 63

<210> 64
<211> 1205
<212> PRT
<213> Human

<400> 64

<210> 65
<211> 573
<212> PRT
<213> Human

<400> 65

<210> 66
<211> 612
<212> PRT
<213> Human

<400> 66

<210> 67
<211> 611
<212> PRT
<213> Human

<400> 67

<210> 68
<211> 671
<212> PRT
<213> Human

<400> 68
Met Cys Phe Ser Pro Ile Leu Glu Ile Asn Met Gln Ser Glu Ser Asn
                5                   10                  15
Ile Thr Val Arg Asp Asp Ile Asp Asp Ile Asn Thr Asn Met Tyr Gln
            20                  25                  30
Pro Leu Ser Tyr Pro Leu Ser Phe Gln Val Ser Leu Thr Gly Phe Leu
        35                  40                  45
Met Leu Glu Ile Val Leu Gly Leu Gly Ser Asn Leu Thr Val Leu Val
    50                  55                  60
Leu Tyr Cys Met Lys Ser Asn Leu Ile Asn Ser Val Ser Asn Ile Ile
65                  70                  75                  80

Thr Met Asn Leu His Val Leu Asp Val Ile Ile Cys Val Gly Cys Ile
                85                  90                  95
Pro Leu Thr Ile Val Ile Leu Leu Leu Ser Leu Glu Ser Asn Thr Ala
            100                 105                 110
Leu Ile Cys Cys Phe His Glu Ala Cys Val Ser Phe Ala Ser Val Ser
        115                 120                 125
Thr Ala Ile Asn Val Phe Ala Ile Thr Leu Asp Arg Tyr Asp Ile Ser
    130                 135                 140
Val Lys Pro Ala Asn Arg Ile Leu Thr Met Gly Arg Ala Val Met Leu
145                 150                 155                 160
Met Ile Ser Ile Trp Ile Phe Ser Phe Phe Ser Phe Leu Ile Pro Phe
                165                 170                 175
Ile Glu Val Asn Phe Phe Ser Leu Gln Ser Gly Asn Thr Trp Glu Asn
            180                 185                 190
Lys Thr Leu Leu Cys Val Ser Thr Asn Glu Tyr Tyr Thr Glu Leu Gly
        195                 200                 205
Met Tyr Tyr His Leu Leu Val Gln Ile Pro Ile Phe Phe Phe Thr Val
    210                 215                 220
Val Val Met Leu Ile Thr Tyr Thr Lys Ile Leu Gln Ala Leu Asn Ile
225                 230                 235                 240
Arg Ile Gly Thr Arg Phe Ser Thr Gly Gln Lys Lys Lys Ala Arg Lys
                245                 250                 255
Lys Lys Thr Ile Ser Leu Thr Thr Gln His Glu Ala Thr Asp Met Ser
            260                 265                 270
Gln Ser Ser Gly Gly Arg Asn Val Val Phe Gly Val Arg Thr Ser Val
        275                 280                 285
Ser Val Ile Ile Ala Leu Arg Arg Ala Val Lys Arg His Arg Glu Arg
    290                 295                 300
Arg Glu Arg Gln Lys Arg Val Phe Arg Met Ser Leu Leu Ile Ile Ser
305                 310                 315                 320
Thr Phe Leu Leu Cys Trp Thr Pro Ile Ser Val Leu Asn Thr Thr Ile
                325                 330                 335
Leu Cys Leu Gly Pro Ser Asp Leu Leu Val Lys Leu Arg Leu Cys Phe
            340                 345                 350
Leu Val Met Ala Tyr Gly Thr Thr Ile Phe His Pro Leu Leu Tyr Ala
        355                 360                 365
Phe Thr Arg Gln Lys Phe Gln Lys Val Leu Lys Ser Lys Met Lys Lys
    370                 375                 380
Arg Val Val Ser Ile Val Glu Ala Asp Pro Leu Pro Asn Asn Ala Val
385                 390                 395                 400
Ile His Asn Ser Trp Ile Asp Pro Lys Arg Asn Lys Lys Ile Thr Phe
                405                 410                 415
Glu Asp Ser Glu Ile Arg Glu Lys Cys Leu Val Pro Gln Val Val Thr
            420                 425                 430
Asp Ala Ser Lys Gly Glu Glu Leu Phe Thr Gly Val Val Pro Ile Leu
        435                 440                 445
Val Glu Leu Asp Gly Asp Val Asn Gly His Lys Phe Ser Val Ser Gly
    450                 455                 460
Glu Gly Glu Gly Asp Ala Thr Tyr Gly Lys Leu Thr Leu Lys Phe Ile
465                 470                 475                 480
Cys Thr Thr Gly Lys Leu Pro Val Pro Trp Pro Thr Leu Val Thr Thr
                485                 490                 495
Leu Cys Tyr Gly Val Gln Cys Phe Ser Arg Tyr Pro Asp His Met Lys
            500                 505                 510
Arg His Asp Phe Phe Lys Ser Ala Met Pro Glu Gly Tyr Val Gln Glu
        515                 520                 525
Arg Thr Ile Phe Phe Lys Asp Asp Gly Asn Tyr Lys Thr Arg Ala Glu
    530                 535                 540
Val Lys Phe Glu Gly Asp Thr Leu Val Asn Arg Ile Glu Leu Lys Gly
545                 550                 555                 560
Ile Asp Phe Lys Glu Asp Gly Asn Ile Leu Gly His Lys Leu Glu Tyr
                565                 570                 575
Asn Tyr Asn Ser His Asn Val Tyr Ile Met Ala Asp Lys Gln Lys Asn
            580                 585                 590
Gly Ile Lys Val Asn Phe Lys Thr Arg His Asn Ile Glu Asp Gly Ser
        595                 600                 605
Val Gln Leu Ala Asp His Tyr Gln Gln Asn Thr Pro Ile Gly Asp Gly
    610                 615                 620
Pro Val Leu Leu Pro Asp Asn His Tyr Leu Ser Thr Gln Ser Ala Leu
625                 630                 635                 640
Ser Lys Asp Pro Asn Glu Lys Arg Asp His Met Val Leu Leu Glu Phe
                645                 650                 655
Val Thr Ala Ala Gly Ile Thr His Gly Met Asp Glu Leu Tyr Asn
            660                 665                 670

<210> 69
<211> 599
<212> PRT
<213> Human

<400> 69

<210> 70
<211> 575
<212> PRT
<213> Human

<400> 70

<210> 71
<211> 727
<212> PRT
<213> Human

<400> 71

<210> 72
<211> 616
<212> PRT
<213> Human

<400> 72

<210> 73
<211> 608
<212> PRT
<213> Human

<400> 73
Met Ala Asn Tyr Ser His Ala Ala Asp Asn Ile Leu Gln Asn Leu Ser
                5                   10                  15
Pro Leu Thr Ala Phe Leu Lys Leu Thr Ser Leu Gly Phe Ile Ile Gly
            20                  25                  30
Val Ser Val Val Gly Asn Leu Leu Ile Ser Ile Leu Leu Val Lys Asp
        35                  40                  45
Lys Thr Leu His Arg Ala Pro Tyr Tyr Phe Leu Leu Asp Leu Cys Cys
    50                  55                  60
Ser Asp Ile Leu Arg Ser Ala Ile Cys Phe Pro Phe Val Phe Asn Ser
65                  70                  75                  80
Val Lys Asn Gly Ser Thr Trp Thr Tyr Gly Thr Leu Thr Cys Lys Val
                85                  90                  95
Ile Ala Phe Leu Gly Val Leu Ser Cys Phe His Thr Ala Phe Met Leu
            100                 105                 110
Phe Cys Ile Ser Val Thr Arg Tyr Leu Ala Ile Ala His His Arg Phe
        115                 120                 125
Tyr Thr Lys Arg Leu Thr Phe Trp Thr Cys Leu Ala Val Ile Cys Met
    130                 135                 140
Val Trp Thr Leu Ser Val Ala Met Ala Phe Pro Pro Val Leu Asp Val
145                 150                 155                 160
Gly Thr Tyr Ser Phe Ile Arg Glu Glu Asp Gln Cys Thr Phe Gln His
                165                 170                 175
Arg Ser Phe Arg Ala Asn Asp Ser Leu Gly Phe Met Leu Leu Leu Ala

            180                 185                 190
Leu Ile Leu Leu Ala Thr Gln Leu Val Tyr Leu Lys Leu Ile Phe Phe
        195                 200                 205
Val His Asp Arg Arg Lys Met Lys Pro Val Gln Phe Val Ala Ala Val
    210                 215                 220
Ser Gln Asn Trp Thr Phe His Gly Pro Gly Ala Ser Gly Gln Ala Ala
225                 230                 235                 240
Ala Asn Trp Leu Ala Gly Phe Gly Arg Gly Pro Thr Pro Pro Thr Leu
                245                 250                 255
Leu Gly Ile Arg Gln Asn Ala Asn Thr Thr Gly Arg Arg Arg Leu Leu
            260                 265                 270
Val Leu Asp Glu Phe Lys Met Glu Lys Arg Ile Ser Arg Met Phe Tyr
        275                 280                 285
Ile Met Thr Phe Leu Phe Leu Thr Leu Trp Gly Pro Tyr Leu Val Ala
    290                 295                 300
Cys Tyr Trp Arg Val Phe Ala Arg Gly Pro Val Val Pro Gly Gly Phe
305                 310                 315                 320
Leu Thr Ala Ala Val Trp Met Ser Phe Ala Gln Ala Gly Ile Asn Pro
                325                 330                 335
Phe Val Cys Ile Phe Ser Asn Arg Glu Leu Arg Arg Cys Phe Ser Thr
            340                 345                 350
Thr Leu Leu Tyr Cys Arg Lys Ser Arg Leu Pro Arg Glu Pro Tyr Cys
        355                 360                 365
Val Ile Ser Arg Lys Gly Glu Glu Leu Phe Thr Gly Val Val Pro Ile
    370                 375                 380
Leu Val Glu Leu Asp Gly Asp Val Asn Gly His Lys Phe Ser Val Ser
385                 390                 395                 400
Gly Glu Gly Glu Gly Asp Ala Thr Tyr Gly Lys Leu Thr Leu Lys Phe
                405                 410                 415
Ile Cys Thr Thr Gly Lys Leu Pro Val Pro Trp Pro Thr Leu Val Thr
            420                 425                 430
Thr Leu Cys Tyr Gly Val Gln Cys Phe Ser Arg Tyr Pro Asp His Met
        435                 440                 445
Lys Arg His Asp Phe Phe Lys Ser Ala Met Pro Glu Gly Tyr Val Gln
    450                 455                 460
Glu Arg Thr Ile Phe Phe Lys Asp Asp Gly Asn Tyr Lys Thr Arg Ala
465                 470                 475                 480
Glu Val Lys Phe Glu Gly Asp Thr Leu Val Asn Arg Ile Glu Leu Lys
                485                 490                 495
Gly Ile Asp Phe Lys Glu Asp Gly Asn Ile Leu Gly His Lys Leu Glu
            500                 505                 510
Tyr Asn Tyr Asn Ser His Asn Val Tyr Ile Met Ala Asp Lys Gln Lys
        515                 520                 525
Asn Gly Ile Lys Val Asn Phe Lys Thr Arg His Asn Ile Glu Asp Gly
    530                 535                 540
Ser Val Gln Leu Ala Asp His Tyr Gln Gln Asn Thr Pro Ile Gly Asp
545                 550                 555                 560
Gly Pro Val Leu Leu Pro Asp Asn His Tyr Leu Ser Thr Gln Ser Ala
                565                 570                 575
Leu Ser Lys Asp Pro Asn Glu Lys Arg Asp His Met Val Leu Leu Glu
            580                 585                 590
Phe Val Thr Ala Ala Gly Ile Thr His Gly Met Asp Glu Leu Tyr Asn
        595                 600                 605

<210> 74
<211> 572
<212> PRT
<213> Human

<400> 74

<210> 75
<211> 613
<212> PRT
<213> Human

<400> 75

<210> 76
<211> 574
<212> PRT
<213> Human

<400> 76

<210> 77
<211> 1061
<212> PRT

<213> Human

<400> 77

<210> 78
<211> 753
<212> PRT
<213> Human

<400> 78

<210> 79
<211> 600
<212> PRT
<213> Human

<400> 79

<210> 80
<211> 672
<212> PRT
<213> Human

<400> 80

<210> 81
<211> 542
<212> PRT
<213> Human

<400> 81

<210> 82
<211> 571
<212> PRT
<213> Human

<400> 82

<210> 83
<211> 577
<212> PRT
<213> Human

<400> 83

<210> 84
<211> 547
<212> PRT
<213> Human

<400> 84

<210> 85
<211> 679
<212> PRT
<213> Human

<400> 85

<210> 86
<211> 625
<212> PRT
<213> Human

<400> 86

<210> 87
<211> 778
<212> PRT
<213> Human

<400> 87

<210> 88
<211> 584
<212> PRT
<213> Human

<400> 88

<210> 89
<211> 585
<212> PRT
<213> Human

<400> 89

<210> 90
<211> 611
<212> PRT
<213> Human

<400> 90

<210> 91
<211> 591
<212> PRT
<213> Rat

<400> 91
Met Glu Val Ser Arg Glu Met Leu Phe Glu Glu Leu Asp Asn Tyr Ser
                5                   10                  15
Tyr Ala Leu Glu Tyr Tyr Ser Gln Glu Pro Asp Ala Glu Glu Asn Val
            20                  25                  30
Tyr Pro Gly Ile Val His Trp Ile Ser Leu Leu Leu Tyr Ala Leu Ala
        35                  40                  45
Phe Val Leu Gly Ile Pro Gly Asn Ala Ile Val Ile Trp Phe Met Gly
    50                  55                  60
Phe Lys Trp Lys Lys Thr Val Thr Thr Leu Trp Phe Leu Asn Leu Ala
65                  70                  75                  80
Ile Ala Asp Phe Val Phe Val Leu Phe Leu Pro Leu Tyr Ile Ser Tyr
                85                  90                  95

Val Ala Leu Ser Phe His Trp Pro Phe Gly Arg Trp Leu Cys Lys Leu
            100                 105                 110
Asn Ser Phe Ile Ala Gln Leu Asn Met Phe Ser Ser Val Phe Phe Leu
        115                 120                 125
Thr Val Ile Ser Leu Asp Arg Tyr Ile His Leu Ile His Pro Gly Leu
    130                 135                 140
Ser His Pro His Arg Thr Leu Lys Asn Ser Leu Leu Val Val Leu Phe
145                 150                 155                 160
Val Trp Leu Leu Ala Ser Leu Leu Gly Gly Pro Thr Leu Tyr Phe Arg
                165                 170                 175
Asp Thr Val Glu Val Asn Asn Arg Ile Ile Cys Tyr Asn Asn Phe Gln
            180                 185                 190
Glu Tyr Glu Leu Thr Leu Met Arg His His Val Leu Thr Trp Val Lys
        195                 200                 205
Phe Leu Phe Gly Tyr Leu Leu Pro Leu Leu Thr Met Ser Ser Cys Tyr
    210                 215                 220
Leu Cys Leu Ile Phe Lys Thr Lys Lys Gln Asn Ile Leu Ile Ser Ser
225                 230                 235                 240
Lys His Leu Trp Met Ile Leu Ser Val Val Ile Ala Phe Met Val Cys
                245                 250                 255
Trp Thr Pro Phe His Leu Phe Ser Ile Trp Glu Leu Ser Ile His His
            260                 265                 270
Asn Ser Ser Phe Gln Asn Val Leu Gln Gly Gly Ile Pro Leu Ser Thr
        275                 280                 285
Gly Leu Ala Phe Leu Asn Ser Cys Leu Asn Pro Ile Leu Tyr Val Leu
    290                 295                 300
Ile Ser Lys Lys Phe Gln Ala Arg Phe Arg Ala Ser Val Ala Glu Val
305                 310                 315                 320
Leu Lys Arg Ser Leu Trp Glu Ala Ser Cys Ser Gly Thr Val Ser Glu
                325                 330                 335
Gln Leu Arg Ser Ala Glu Thr Lys Ser Leu Ser Leu Leu Glu Thr Ala
            340                 345                 350
Gln Ala Ser Lys Gly Glu Glu Leu Phe Thr Gly Val Val Pro Ile Leu
        355                 360                 365
Val Glu Leu Asp Gly Asp Val Asn Gly His Lys Phe Ser Val Ser Gly
    370                 375                 380
Glu Gly Glu Gly Asp Ala Thr Tyr Gly Lys Leu Thr Leu Lys Phe Ile
385                 390                 395                 400
Cys Thr Thr Gly Lys Leu Pro Val Pro Trp Pro Thr Leu Val Thr Thr
                405                 410                 415
Leu Cys Tyr Gly Val Gln Cys Phe Ser Arg Tyr Pro Asp His Met Lys
            420                 425                 430
Arg His Asp Phe Phe Lys Ser Ala Met Pro Glu Gly Tyr Val Gln Glu
        435                 440                 445
Arg Thr Ile Phe Phe Lys Asp Asp Gly Asn Tyr Lys Thr Arg Ala Glu
    450                 455                 460
Val Lys Phe Glu Gly Asp Thr Leu Val Asn Arg Ile Glu Leu Lys Gly
465                 470                 475                 480
Ile Asp Phe Lys Glu Asp Gly Asn Ile Leu Gly His Lys Leu Glu Tyr
                485                 490                 495
Asn Tyr Asn Ser His Asn Val Tyr Ile Met Ala Asp Lys Gln Lys Asn
            500                 505                 510
Gly Ile Lys Val Asn Phe Lys Thr Arg His Asn Ile Glu Asp Gly Ser
        515                 520                 525
Val Gln Leu Ala Asp His Tyr Gln Gln Asn Thr Pro Ile Gly Asp Gly
    530                 535                 540
Pro Val Leu Leu Pro Asp Asn His Tyr Leu Ser Thr Gln Ser Ala Leu
545                 550                 555                 560
Ser Lys Asp Pro Asn Glu Lys Arg Asp His Met Val Leu Leu Glu Phe
                565                 570                 575
Val Thr Ala Ala Gly Ile Thr His Gly Met Asp Glu Leu Tyr Asn
            580                 585                 590

<210> 92
<211> 568
<212> PRT
<213> Human

<400> 92
Met Met Trp Gly Ala Gly Ser Pro Leu Ala Trp Leu Ser Ala Gly Ser
                5                   10                  15
Gly Asn Val Asn Val Ser Ser Val Gly Pro Ala Glu Gly Pro Thr Gly
            20                  25                  30
Pro Ala Ala Pro Leu Pro Ser Pro Lys Ala Trp Asp Val Val Leu Cys
        35                  40                  45

Ile Ser Gly Thr Leu Val Ser Cys Glu Asn Ala Leu Val Val Ala Ile
    50                  55                  60
Ile Val Gly Thr Pro Ala Phe Arg Ala Pro Met Phe Leu Leu Val Gly
65                  70                  75                  80
Ser Leu Ala Val Ala Asp Leu Leu Ala Gly Leu Gly Leu Val Leu His
                85                  90                  95
Phe Ala Ala Val Phe Cys Ile Gly Ser Ala Glu Met Ser Leu Val Leu
            100                 105                 110
Val Gly Val Leu Ala Met Ala Phe Thr Ala Ser Ile Gly Ser Leu Leu
        115                 120                 125
Ala Ile Thr Val Asp Arg Tyr Leu Ser Leu Tyr Asn Ala Leu Thr Tyr
    130                 135                 140
Tyr Ser Glu Thr Thr Val Thr Arg Thr Tyr Val Met Leu Ala Leu Val
145                 150                 155                 160
Trp Gly Gly Ala Leu Gly Leu Gly Leu Leu Pro Val Leu Ala Trp Asn
                165                 170                 175
Cys Leu Asp Gly Leu Thr Thr Cys Gly Val Val Tyr Pro Leu Ser Lys
            180                 185                 190
Asn His Leu Val Val Leu Ala Ile Ala Phe Phe Met Val Phe Gly Ile
        195                 200                 205
Met Leu Gln Leu Tyr Ala Gln Ile Cys Arg Ile Val Cys Arg His Ala
    210                 215                 220
Gln Gln Ile Ala Leu Gln Arg His Leu Leu Pro Ala Ser His Tyr Val
225                 230                 235                 240
Ala Thr Arg Lys Gly Ile Ala Thr Leu Ala Val Val Leu Gly Ala Phe
                245                 250                 255
Ala Ala Cys Trp Leu Pro Phe Thr Val Tyr Cys Leu Leu Gly Asp Ala
            260                 265                 270
His Ser Pro Pro Leu Tyr Thr Tyr Leu Thr Leu Leu Pro Ala Thr Tyr
        275                 280                 285
Asn Ser Met Ile Asn Pro Ile Ile Tyr Ala Phe Arg Asn Gln Asp Val
    290                 295                 300
Gln Lys Val Leu Trp Ala Val Cys Cys Cys Cys Ser Ser Ser Lys Ile
305                 310                 315                 320
Pro Phe Arg Ser Arg Ser Pro Ser Asp Val Ala Ser Lys Gly Glu Glu
                325                 330                 335
Leu Phe Thr Gly Val Val Pro Ile Leu Val Glu Leu Asp Gly Asp Val
            340                 345                 350
Asn Gly His Lys Phe Ser Val Ser Gly Glu Gly Glu Gly Asp Ala Thr
        355                 360                 365
Tyr Gly Lys Leu Thr Leu Lys Phe Ile Cys Thr Thr Gly Lys Leu Pro
    370                 375                 380
Val Pro Trp Pro Thr Leu Val Thr Thr Leu Cys Tyr Gly Val Gln Cys
385                 390                 395                 400
Phe Ser Arg Tyr Pro Asp His Met Lys Arg His Asp Phe Phe Lys Ser
                405                 410                 415
Ala Met Pro Glu Gly Tyr Val Gln Glu Arg Thr Ile Phe Phe Lys Asp
            420                 425                 430
Asp Gly Asn Tyr Lys Thr Arg Ala Glu Val Lys Phe Glu Gly Asp Thr
        435                 440                 445
Leu Val Asn Arg Ile Glu Leu Lys Gly Ile Asp Phe Lys Glu Asp Gly
    450                 455                 460
Asn Ile Leu Gly His Lys Leu Glu Tyr Asn Tyr Asn Ser His Asn Val
465                 470                 475                 480
Tyr Ile Met Ala Asp Lys Gln Lys Asn Gly Ile Lys Val Asn Phe Lys
                485                 490                 495
Thr Arg His Asn Ile Glu Asp Gly Ser Val Gln Leu Ala Asp His Tyr
            500                 505                 510
Gln Gln Asn Thr Pro Ile Gly Asp Gly Pro Val Leu Leu Pro Asp Asn
        515                 520                 525
His Tyr Leu Ser Thr Gln Ser Ala Leu Ser Lys Asp Pro Asn Glu Lys
    530                 535                 540
Arg Asp His Met Val Leu Leu Glu Phe Val Thr Ala Ala Gly Ile Thr
545                 550                 555                 560
His Gly Met Asp Glu Leu Tyr Asn
                565

<210> 93
<211> 600
<212> PRT
<213> Human

<400> 93

<210> 94
<211> 595
<212> PRT
<213> Human

<400> 94

<210> 95
<211> 657
<212> PRT
<213> Human

<400> 95
Met Leu Ala Ala Ala Phe Ala Asp Ser Asn Ser Ser Ser Met Asn Val
                5                   10                  15
Ser Phe Ala His Leu His Phe Ala Gly Gly Tyr Leu Pro Ser Asp Ser
            20                  25                  30
Gln Asp Trp Arg Thr Ile Ile Pro Ala Leu Leu Val Ala Val Cys Leu
        35                  40                  45
Val Gly Phe Val Gly Asn Leu Cys Val Ile Gly Ile Leu Leu His Asn
    50                  55                  60
Ala Trp Lys Gly Lys Pro Ser Met Ile His Ser Leu Ile Leu Asn Leu
65                  70                  75                  80
Ser Leu Ala Asp Leu Ser Leu Leu Leu Phe Ser Ala Pro Ile Arg Ala
                85                  90                  95
Thr Ala Tyr Ser Lys Ser Val Trp Asp Leu Gly Trp Phe Val Cys Lys
            100                 105                 110
Ser Ser Asp Trp Phe Ile His Thr Cys Met Ala Ala Lys Ser Leu Thr
        115                 120                 125
Ile Val Val Val Ala Lys Val Cys Phe Met Tyr Ala Ser Asp Pro Ala
    130                 135                 140
Lys Gln Val Ser Ile His Asn Tyr Thr Ile Trp Ser Val Leu Val Ala
145                 150                 155                 160
Ile Trp Thr Val Ala Ser Leu Leu Pro Leu Pro Glu Trp Phe Phe Ser
                165                 170                 175
Thr Ile Arg His His Glu Gly Val Glu Met Cys Leu Val Asp Val Pro
            180                 185                 190
Ala Val Ala Glu Glu Phe Met Ser Met Phe Gly Lys Leu Tyr Pro Leu
        195                 200                 205
Leu Ala Phe Gly Leu Pro Leu Phe Phe Ala Ser Phe Tyr Phe Trp Arg
    210                 215                 220
Ala Tyr Asp Gln Cys Lys Lys Arg Gly Thr Lys Thr Gln Asn Leu Arg
225                 230                 235                 240
Asn Gln Ile Arg Ser Lys Gln Val Thr Val Met Leu Leu Ser Ile Ala
                245                 250                 255
Ile Ile Ser Ala Leu Leu Trp Leu Pro Glu Trp Val Ala Trp Leu Trp
            260                 265                 270
Val Trp His Leu Lys Ala Ala Gly Pro Ala Pro Pro Gln Gly Phe Ile
        275                 280                 285
Ala Leu Ser Gln Val Leu Met Phe Ser Ile Ser Ser Ala Asn Pro Leu
    290                 295                 300
Ile Phe Leu Val Met Ser Glu Glu Phe Arg Glu Gly Leu Lys Gly Val
305                 310                 315                 320
Trp Lys Trp Met Ile Thr Lys Lys Pro Pro Thr Val Ser Glu Ser Gln
                325                 330                 335
Glu Thr Pro Ala Gly Asn Ser Glu Gly Leu Pro Asp Lys Val Pro Ser
            340                 345                 350
Pro Glu Ser Pro Ala Ser Ile Pro Glu Lys Glu Lys Pro Ser Ser Pro
        355                 360                 365
Ser Ser Gly Lys Gly Lys Thr Glu Lys Ala Glu Ile Pro Ile Leu Pro
    370                 375                 380
Asp Val Glu Gln Phe Trp His Glu Arg Asp Thr Val Pro Ser Val Gln
385                 390                 395                 400
Asp Asn Asp Pro Ile Pro Trp Glu His Glu Asp Gln Glu Thr Gly Glu
                405                 410                 415
Gly Val Lys Thr Ser Lys Gly Glu Glu Leu Phe Thr Gly Val Val Pro
            420                 425                 430
Ile Leu Val Glu Leu Asp Gly Asp Val Asn Gly His Lys Phe Ser Val
        435                 440                 445
Ser Gly Glu Gly Glu Gly Asp Ala Thr Tyr Gly Lys Leu Thr Leu Lys
    450                 455                 460
Phe Ile Cys Thr Thr Gly Lys Leu Pro Val Pro Trp Pro Thr Leu Val
465                 470                 475                 480
Thr Thr Leu Cys Tyr Gly Val Gln Cys Phe Ser Arg Tyr Pro Asp His

                485                 490                 495
Met Lys Arg His Asp Phe Phe Lys Ser Ala Met Pro Glu Gly Tyr Val
            500                 505                 510
Gln Glu Arg Thr Ile Phe Phe Lys Asp Asp Gly Asn Tyr Lys Thr Arg
        515                 520                 525
Ala Glu Val Lys Phe Glu Gly Asp Thr Leu Val Asn Arg Ile Glu Leu
    530                 535                 540
Lys Gly Ile Asp Phe Lys Glu Asp Gly Asn Ile Leu Gly His Lys Leu
545                 550                 555                 560
Glu Tyr Asn Tyr Asn Ser His Asn Val Tyr Ile Met Ala Asp Lys Gln
                565                 570                 575
Lys Asn Gly Ile Lys Val Asn Phe Lys Thr Arg His Asn Ile Glu Asp
            580                 585                 590
Gly Ser Val Gln Leu Ala Asp His Tyr Gln Gln Asn Thr Pro Ile Gly
        595                 600                 605
Asp Gly Pro Val Leu Leu Pro Asp Asn His Tyr Leu Ser Thr Gln Ser
    610                 615                 620
Ala Leu Ser Lys Asp Pro Asn Glu Lys Arg Asp His Met Val Leu Leu
625                 630                 635                 640
Glu Phe Val Thr Ala Ala Gly Ile Thr His Gly Met Asp Glu Leu Tyr
                645                 650                 655
Asn

<210> 96
<211> 657
<212> PRT
<213> Human

<400> 96

<210> 97
<211> 1232
<212> PRT
<213> Human

<400> 97

<210> 98
<211> 931
<212> PRT
<213> Human

<400> 98

<210> 99
<211> 1584
<212> PRT
<213> Human

<400> 99

<210> 100
<211> 645
<212> PRT
<213> Human

<400> 100

<210> 101
<211> 637
<212> PRT
<213> Human

<400> 101

<210> 102
<211> 613
<212> PRT
<213> Human

<400> 102
Met Ala Asn Ala Ser Glu Pro Gly Gly Ser Gly Gly Gly Glu Ala Ala
                5                   10                  15
Ala Leu Gly Leu Lys Leu Ala Thr Leu Ser Leu Leu Leu Cys Val Ser
            20                  25                  30
Leu Ala Gly Asn Val Leu Phe Ala Leu Leu Ile Val Arg Glu Arg Ser
        35                  40                  45
Leu His Arg Ala Pro Tyr Tyr Leu Leu Leu Asp Leu Cys Leu Ala Asp
    50                  55                  60
Gly Leu Arg Ala Leu Ala Cys Leu Pro Ala Val Met Leu Ala Ala Arg
65                  70                  75                  80
Arg Ala Ala Ala Ala Ala Gly Ala Pro Pro Gly Ala Leu Gly Cys Lys
                85                  90                  95
Leu Leu Ala Phe Leu Ala Ala Leu Phe Cys Phe His Ala Ala Phe Leu
            100                 105                 110
Leu Leu Gly Val Gly Val Thr Arg Tyr Leu Ala Ile Ala His His Arg
        115                 120                 125
Phe Tyr Ala Glu Arg Leu Ala Gly Trp Pro Cys Ala Ala Met Leu Val
    130                 135                 140
Cys Ala Ala Trp Ala Leu Ala Leu Ala Ala Ala Phe Pro Pro Val Leu
145                 150                 155                 160
Asp Gly Gly Gly Asp Asp Glu Asp Ala Pro Cys Ala Leu Glu Gln Arg
                165                 170                 175
Pro Asp Gly Ala Pro Gly Ala Leu Gly Phe Leu Leu Leu Leu Ala Val
            180                 185                 190
Val Val Gly Ala Thr His Leu Val Tyr Leu Arg Leu Leu Phe Phe Ile
        195                 200                 205
His Asp Arg Arg Lys Met Arg Pro Ala Arg Leu Val Pro Ala Val Ser
    210                 215                 220
His Asp Trp Thr Phe His Gly Pro Gly Ala Thr Gly Gln Ala Ala Ala
225                 230                 235                 240
Asn Trp Thr Ala Gly Phe Gly Arg Gly Pro Thr Pro Pro Ala Leu Val
                245                 250                 255
Gly Ile Arg Pro Ala Gly Pro Gly Arg Gly Ala Arg Arg Leu Leu Val
            260                 265                 270
Leu Glu Glu Phe Lys Thr Glu Lys Arg Leu Cys Lys Met Phe Tyr Ala
        275                 280                 285
Val Thr Leu Leu Phe Leu Leu Leu Trp Gly Pro Tyr Val Val Ala Ser
    290                 295                 300
Tyr Leu Arg Val Leu Val Arg Pro Gly Ala Val Pro Gln Ala Tyr Leu

305                 310                 315                 320
Thr Ala Ser Val Trp Leu Thr Phe Ala Gln Ala Gly Ile Asn Pro Val
                325                 330                 335
Val Cys Phe Leu Phe Asn Arg Glu Leu Arg Asp Cys Phe Arg Ala Gln
            340                 345                 350
Phe Pro Cys Cys Gln Ser Pro Arg Thr Thr Gln Ala Thr His Pro Cys
        355                 360                 365
Asp Leu Lys Gly Ile Gly Leu Ala Ser Lys Gly Glu Glu Leu Phe Thr
    370                 375                 380
Gly Val Val Pro Ile Leu Val Glu Leu Asp Gly Asp Val Asn Gly His
385                 390                 395                 400
Lys Phe Ser Val Ser Gly Glu Gly Glu Gly Asp Ala Thr Tyr Gly Lys
                405                 410                 415
Leu Thr Leu Lys Phe Ile Cys Thr Thr Gly Lys Leu Pro Val Pro Trp
            420                 425                 430
Pro Thr Leu Val Thr Thr Leu Cys Tyr Gly Val Gln Cys Phe Ser Arg
        435                 440                 445
Tyr Pro Asp His Met Lys Arg His Asp Phe Phe Lys Ser Ala Met Pro
    450                 455                 460
Glu Gly Tyr Val Gln Glu Arg Thr Ile Phe Phe Lys Asp Asp Gly Asn
465                 470                 475                 480
Tyr Lys Thr Arg Ala Glu Val Lys Phe Glu Gly Asp Thr Leu Val Asn
                485                 490                 495
Arg Ile Glu Leu Lys Gly Ile Asp Phe Lys Glu Asp Gly Asn Ile Leu
            500                 505                 510
Gly His Lys Leu Glu Tyr Asn Tyr Asn Ser His Asn Val Tyr Ile Met
        515                 520                 525
Ala Asp Lys Gln Lys Asn Gly Ile Lys Val Asn Phe Lys Thr Arg His
    530                 535                 540
Asn Ile Glu Asp Gly Ser Val Gln Leu Ala Asp His Tyr Gln Gln Asn
545                 550                 555                 560
Thr Pro Ile Gly Asp Gly Pro Val Leu Leu Pro Asp Asn His Tyr Leu
                565                 570                 575
Ser Thr Gln Ser Ala Leu Ser Lys Asp Pro Asn Glu Lys Arg Asp His
            580                 585                 590
Met Val Leu Leu Glu Phe Val Thr Ala Ala Gly Ile Thr His Gly Met
        595                 600                 605
Asp Glu Leu Tyr Asn
    610

<210> 103
<211> 1145
<212> PRT
<213> Human

<400> 103

<210> 104
<211> 560
<212> PRT
<213> Human

<400> 104

<210> 105
<211> 568
<212> PRT
<213> Human

<400> 105

<210> 106
<211> 550
<212> PRT
<213> Human

<400> 106

<210> 107
<211> 561
<212> PRT
<213> Human

<400> 107

<210> 108
<211> 591
<212> PRT
<213> Human

<400> 108
Met Glu His Thr His Ala His Leu Ala Ala Asn Ser Ser Leu Ser Trp
                5                   10                  15
Trp Ser Pro Gly Ser Ala Cys Gly Leu Gly Phe Val Pro Val Val Tyr
            20                  25                  30
Tyr Ser Leu Leu Leu Cys Leu Gly Leu Pro Ala Asn Ile Leu Thr Val
        35                  40                  45
Ile Ile Leu Ser Gln Leu Val Ala Arg Arg Gln Lys Ser Ser Tyr Asn
    50                  55                  60
Tyr Leu Leu Ala Leu Ala Ala Ala Asp Ile Leu Val Leu Phe Phe Ile
65                  70                  75                  80
Val Phe Val Asp Phe Leu Leu Glu Asp Phe Ile Leu Asn Met Gln Met
                85                  90                  95
Pro Gln Val Pro Asp Lys Ile Ile Glu Val Leu Glu Phe Ser Ser Ile
            100                 105                 110
His Thr Ser Ile Trp Ile Thr Val Pro Leu Thr Ile Asp Arg Tyr Ile
        115                 120                 125
Ala Val Cys His Pro Leu Lys Tyr His Thr Val Ser Tyr Pro Ala Arg

130                 135                 140
Thr Arg Lys Val Ile Val Ser Val Tyr Ile Thr Cys Phe Leu Thr Ser
145                 150                 155                 160
Ile Pro Tyr Tyr Trp Trp Pro Asn Ile Trp Thr Glu Asp Tyr Ile Ser
                165                 170                 175
Thr Ser Val His His Val Leu Ile Trp Ile His Cys Phe Thr Val Tyr
            180                 185                 190
Leu Val Pro Cys Ser Ile Phe Phe Ile Leu Asn Ser Ile Ile Val Tyr
        195                 200                 205
Lys Leu Arg Arg Lys Ser Asn Phe Arg Leu Arg Gly Tyr Ser Thr Gly
    210                 215                 220
Lys Thr Thr Ala Ile Leu Phe Thr Ile Thr Ser Ile Phe Ala Thr Leu
225                 230                 235                 240
Trp Ala Pro Arg Ile Ile Met Ile Leu Tyr His Leu Tyr Gly Ala Pro
                245                 250                 255
Ile Gln Asn Arg Trp Leu Val His Ile Met Ser Asp Ile Ala Asn Met
            260                 265                 270
Leu Ala Leu Leu Asn Thr Ala Ile Asn Phe Phe Leu Tyr Cys Phe Ile
        275                 280                 285
Ser Lys Arg Phe Arg Thr Met Ala Ala Ala Thr Leu Lys Ala Phe Phe
    290                 295                 300
Lys Cys Gln Lys Gln Pro Val Gln Phe Tyr Thr Asn His Asn Phe Ser
305                 310                 315                 320
Ile Thr Ser Ser Pro Trp Ile Ser Pro Ala Asn Ser His Cys Ile Lys
                325                 330                 335
Met Leu Val Tyr Gln Tyr Asp Lys Asn Gly Lys Pro Ile Lys Val Ser
            340                 345                 350
Pro Ser Ser Lys Gly Glu Glu Leu Phe Thr Gly Val Val Pro Ile Leu
        355                 360                 365
Val Glu Leu Asp Gly Asp Val Asn Gly His Lys Phe Ser Val Ser Gly
    370                 375                 380
Glu Gly Glu Gly Asp Ala Thr Tyr Gly Lys Leu Thr Leu Lys Phe Ile
385                 390                 395                 400
Cys Thr Thr Gly Lys Leu Pro Val Pro Trp Pro Thr Leu Val Thr Thr
                405                 410                 415
Leu Cys Tyr Gly Val Gln Cys Phe Ser Arg Tyr Pro Asp His Met Lys
            420                 425                 430
Arg His Asp Phe Phe Lys Ser Ala Met Pro Glu Gly Tyr Val Gln Glu
        435                 440                 445
Arg Thr Ile Phe Phe Lys Asp Asp Gly Asn Tyr Lys Thr Arg Ala Glu
    450                 455                 460
Val Lys Phe Glu Gly Asp Thr Leu Val Asn Arg Ile Glu Leu Lys Gly
465                 470                 475                 480
Ile Asp Phe Lys Glu Asp Gly Asn Ile Leu Gly His Lys Leu Glu Tyr
                485                 490                 495
Asn Tyr Asn Ser His Asn Val Tyr Ile Met Ala Asp Lys Gln Lys Asn
            500                 505                 510
Gly Ile Lys Val Asn Phe Lys Thr Arg His Asn Ile Glu Asp Gly Ser
        515                 520                 525
Val Gln Leu Ala Asp His Tyr Gln Gln Asn Thr Pro Ile Gly Asp Gly
    530                 535                 540
Pro Val Leu Leu Pro Asp Asn His Tyr Leu Ser Thr Gln Ser Ala Leu
545                 550                 555                 560
Ser Lys Asp Pro Asn Glu Lys Arg Asp His Met Val Leu Leu Glu Phe
                565                 570                 575
Val Thr Ala Ala Gly Ile Thr His Gly Met Asp Glu Leu Tyr Asn
            580                 585                 590

<210> 109
<211> 596
<212> PRT
<213> Human

<400> 109

<210> 110
<211> 543
<212> PRT
<213> Human

<400> 110

<210> 111
<211> 577
<212> PRT
<213> Human

<400> 111

<210> 112
<211> 569
<212> PRT
<213> Human

<400> 112

<210> 113
<211> 787
<212> PRT
<213> Human

<400> 113

<210> 114
<211> 608
<212> PRT
<213> Human

<400> 114

<210> 115
<211> 577
<212> PRT
<213> Human

<400> 115

<210> 116
<211> 851
<212> PRT
<213> Human

<400> 116

<210> 117
<211> 719
<212> PRT
<213> Human

<400> 117

<210> 118
<211> 591
<212> PRT
<213> Human

<400> 118

<210> 119
<211> 594
<212> PRT
<213> Human

<400> 119

<210> 120
<211> 1459
<212> PRT
<213> Human

<400> 120

<210> 121
<211> 637
<212> PRT
<213> Human

<400> 121

<210> 122
<211> 691
<212> PRT
<213> Human

<400> 122

<210> 123
<211> 661
<212> PRT
<213> Human

<400> 123

<210> 124
<211> 634
<212> PRT
<213> Human

<400> 124

<210> 125
<211> 587
<212> PRT
<213> Human

<400> 125
Met Asn Ser Thr Leu Asp Gly Asn Gln Ser Ser His Pro Phe Cys Leu
                5                   10                  15
Leu Ala Phe Gly Tyr Leu Glu Thr Val Asn Phe Cys Leu Leu Glu Val
            20                  25                  30
Leu Ile Ile Val Phe Leu Thr Val Leu Ile Ile Ser Gly Asn Ile Ile
        35                  40                  45
Val Ile Phe Val Phe His Cys Ala Pro Leu Leu Asn His His Thr Thr
    50                  55                  60
Ser Tyr Phe Ile Gln Thr Met Ala Tyr Ala Asp Leu Phe Val Gly Val
65                  70                  75                  80
Ser Cys Val Val Pro Ser Leu Ser Leu Leu His His Pro Leu Pro Val
                85                  90                  95
Glu Glu Ser Leu Thr Cys Gln Ile Phe Gly Phe Val Val Ser Val Leu
            100                 105                 110
Lys Ser Val Ser Met Ala Ser Leu Ala Cys Ile Ser Ile Asp Arg Tyr
        115                 120                 125
Ile Ala Ile Thr Lys Pro Leu Thr Tyr Asn Thr Leu Val Thr Pro Trp
    130                 135                 140
Arg Leu Arg Leu Cys Ile Phe Leu Ile Trp Leu Tyr Ser Thr Leu Val
145                 150                 155                 160
Phe Leu Pro Ser Phe Phe His Trp Gly Lys Pro Gly Tyr His Gly Asp
                165                 170                 175
Val Phe Gln Trp Cys Ala Glu Ser Trp His Thr Asp Ser Tyr Phe Thr
            180                 185                 190
Leu Phe Ile Val Met Met Leu Tyr Ala Pro Ala Ala Leu Ile Val Cys
        195                 200                 205
Phe Thr Tyr Phe Asn Ile Phe Arg Ile Cys Gln Gln His Thr Lys Asp
    210                 215                 220
Ile Ser Glu Arg Gln Ala Arg Phe Ser Ser Gln Ser Gly Glu Thr Gly
225                 230                 235                 240
Glu Val Gln Ala Cys Pro Asp Lys Arg Tyr Ala Met Val Leu Phe Arg
                245                 250                 255
Ile Thr Ser Val Phe Tyr Ile Leu Trp Leu Pro Tyr Ile Ile Tyr Phe
            260                 265                 270
Leu Leu Glu Ser Ser Thr Gly His Ser Asn Arg Phe Ala Ser Phe Leu
        275                 280                 285
Thr Thr Trp Leu Ala Ile Ser Asn Ser Phe Cys Asn Cys Val Ile Tyr
    290                 295                 300
Ser Leu Ser Asn Ser Val Phe Gln Arg Gly Leu Lys Arg Leu Ser Gly
305                 310                 315                 320
Ala Met Cys Thr Ser Cys Ala Ser Gln Thr Thr Ala Asn Asp Pro Tyr
                325                 330                 335
Thr Val Arg Ser Lys Gly Pro Leu Asn Gly Cys His Ile Ala Ser Lys
            340                 345                 350
Gly Glu Glu Leu Phe Thr Gly Val Val Pro Ile Leu Val Glu Leu Asp
        355                 360                 365
Gly Asp Val Asn Gly His Lys Phe Ser Val Ser Gly Glu Gly Glu Gly
    370                 375                 380
Asp Ala Thr Tyr Gly Lys Leu Thr Leu Lys Phe Ile Cys Thr Thr Gly
385                 390                 395                 400
Lys Leu Pro Val Pro Trp Pro Thr Leu Val Thr Thr Leu Cys Tyr Gly
                405                 410                 415
Val Gln Cys Phe Ser Arg Tyr Pro Asp His Met Lys Arg His Asp Phe
            420                 425                 430
Phe Lys Ser Ala Met Pro Glu Gly Tyr Val Gln Glu Arg Thr Ile Phe
        435                 440                 445
Phe Lys Asp Asp Gly Asn Tyr Lys Thr Arg Ala Glu Val Lys Phe Glu
    450                 455                 460
Gly Asp Thr Leu Val Asn Arg Ile Glu Leu Lys Gly Ile Asp Phe Lys
465                 470                 475                 480
Glu Asp Gly Asn Ile Leu Gly His Lys Leu Glu Tyr Asn Tyr Asn Ser
                485                 490                 495
His Asn Val Tyr Ile Met Ala Asp Lys Gln Lys Asn Gly Ile Lys Val
            500                 505                 510
Asn Phe Lys Thr Arg His Asn Ile Glu Asp Gly Ser Val Gln Leu Ala
        515                 520                 525
Asp His Tyr Gln Gln Asn Thr Pro Ile Gly Asp Gly Pro Val Leu Leu
    530                 535                 540
Pro Asp Asn His Tyr Leu Ser Thr Gln Ser Ala Leu Ser Lys Asp Pro
545                 550                 555                 560
Asn Glu Lys Arg Asp His Met Val Leu Leu Glu Phe Val Thr Ala Ala
                565                 570                 575
Gly Ile Thr His Gly Met Asp Glu Leu Tyr Asn
            580                 585

<210> 126
<211> 1189
<212> PRT
<213> Human

<400> 126

<210> 127
<211> 586
<212> PRT
<213> Human

<400> 127

<210> 128
<211> 1810
<212> PRT
<213> Human

<400> 128
Met Thr Pro Ala Cys Pro Leu Leu Leu Ser Val Ile Leu Ser Leu Arg
                5                    10                    15
Leu Ala Thr Ala Phe Asp Pro Ala Pro Ser Ala Cys Ser Ala Leu Ala
            20                    25                    30
Ser Gly Val Leu Tyr Gly Ala Phe Ser Leu Gln Asp Leu Phe Pro Thr
        35                    40                    45
Ile Ala Ser Gly Cys Ser Trp Thr Leu Glu Asn Pro Asp Pro Thr Lys
    50                    55                    60
Tyr Ser Leu Tyr Leu Arg Phe Asn Arg Gln Glu Gln Val Cys Ala His
65                    70                    75                    80
Phe Ala Pro Arg Leu Leu Pro Leu Asp His Tyr Leu Val Asn Phe Thr
                85                    90                    95
Cys Leu Arg Pro Ser Pro Glu Glu Ala Val Ala Gln Ala Glu Ser Glu
            100                    105                    110
Val Gly Arg Pro Glu Glu Glu Glu Ala Glu Ala Ala Ala Gly Leu Glu
        115                    120                    125
Leu Cys Ser Gly Ser Gly Pro Phe Thr Phe Leu His Phe Asp Lys Asn
    130                    135                    140
Phe Val Gln Leu Cys Leu Ser Ala Glu Pro Ser Glu Ala Pro Arg Leu
145                    150                    155                    160
Leu Ala Pro Ala Ala Leu Ala Phe Arg Phe Val Glu Val Leu Leu Ile
                165                    170                    175
Asn Asn Asn Asn Ser Ser Gln Phe Thr Cys Gly Val Leu Cys Arg Trp
            180                    185                    190
Ser Glu Glu Cys Gly Arg Ala Ala Gly Arg Ala Cys Gly Phe Ala Gln
        195                    200                    205
Pro Gly Cys Ser Cys Pro Gly Glu Ala Gly Ala Gly Ser Thr Thr Thr
    210                    215                    220
Thr Ser Pro Gly Pro Pro Ala Ala His Thr Leu Ser Asn Ala Leu Val
225                    230                    235                    240
Pro Gly Gly Pro Ala Pro Pro Ala Glu Ala Asp Leu His Ser Gly Ser
                245                    250                    255
Ser Asn Asp Leu Phe Thr Thr Glu Met Arg Tyr Gly Glu Glu Pro Glu
            260                    265                    270
Glu Glu Pro Lys Val Lys Thr Gln Trp Pro Arg Ser Ala Asp Glu Pro
        275                    280                    285
Gly Leu Tyr Met Ala Gln Thr Gly Asp Pro Ala Ala Glu Glu Trp Ser
    290                    295                    300
Pro Trp Ser Val Cys Ser Leu Thr Cys Gly Gln Gly Leu Gln Val Arg
305                    310                    315                    320
Thr Arg Ser Cys Val Ser Ser Pro Tyr Gly Thr Leu Cys Ser Gly Pro
                325                    330                    335
Leu Arg Glu Thr Arg Pro Cys Asn Asn Ser Ala Thr Cys Pro Val His
            340                    345                    350
Gly Val Trp Glu Glu Trp Gly Ser Trp Ser Leu Cys Ser Arg Ser Cys
        355                    360                    365
Gly Arg Gly Ser Arg Ser Arg Met Arg Thr Cys Val Pro Pro Gln His
    370                    375                    380
Gly Gly Lys Ala Cys Glu Gly Pro Glu Leu Gln Thr Lys Leu Cys Ser
385                    390                    395                    400
Met Ala Ala Cys Pro Val Glu Gly Gln Trp Leu Glu Trp Gly Pro Trp
                405                    410                    415
Gly Pro Cys Ser Thr Ser Cys Ala Asn Gly Thr Gln Gln Arg Ser Arg
            420                    425                    430
Lys Cys Ser Val Ala Gly Pro Ala Trp Ala Thr Cys Thr Gly Ala Leu

Thr Asp Thr Arg Glu Cys Ser Asn Leu Glu Cys Pro Ala Thr Asp Ser
        435                 440                 445
Lys Trp Gly Pro Trp Asn Ala Trp Ser Leu Cys Ser Lys Thr Cys Asp
    450                 455                 460
Thr Gly Trp Gln Arg Arg Phe Arg Met Cys Gln Ala Thr Gly Thr Gln
465                 470                 475                 480
Gly Tyr Pro Cys Glu Gly Thr Gly Glu Glu Val Lys Pro Cys Ser Glu
                485                 490                 495
Lys Arg Cys Pro Ala Phe His Glu Met Cys Arg Asp Glu Tyr Val Met
            500                 505                 510
Leu Met Thr Trp Lys Lys Ala Ala Ala Gly Glu Ile Ile Tyr Asn Lys
        515                 520                 525
Cys Pro Pro Asn Ala Ser Gly Ser Ala Ser Arg Arg Cys Leu Leu Ser
    530                 535                 540
Ala Gln Gly Val Ala Tyr Trp Gly Leu Pro Ser Phe Ala Arg Cys Ile
545                 550                 555                 560
Ser His Glu Tyr Arg Tyr Leu Tyr Leu Ser Leu Arg Glu His Leu Ala
                565                 570                 575
Lys Gly Gln Arg Met Leu Ala Gly Glu Gly Met Ser Gln Val Val Arg
            580                 585                 590
Ser Leu Gln Glu Leu Leu Ala Arg Arg Thr Tyr Tyr Ser Gly Asp Leu
        595                 600                 605
Leu Phe Ser Val Asp Ile Leu Arg Asn Val Thr Asp Thr Phe Lys Arg
    610                 615                 620
Ala Thr Tyr Val Pro Ser Ala Asp Asp Val Gln Arg Phe Phe Gln Val
625                 630                 635                 640
Val Ser Phe Met Val Asp Ala Glu Asn Lys Glu Lys Trp Asp Asp Ala
                645                 650                 655
Gln Gln Val Ser Pro Gly Ser Val His Leu Leu Arg Val Val Glu Asp
            660                 665                 670
Phe Ile His Leu Val Gly Asp Ala Leu Lys Ala Phe Gln Ser Ser Leu
        675                 680                 685
Ile Val Thr Asp Asn Leu Val Ile Ser Ile Gln Arg Glu Pro Val Ser
    690                 695                 700
Ala Val Ser Ser Asp Ile Thr Phe Pro Met Arg Gly Arg Arg Gly Met
705                 710                 715                 720
Lys Asp Trp Val Arg His Ser Glu Asp Arg Leu Phe Leu Pro Lys Glu
                725                 730                 735
Val Leu Ser Leu Ser Ser Pro Gly Lys Pro Ala Thr Ser Gly Ala Ala
            740                 745                 750
Gly Ser Pro Gly Arg Gly Arg Gly Pro Gly Thr Val Pro Pro Gly Pro
        755                 760                 765
Gly His Ser His Gln Arg Leu Leu Pro Ala Asp Pro Asp Glu Ser Ser
    770                 775                 780
Tyr Phe Val Ile Gly Ala Val Leu Tyr Arg Thr Leu Gly Leu Ile Leu
785                 790                 795                 800
Pro Pro Pro Arg Pro Pro Leu Ala Val Thr Ser Arg Val Met Thr Val
                805                 810                 815
Thr Val Arg Pro Pro Thr Gln Pro Pro Ala Glu Pro Leu Ile Thr Val
            820                 825                 830
Glu Leu Ser Tyr Ile Ile Asn Gly Thr Thr Asp Pro His Cys Ala Ser
        835                 840                 845
Trp Asp Tyr Ser Arg Ala Asp Ala Ser Ser Gly Asp Trp Asp Thr Glu
    850                 855                 860
Asn Cys Gln Thr Leu Glu Thr Gln Ala Ala His Thr Arg Cys Gln Cys
865                 870                 875                 880
Gln His Leu Ser Thr Phe Ala Val Leu Ala Gln Pro Pro Lys Asp Leu
                885                 890                 895
Thr Leu Glu Leu Ala Gly Ser Pro Ser Val Pro Leu Val Ile Gly Cys
            900                 905                 910
Ala Val Ser Cys Met Ala Leu Leu Thr Leu Leu Ala Ile Tyr Ala Ala
        915                 920                 925
Phe Trp Arg Phe Ile Lys Ser Glu Arg Ser Ile Ile Leu Leu Asn Phe
    930                 935                 940
Cys Leu Ser Ile Leu Ala Ser Asn Ile Leu Ile Leu Val Gly Gln Ser
945                 950                 955                 960
Arg Val Leu Ser Lys Gly Val Cys Thr Met Thr Ala Ala Phe Leu His
                965                 970                 975
Phe Phe Phe Leu Ser Ser Phe Cys Trp Val Leu Thr Glu Ala Trp Gln
            980                 985                 990
Ser Tyr Leu Ala Val Ile Gly Arg Met Arg Thr Arg Leu Val Arg Lys
        995                 1000                1005
Arg Phe Leu Cys Leu Gly Trp Gly Leu Pro Ala Leu Val Val Ala Val
    1010                1015                1020
1025                1030                1035                1040

Ser Val Gly Phe Thr Arg Thr Lys Gly Tyr Gly Thr Ser Ser Tyr Cys
                1045                1050                1055
Trp Leu Ser Leu Glu Gly Gly Leu Leu Tyr Ala Phe Val Gly Pro Ala
            1060                1065                1070
Ala Val Ile Val Leu Val Asn Met Leu Ile Gly Ile Ile Val Phe Asn
        1075                1080                1085
Lys Leu Met Ala Arg Asp Gly Ile Ser Asp Lys Ser Lys Lys Gln Arg
    1090                1095                1100
Ala Gly Ser Glu Arg Cys Pro Trp Ala Ser Leu Leu Leu Pro Cys Ser
1105                1110                1115                1120
Ala Cys Gly Ala Val Pro Ser Pro Leu Leu Ser Ser Ala Ser Ala Arg
                1125                1130                1135
Asn Ala Met Ala Ser Leu Trp Ser Ser Cys Val Val Leu Pro Leu Leu
            1140                1145                1150
Ala Leu Thr Trp Met Ser Ala Val Leu Ala Met Thr Asp Arg Arg Ser
        1155                1160                1165
Val Leu Phe Gln Ala Leu Phe Ala Val Phe Asn Ser Ala Gln Gly Phe
    1170                1175                1180
Val Ile Thr Ala Val His Cys Phe Leu Arg Arg Glu Val Gln Asp Val
1185                1190                1195                1200
Val Lys Cys Gln Met Gly Val Cys Arg Ala Asp Glu Ser Glu Asp Ser
                1205                1210                1215
Pro Asp Ser Cys Lys Asn Gly Gln Leu Gln Ile Leu Ser Asp Phe Glu
            1220                1225                1230
Lys Asp Val Asp Leu Ala Cys Gln Thr Val Leu Phe Lys Glu Val Asn
        1235                1240                1245
Thr Cys Asn Pro Ser Thr Ile Thr Gly Thr Leu Ser Arg Leu Ser Leu
    1250                1255                1260
Asp Glu Asp Glu Glu Pro Lys Ser Cys Leu Val Gly Pro Glu Gly Ser
1265                1270                1275                1280
Leu Ser Phe Ser Pro Leu Pro Gly Asn Ile Leu Val Pro Met Ala Ala
                1285                1290                1295
Ser Pro Gly Leu Gly Glu Pro Pro Pro Pro Gln Glu Ala Asn Pro Val
            1300                1305                1310
Tyr Met Cys Gly Glu Gly Gly Leu Arg Gln Leu Asp Leu Thr Trp Leu
        1315                1320                1325
Arg Pro Thr Glu Pro Gly Ser Glu Gly Asp Tyr Met Val Leu Pro Arg
    1330                1335                1340
Arg Thr Leu Ser Leu Gln Pro Gly Gly Gly Gly Gly Gly Gly Glu Asp
1345                1350                1355                1360
Ala Pro Arg Ala Arg Pro Glu Gly Thr Pro Arg Arg Ala Ala Lys Thr
                1365                1370                1375
Val Ala His Thr Glu Gly Tyr Pro Ser Phe Leu Ser Val Asp His Ser
            1380                1385                1390
Gly Leu Gly Leu Gly Pro Ala Tyr Gly Ser Leu Gln Asn Pro Tyr Gly
        1395                1400                1405
Met Thr Phe Gln Pro Pro Pro Pro Thr Pro Ser Ala Arg Gln Val Pro
    1410                1415                1420
Glu Pro Gly Glu Arg Ser Arg Thr Met Pro Arg Thr Val Pro Gly Ser
1425                1430                1435                1440
Thr Met Lys Met Gly Ser Leu Glu Arg Lys Lys Leu Arg Tyr Ser Asp
                1445                1450                1455
Leu Asp Phe Glu Val Met His Thr Arg Lys Arg His Ser Glu Leu Tyr
            1460                1465                1470
His Glu Leu Asn Gln Lys Phe His Thr Phe Asp Arg Tyr Arg Ser Gln
        1475                1480                1485
Ser Thr Ala Lys Arg Glu Lys Arg Trp Ser Val Ser Ser Gly Gly Ala
    1490                1495                1500
Ala Glu Arg Ser Val Cys Thr Asp Lys Pro Ser Pro Gly Glu Arg Pro
1505                1510                1515                1520
Ser Leu Ser Gln His Arg Arg His Gln Ser Trp Ser Thr Phe Lys Ser
                1525                1530                1535
Met Thr Leu Gly Ser Leu Pro Pro Lys Pro Arg Glu Arg Leu Thr Leu
            1540                1545                1550
His Arg Ala Ala Ala Trp Glu Pro Thr Glu Pro Pro Asp Gly Asp Phe
        1555                1560                1565
Gln Thr Glu Val Ala Ser Lys Gly Glu Glu Leu Phe Thr Gly Val Val
    1570                1575                1580
Pro Ile Leu Val Glu Leu Asp Gly Asp Val Asn Gly His Lys Phe Ser
1585                1590                1595                1600
Val Ser Gly Glu Gly Glu Gly Asp Ala Thr Tyr Gly Lys Leu Thr Leu
                1605                1610                1615
Lys Phe Ile Cys Thr Thr Gly Lys Leu Pro Val Pro Trp Pro Thr Leu
            1620                1625                1630
Val Thr Thr Leu Cys Tyr Gly Val Gln Cys Phe Ser Arg Tyr Pro Asp

1635                1640                1645
His Met Lys Arg His Asp Phe Phe Lys Ser Ala Met Pro Glu Gly Tyr
    1650                1655                1660
Val Gln Glu Arg Thr Ile Phe Phe Lys Asp Asp Gly Asn Tyr Lys Thr
1665                1670                1675                1680
Arg Ala Glu Val Lys Phe Glu Gly Asp Thr Leu Val Asn Arg Ile Glu
                1685                1690                1695
Leu Lys Gly Ile Asp Phe Lys Glu Asp Gly Asn Ile Leu Gly His Lys
            1700                1705                1710
Leu Glu Tyr Asn Tyr Asn Ser His Asn Val Tyr Ile Met Ala Asp Lys
        1715                1720                1725
Gln Lys Asn Gly Ile Lys Val Asn Phe Lys Thr Arg His Asn Ile Glu
    1730                1735                1740
Asp Gly Ser Val Gln Leu Ala Asp His Tyr Gln Gln Asn Thr Pro Ile
1745                1750                1755                1760
Gly Asp Gly Pro Val Leu Leu Pro Asp Asn His Tyr Leu Ser Thr Gln
                1765                1770                1775
Ser Ala Leu Ser Lys Asp Pro Asn Glu Lys Arg Asp His Met Val Leu
            1780                1785                1790
Leu Glu Phe Val Thr Ala Ala Gly Ile Thr His Gly Met Asp Glu Leu
        1795                1800                1805
Tyr Asn
    1810

<210> 129
<211> 563
<212> PRT
<213> Human

<400> 129

<210> 130
<211> 707
<212> PRT
<213> Human

<400> 130

<210> 131
<211> 746
<212> PRT
<213> Human

<400> 131

<210> 132
<211> 1635
<212> DNA
<213> Human

<400> 132
   atgtattcat ttatggcagg atccatattc atcacaatat ttggcaatct tgccatgata 60
   atttccattt cctacttcaa gcagcttcac acaccaacca acttcctcat cctctccatg 120
   gccatcactg atttcctcct gggattcacc atcatgccat atagtatgat cagatcggtg 180
   gagaactgct ggtattttgg gcttacattt tgcaagattt attatagttt tgacctgatg 240
   cttagcataa catccatttt tcatctttgc tcagtggcca ttgatagatt ttatgctata 300
   tgttacccat tactttattc caccaaaata actattccag tcattaaaag attgctactt 360
   ctatgttggt cggtccctgg agcatttgcc ttcggggtgg tcttctcaga ggcctatgca 420
   gatggaatag agggctatga catcttggtt gcttgttcca gttcctgccc agtgatgttc 480
   aacaagctat ggggggacac cttgtttatg gcaggtttct tcactcctgg gtctatgatg 540
   gtggggattt atggcaaaat ttttgcagta tccagaaaac atgctcatgc catcaataac 600
   ttgcgagaaa atcaaaataa tcaagtgaag aaagacaaaa aagctgccaa aactttagga 660
   atagtgatag gagttttctt attatgttgg tttccttgtt tcttcacaat tttattggat 720
   ccctttttga acttctctac tcctgtagtt ttgtttgatg ccttgacatg gtttggctat 780
   tttaactcca catgtaatcc gttaatatat ggtttcttct atccctggtt tcgcagagca 840
   ctgaagtaca ttttgctagg taaaattttc agctcatgtt tccataatac tattttgtgt 900
   atgcaaaaag aaagtgaggc tagcaaagga gaagaactct tcactggagt tgtcccaatt 960
   cttgttgaat tagatggtga tgttaacggc cacaagttct ctgtcagtgg agagggtgaa 1020
   ggtgatgcaa catacggaaa acttaccctg aagttcatct gcactactgg caaactgcct 1080
   gttccatggc caacactagt cactactctg tgctatggtg ttcaatgctt ttcaagatac 1140
   ccggatcata tgaaacggca tgactttttc aagagtgcca tgcccgaagg ttatgtacag 1200
   gaaaggacca tcttcttcaa agatgacggc aactacaaga cacgtgctga agtcaagttt 1260
   gaaggtgata cccttgttaa tagaatcgag ttaaaaggta ttgacttcaa ggaagatggc 1320
   aacattctgg gacacaaatt ggaatacaac tataactcac acaatgtata catcatggca 1380
   gacaaacaaa agaatggaat caaagtgaac ttcaagaccc gccacaacat tgaagatgga 1440
   agcgttcaac tagcagacca ttatcaacaa aatactccaa ttggcgatgg ccctgtcctt 1500
   ttaccagaca accattacct gtccacacaa tctgcccttt cgaaagatcc caacgaaaag 1560
   agagaccaca tggtccttct tgagtttgta acagctgctg ggattacaca tggcatggat 1620
   gaactgtaca actga 1635

<210> 133
<211> 1839
<212> DNA
<213> Human

<400> 133
atgcccacac tcaatacttc tgcctctcca cccacattct tctgggccaa tgcctccgga 60
ggcagtgtgc tgagtgctga tgatgctccg atgcctgtca aattcctagc cctgaggctc 120
atggttgccc tggcctatgg gcttgtgggg gccattggct tgctgggaaa tttggcggtg 180
ctgtgggtac tgagtaactg tgcccggaga gcccctggcc caccttcaga caccttcgtc 240
ttcaacctgg ctctggcgga cctgggactg gcactcactc tccccttttg ggcagccgag 300
tcggcactgg actttcactg gcccttcgga ggtgccctct gcaagatggt tctgacggcc 360
actgtcctca acgtctatgc cagcatcttc ctcatcacag cgctgagcgt tgctcgctac 420
tgggtggtgg ccatggctgc ggggccaggc acccacctct cactcttctg ggcccgaata 480
gccaccctgg cagtgtgggc ggcggctgcc ctggtgacgg tgcccacagc tgtcttcggg 540
gtggagggtg aggtgtgtgg tgtgcgcctt tgcctgctgc gtttccccag caggtactgg 600
ctgggggcct accagctgca gagggtggtg ctggctttca tggtgccctt gggcgtcatc 660
accaccagct acctgctgct gctggccttc ctgcagcggc ggcaacggcg gcggcaggac 720
agcagggtcg tggcccgctc tgtccgcatc ctggtggctt ccttcttcct ctgctggttt 780
cccaaccatg tggtcactct ctggggtgtc ctggtgaagt ttgacctggt gccctggaac 840
agtactttct atactatcca gacgtatgtc ttccctgtca ctacttgctt ggcacacagc 900
aatagctgcc tcaaccctgt gctgtactgt ctcctgaggc gggagccccg gcaggctctg 960
gcaggcacct tcagggatct gcggtcgagg ctgtggcccc agggcggagg ctgggtgcaa 1020
caggtggccc taaagcaggt aggcaggcgg tgggtcgcaa gcaacccccg ggagagccgc 1080
ccttctaccc tgctcaccaa cctggacaga gggacacccg ggctagcaa aggagaagaa 1140
ctcttcactg gagttgtccc aattcttgtt gaattagatg gtgatgttaa cggccacaag 1200
ttctctgtca gtggagaggg tgaaggtgat gcaacatacg gaaaacttac cctgaagttc 1260
atctgcacta ctggcaaact gcctgttcca tggccaacac tagtcactac tctgtgctat 1320
ggtgttcaat gcttttcaag atacccggat catatgaaac ggcatgactt tttcaagagt 1380
gccatgcccg aaggttatgt acaggaaagg accatcttct tcaaagatga cggcaactac 1440
aagacacgtg ctgaagtcaa gtttgaaggt gatacccttg ttaatagaat cgagttaaaa 1500
ggtattgact tcaaggaaga tggcaacatt ctgggacaca aattggaata caactataac 1560
tcacacaatg tatacatcat ggcagacaaa caaaagaatg gaatcaaagt gaacttcaag 1620
acccgccaca acattgaaga tggaagcgtt caactagcag accattatca acaaaatact 1680
ccaattggcg atggccctgt ccttttacca gacaaccatt acctgtccac acaatctgcc 1740
ctttcgaaag atcccaacga aaagagagac cacatggtcc ttcttgagtt tgtaacagct 1800
gctgggatta cacatggcat ggatgaactg tacaactga 1839

<210> 134
<211> 1800

<212> DNA
<213> Human

<400> 134
atgtcccctg aatgcgcgcg ggcagcgggc gacgcgccct tgcgcagcct ggagcaagcc 60
aaccgcaccc gctttccctt cttctccgac gtcaagggcg accaccggct ggtgctggcc 120
gcggtggaga caaccgtgct ggtgctcatc tttgcagtgt cgctgctggg caacgtgtgc 180
gccctggtgc tggtggcgcg ccgacgacgc cgcggcgcga ctgcctgcct ggtactcaac 240
ctcttctgcg cggacctgct cttcatcagc gctatccctc tggtgctggc cgtgcgctgg 300
actgaggcct ggctgctggg ccccgttgcc tgccacctgc tcttctacgt gatgaccctg 360
agcggcagcg tcaccatcct cacgctggcc gcggtcagcc tggagcgcat ggtgtgcatc 420
gtgcacctgc agcgcggcgt gcggggtcct gggcggcggg cgcgggcagt gctgctggcg 480
ctcatctggg gctattcggc ggtcgccgct ctgcctctct gcgtcttctt ccgagtcgtc 540
ccgcaacggc tccccggcgc cgaccaggaa atttcgattt gcacactgat ttggcccacc 600
attcctggag agatctcgtg ggatgtctct tttgttactt tgaacttctt ggtgccagga 660
ctggtcattg tgatcagtta ctccaaaatt ttacagatca caaaggcatc aaggaagagg 720
ctcacggtaa gcctggccta ctcggagagc caccagatcc gcgtgtccca gcaggacttc 780
cggctcttcc gcaccctctt cctcctcatg gtctccttct tcatcatgtg gagccccatc 840
atcatcacca tcctcctcat cctgatccag aacttcaagc aagacctggt catctggccg 900
tccctcttct tctgggtggt ggccttcaca tttgctaatt cagccctaaa ccccatcctc 960
tacaacatga cactgtgcag gaatgagtgg aagaaaattt tttgctgctt ctggttccca 1020
gaaaagggag ccattttaac agacacatct gtcaaaagaa atgacttgtc gattatttct 1080
ggcgctagca aaggagaaga actcttcact ggagttgtcc caattcttgt tgaattagat 1140
ggtgatgtta acggccacaa gttctctgtc agtggagagg gtgaaggtga tgcaacatac 1200
ggaaaactta ccctgaagtt catctgcact actggcaaac tgcctgttcc atggccaaca 1260
ctagtcacta ctctgtgcta tggtgttcaa tgcttttcaa gatacccgga tcatatgaaa 1320
cggcatgact ttttcaagag tgccatgccc gaaggttatg tacaggaaag gaccatcttc 1380
ttcaaagatg acggcaacta caagacacgt gctgaagtca agtttgaagg tgataccctt 1440
gttaatagaa tcgagttaaa aggtattgac ttcaaggaag atggcaacat tctgggacac 1500
aaattggaat acaactataa ctcacacaat gtatacatca tggcagacaa acaaaagaat 1560
ggaatcaaag tgaacttcaa gacccgccac aacattgaag atggaagcgt tcaactagca 1620
gaccattatc aacaaaatac tccaattggc gatggccctg tccttttacc agacaaccat 1680
tacctgtcca cacaatctgc cctttcgaaa gatcccaacg aaaagagaga ccacatggtc 1740
cttcttgagt ttgtaacagc tgctgggatt acacatggca tggatgaact gtacaactga 1800

<210> 135
<211> 2673
<212> DNA
<213> Human

<400> 135
   atgcagggac cattgcttct tccaggcctc tgctttctgc tgagcctctt tggagctgtg 60
   actcagaaaa ccaaaacttc ctgtgctaag tgccccccaa atgcttcctg tgtcaataac 120
   actcactgca cctgcaacca tggatatact tctggatctg ggcagaaact attcacattc 180
   cccttggaga catgtaacga cattaatgaa tgcacaccac cctatagtgt atattgtgga 240
   tttaacgctg tgtgttacaa tgtcgaagga agtttctact gtcaatgtgt cccaggatat 300
   agactgcatt ctgggaatga acaattcagt aattccaatg agaacacctg tcaggacacc 360
   acctcctcaa agacaaccca gggcaggaaa gagctgcaaa agattgtgga caaatttgag 420
   tcacttctca ccaatcagac tttatggaga acagaaggga gacaagaaat ctcatccaca 480
   gctaccacta ttctccggga tgtggaatcg aaagttctag aaactgcctt gaaagatcca 540
   gaacaaaaag tcctgaaaat ccaaaacgat agtgtagcta ttgaaactca agcgattaca 600
   gacaattgct ctgaagaaag aaagacattc aacttgaacg tccaaatgaa ctcaatggac 660
   atccgttgca gtgacatcat ccagggagac acacaaggtc ccagtgccat tgcctttatc 720
   tcatattctt ctcttggaaa catcataaat gcaacttttt ttgaagagat ggataagaaa 780
   gatcaagtgt atctgaactc tcaggttgtg agtgctgcta ttggacccaa aaggaacgtg 840
   tctctctcca agtctgtgac gctgactttc cagcacgtga agatgacccc cagtaccaaa 900
   aaggtcttct gtgtctactg gaagagcaca gggcagggca gccagtggtc cagggatggc 960
   tgcttcctga tacacgtgaa caagagtcac accatgtgta attgcagtca cctgtccagc 1020
   ttcgctgtcc tgatggccct gaccagccag gaggaggatc ccgtgctgac tgtcatcacc 1080
   tacgtggggc tgagcgtctc tctgctgtgc ctcctcctgg cggccctcac ttttctcctg 1140
   tgtaaagcca tccagaacac cagcacctca ctgcatctgc agctctcgct ctgcctcttc 1200
   ctggcccacc tcctcttcct cgtggggatt gatcgaactg aacccaaggt gctgtgctcc 1260
   atcatcgccg gtgctttgca ctatctctac ctggccgcct tcacctggat gctgctggag 1320
   ggtgtgcacc tcttcctcac tgcacggaac ctgacagtgg tcaactactc aagcatcaat 1380
   agactcatga agtggatcat gttcccagtc ggctatggcg ttcccgctgt gactgtggcc 1440
   atttctgcag cctcctggcc tcacctttat ggaactgctg atcgatgctg gctccacctg 1500
   gaccagggat tcatgtggag tttccttggc ccagtctgtg ccattttctc tgcgaattta 1560
   gtattgttta tcttggtctt ttggattttg aaaagaaaac tttcctccct caatagtgaa 1620
   gtgtcaacca tccagaacac aaggatgctg gctttcaaag caacagctca gctcttcatc 1680
   ctgggctgca catggtgtct gggcttgcta caggtgggtc cagctgccca ggtcatggcc 1740
   tacctcttca ccatcatcaa cagcctccaa ggcttcttca tcttcttggt ctactgcctc 1800
   ctcagccagc aggtccagaa acaatatcaa aagtggttta gagagatcgt aaaatcaaaa 1860
   tctgagtctg agacatacac actttccagc aagatgggtc ctgactcaaa acccagtgag 1920
   ggggatgttt ttccaggaca agtgaagaga aaatatgcta gcaaaggaga agaactcttc 1980
   actggagttg tcccaattct tgttgaatta gatggtgatg ttaacggcca caagttctct 2040
   gtcagtggag agggtgaagg tgatgcaaca tacggaaaac ttaccctgaa gttcatctgc 2100
   actactggca aactgcctgt tccatggcca acactagtca ctactctgtg ctatggtgtt 2160
   caatgctttt caagataccc ggatcatatg aaacggcatg actttttcaa gagtgccatg 2220
   cccgaaggtt atgtacagga aaggaccatc ttcttcaaag atgacggcaa ctacaagaca 2280
   cgtgctgaag tcaagtttga aggtgatacc cttgttaata gaatcgagtt aaaaggtatt 2340
   gacttcaagg aagatggcaa cattctggga cacaaattgg aatacaacta taactcacac 2400
   aatgtataca tcatggcaga caaacaaaag aatggaatca aagtgaactt caagacccgc 2460
   cacaacattg aagatggaag cgttcaacta gcagaccatt atcaacaaaa tactccaatt 2520
   ggcgatggcc ctgtcctttt accagacaac cattacctgt ccacacaatc tgccctttcg 2580
   aaagatccca acgaaaagag agaccacatg gtccttcttg agtttgtaac agctgctggg 2640
   attacacatg gcatggatga actgtacaac tga 2673

<210> 136
<211> 1797
<212> DNA
<213> Human

<400> 136
atggacccag aagaaacttc agtttatttg gattattact atgctacgag cccaaactct 60
gacatcaggg agacccactc ccatgttcct tacacctctg tcttccttcc agtcttttac 120
acagctgtgt tcctgactgg agtgctgggg aaccttgttc tcatgggagc gttgcatttc 180
aaacccggca gccgaagact gatcgacatc tttatcatca atctggctgc ctctgacttc 240
atttttcttg tcacattgcc tctctgggtg gataaagaag catctctagg actgtggagg 300
acgggctcct tcctgtgcaa agggagctcc tacatgatct ccgtcaatat gcactgcagt 360
gtcctcctgc tcacttgcat gagtgttgac cgctacctgg ccattgtgtg gccagtcgta 420
tccaggaaat tcagaaggac agactgtgca tatgtagtct gtgccagcat ctggtttatc 480
tcctgcctgc tggggttgcc tactcttctg tccagggagc tcacgctgat tgatgataag 540
ccatactgtg cagagaaaaa ggcaactcca attaaactca tatggtccct ggtggcctta 600
attttcacct tttttgtccc tttgttgagc attgtgacct gctactgttg cattgcaagg 660
aagctgtgtg cccattacca gcaatcagga aagcacaaca aaaagctgaa gaaatctata 720
aagatcatct ttattgtcgt ggcagccttt cttgtctcct ggctgccctt caatactttc 780
aagttcctgg ccattgtctc tgggttgcgg caagaacact atttaccctc agctattctt 840
cagcttggta tggaggtgag tggacccttg gcatttgcca acagctgtgt caaccctttc 900
atttactata tcttcgacag ctacatccgc cgggccattg tccactgctt gtgcccttgc 960
ctgaaaaact atgactttgg gagtagcact gagacatcag atagtcacct cactaaggct 1020
ctctccacct tcattcatgc agaagatttt gccaggagga ggaagaggtc tgtgtcactc 1080
gctagcaaag gagaagaact cttcactgga gttgtcccaa ttcttgttga attagatggt 1140
gatgttaacg gccacaagtt ctctgtcagt ggagagggtg aaggtgatgc aacatacgga 1200
aaacttaccc tgaagttcat ctgcactact ggcaaactgc ctgttccatg gccaacacta 1260
gtcactactc tgtgctatgg tgttcaatgc ttttcaagat acccggatca tatgaaacgg 1320
catgactttt tcaagagtgc catgcccgaa ggttatgtac aggaaaggac catcttcttc 1380
aaagatgacg gcaactacaa gacacgtgct gaagtcaagt ttgaaggtga tacccttgtt 1440
aatagaatcg agttaaaagg tattgacttc aaggaagatg gcaacattct gggacacaaa 1500
ttggaataca actataactc acacaatgta tacatcatgg cagacaaaca aaagaatgga 1560
atcaaagtga acttcaagac ccgccacaac attgaagatg gaagcgttca actagcagac 1620
cattatcaac aaaatactcc aattggcgat ggccctgtcc ttttaccaga caaccattac 1680
ctgtccacac aatctgccct ttcgaaagat cccaacgaaa agagagacca catggtcctt 1740
cttgagtttg taacagctgc tgggattaca catggcatgg atgaactgta caactga 1797

<210> 137
<211> 1674
<212> DNA
<213> Human

<400> 137
atgccattcc caaactgctc agcccccagc actgtggtgg ccacagctgt gggtgtcttg 60
ctggggctgg agtgtgggct gggtctgctg ggcaacgcgg tggcgctgtg gaccttcctg 120
ttccgggtca gggtgtggaa gccgtacgct gtctacctgc tcaacctggc cctggctgac 180
ctgctgttgg ctgcgtgcct gcctttcctg gccgccttct acctgagcct ccaggcttgg 240
catctgggcc gtgtgggctg ctgggccctg cgcttcctgc tggacctcag ccgcagcgtg 300
gggatggcct tcctggccgc cgtggctttg gaccggtacc tccgtgtggt ccaccctcgg 360
cttaaggtca acctgctgtc tcctcaggcg gccctggggg tctcgggcct cgtctggctc 420
ctgatggtcg ccctcacctg cccgggcttg ctcatctctg aggccgccca gaactccacc 480
aggtgccaca gtttctactc cagggcagac ggctccttca gcatcatctg gcaggaagca 540
ctctcctgcc ttcagtttgt cctccccttt ggcctcatcg tgttctgcaa tgcaggcatc 600
atcagggctc tccagaaaag actccgggag cctgagaaac agcccaagct tcagcgggcc 660
caggcactgg tcaccttggt ggtggtgctg tttgctctgt gctttctgcc ctgcttcctg 720
gccagagtcc tgatgcacat cttccagaat ctggggagct gcagggccct ttgtgcagtg 780
gctcatacct cggatgtcac gggcagcctc acctacctgc acagtgtgct caaccccgtg 840
gtatactgct tctccagccc caccttcagg agctcctatc ggagggtctt ccacaccctc 900
cgaggcaaag ggcaggcagc agagccccca gatttcaacc ccagagactc ctattccgct 960
agcaaaggag aagaactctt cactggagtt gtcccaattc ttgttgaatt agatggtgat 1020
gttaacggcc acaagttctc tgtcagtgga gagggtgaag gtgatgcaac atacggaaaa 1080
cttaccctga agttcatctg cactactggc aaactgcctg ttccatggcc aacactagtc 1140
actactctgt gctatggtgt tcaatgcttt tcaagatacc cggatcatat gaaacggcat 1200

gactttttca agagtgccat gcccgaaggt tatgtacagg aaaggaccat cttcttcaaa 1260
gatgacggca actacaagac acgtgctgaa gtcaagtttg aaggtgatac ccttgttaat 1320
agaatcgagt taaaaggtat tgacttcaag gaagatggca acattctggg acacaaattg 1380
gaatacaact ataactcaca caatgtatac atcatggcag acaaacaaaa gaatggaatc 1440
aaagtgaact tcaagacccg ccacaacatt gaagatggaa gcgttcaact agcagaccat 1500
tatcaacaaa atactccaat tggcgatggc cctgtccttt taccagacaa ccattacctg 1560
tccacacaat ctgccctttc gaaagatccc aacgaaaaga gagaccacat ggtccttctt 1620
gagtttgtaa cagctgctgg gattacacat ggcatggatg aactgtacaa ctga 1674

<210> 138
<211> 1926
<212> DNA
<213> Human

<400> 138
atgttcgtgg catcagagag aaagatgaga gctcaccagg tgctcacctt cctcctgctc 60
ttcgtgatca cctcggtggc ctctgaaaac gccagcacat cccgaggctg tgggctggac 120
ctcctccctc agtacgtgtc cctgtgcgac ctggacgcca tctggggcat tgtggtggag 180
gcggtggccg gggcgggcgc cctgatcaca ctgctcctga tgctcatcct cctggtgcgg 240
ctgcccttca tcaaggagaa ggagaagaag agccctgtgg gcctccactt tctgttcctc 300
ctggggaccc tgggcctctt tgggctgacg tttgccttca tcatccagga ggacgagacc 360
atctgctctg tccgccgctt cctctggggc gtcctctttg cgctctgctt ctcctgcctg 420
ctgagccagg catggcgcgt gcggaggctg gtgcggcatg gcacgggccc cgcgggctgg 480
cagctggtgg gcctggcgct gtgcctgatg ctggtgcaag tcatcatcgc tgtggagtgg 540
ctggtgctca ccgtgctgcg tgacacaagg ccagcctgcg cctacgagcc catggacttt 600
gtgatggccc tcatctacga catggtactg cttgtggtca ccctggggct ggccctcttc 660
actctgtgcg gcaagttcaa gaggtggaag ctgaacgggg ccttcctcct catcacagcc 720
ttcctctctg tgctcatctg ggtggcctgg atgaccatgt acctcttcgg caatgtcaag 780
ctgcagcagg gggatgcctg gaacgacccc accttggcca tcacgctggc ggccagcggc 840
tgggtcttcg tcatcttcca cgccatccct gagatccact gcacccttct gccagccctg 900
caggagaaca cgcccaacta cttcgacacg tcgcagccca ggatgcggga gacggccttc 960
gaggaggacg tgcagctgcc gcgggcctat atggagaaca aggccttctc catggatgaa 1020
cacaatgcag ctctccgaac agcaggattt cccaacggca gcttgggaaa aagacccagt 1080
ggcagcttgg ggaaaagacc cagcgctccg tttagaagca acgtgtatca gccaactgag 1140
atggccgtcg tgctcaacgg tgggaccatc ccaactgctc cgccaagtca cacaggaaga 1200
cacctttggg ctagcaaagg agaagaactc ttcactggag ttgtcccaat tcttgttgaa 1260
ttagatggtg atgttaacgg ccacaagttc tctgtcagtg gagagggtga aggtgatgca 1320
acatacggaa aacttaccct gaagttcatc tgcactactg gcaaactgcc tgttccatgg 1380
ccaacactag tcactactct gtgctatggt gttcaatgct tttcaagata cccggatcat 1440
atgaaacggc atgacttttt caagagtgcc atgcccgaag gttatgtaca ggaaaggacc 1500
atcttcttca aagatgacgg caactacaag acacgtgctg aagtcaagtt tgaaggtgat 1560
acccttgtta atagaatcga gttaaaaggt attgacttca aggaagatgg caacattctg 1620
ggacacaaat tggaatacaa ctataactca cacaatgtat acatcatggc agacaaacaa 1680
aagaatggaa tcaaagtgaa cttcaagacc cgccacaaca ttgaagatgg aagcgttcaa 1740
ctagcagacc attatcaaca aaatactcca attggcgatg gccctgtcct tttaccagac 1800
aaccattacc tgtccacaca atctgccctt tcgaaagatc ccaacgaaaa gagagaccac 1860
atggtccttc ttgagtttgt aacagctgct gggattacac atggcatgga tgaactgtac 1920
aactga 1926

<210> 139
<211> 1746
<212> DNA
<213> Human

<400> 139
atggctggaa actgctcctg ggaggcccat cccggcaaca ggaacaggat gtgccctggc 60
ctgagcgagg cccccggaact ctacagccgg ggcttcctga ccatcgagca gatcgcgatg 120
ctgccgcctc cggccgtcat gaactacatc ttcctgctcc tctgcctgtg tggcctggtg 180
ggcaacgggc tggtcctctg gtttttcggc ttctccatca agaggaaccc cttctccatc 240
tacttcctgc acctggccag cgccgatgtg ggctacctct tcagcaaggc ggtgttctcc 300
atcctgaaca cgggggggctt cctgggcacg tttgccgact acatccgcag cgtgtgccgg 360
gtcctggggc tctgcatgtt ccttaccggc gtgagcctcc tgccggccgt cagcgccgag 420
cgctgcgcct cggtcatctt ccccgcctgg tactggcgcc ggcggcccaa gcgcctgtcg 480
gccgtggtgt gcgccctgct gtgggtcctg tccctcctgg tcacctgcct gcacaactac 540
ttctgcgtgt tcctgggccg cggggccccc ggcgcggcct gcaggcacat ggacatcttc 600
ctgggcatcc tcctgttcct gctctgctgc ccgctcatgg tgctgccctg cctggccctc 660
atcctgcacg tggagtgccg ggcccgacgg cgccagcgct ctgccaagct caaccacgtc 720
atcctggcca tggtctccgt cttcctggtg tcctccatct acttagggat cgactggttc 780
ctcttctggg tcttccagat ccccgccccc ttccccgagt acgtcactga cctgtgcatc 840
tgcatcaaca gcagcgccaa gcccatcgtc tacttcctgg ccgggaggga caagtcgcag 900
cggctgtggg agccgctcag ggtggtcttc cagcgggccc tgcgggacgg cgctgagctg 960
ggggaggccg gggcagcac gcccaacaca gtcaccatgg agatgcagtg tcccccgggg 1020
aacgcctccg ctagcaaagg agaagaactc ttcactggag ttgtcccaat tcttgttgaa 1080
ttagatggtg atgttaacgg ccacaagttc tctgtcagtg gagagggtga aggtgatgca 1140
acatacggaa aacttaccct gaagttcatc tgcactactg gcaaactgcc tgttccatgg 1200

ccaacactag tcactactct gtgctatggt gttcaatgct tttcaagata cccggatcat 1260
atgaaacggc atgacttttt caagagtgcc atgcccgaag gttatgtaca ggaaaggacc 1320
atcttcttca aagatgacgg caactacaag acacgtgctg aagtcaagtt tgaaggtgat 1380
acccttgtta atagaatcga gttaaaaggt attgacttca aggaagatgg caacattctg 1440
ggacacaaat tggaatacaa ctataactca cacaatgtat acatcatggc agacaaacaa 1500
aagaatggaa tcaaagtgaa cttcaagacc cgccacaaca ttgaagatgg aagcgttcaa 1560
ctagcagacc attatcaaca aaatactcca attggcgatg gccctgtcct tttaccagac 1620
aaccattacc tgtccacaca atctgccctt tcgaaagatc ccaacgaaaa gagagaccac 1680
atggtccttc ttgagtttgt aacagctgct gggattacac atggcatgga tgaactgtac 1740
aactga 1746

<210> 140
<211> 3759
<212> DNA
<213> Human

<400> 140
atggtttctc ctgtcaggca gtgtggccat gttggcagaa ctgaagaagt tttactgacg 60
ttcaagatat tccttgtcat catttgtctt catgtcgttc tggtaacatc cctggaagaa 120
gatactgata attccagttt gtcaccacca cctgctaaat tatctgttgt cagttttgcc 180
ccctcctcca atgaggttga aacaacaagc ctcaatgatg ttactttaag cttactccct 240
tcaaacgaaa cagaaaaaac taaaatcact atagtaaaaa ccttcaatgc atcaggcgtc 300
aaacccagaa gaaatatctg caatttgtca tctatttgca atgactcagc atttttttaga 360
ggtgagatca tgtttcaata tgataaagaa agcactgttc cccagaatca acatataacg 420
aatggcacct taactggagt cctgtctcta agtgaattaa aacgctcaga gctcaacaaa 480
accctgcaaa ccctaagtga gacttacttt ataatgtgtg ctacagcaga ggcccaaagc 540
acattaaatt gtacattcac aataaaactg aataatacaa tgaatgcatg tgctgtaata 600
gctgctttgg aaagagtaaa gattcgacca atggaacact gctgctgttc tgtcaggata 660
ccctgccctt cctccccaga agagttggaa aagcttcagt gtgacctgca ggatcccatt 720
gtctgtcttg ctgaccatcc acgtggccca ccatttcttc ccagccaatc catcccagtg 780
gtgcctcggg ccactgtgct ttcccaggtc cccaaagcta cctcttttgc tgagcctcca 840
gattattcac ctgtgaccca caatgttccc tctccaatag gggagattca acccctttca 900
ccccagcctt cagctcccat agcttccagc cctgccattg acatgccccc acagtctgaa 960
acgatctctt cccctatgcc ccaaacccat gtctccggca ccccacctcc tgtgaaagcc 1020
tcattttcct ctcccaccgt gtctgcccct gcgaatgtca acactaccag cgcacctcct 1080
gtccagacag acatcgtcaa caccagcagt atttctgatc ttgagaacca agtgttgcag 1140
atggagaagg ctctgtcctt gggcagcctg gagcctaacc tcgcaggaga aatgatcaac 1200
caagtcagca gactccttca ttccccgcct gacatgctgg cccctctggc tcaaagattg 1260
ctgaaagtag tggatgacat tggcctacag ctgaactttt caaacacgac tataagtcta 1320
acctcccctt ctttggctct ggctgtgatc agagtgaatg ccagtagttt caacacaact 1380
acctttgtgg cccaagaccc tgcaaatctt caggtttctc tggaaaccca agctcctgag 1440
aacagtattg gcacaattac tcttccttca tcgctgatga ataatttacc agctcatgac 1500
atggagctag cttccagggt tcagttcaat ttttttgaaa cacctgcttt gtttcaggat 1560
ccttccctgg agaacctctc tctgatcagc tacgtcatat catcgagtgt tgcaaacctg 1620
accgtcagga acttgacaag aaacgtgaca gtcacattaa agcacatcaa cccgagccag 1680
gatgagttaa cagtgagatg tgtattttgg gacttgggca gaaatggtgg cagaggaggc 1740
tggtcagaca atggctgctc tgtcaaagac aggagattga atgaaaccat ctgtacctgt 1800
agccatctaa caagcttcgg cgttctgctg gacctatcta ggacatctgt gctgcctgct 1860
caaatgatgg ctctgacgtt cattacatat attggttgtg ggctttcatc aatttttctg 1920
tcagtgactc ttgtaaccta catagctttt gaaaagatcc ggagggatta cccttccaaa 1980
atcctcatcc agctgtgtgc tgctctgctt ctgctgaacc tggtcttcct cctggactcg 2040
tggattgctc tgtataagat gcaaggcctc tgcatctcag tggctgtatt tcttcattat 2100
tttctcttgg tctcattcac atggatgggc ctagaagcat tccatatgta cctggccctt 2160
gtcaaagtat ttaatactta catccgaaaa tacatcctta aattctgcat tgtcggttgg 2220
ggggtaccag ctgtggttgt gaccatcatc ctgactatat ccccagataa ctatgggctt 2280
ggatcctatg ggaaattccc caatggttca ccggatgact tctgctggat caacaacaat 2340
gcagtattct acattacggt ggtgggatat ttctgtgtga tatttttgct gaacgtcagc 2400
atgttcattg tggtcctggt tcagctctgt cgaattaaaa agaagaagca actgggagcc 2460
cagcgaaaaa ccagtattca agacctcagg agtatcgctg gccttacatt tttactggga 2520
ataacttggg gctttgcctt ctttgcctgg ggaccagtta acgtgacctt catgtatctg 2580
tttgccatct ttaatacctt acaaggattt ttcatattca tcttttactg tgtggccaaa 2640
gaaaatgtca ggaagcaatg gaggcggtat ctttgttgtg gaaagttacg gctggctgaa 2700
aattctgact ggagtaaaac tgctactaat ggtttaaaga agcagactgt aaaccaagga 2760
gtgtccagct cttcaaattc cttacagtca agcagtaact ccactaactc caccacactg 2820
ctagtgaata atgattgctc agtacacgca agcgggaatg gaaatgcttc tacagagagg 2880
aatggggtct cttttagtgt tcagaatgga gatgtgtgcc ttcacgattt cactggaaaa 2940
cagcacatgt ttaacgagaa ggaagattcc tgcaatggga aaggccgtat ggctctcaga 3000
aggacttcaa agcggggaag cttacacttt attgagcaaa tgtctagcaa aggagaagaa 3060
ctcttcactg gagttgtccc aattcttgtt gaattagatg gtgatgttaa cggccacaag 3120
ttctctgtca gtggagaggg tgaaggtgat gcaacatacg gaaaacttac cctgaagttc 3180
atctgcacta ctggcaaact gcctgttcca tggccaacac tagtcactac tctgtgctat 3240
ggtgttcaat gcttttcaag atacccggat catatgaaac ggcatgactt tttcaagagt 3300
gccatgcccg aaggttatgt acaggaaagg accatcttct tcaaagatga cggcaactac 3360
aagacacgtg ctgaagtcaa gtttgaaggt gatacccttg ttaatagaat cgagttaaaa 3420
ggtattgact tcaaggaaga tggcaacatt ctgggacaca aattggaata caactataac 3480

tcacacaatg tatacatcat ggcagacaaa caaaagaatg gaatcaaagt gaacttcaag 3540
acccgccaca acattgaaga tggaagcgtt caactagcag accattatca acaaaatact 3600
ccaattggcg atggccctgt ccttttacca gacaaccatt acctgtccac acaatctgcc 3660
ctttcgaaag atcccaacga aaagagagac cacatggtcc ttcttgagtt tgtaacagct 3720
gctgggatta cacatggcat ggatgaactg tacaactga 3759

<210> 141
<211> 1968
<212> DNA
<213> Human

<400> 141
atggagtcct caccccatcc ccagtcatca gggaactctt ccactttggg gagggtccct 60
caaaccccag gtccctctac tgccagtggg gtcccggagg tggggctacg ggatgttgct 120
tcggaatctg tggccctctt cttcatgctc ctgctggact tgactgctgt ggctggcaat 180
gccgctgtga tggccgtgat cgccaagacg cctgccctcc gaaaatttgt cttcgtcttc 240
cacctctgcc tggtggacct gctggctgcc ctgaccctca tgcccctggc catgctctcc 300
agctctgccc tctttgacca cgccctcttt ggggaggtgg cctgccgcct ctacttgttt 360
ctgagcgtgt gctttgtcag cctggccatc ctctcggtgt cagccatcaa tgtggagcgc 420
tactattacg tagtccaccc catgcgctac gaggtgcgca tgacgctggg gctggtggcc 480
tctgtgctgg tgggtgtgtg ggtgaaggcc ttggccatgg cttctgtgcc agtgttggga 540
agggtctcct gggaggaagg agctcccagt gtccccccag gctgttcact ccagtggagc 600
cacagtgcct actgccagct ttttgtggtg gtctttgctg tcctttactt tctgttgccc 660
ctgctcctca tacttgtggt ctactgcagc atgttccgag tggcccgcgt ggctgccatg 720
cagcacgggc cgctgcccac gtggatggag acaccccggc aacgctccga atctctcagc 780
agccgctcca cgatggtcac cagctcgggg gccccccaga ccaccccaca ccggacgttt 840
gggggaggga aagcagcagt ggttctcctg gctgtggggg gacagttcct gctctgttgg 900
ttgccctact tctctttcca cctctatgtt gccctgagtg ctcagcccat ttcaactggg 960
caggtggaga gtgtggtcac ctggattggc tacttttgct tcacttccaa ccctttcttc 1020
tatggatgtc tcaaccggca gatccggggg gagctcagca agcagtttgt ctgcttcttc 1080
aagccagctc cagaggagga gctgaggctg cctagccggg agggctccat tgaggagaac 1140
ttcctgcagt tccttcaggg gactggctgt ccttctgagt cctgggtttc ccgaccccta 1200
cccagcccca agcaggagcc acctgctgtt gactttcgaa tccaggccag agctagcaaa 1260
ggagaagaac tcttcactgg agttgtccca attcttgttg aattagatgg tgatgttaac 1320
ggccacaagt tctctgtcag tggagagggt gaaggtgatg caacatacgg aaaacttacc 1380
ctgaagttca tctgcactac tggcaaactg cctgttccat ggccaacact agtcactact 1440
ctgtgctatg gtgttcaatg cttttcaaga tacccggatc atatgaaacg gcatgacttt 1500
ttcaagagtg ccatgcccga aggttatgta caggaaagga ccatcttctt caaagatgac 1560
ggcaactaca agacacgtgc tgaagtcaag tttgaaggtg atacccttgt taatagaatc 1620
gagttaaaag gtattgactt caaggaagat ggcaacattc tgggacacaa attggaatac 1680
aactataact cacacaatgt atacatcatg gcagacaaac aaaagaatgg aatcaaagtg 1740
aacttcaaga cccgccacaa cattgaagat ggaagcgttc aactagcaga ccattatcaa 1800
caaaatactc caattggcga tggccctgtc cttttaccag acaaccatta cctgtccaca 1860
caatctgccc tttcgaaaga tcccaacgaa aagagagacc acatggtcct tcttgagttt 1920
gtaacagctg ctgggattac acatggcatg gatgaactgt acaactga 1968

<210> 142
<211> 1722
<212> DNA
<213> Human

<400> 142
atgcagccgt ccccgccgcc caccgagctg gtgccgtcgg agcgcgccgt ggtgctgctg 60
tcgtgcgcac tctccgcgct cggctcgggc ctgctggtgg ccacgcacgc cctgtggccc 120
gacctgcgca gccgggcacg gcgcctgctg ctcttcctgt cgctggccga cctgctctcg 180
gccgcctcct acttctacgg agtgctgcag aacttcgcgg gcccgtcgtg ggactgcgtg 240
ctgcagggcg cgctgtccac cttcgccaac accagctcct tcttctggac cgtggccatt 300
gcgctctact tgtacctcag catcgtccgc gccgcgcgcg ggcctcgcac agatcgcctg 360
ctttgggcct tccatgtcgt cagctggggg gtcccgttgg tcatcactgt ggcagccgtc 420
gccctgaaga agattggcta tgacgcctcg gacgtgtctg tgggctggtg ctggatcgac 480
ctggaggcca aggaccatgt cctgtggatg ctgctgacgg ggaagctgtg ggagatgctg 540
gcatatgtgc tgctgcctct gctgtacctc ctggtccgga agcacatcaa cagagcgcac 600
acggcactct ctgagtaccg gcccatcctc tcccaggagc accgcctgct gcgccactcc 660
tccatggcgg acaagaagct ggtgctcatc ccgctcatct tcatcggcct cagggtctgg 720
agcaccgtgc ggttcgtgct gaccctctgt ggctccccgg ccgtgcagac gccggtgctg 780
gtggttctgc atggtatcgg gaacacgttt cagggaggtg ccaactgcat catgttcgtc 840
ctctgcaccc gcgccgtccg aactcggctc ttctctctct gttgctgctg ctgctcttct 900
cagcctccca ccaagagccc ggctggcact cccaaggctc ccgcgccttc caagccagga 960
gaatctcagg aatcccaagg gaccccaggg gaacttccaa gcactgctag caaaggagaa 1020
gaactcttca ctggagttgt cccaattctt gttgaattag atggtgatgt taacggccac 1080
aagttctctg tcagtggaga gggtgaaggt gatgcaacat acggaaaact taccctgaag 1140
ttcatctgca ctactggcaa actgcctgtt ccatggccaa cactagtcac tactctgtgc 1200
tatggtgttc aatgcttttc aagatacccg gatcatatga aacggcatga ctttttcaag 1260
agtgccatgc ccgaaggtta tgtacaggaa aggaccatct tcttcaaaga tgacggcaac 1320
tacaagacac gtgctgaagt caagtttgaa ggtgataccc ttgttaatag aatcgagtta 1380

aaaggtattg acttcaagga agatggcaac attctgggac acaaattgga atacaactat 1440
aactcacaca atgtatacat catggcagac aaacaaaaga atggaatcaa agtgaacttc 1500
aagacccgcc acaacattga agatggaagc gttcaactag cagaccatta tcaacaaaat 1560
actccaattg gcgatggccc tgtcctttta ccagacaacc attacctgtc cacacaatct 1620
gccctttcga aagatcccaa cgaaaagaga gaccacatgg tccttcttga gtttgtaaca 1680
gctgctggga ttacacatgg catggatgaa ctgtacaact ga 1722

<210> 143
<211> 2763
<212> DNA
<213> Human

<400> 143
atggcagtcc ggttgtgcag tctctctcta tatggagaga tagaattaca aaaagtgaca 60
ataggaaatt gcaatgaaaa tctggaaacc ctggaaaagc aggtaaagga tgtcacagca 120
ccacttaata acatttcttc tgaagtccag attttaacat ctgatgccaa taaattaact 180
gctgagaaca tcactagtgc tacgcgagtg gttggacaga tattcaacac ttccagaaat 240
gcttcacctg aggcaaagaa agttgccata gtaacagtga gtcaactcct agatgccagt 300
gaagatgctt ttcaaagagt tgctgctact gctaatgatg atgcccttac aacgcttatt 360
gagcaaatgg agacttattc cttgtctttg ggtaatcaat cagtggtgga acctaacata 420
gcaatacagt cagcaaattt ctcttcagaa aatgcggtgg ggccttcaaa tgttcgcttc 480
tctgtgcaga aaggagctag cagttctcta gtttctagtt caacatttat acatacaaat 540
gtggatggcc ttaacccaga tgcacagact gagcttcagg tcttgcttaa tatgacgaaa 600
aattacacca agacatgcgg ctttgtagtt tatcaaaatg acaagctttt ccaatcaaaa 660
acttttacag ctaaatcgga ttttagtcaa aaaattatct caagcaaaac tgatgaaaat 720
gagcaagatc agagtgcttc tgttgacatg gtctttagtc caaagtacaa ccaaaaagaa 780
tttcaactct attcctatgc ctgtgtctat tggaatttgt cagcgaagga ctgggacaca 840
tatggctgtc aaaaagacaa gggcactgat ggattcctgc gctgccgctg caaccatact 900
actaattttg ctgtattaat gactttcaaa aaggattatc aatatcccaa atcacttgac 960
atattatcca acgttggatg tgcactgtct gttactggtc tggctctcac agttatattt 1020
cagattgtca ccaggaaagt cagaaaaacc tcagtaacct gggttttggt caatctgtgc 1080
atatcaatgt tgattttcaa cctcctcttt gtgtttggaa ttgaaaactc caataagaac 1140
ttgcagacaa gtgatggtga catcaataat attgactttg acaataatga catacccagg 1200
acagacacca ttaacatccc gaatcccatg tgcactgcga ttgccgcctt actgcactat 1260
tttctgttag tgacatttac ctggaacgca ctcagcgctg cacagctcta ttaccttcta 1320
ataaggacca tgaagcctct tcctcggcat ttcattcttt tcatctcatt aattggatgg 1380
ggagtcccag ctatagtagt ggctataaca gtgggagtta tttattctca gaatggaaat 1440
aatccacagt gggaattaga ctaccggcaa gagaaaatct gctggctggc aattccagaa 1500
cccaatggtg ttataaaaag tccgctgttg tggtcattca tcgtacctgt aaccattatc 1560
ctcatcagca atgttgttat gtttattaca atctcgatca aagtgctgtg gaagaataac 1620
cagaacctga caagcacaaa aaaagtttca tccatgaaga agattgttag cacattatct 1680
gttgcagttg tttttggaat tacctggatt ctagcatacc tgatgctagt taatgatgat 1740
agcatcagga tcgtcttcag ctacatattc tgccttttca acactacaca gggattgcaa 1800
atttttatcc tgtacactgt tagaacaaaa gtcttccaga gtgaagcttc caaagtgttg 1860
atgttgctat cgtctattgg gagaaggaag tcattgcctt cagtgacgcg gccgaggctg 1920
cgtgtaaaga tgtataattt cctcaggtca ttgccaacct tacatgaacg ctttaggcta 1980
ctggaaacct ctccgagtac tgaggaaatc acactctctg aaagtgacaa tgcaaaggaa 2040
agcatcgcta gcaaaggaga agaactcttc actggagttg tcccaattct tgttgaatta 2100
gatggtgatg ttaacggcca caagttctct gtcagtggag agggtgaagg tgatgcaaca 2160
tacggaaaac ttaccctgaa gttcatctgc actactggca aactgcctgt tccatggcca 2220
acactagtca ctactctgtg ctatggtgtt caatgctttt caagataccc ggatcatatg 2280
aaacggcatg actttttcaa gagtgccatg cccgaaggtt atgtacagga aaggaccatc 2340
ttcttcaaag atgacggcaa ctacaagaca cgtgctgaag tcaagtttga aggtgatacc 2400
cttgttaata gaatcgagtt aaaaggtatt gacttcaagg aagatggcaa cattctggga 2460
cacaaattgg aatacaacta taactcacac aatgtataca tcatggcaga caaacaaaag 2520
aatggaatca aagtgaactt caagacccgc cacaacattg aagatggaag cgttcaacta 2580
gcagaccatt atcaacaaaa tactccaatt ggcgatggcc ctgtcctttt accagacaac 2640
cattacctgt ccacacaatc tgccctttcg aaagatccca acgaaaagag agaccacatg 2700
gtccttcttg agtttgtaac agctgctggg attacacatg gcatggatga actgtacaac 2760
tga 2763

<210> 144
<211> 2301
<212> DNA
<213> Human

<400> 144
   atggatcact gtggtgccct tttcctgtgc ctgtgccttc tgactttgca gaatgcaaca 60
   acagagacat gggaagaact cctgagctac atggagaata tgcaggtgtc caggggccgg 120
   agctcagttt tttcctctcg tcaactccac cagctggagc agatgctact gaacaccagc 180
   ttcccaggct acaacctgac cttgcagaca cccaccatcc agtctctggc cttcaagctg 240
   agctgtgact tctctggcct ctcgctgacc agtgccactc tgaagcgggt gccccaggca 300
   ggaggtcagc atgcccgggg tcagcacgcc atgcagttcc ccgccgagct gacccgggac 360
   gcctgcaaga cccgccccag ggagctgcgg ctcatctgta tctacttctc caacacccac 420
   tttttcaagg atgaaaacaa ctcatctctg ctgaataact acgtcctggg ggcccagctg 480
   agtcatgggc acgtgaacaa cctcagggat cctgtgaaca tcagcttctg gcacaaccaa 540
   agcctggaag gctacaccct gacctgtgtc ttctggaagg agggagccag gaaacagccc 600
   tgggggggct ggagccctga gggctgtcgt acagagcagc cctcccactc tcaggtgctc 660
   tgccgctgca accacctcac ctactttgct gttctcatgc aactctcccc agccctggtc 720
   cctgcagagt tgctggcacc tcttacgtac atctccctcg tgggctgcag catctccatc 780
   gtggcctcgc tgatcacagt cctgctgcac ttccatttca ggaagcagag tgactcctta 840
   acacgcatcc acatgaacct gcatgcctcc gtgctgctcc tgaacatcgc cttcctgctg 900
   agccccgcat tcgcaatgtc tcctgtgccc gggtcagcat gcacggctct ggccgctgcc 960
   ctgcactacg cgctgctcag ctgcctcacc tggatggcca tcgagggctt caacctctac 1020
   ctcctcctcg ggcgtgtcta caacatctac atccgcagat atgtgttcaa gcttggtgtg 1080
   ctaggctggg gggccccagc cctcctggtg ctgctttccc tctctgtcaa gagctcggta 1140
   tacggaccct gcacaatccc cgtcttcgac agctgggaga atggcacagg cttccagaac 1200
   atgtccatat gctgggtgcg gagccccgtg gtgcacagtg tcctggtcat gggctacggc 1260
   ggcctcacgt ccctcttcaa cctggtggtg ctggcctggg cgctgtggac cctgcgcagg 1320
   ctgcgggagc gggcggatgc accaagtgtc agggcctgcc atgacactgt cactgtgctg 1380
   ggcctcaccg tgctgctggg aaccacctgg gccttggcct tcttttcttt tggcgtcttc 1440
   ctgctgcccc agctgttcct cttcaccatc ttaaactcgc tctacggttt cttccttttc 1500
   ctgtggttct gctcccagcg gtgccgctca gaagcagagg ccaaggcaca gatagaggcc 1560
   ttcagctcct cccaaacaac acaggctagc aaaggagaag aactcttcac tggagttgtc 1620
   ccaattcttg ttgaattaga tggtgatgtt aacggccaca agttctctgt cagtggagag 1680
   ggtgaaggtg atgcaacata cggaaaactt accctgaagt tcatctgcac tactggcaaa 1740
   ctgcctgttc catggccaac actagtcact actctgtgct atggtgttca atgcttttca 1800
   agatacccgg atcatatgaa acggcatgac tttttcaaga gtgccatgcc cgaaggttat 1860
   gtacaggaaa ggaccatctt cttcaaagat gacggcaact acaagacacg tgctgaagtc 1920
   aagtttgaag gtgataccct tgttaataga atcgagttaa aaggtattga cttcaaggaa 1980
   gatggcaaca ttctgggaca caaattggaa tacaactata actcacacaa tgtatacatc 2040
   atggcagaca aacaaaagaa tggaatcaaa gtgaacttca agacccgcca caacattgaa 2100
   gatggaagcg ttcaactagc agaccattat caacaaaata ctccaattgg cgatggccct 2160
   gtccttttac cagacaacca ttacctgtcc acacaatctg ccctttcgaa agatcccaac 2220
   gaaaagagag accacatggt ccttcttgag tttgtaacag ctgctgggat tacacatggc 2280
   atggatgaac tgtacaactg a 2301

<210> 145
<211> 3375
<212> DNA
<213> Human

<400> 145
atgcgtggct tcaacctgct cctcttctgg ggatgttgtg ttatgcacag ctgggaaggg 60
cacataagac ccacacggaa accaaacaca aagggtaata actgtagaga cagtaccttg 120
tgcccagctt atgccacctg caccaataca gtggacagtt actattgcac ttgcaaacaa 180
ggcttcctgt ccagcaatgg gcaaaatcac ttcaaggatc caggagtgcg atgcaaagat 240
attgatgaat gttctcaaag cccccagccc tgtggtccta actcatcctg caaaaacctg 300
tcagggaggt acaagtgcag ctgtttagat ggtttctctt ctcccactgg aaatgactgg 360
gtcccaggaa agccgggcaa tttctcctgt actgatatca atgagtgcct caccagcagg 420
gtctgccctg agcattctga ctgtgtcaac tccatgggaa gctacagttg cagctgtcaa 480
gttggattca tctctagaaa ctccacctgt gaagacgtga atgaatgtgc agatccaaga 540
gcttgcccag agcatgcaac ttgtaataac actgttggaa actactcttg tttctgcaac 600
ccaggatttg aatccagcag tggccacttg agtttccagg gtctcaaagc atcgtgtgaa 660
gatattgatg aatgcactga aatgtgcccc atcaattcaa catgcaccaa cactcctggg 720
agctactttt gcacctgcca ccctggcttt gcaccaagca gtggacagtt gaatttcaca 780
gaccaaggag tggaatgtag agatattgat gagtgccgcc aagatccatc aacctgtggt 840
cctaattcta tctgcaccaa tgccctgggc tcctacagct gtggctgcat tgtaggcttt 900
catcccaatc cagaaggctc ccagaaagat ggcaacttca gctgccaaag ggttctcttc 960
aaatgtaagg aagatgtgat acccgataat aagcagatcc agcaatgcca agagggaacc 1020
gcagtgaaac ctgcatatgt ctccttttgt gcacaaataa ataacatctt cagcgttctg 1080
gacaaagtgt gtgaaaataa aacgaccgta gtttctctga agaatacaac tgagagcttt 1140
gtccctgtgc ttaaacaaat atccacgtgg actaaattca ccaaggaaga gacgtcctcc 1200
ctggccacag tcttcctgga gagtgtggaa agcatgacac tggcatcttt ttggaaaccc 1260
tcagcaaatg tcactccggc tgttcggacg gaatacttag acattgagag caaagttatc 1320
aacaaagaat gcagtgaaga gaatgtgacg ttggacttgg tagccaaggg ggataagatg 1380
aagatcgggt gttccacaat tgaggaatct gaatccacag agaccactgg tgtggctttt 1440
gtctcctttg tgggcatgga atcggtttta aatgagcgct tcttccaaga ccaccaggct 1500
cccttgacca cctctgagat caagctgaag atgaattctc gagtcgttgg gggcataatg 1560
actggagaga agaaagacgg cttctcagat ccaatcatct acactctgga gaacgttcag 1620
ccaaagcaga agtttgagag gcccatctgt gtttcctgga gcactgatgt gaagggtgga 1680
agatggacat cctttggctg tgtgatcctg gaagcttctg agacatatac catctgcagc 1740
tgtaatcaga tggcaaatct tgccgttatc atggcgtctg gggagctcac gatggacttt 1800
tccttgtaca tcattagcca tgtaggcatt atcatctcct tggtgtgcct cgtcttggcc 1860
atcgccacct ttctgctgtg tcgctccatc cgaaatcaca acacctacct ccacctgcac 1920
ctctgcgtgt gtctcctctt ggcgaagact ctcttcctcg ccggtataca caagactgac 1980
aacaagacgg gctgcgccat catcgcgggc ttcctgcact accttttcct tgcctgcttc 2040
ttctggatgc tggtggaggc tgtgatactg ttcttgatgg tcagaaacct gaaggtggtg 2100
aattacttca gctctcgcaa catcaagatg ctgcacatct gtgcctttgg ttatgggctg 2160
ccgatgctgg tggtggtgat ctctgccagt gtgcagccac agggctatgg aatgcataat 2220

cgctgctggc tgaatacaga gacagggttc atctggagtt tcttggggcc agtttgcaca 2280
gttatagtga tcaactccct tctcctgacc tggaccttgt ggatcctgag gcagaggctt 2340
tccagtgtta atgccgaagt ctcaacgcta aaagacacca ggttactgac cttcaaggcc 2400
tttgcccagc tcttcatcct gggctgctcc tgggtgctgg gcatttttca gattggacct 2460
gtggcaggtg tcatggctta cctgttcacc atcatcaaca gcctgcaggg ggccttcatc 2520
ttcctcatcc actgtctgct caacggccag gtacgagaag aatacaagag gtggatcact 2580
gggaagacga agcccagctc ccagtcccag acctcaagga tcttgctgtc ctccatgcca 2640
tccgcttcca agacgggtgc tagcaaagga gaagaactct tcactggagt tgtcccaatt 2700
cttgttgaat tagatggtga tgttaacggc cacaagttct ctgtcagtgg agagggtgaa 2760
ggtgatgcaa catacggaaa acttaccctg aagttcatct gcactactgg caaactgcct 2820
gttccatggc caacactagt cactactctg tgctatggtg ttcaatgctt ttcaagatac 2880
ccggatcata tgaaacggca tgactttttc aagagtgcca tgcccgaagg ttatgtacag 2940
gaaaggacca tcttcttcaa agatgacggc aactacaaga cacgtgctga agtcaagttt 3000
gaaggtgata cccttgttaa tagaatcgag ttaaaaggta ttgacttcaa ggaagatggc 3060
aacattctgg gacacaaatt ggaatacaac tataactcac acaatgtata catcatggca 3120
gacaaacaaa agaatggaat caaagtgaac ttcaagaccc gccacaacat tgaagatgga 3180
agcgttcaac tagcagacca ttatcaacaa aatactccaa ttggcgatgg ccctgtcctt 3240
ttaccagaca accattacct gtccacacaa tctgcccttt cgaaagatcc caacgaaaag 3300
agagaccaca tggtccttct tgagtttgta acagctgctg ggattacaca tggcatggat 3360
gaactgtaca actga 3375

<210> 146
<211> 1800
<212> DNA
<213> Human

<400> 146
atggccccca cagagccctg gagccccagc ccggggtcag cgccctggga ctactcgggg 60
ttggacggcc tggaggagct ggagctgtgt ccggccgggg acctgcccta cggctacgtc 120
tacatccccg cgctctacct ggcggccttc gccgtgggcc tgctgggcaa cgcctttgtg 180
gtgtggctgc tggccgggcg gcgggggccg cggcggctgg tggatacctt cgtgctgcac 240
ctggcggcag ctgacctggg cttcgtgctc acgctgccgc tgtgggccgc ggcggcggcg 300
ctaggcggcc gctggccgtt cggcgatggc ctctgcaagc tcagcagctt cgcgctggcg 360
ggcacgcgct gcgcgggcgc gctgctgctg gcgggcatga gcgtggaccg ctacctggcc 420
gtggtgaagc tgctcgaggc gaggccactg cgcaccccgc gctgcgcgct ggcctcgtgc 480
tgcggcgtct gggccgtggc gctgctggcc ggcctgccct ccctggtcta ccgggggttg 540
cagcccctgc ctgggggcca ggacagccag tgcggcgagg agccctccca cgccttccag 600
ggcctcagct tgctgctgct gctgctgacc ttcgtgctgc ccctggtcgt caccctcttc 660
tgctactgcc gcatctcgcg ccgcctgcga cggccgccgc acgtgggtcg ggcccggagg 720
aactcgctgc gcatcatctt cgccatcgag agcacgtttg tgggctcctg gctgcccttc 780
agcgccctgc gggccgtctt ccacctggcg cgtctggggg cgctgccgct gccgtgcccc 840
ctgctgctgg cgctgcgctg gggcctcacc attgccacct gcctggcctt cgtcaacagc 900
tgcgccaacc cgctcatcta cctcctgctg gaccgctcat tccgagcccg ggcgctggac 960
ggggcctgcg ggcgcaccgg ccgcctggcg cgaaggatca gctcagcctc ctcgctctcc 1020
agggacgaca gttccgtgtt ccgttgccgg gcccaggccg cgaacactgc ctcggcctcc 1080
tgggctagca aaggagaaga actcttcact ggagttgtcc caattcttgt tgaattagat 1140
ggtgatgtta acggccacaa gttctctgtc agtggagagg gtgaaggtga tgcaacatac 1200
ggaaaactta ccctgaagtt catctgcact actggcaaac tgcctgttcc atggccaaca 1260
ctagtcacta ctctgtgcta tggtgttcaa tgcttttcaa gatacccgga tcatatgaaa 1320
cggcatgact ttttcaagag tgccatgccc gaaggttatg tacaggaaag gaccatcttc 1380
ttcaaagatg acggcaacta caagacacgt gctgaagtca agtttgaagg tgataccctt 1440
gttaatagaa tcgagttaaa aggtattgac ttcaaggaag atggcaacat tctgggacac 1500
aaattggaat acaactataa ctcacacaat gtatacatca tggcagacaa acaaaagaat 1560
ggaatcaaag tgaacttcaa gacccgccac aacattgaag atggaagcgt tcaactagca 1620
gaccattatc aacaaaatac tccaattggc gatggccctg tccttttacc agacaaccat 1680
tacctgtcca cacaatctgc cctttcgaaa gatcccaacg aaaagagaga ccacatggtc 1740
cttcttgagt ttgtaacagc tgctgggatt acacatggca tggatgaact gtacaactga 1800

<210> 147
<211> 1674
<212> DNA
<213> Human

<400> 147
atgagtcagc aaaacaccag tggggactgc ctgtttgacg gtgtcaacga gctgatgaaa 60
accctacagt ttgcagtcca catccccacc ttcgtcctgg gcctgctcct caacctgctg 120
gccatccatg gcttcagcac cttccttaag aacaggtggc ccgattatgc tgccacctcc 180
atctacatga tcaacctggc agtctttgac ctgctgctgg tgctctccct ccccttcaag 240
atggtcctgt cccaggtaca gtcccccttc ccgtccctgt gcaccctggt ggagtgcctt 300
tacttcgtca gcatgtacgg aagcgtcttc accatctgct tcatcagcat ggaccggttc 360
ttggccatcc gttacccgct actggtgagc cacctccggt cccccaggaa gatctttggg 420
atctgctgca ccatctgggt cctggtgtgg accggaagca tccctatcta cagtttccat 480
gggaaagtgg aaaaatacat gtgcttccac aacatgtctg atgatacctg gagcgccaag 540
gtcttcttcc cgctggaggt gtttggcttc ctccttccca tgggcatcat gggcttctgc 600
tgctccagga gcatccacat cctgctgggc cgccgagacc acacccagga ctgggtgcag 660

cagaaagcct gcatctacag catcgcagcc agcctggctg tcttcgtggt ctccttcctc 720
ccagtccacc tggggttctt cctgcagttc ctggtgagaa acagctttat cgtagagtgc 780
agagccaagc agagcatcag cttcttcttg caattgtcca tgtgtttctc caacgtcaac 840
tgctgcctgg atgttttctg ctactacttt gtcatcaaag aattccgcat gaacatcagg 900
gcccaccggc cttccagggt ccagctggtc ctgcaggaca ccacgatctc ccggggcgct 960
agcaaaggag aagaactctt cactggagtt gtcccaattc ttgttgaatt agatggtgat 1020
gttaacggcc acaagttctc tgtcagtgga gagggtgaag gtgatgcaac atacggaaaa 1080
cttaccctga agttcatctg cactactggc aaactgcctg ttccatggcc aacactagtc 1140
actactctgt gctatggtgt tcaatgcttt tcaagatacc cggatcatat gaaacggcat 1200
gactttttca agagtgccat gcccgaaggt tatgtacagg aaaggaccat cttcttcaaa 1260
gatgacggca actacaagac acgtgctgaa gtcaagtttg aaggtgatac ccttgttaat 1320
agaatcgagt taaaaggtat tgacttcaag gaagatggca acattctggg acacaaattg 1380
gaatacaact ataactcaca caatgtatac atcatggcag acaaacaaaa gaatggaatc 1440
aaagtgaact tcaagacccg ccacaacatt gaagatggaa gcgttcaact agcagaccat 1500
tatcaacaaa atactccaat tggcgatggc cctgtccttt taccagacaa ccattacctg 1560
tccacacaat ctgccctttc gaaagatccc aacgaaaaga gagaccacat ggtccttctt 1620
gagtttgtaa cagctgctgg gattacacat ggcatggatg aactgtacaa ctga 1674

<210> 148
<211> 2127
<212> DNA
<213> Human

<400> 148
atggacacta ccatggaagc tgacctgggt gccactggcc acaggccccg cacagagctt 60
gatgatgagg actcctaccc ccaaggtggc tgggacacgg ttttcctggt ggccctgctg 120
ctccttgggc tgccagccaa tgggttgatg gcgtggctgg ccggctccca ggcccggcat 180
ggagctggca cgcgtctggc gctgctcctg ctcagcctgg ccctctctga cttcttgttc 240
ctggcagcag cggccttcca gatcctagag atccggcatg gggacactgg gccgctgggg 300
acagctgcct gccgcttcta ctacttccta tggggcgtgt cctactcctc cggcctcttc 360
ctgctggccg ccctcagcct cgaccgctgc ctgctggcgc tgtgcccaca ctggtaccct 420
gggcaccgcc cagtccgcct gcccctctgg gtctgcgccg gtgtctgggt gctggccaca 480
ctcttcagcg tgccctggct ggtcttcccc gaggctgccg tctggtggta cgacctggtc 540
atctgcctgg acttctggga cagcgaggag ctgtcgctga ggatgctgga ggtcctgggg 600
ggcttcctgc ctttcctcct gctgctcgtc tgccacgtgc tcacccaggc cacagcctgt 660
cgcacctgcc accgccaaca gcagcccgca gcctgccggg gcttcgcccg tgtggccagg 720
accattctgt cagcctatgt ggtcctgagg ctgccctacc agctggccca gctgctctac 780
ctggccttcc tgtgggacgt ctactctggc tacctgctct gggaggccct ggtctactcc 840
gactacctga tcctactcaa cagctgcctc agccccttcc tctgcctcat ggccagtgcc 900
gacctccgga ccctgctgcg ctccgtgctc tcgtccttcg cggcagctct ctgcgaggag 960
cggccgggca gcttcacgcc cactgagcca cagacccagc tagattctga gggtccaact 1020
ctgccagagc cgatggcaga ggcccagtca cagatggatc ctgtggccca gcctcaggtg 1080
aaccccacac tccagccacg atcggatccc acagctcagc cacagctgaa ccctacggcc 1140
cagccacagt cggatcccac agcccagcca cagctgaacc tcatggccca gccacagtca 1200
gattctgtgg cccagccaca ggcagacact aacgtccaga cccctgcacc tgctgccagt 1260
tctgtgccca gtccctgtga tgaagcttcc ccaaccccat cctcgcatcc taccccaggg 1320
gcccttgagg acccagccac acctcctgcc tctgaaggag aaagccccag cagcaccccg 1380
ccagaggcgg ccccgggcgc aggccccacg gctagcaaag gagaagaact cttcactgga 1440
gttgtcccaa ttcttgttga attagatggt gatgttaacg gccacaagtt ctctgtcagt 1500
ggagagggtg aaggtgatgc aacatacgga aaacttaccc tgaagttcat ctgcactact 1560
ggcaaactgc ctgttccatg gccaacacta gtcactactc tgtgctatgg tgttcaatgc 1620
ttttcaagat acccggatca tatgaaacgg catgactttt tcaagagtgc catgcccgaa 1680
ggttatgtac aggaaaggac catcttcttc aaagatgacg gcaactacaa gacacgtgct 1740
gaagtcaagt ttgaaggtga tacccttgtt aatagaatcg agttaaaagg tattgacttc 1800
aaggaagatg gcaacattct gggacacaaa ttggaataca actataactc acacaatgta 1860
tacatcatgg cagacaaaca aaagaatgga atcaaagtga acttcaagac ccgccacaac 1920
attgaagatg gaagcgttca actagcagac cattatcaac aaaatactcc aattggcgat 1980
ggccctgtcc ttttaccaga caaccattac ctgtccacac aatctgccct ttcgaaagat 2040
cccaacgaaa agagagacca catggtcctt cttgagtttg taacagctgc tgggattaca 2100
catggcatgg atgaactgta caactga 2127

<210> 149
<211> 1914
<212> DNA
<213> Human

<400> 149
atgaggcccg agcgtccccg gccgcgcggc agcgcccccg gcccgatgga gaccccgccg 60
tgggacccag cccgcaacga ctcgctgccg cccacgctga ccccggccgt gcccccctac 120
gtgaagcttg gcctcaccgt cgtctacacc gtgttctacg cgctgctctt cgtgttcatc 180
tacgtgcagc tctggctggt gctgcgttac cgccacaagc ggctcagcta ccagagcgtc 240
ttcctctttc tctgcctctt ctgggcctcc ctgcggaccg tcctcttctc cttctacttc 300
aaagacttcg tggcggccaa ttcgctcagc cccttcgtct tctggctgct ctactgcttc 360
cctgtgtgcc tgcagttttt caccctcacg ctgatgaact tgtacttcac gcaggtgatt 420
ttcaaagcca agtcaaaata ttctccagaa ttactcaaat accggttgcc cctctacctg 480

ページ(152)

gcctccctct tcatcagcct tgttttcctg ttggtgaatt taacctgtgc tgtgctggta 540
aagacgggaa attgggagag gaaggttatc gtctctgtgc gagtggccat taatgacacg 600
ctcttcgtgc tgtgtgccgt ctctctctcc atctgtctct acaaaatctc taagatgtcc 660
ttagccaaca tttacttgga gtccaagggc tcctccgtgt gtcaagtgac tgccatcggt 720
gtcaccgtga tactgcttta cacctctcgg gcctgctaca acctgttcat cctgtcattt 780
tctcagaaca agagcgtcca ttcctttgat tatgactggt acaatgtatc agaccaggca 840
gatttgaaga atcagctggg agatgctgga tacgtattat ttggagtggt gttatttgtt 900
tgggaactct tacctaccac cttagtcgtt tatttcttcc gagttagaaa tcctacaaag 960
gaccttacca accctggaat ggtccccagc catggattca gtcccagatc ttatttcttt 1020
gacaaccctc gaagatatga cagtgatgat gaccttgcct ggaacattgc ccctcaggga 1080
cttcagggag gttttgctcc agattactat gattggggac aacaaactaa cagcttcctg 1140
gcacaagcag gaactttgca agactcaact ttggatcctg acaaaccaag ccttggggct 1200
agcaaaggag aagaactctt cactggagtt gtcccaattc ttgttgaatt agatggtgat 1260
gttaacggcc acaagttctc tgtcagtgga gagggtgaag gtgatgcaac atacggaaaa 1320
cttaccctga agttcatctg cactactggc aaactgcctg ttccatggcc aacactagtc 1380
actactctgt gctatggtgt tcaatgcttt tcaagatacc cggatcatat gaaacggcat 1440
gactttttca agagtgccat gcccgaaggt tatgtacagg aaaggaccat cttcttcaaa 1500
gatgacggca actacaagac acgtgctgaa gtcaagtttg aaggtgatac ccttgttaat 1560
agaatcgagt taaaaggtat tgacttcaag gaagatggca acattctggg acacaaattg 1620
gaatacaact ataactcaca caatgtatac atcatggcag acaaacaaaa gaatggaatc 1680
aaagtgaact tcaagacccg ccacaacatt gaagatggaa gcgttcaact agcagaccat 1740
tatcaacaaa atactccaat tggcgatggc cctgtccttt taccagacaa ccattacctg 1800
tccacacaat ctgccctttc gaaagatccc aacgaaaaga gagaccacat ggtccttctt 1860
gagtttgtaa cagctgctgg gattacacat ggcatggatg aactgtacaa ctga 1914

<210> 150
<211> 1974
<212> DNA
<213> Human

<400> 150
   atggtcttct cggcagtgtt gactgcgttc cataccggga catccaacac aacatttgtc 60
   gtgtatgaaa acacctacat gaatattaca ctccctccac cattccagca tcctgacctc 120
   agtccattgc ttagatatag ttttgaaacc atggctccca ctggtttgag ttccttgacc 180
   gtgaatagta cagctgtgcc cacaacacca gcagcattta agagcctaaa cttgcctctt 240
   cagatcaccc tttctgctat aatgatattc attctgtttg tgtcttttct tgggaacttg 300
   gttgtttgcc tcatggttta ccaaaaagct gccatgaggt ctgcaattaa catcctcctt 360
   gccagcctag cttttgcaga catgttgctt gcagtgctga acatgccctt tgccctggta 420
   actattctta ctacccgatg gatttttggg aaattcttct gtagggtatc tgctatgttt 480
   ttctggttat ttgtgataga aggagtagcc atcctgctca tcattagcat agataggttc 540
   cttattatag tccagaggca ggataagcta aacccatata gagctaaggt tctgattgca 600
   gtttcttggg caacttcctt ttgtgtagct tttcctttag ccgtaggaaa ccccgacctg 660
   cagatacctt cccgagctcc ccagtgtgtg tttgggtaca caaccaatcc aggctaccag 720
   gcttatgtga ttttgatttc tctcatttct ttcttcatac ccttcctggt aatactgtac 780
   tcatttatgg gcatactcaa cacccttcgg cacaatgcct tgaggatcca tagctaccct 840
   gaaggtatat gcctcagcca ggccagcaaa ctgggtctca tgagtctgca gagacctttc 900
   cagatgagca ttgacatggg ctttaaaaca cgtgccttca ccactatttt gattctcttt 960
   gctgtcttca ttgtctgctg ggccccattc accacttaca gccttgtggc aacattcagt 1020
   aagcactttt actatcagca caactttttt gagattagca cctggctact gtggctctgc 1080
   tacctcaagt ctgcattgaa tccgctgatc tactactgga ggattaagaa attccatgat 1140
   gcttgcctgg acatgatgcc taagtccttc aagtttttgc cgcagctccc tggtcacaca 1200
   aagcgacgga tacgtcctag tgctgtctat gtgtgtgggg aacatcggac ggtggtggct 1260
   agcaaaggag aagaactctt cactggagtt gtcccaattc ttgttgaatt agatggtgat 1320
   gttaacggcc acaagttctc tgtcagtgga gagggtgaag gtgatgcaac atacggaaaa 1380
   cttaccctga agttcatctg cactactggc aaactgcctg ttccatggcc aacactagtc 1440
   actactctgt gctatggtgt tcaatgcttt tcaagatacc cggatcatat gaaacggcat 1500
   gactttttca agagtgccat gcccgaaggt tatgtacagg aaaggaccat cttcttcaaa 1560
   gatgacggca actacaagac acgtgctgaa gtcaagtttg aaggtgatac ccttgttaat 1620
   agaatcgagt taaaaggtat tgacttcaag gaagatggca acattctggg acacaaattg 1680
   gaatacaact ataactcaca caatgtatac atcatggcag acaaacaaaa gaatggaatc 1740
   aaagtgaact tcaagacccg ccacaacatt gaagatggaa gcgttcaact agcagaccat 1800
   tatcaacaaa atactccaat tggcgatggc cctgtccttt taccagacaa ccattacctg 1860
   tccacacaat ctgccctttc gaaagatccc aacgaaaaga gagaccacat ggtccttctt 1920
   gagtttgtaa cagctgctgg gattacacat ggcatggatg aactgtacaa ctga 1974

<210> 151
<211> 1836
<212> DNA
<213> Human

<400> 151
atggccaaca ctaccggaga gcctgaggag gtgagcggcg ctctgtcccc accgtccgca 60
tcagcttatg tgaagctggt actgctggga ctgattatgt gcgtgagcct ggcgggtaac 120
gccatcttgt ccctgctggt gctcaaggag cgtgccctgc acaaggctcc ttactacttc 180
ctgctggacc tgtgcctggc cgatggcata cgctctgccg tctgcttccc ctttgtgctg 240

gcttctgtgc gccacggctc ttcatggacc ttcagtgcac tcagctgcaa gattgtggcc 300
tttatggccg tgctcttttg cttccatgcg gccttcatgc tgttctgcat cagcgtcacc 360
cgctacatgg ccatcgccca ccaccgcttc tacgccaagc gcatgacact ctggacatgc 420
gcggctgtca tctgcatggc ctggaccctg tctgtggcca tggccttccc acctgtcttt 480
gacgtgggca cctacaagtt tattcgggag gaggaccagt gcatctttga gcatcgctac 540
ttcaaggcca atgacacgct gggcttcatg cttatgttgg ctgtgctcat ggcagctacc 600
catgctgtct acggcaagct gctcctcttc gagtatcgtc accgcaagat gaagccagtg 660
cagatggtgc cagccatcag ccagaactgg acattccatg gtcccggggc caccggccag 720
gctgctgcca actggatcgc cggctttggc cgtgggccca tgccaccaac cctgctgggt 780
atccggcaga atgggcatgc agccagccgg cggctactgg gcatggacga ggtcaagggt 840
gaaaagcagc tgggccgcat gttctacgcg atcacactgc tctttctgct cctctggtca 900
ccctacatcg tggcctgcta ctggcgagtg tttgtgaaag cctgtgctgt gccccaccgc 960
tacctggcca ctgctgtttg gatgagcttc gcccaggctg ccgtcaaccc aattgtctgc 1020
ttcctgctca acaaggacct caagaagtgc ctgaggactc acgcccccctg ctggggcaca 1080
ggaggtgccc cggctcccag agaaccctac tgtgtcatgg ctagcaaagg agaagaactc 1140
ttcactggag ttgtcccaat tcttgttgaa ttagatggtg atgttaacgg ccacaagttc 1200
tctgtcagtg gagagggtga aggtgatgca acatacggaa aacttaccct gaagttcatc 1260
tgcactactg gcaaactgcc tgttccatgg ccaacactag tcactactct gtgctatggt 1320
gttcaatgct tttcaagata cccggatcat atgaaacggc atgacttttt caagagtgcc 1380
atgcccgaag gttatgtaca ggaaaggacc atcttcttca aagatgacgg caactacaag 1440
acacgtgctg aagtcaagtt tgaaggtgat acccttgtta atagaatcga gttaaaaggt 1500
attgacttca aggaagatgg caacattctg ggacacaaat tggaatacaa ctataactca 1560
cacaatgtat acatcatggc agacaaacaa aagaatggaa tcaaagtgaa cttcaagacc 1620
cgccacaaca ttgaagatgg aagcgttcaa ctagcagacc attatcaaca aaatactcca 1680
attggcgatg gccctgtcct tttaccagac aaccattacc tgtccacaca atctgccctt 1740
tcgaaagatc ccaacgaaaa gagagaccac atggtccttc ttgagtttgt aacagctgct 1800
gggattacac atggcatgga tgaactgtac aactga 1836

<210> 152
<211> 2397
<212> DNA
<213> Human

<400> 152
atggatctga agacagtgct ctccctgccc cgctacccag gggagttcct gcaccccgtg 60
gtgtacgcgt gcacggccgt catgctgctc tgcctcctgg cctccttcgt cacctacatc 120
gtgcaccaga gcgccatccg catcagccgc aagggccggc acacgctcct gaatttctgc 180
ttccacgcgg ccctgacctt cactgtgttc gccggcggca tcaatcgcac caagtacccc 240
atcctgtgcc aggcggtggg catcgtgctg cactattcta cactgtccac catgctgtgg 300
ataggagtga ccgccaggaa catctacaag caggtgacca agaaggcccc tctgtgcctg 360
gacacagacc agccaccgta ccccaggcag cccctgctca ggttttacct cgtcagcgga 420
ggggtcccct ttatcatctg tggggtcacg gctgccacga acatcaggaa ttacgggaca 480
gaggacgagg acacggcgta ctgctggatg gcctgggagc ccagcctggg cgccttctac 540
ggcccagccg ccatcatcac cctggtcacc tgtgtgtact tcctgggcac ctacgtgcag 600
ctgcggcgcc acccagggcg caggtacgag ctgcgcacac agcccgagga gcagcggcgg 660
ctggcgacac ccgagggcgg ccgtgggatc cggccaggca ccccacccgc acacgatgcc 720
cccggcgcct ccgtgctgca gaacgagcac tcattccagg cacagctgcg cgccgccgcc 780
ttcacgctgt tcctgttcac ggccacgtgg gccttcgggg cgctggcggt gtcacagggc 840
cacttcctgg acatggtctt cagctgcctg tacggcgcct tctgcgtgac cctgggactc 900
ttcgtgctca tccaccactg cgccaagcgt gaggacgtgt ggcagtgctg gtgggcatgc 960
tgcccgcccc gcaaggacgc ccaccccgca cttgacgcca acggggccgc gctgggccgc 1020
gccgcctgcc tgcactcgcc gggactgggc cagccacggg gcttcgcgca cccaccgggc 1080
ccctgcaaga tgaccaacct gcaggccgcg cagggccacg ccagttgcct gtcaccggcc 1140
accccgtgct gcgccaagat gcactgcgag ccactgacgg cggacgaggc gcacgtgcac 1200
ctgcaggagg agggcgcctt cgggcacgac ccccacctgc acgggtgcct tcagggcaga 1260
actaagccgc cctactttag ccggcaccca gcagaggagc ccgagtacgc ctaccacatc 1320
ccatccagcc tggatggcag cccccgcagc tcgcgcacag acagcccccc cagctctctg 1380
gatggcccgg cggggacaca cacgctggcc tgctgcaccc agggcgaccc cttccccatg 1440
gtcacccagc ccgagggcag tgatgggagc cctgccctct acagctgccc cacgcagccg 1500
ggcagggagg cagcgctcgg gcccggccac ttggagatgc tgcggaggac acagtccctg 1560
ccctttggtg gccccagcca gaacgggctg cccaagggta aattgctaga aggcctgccg 1620
tttggcaccg acgggaccgg caacatccga acgggaccct ggaaaaacga aactactgtg 1680
gctagcaaag gagaagaact cttcactgga gttgtcccaa ttcttgttga attagatggt 1740
gatgttaacg gccacaagtt ctctgtcagt ggagagggtg aaggtgatgc aacatacgga 1800
aaacttaccc tgaagttcat ctgcactact ggcaaactgc ctgttccatg gccaacacta 1860
gtcactactc tgtgctatgg tgttcaatgc ttttcaagat acccggatca tatgaaacgg 1920
catgactttt tcaagagtgc catgcccgaa ggttatgtac aggaaaggac catcttcttc 1980
aaagatgacg gcaactacaa gacacgtgct gaagtcaagt ttgaaggtga tacccttgtt 2040
aatagaatcg agttaaaagg tattgacttc aaggaagatg gcaacattct gggacacaaa 2100
ttggaataca actataactc acacaatgta tacatcatgg cagacaaaca aaagaatgga 2160
atcaaagtga acttcaagac ccgccacaac attgaagatg gaagcgttca actagcagac 2220
cattatcaac aaaatactcc aattggcgat ggccctgtcc ttttaccaga caaccattac 2280
ctgtccacac aatctgccct ttcgaaagat cccaacgaaa agagagacca catggtcctt 2340
cttgagtttg taacagctgc tgggattaca catggcatgg atgaactgta caactga 2397

<210> 153
<211> 1674
<212> DNA
<213> Human

<400> 153
atgacaaaca gttcgttctt ctgcccagtt tataaagatc tggagccatt cacgtatttt 60
ttttatttag ttttccttgt tggaattatt ggaagttgtt ttgcaacctg ggcttttata 120
cagaagaata cgaatcacag gtgtgtgagc atctacttaa ttaatttgct tacagccgat 180
ttcctgctta ctctggcatt accagtgaaa attgttgttg acttgggtgt ggcaccttgg 240
aagctgaaga tattccactg ccaagtaaca gcctgcctca tctatatcaa tatgtattta 300
tcaattatct tcttagcatt tgtcagcatt gaccgctgtc ttcagctgac acacagctgc 360
aagatctacc gaatacaaga acccggattt gccaaaatga tatcaaccgt tgtgtggcta 420
atggtccttc ttataatggt gccaaatatg atgattccca tcaaagacat caaggaaaag 480
tcaaatgtgg gttgtatgga gtttaaaaag gaatttggaa gaaattggca tttgctgaca 540
aatttcatat gtgtagcaat atttttaaat ttctcagcca tcattttaat atccaattgc 600
cttgtaattc gacagctcta cagaaacaaa gataatgaaa attacccaaa tgtgaaaaag 660
gctctcatca acatactttt agtgaccacg ggctacatca tatgctttgt tccttaccac 720
attgtccgaa tcccgtatac cctcagccag acagaagtca taactgattg ctcaaccagg 780
atttcactct tcaaagccaa agaggctaca ctgctcctgg ctgtgtcgaa cctgtgcttt 840
gatcctatcc tgtactatca cctctcaaaa gcattccgct caaaggtcac tgagactttt 900
gcctcaccta aagagaccaa ggctcagaaa gaaaaattaa gatgtgaaaa taatgcagct 960
agcaaaggag aagaactctt cactggagtt gtcccaattc ttgttgaatt agatggtgat 1020
gttaacggcc acaagttctc tgtcagtgga gagggtgaag gtgatgcaac atacggaaaa 1080
cttaccctga agttcatctg cactactggc aaactgcctg ttccatggcc aacactagtc 1140
actactctgt gctatggtgt tcaatgcttt tcaagatacc cggatcatat gaaacggcat 1200
gactttttca agagtgccat gcccgaaggt tatgtacagg aaaggaccat cttcttcaaa 1260
gatgacggca actacaagac acgtgctgaa gtcaagtttg aaggtgatac ccttgttaat 1320
agaatcgagt taaaaggtat tgacttcaag gaagatggca acattctggg acacaaattg 1380
gaatacaact ataactcaca caatgtatac atcatggcag acaaacaaaa gaatggaatc 1440
aaagtgaact tcaagacccg ccacaacatt gaagatggaa gcgttcaact agcagaccat 1500
tatcaacaaa atactccaat tggcgatggc cctgtccttt taccagacaa ccattacctg 1560
tccacacaat ctgccctttc gaaagatccc aacgaaaaga gagaccacat ggtccttctt 1620
gagtttgtaa cagctgctgg gattacacat ggcatggatg aactgtacaa ctga 1674

<210> 154
<211> 1791
<212> DNA
<213> Human

<400> 154
   atggggttca acttgacgct tgcaaaatta ccaaataacg agctgcacgg ccaagagagt 60
   cacaattcag gcaacaggag cgacgggcca ggaaagaaca ccacccttca caatgaattt 120
   gacacaattg tcttgccggt gctttatctc attatatttg tggcaagcat cttgctgaat 180
   ggtttagcag tgtggatctt cttccacatt aggaataaaa ccagcttcat attctatctc 240
   aaaaacatag tggttgcaga cctcataatg acgctgacat ttccatttcg aatagtccat 300
   gatgcaggat ttggaccttg gtacttcaag tttattctct gcagatacac ttcagttttg 360
   ttttatgcaa acatgtatac ttccatcgtg ttccttgggc tgataagcat tgatcgctat 420
   ctgaaggtgg tcaagccatt tggggactct cggatgtaca gcataacctt cacgaaggtt 480
   ttatctgttt gtgtttgggt gatcatggct gttttgtctt tgccaaacat catcctgaca 540
   aatggtcagc caacagagga caatatccat gactgctcaa aacttaaaag tcctttgggg 600
   gtcaaatggc atacggcagt cacctatgtg aacagctgct tgtttgtggc cgtgctggtg 660
   attctgatcg gatgttacat agccatatcc aggtacatcc acaaatccag caggcaattc 720
   ataagtcagt caagccgaaa gcgaaaacat aaccagagca tcagggttgt tgtggctgtg 780
   tttttaccct gctttctacc atatcacttg tgcagaattc cttttacttt tagtcactta 840
   gacaggcttt tagatgaatc tgcacaaaaa atcctatatt actgcaaaga aattacactt 900
   ttcttgtctg cgtgtaatgt ttgcctggat ccaataattt actttttcat gtgtaggtca 960
   ttttcaagaa ggctgttcaa aaaatcaaat atcagaacca ggagtgaaag catcagatca 1020
   ctgcaaagtg tgagaagatc ggaagttcgc atatattatg attacactga tgtggctagc 1080
   aaaggagaag aactcttcac tggagttgtc ccaattcttg ttgaattaga tggtgatgtt 1140
   aacggccaca agttctctgt cagtggagag ggtgaaggtg atgcaacata cggaaaactt 1200
   accctgaagt tcatctgcac tactggcaaa ctgcctgttc catggccaac actagtcact 1260
   actctgtgct atggtgttca atgcttttca agatacccgg atcatatgaa acggcatgac 1320
   tttttcaaga gtgccatgcc cgaaggttat gtacaggaaa ggaccatctt cttcaaagat 1380
   gacggcaact acaagacacg tgctgaagtc aagtttgaag gtgataccct tgttaataga 1440
   atcgagttaa aaggtattga cttcaaggaa gatggcaaca ttctgggaca caaattggaa 1500
   tacaactata actcacacaa tgtatacatc atggcagaca aacaaaagaa tggaatcaaa 1560
   gtgaacttca agacccgcca caacattgaa gatggaagcg ttcaactagc agaccattat 1620
   caacaaaata ctccaattgg cgatggccct gtccttttac cagacaacca ttacctgtcc 1680
   acacaatctg ccctttcgaa agatcccaac gaaaagagag accacatggt ccttcttgag 1740
   tttgtaacag ctgctgggat tacacatggc atggatgaac tgtacaactg a 1791

<210> 155
<211> 1719
<212> DNA

<213> Human

<400> 155
atgctgggga tcatggcatg gaatgcaact tgcaaaaact ggctggcagc agaggctgcc 60
ctggaaaagt actacctttc catttttat gggattgagt tcgttgtggg agtccttgga 120
aataccattg ttgtttacgg ctacatcttc tctctgaaga actggaacag cagtaatatt 180
tatctcttta acctctctgt ctctgactta gcttttctgt gcaccctccc catgctgata 240
aggagttatg ccaatggaaa ctggatatat ggagacgtgc tctgcataag caaccgatat 300
gtgcttcatg ccaacctcta taccagcatt ctctttctca cttttatcag catagatcga 360
tacttgataa ttaagtatcc tttccgagaa caccttctgc aaaagaaaga gtttgctatt 420
ttaatctcct tggccatttg ggttttagta accttagagt tactacccat acttcccctt 480
ataaatcctg ttataactga caatggcacc acctgtaatg attttgcaag ttctggagac 540
cccaactaca acctcattta cagcatgtgt ctaacactgt tggggttcct tattcctctt 600
tttgtgatgt gtttctttta ttacaagatt gctctcttcc taaagcagag gaataggcag 660
gttgctactg ctctgcccct tgaaaagcct ctcaacttgg tcatcatggc agtggtaatc 720
ttctctgtgc cttttacacc ctatcacgtc atgcggaatg tgaggatcgc ttcacgcctg 780
ggagttgga agcagtatca gtgcactcag gtcgtcatca actcctttta cattgtgaca 840
cggcctttgg cctttctgaa cagtgtcatc aaccctgtct tctattttct tttgggagat 900
cacttcaggg acatgctgat gaatcaactg agacacaact tcaaatccct tacatccttt 960
agcagatggg ctcatgaact cctactttca ttcagagaaa aggctagcaa aggagaagaa 1020
ctcttcactg gagttgtccc aattcttgtt gaattagatg gtgatgttaa cggccacaag 1080
ttctctgtca gtggagaggg tgaaggtgat gcaacatacg gaaaacttac cctgaagttc 1140
atctgcacta ctggcaaact gcctgttcca tggccaacac tagtcactac tctgtgctat 1200
ggtgttcaat gcttttcaag atacccggat catatgaaac ggcatgactt tttcaagagt 1260
gccatgcccg aaggttatgt acaggaaagg accatcttct tcaaagatga cggcaactac 1320
aagacacgtg ctgaagtcaa gtttgaaggt gatacccttg ttaatagaat cgagttaaaa 1380
ggtattgact tcaaggaaga tggcaacatt ctgggacaca aattggaata caactataac 1440
tcacacaatg tatacatcat ggcagacaaa caaaagaatg gaatcaaagt gaacttcaag 1500
acccgccaca acattgaaga tggaagcgtt caactagcag accattatca acaaaatact 1560
ccaattggcg atggccctgt ccttttacca gacaaccatt acctgtccac acaatctgcc 1620
ctttcgaaag atcccaacga aaagagagac cacatggtcc ttcttgagtt tgtaacagct 1680
gctgggatta cacatggcat ggatgaactg tacaactga 1719

<210> 156
<211> 1728
<212> DNA
<213> Human

<400> 156
   atgagagctg tcttcatcca aggtgctgaa gagcaccctg cggcattctg ctaccaggtg 60
   aatgggtctt gccccaggac agtacatact ctgggcatcc agttggtcat ctacctggcc 120
   tgtgcagcag gcatgctgat tatcgtgcta gggaatgtat ttgtggcatt tgctgtgtcc 180
   tacttcaaag cgcttcacac gcccaccaac ttcctgctgc tctccctggc cctggctgac 240
   atgtttctgg gtctgctggt gctgcccctc agcaccattc gctcagtgga gagctgctgg 300
   ttcttcgggg acttcctctg ccgcctgcac acctacctgg acaccctctt ctgcctcacc 360
   tccatcttcc atctctgttt catttccatt gaccgccact gtgccatctg tgaccccctg 420
   ctctatccct ccaagttcac agtgagggtg gctctcaggt acatcctggc aggatggggg 480
   gtgcccgcag catacacttc gttattcctc tacacagatg tggtagagac aaggctcagc 540
   cagtggctgg aagagatgcc ttgtgtgggc agttgccagc tgctgctcaa taaattttgg 600
   ggctggttaa acttcccttt gttctttgtc ccctgcctca ttatgatcag cttgtatgtg 660
   aagatctttg tggttgctac cagacaggct cagcagatta ccacattgag caaaagcctg 720
   gctggggctg ccaagcatga gagaaaagct gccaagaccc tgggcattgc tgtgggcata 780
   tacctcttgt gctggctgcc cttcaccata gacacgatgg tcgacagcct ccttcacttt 840
   atcacacccc cactggtctt tgacatcttt atctggtttg cttacttcaa ctcagcctgc 900
   aaccccatca tctatgtctt ttcctaccag tggtttcgga aggcactgaa actcacactg 960
   agccagaagg tcttctcacc gcagacacgc actgttgatt tgtaccaaga agctagcaaa 1020
   ggagaagaac tcttcactgg agttgtccca attcttgttg aattagatgg tgatgttaac 1080
   ggccacaagt tctctgtcag tggagagggt gaaggtgatg caacatacgg aaaacttacc 1140
   ctgaagttca tctgcactac tggcaaactg cctgttccat ggccaacact agtcactact 1200
   ctgtgctatg gtgttcaatg cttttcaaga tacccggatc atatgaaacg gcatgacttt 1260
   ttcaagagtg ccatgcccga aggttatgta caggaaagga ccatcttctt caaagatgac 1320
   ggcaactaca agacacgtgc tgaagtcaag tttgaaggtg atacccttgt taatagaatc 1380
   gagttaaaag gtattgactt caaggaagat ggcaacattc tgggacacaa attggaatac 1440
   aactataact cacacaatgt atacatcatg gcagacaaac aaaagaatgg aatcaaagtg 1500
   aacttcaaga cccgccacaa cattgaagat ggaagcgttc aactagcaga ccattatcaa 1560
   caaaatactc caattggcga tggccctgtc cttttaccag acaaccatta cctgtccaca 1620
   caatctgccc tttcgaaaga tcccaacgaa aagagagacc acatggtcct tcttgagttt 1680
   gtaacagctg ctgggattac acatggcatg gatgaactgt acaactga 1728

<210> 157
<211> 1743
<212> DNA
<213> Human

<400> 157

atgaccagca atttttccca acctgttgtg cagctttgct atgaggatgt gaatggatct 60
tgtattgaaa ctccctattc tcctgggtcc cgggtaattc tgtacacggc gtttagcttt 120
gggtctttgc tggctgtatt tggaaatctc ttagtaatga cttctgttct tcattttaag 180
cagctgcact ctccaaccaa ttttctcatt gcctctctgg cctgtgctga cttcttggta 240
ggtgtgactg tgatgctttt cagcatggtc aggacggtgg agagctgctg gtattttgga 300
gccaaatttt gtactcttca cagttgctgt gatgtggcat tttgttactc ttctgtcctc 360
cacttgtgct tcatctgcat cgacaggtac attgtggtta ctgatcccct ggtctatgct 420
accaagttca ccgtgtctgt gtcgggaatt tgcatcagcg tgtcctggat tctgcctctc 480
acgtacagcg gtgctgtgtt ctacacaggt gtcaatgatg atgggctgga ggaattagta 540
agtgctctca actgcgtagg tggctgtcaa attattgtaa gtcaaggctg ggtgttgata 600
gattttctgt tattcttcat acctaccctt gttatgataa ttctttacag taagattttt 660
cttatagcta aacaacaagc tataaaaatt gaaactacta gtagcaaagt agaatcatcc 720
tcagagagtt ataaaatcag agtggccaag agagagagga aagcagctaa aaccctgggg 780
gtcacggtac tagcatttgt tatttcatgg ttaccgtata cagttgatat attaattgat 840
gcctttatgg gcttcctgac ccctgcctat atctatgaaa tttgctgttg gagtgcttat 900
tataactcag ccatgaatcc tttgatttat gctctatttt atccttggtt taggaaagcc 960
ataaaactta ttttaagtgg agatgtttta aaggctagtt catcaaccat tagtttattt 1020
ttagaagcta gcaaaggaga agaactcttc actggagttg tcccaattct tgttgaatta 1080
gatggtgatg ttaacggcca caagttctct gtcagtggag agggtgaagg tgatgcaaca 1140
tacggaaaac ttaccctgaa gttcatctgc actactggca aactgcctgt tccatggcca 1200
acactagtca ctactctgtg ctatggtgtt caatgctttt caagataccc ggatcatatg 1260
aaacggcatg actttttcaa gagtgccatg cccgaaggtt atgtacagga aaggaccatc 1320
ttcttcaaag atgacggcaa ctacaagaca cgtgctgaag tcaagtttga aggtgatacc 1380
cttgttaata gaatcgagtt aaaaggtatt gacttcaagg aagatggcaa cattctggga 1440
cacaaattgg aatacaacta taactcacac aatgtataca tcatggcaga caaacaaaag 1500
aatggaatca aagtgaactt caagacccgc cacaacattg aagatggaag cgttcaacta 1560
gcagaccatt atcaacaaaa tactccaatt ggcgatggcc ctgtcctttt accagacaac 1620
cattacctgt ccacacaatc tgccctttcg aaagatccca acgaaaagag agaccacatg 1680
gtccttcttg agtttgtaac agctgctggg attacacatg gcatggatga actgtacaac 1740
tga 1743

<210> 158
<211> 1752
<212> DNA
<213> Human

<400> 158
   atgagcagca attcatccct gctggtggct gtgcagctgt gctacgcgaa cgtgaatggg 60
   tcctgtgtga aaatcccctt ctcgccggga tcccgggtga ttctgtacat agtgtttggc 120
   tttggggctg tgctggctgt gtttggaaac ctcctggtga tgatttcaat cctccatttc 180
   aagcagctgc actctccgac caattttctc gttgcctctc tggcctgcgc tgatttcttg 240
   gtgggtgtga ctgtgatgcc cttcagcatg gtcaggacgg tggagagctg ctggtatttt 300
   gggaggagtt tttgtacttt ccacacctgc tgtgatgtgg cattttgtta ctcttctctc 360
   tttcacttgt gcttcatctc catcgacagg tacattgcgg ttactgaccc cctggtctat 420
   cctaccaagt tcaccgtatc tgtgtcagga atttgcatca gcgtgtcctg gatcctgccc 480
   ctcatgtaca gcggtgctgt gttctacaca ggtgtctatg acgatgggct ggaggaatta 540
   tctgatgccc taaactgtat aggaggttgt cagaccgttg taaatcaaaa ctgggtgttg 600
   acagattttc tatccttctt tatacctacc tttattatga taattctgta tggtaacata 660
   tttcttgtgg ctagacgaca ggcgaaaaag atagaaaata ctggtagcaa gacagaatca 720
   tcctcagaga gttacaaagc cagagtggcc aggagagaga gaaaagcagc taaaaccctg 780
   ggggtcacag tggtagcatt tatgatttca tggttaccat atagcattga ttcattaatt 840
   gatgccttta tgggctttat aacccctgcc tgtatttatg agatttgctg ttggtgtgct 900
   tattataact cagccatgaa tcctttgatt tatgctttat tttacccatg gtttaggaaa 960
   gcaataaaag ttattgtaac tggtcaggtt ttaaagaaca gttcagcaac catgaatttg 1020
   ttttctgaac atatagctag caaaggagaa gaactcttca ctggagttgt cccaattctt 1080
   gttgaattag atggtgatgt taacggccac aagttctctg tcagtggaga gggtgaaggt 1140
   gatgcaacat acggaaaact taccctgaag ttcatctgca ctactggcaa actgcctgtt 1200
   ccatggccaa cactagtcac tactctgtgc tatggtgttc aatgcttttc aagatacccg 1260
   gatcatatga aacggcatga ctttttcaag agtgccatgc ccgaaggtta tgtacaggaa 1320
   aggaccatct tcttcaaaga tgacggcaac tacaagacac gtgctgaagt caagtttgaa 1380
   ggtgataccc ttgttaatag aatcgagtta aaaggtattg acttcaagga agatggcaac 1440
   attctgggac acaaattgga atacaactat aactcacaca atgtatacat catggcagac 1500
   aaacaaaaga atggaatcaa agtgaacttc aagacccgcc acaacattga agatggaagc 1560
   gttcaactag cagaccatta tcaacaaaat actccaattg gcgatggccc tgtcctttta 1620
   ccagacaacc attacctgtc cacacaatct gccctttcga aagatcccaa cgaaaagaga 1680
   gaccacatgg tccttcttga gtttgtaaca gctgctggga ttacacatgg catggatgaa 1740
   ctgtacaact ga 1752

<210> 159
<211> 2556
<212> DNA
<213> Human

<400> 159
atggggccca ccctagcggt tcccaccccc tatggctgta ttggctgtaa gctaccccag 60

ccagaatacc caccggctct aatcatcttt atgttctgcg cgatggttat caccatcgtt 120
gtagacctaa tcggcaactc catggtcatt ttggctgtga cgaagaacaa gaagctccgg 180
aattctggca acatcttcgt ggtcagtctc tctgtggccg atatgctggt ggccatctac 240
ccataccctt tgatgctgca tgccatgtcc attgggggct gggatctgag ccagttacag 300
tgccagatgg tcgggttcat cacagggctg agtgtggtcg gctccatctt caacatcgtg 360
gcaatcgcta tcaaccgtta ctgctacatc tgccacagcc tccagtacga acggatcttc 420
agtgtgcgca atacctgcat ctacctggtc atcacctgga tcatgaccgt cctggctgtc 480
ctgcccaaca tgtacattgg caccatcgag tacgatcctc gcacctacac ctgcatcttc 540
aactatctga acaaccctgt cttcactgtt accatcgtct gcatccactt cgtcctccct 600
ctcctcatcg tgggtttctg ctacgtgagg atctggacca aagtgctggc ggcccgtgac 660
cctgcagggc agaatcctga caaccaactt gctgaggttc gcaattttct aaccatgttt 720
gtgatcttcc tcctctttgc agtgtgctgg tgccctatca acgtgctcac tgtcttggtg 780
gctgtcagtc cgaaggagat ggcaggcaag atccccaact ggctttatct tgcagcctac 840
ttcatagcct acttcaacag ctgcctcaac gctgtgatct acgggctcct caatgagaat 900
ttccgaagag aatactggac catcttccat gctatgcggc accctatcat attcttccct 960
ggcctcatca gtgatattcg tgagatgcag gaggcccgta ccctggcccg cgcccgtgcc 1020
catgctcgcg accaagctcg tgaacaagac cgtgcccatg cctgtcctgc tgtggaggaa 1080
accccgatga atgtccggaa tgttccatta cctggtgatg ctgcagctgg ccaccccgac 1140
cgtgcctctg gccaccctaa gccccattcc agatcctcct ctgcctatcg caaatctgcc 1200
tctacccacc acaagtctgt ctttagccac tccaaggctg cctctggtca cctcaagcct 1260
gtctctggcc actccaagcc tgcctctggt caccccaagt ctgccactgt ctaccctaag 1320
cctgcctctg tccatttcaa gggtgactct gtccatttca agggtgactc tgtccatttc 1380
aagcctgact ctgttcattt caagcctgct tccagcaacc ccaagcccat cactggccac 1440
catgtctctg ctggcagcca ctccaagtct gccttcagtg ctgccaccag ccaccctaaa 1500
cccatcaagc cagctaccag ccatgctgag cccaccactg ctgactatcc caagcctgcc 1560
actaccagcc accctaagcc cgctgctgct gacaaccctg agctctctgc ctcccattgc 1620
cccgagatcc ctgccattgc ccaccctgtg tctgacgaca gtgacctccc tgagtcggcc 1680
tctagccctg ccgctgggcc caccaagcct gctgccagcc agctggagtc tgacaccatc 1740
gctgaccttc ctgaccctac tgtagtcact accagtacca atgattacca tgatgtcgtg 1800
gttgttgatg ttgaagatga tcctgatgaa atggctgtgg ctagcaaagg agaagaactc 1860
ttcactggag ttgtcccaat tcttgttgaa ttagatggtg atgttaacgg ccacaagttc 1920
tctgtcagtg gagagggtga aggtgatgca acatacggaa aacttaccct gaagttcatc 1980
tgcactactg gcaaactgcc tgttccatgg ccaacactag tcactactct gtgctatggt 2040
gttcaatgct tttcaagata cccggatcat atgaaacggc atgacttttt caagagtgcc 2100
atgcccgaag gttatgtaca ggaaaggacc atcttcttca aagatgacgg caactacaag 2160
acacgtgctg aagtcaagtt tgaaggtgat acccttgtta atagaatcga gttaaaaggt 2220
attgacttca aggaagatgg caacattctg ggacacaaat tggaatacaa ctataactca 2280
cacaatgtat acatcatggc agacaaacaa aagaatggaa tcaaagtgaa cttcaagacc 2340
cgccacaaca ttgaagatgg aagcgttcaa ctagcagacc attatcaaca aaatactcca 2400
attggcgatg gccctgtcct tttaccagac aaccattacc tgtccacaca atctgccctt 2460
tcgaaagatc ccaacgaaaa gagagaccac atggtccttc ttgagtttgt aacagctgct 2520
gggattacac atggcatgga tgaactgtac aactga 2556

<210> 160
<211> 2688
<212> DNA
<213> Human

<400> 160
atgaaaatga agtcccaggc aaccatgatt tgctgcttag tgttctttct gtccacagaa 60
tgttcccact atagatccaa gattcaccta aaagctggag ataaacttca aagccctgaa 120
gggaaaccca agactggaag gatccaagag aaatgcgaag gaccttgtat ttcttcttcc 180
aactgcagcc agccctgtgc taaggacttt catggagaaa taggatttac atgtaatcaa 240
aaaaagtggc aaaaatcagc tgaaacatgt acaagccttt ctgtggaaaa actctttaag 300
gactcaactg gtgcatctcg cctttctgta gcagcaccat ctatacctct gcatattcta 360
gactttcgag ctccagagac cattgagagt gtagctcaag gaatccgtaa gaactgcccc 420
tttgattatg cctgcatcac tgacatggtg aaatcatcag aaacaacatc tggaaatatt 480
gcatttatag tggagttatt aaaaaatatt tctacagact tgtctgataa tgttactcga 540
gagaaaatga agagctatag tgaagtggcc aaccacatcc tcgacacagc agccatttca 600
aactgggctt tcattcccaa caaaaatgcc agctcggatt tgttgcagtc agtgaatttg 660
tttgccagac aactccacat ccacaataat tctgagaaca ttgtgaatga actcttcatt 720
cagacaaaag ggtttcacat caaccataat acctcagaga aaagcctcaa tttctccatg 780
agcatgaaca ataccacaga agatatctta ggaatggtac agattcccag gcaagagcta 840
aggaagctgt ggccaaatgc atcccaagcc attagcatag ctttcccaac cttgggggct 900
atcctgagag aagcccactt gcaaaatgtg agtcttccca gacaggtaaa tggtctggtg 960
ctatcagtgg ttttaccaga aaggttgcaa gaaatcatac tcaccttcga aaagatcaat 1020
aaaacccgca atgccagagc ccagtgtgtt ggctggcact ccaagaaaag gagatgggat 1080
gagaaagcgt gccaaatgat gttggatatc aggaacgaag tgaaatgccg ctgtaactac 1140
accagtgtgg tgatgtcttt ttccattctc atgtcctcca aatcgatgac cgacaaagtt 1200
ctggactaca tcacctgcat tgggctcagc gtctcaatcc taagcttggt tctttgcctg 1260
atcattgaag ccacagtgtg gtcccgggtg gttgtgacgg agatatcata catgcgtcac 1320
gtgtgcatcg tgaatatagc agtgtccctt ctgactgcca atgtgtggtt tatcataggc 1380
tctcacttta acattaaggc ccaggactac aacatgtgtg ttgcagtgac atttttcagc 1440
cacttttcct acctctctct gtttttctgg atgctcttca aagcattgct catcatttat 1500
ggaatattgg tcattttccg taggatgatg aagtcccgaa tgatggtcat tggctttgcc 1560

attggctatg ggtgcccatt gatcattgct gtcactacag ttgctatcac agagccagag 1620
aacggctaca tgagacctga ggcctgttgg cttaactggg acaataccaa agccctttta 1680
gcatttgcca tcccggcgtt cgtcattgtg gctgtaaatc tgattgtggt tttggttgtt 1740
gctgtcaaca ctcagaggcc ctctattggc agttccaagt ctcaggatgt ggtcataatt 1800
atgaggatca gcaaaaatgt tgccatcctc actccactgc tgggactgac ctggggtttt 1860
ggaatagcca ctctcataga aggcacttcc ttgacgttcc atataatttt tgccttgctc 1920
aatgctttcc aggtaagttc caagagggag acttttctgt gttactccga cgctagcaaa 1980
ggagaagaac tcttcactgg agttgtccca attcttgttg aattagatgg tgatgttaac 2040
ggccacaagt tctctgtcag tggagagggt gaaggtgatg caacatacgg aaaacttacc 2100
ctgaagttca tctgcactac tggcaaactg cctgttccat ggccaacact agtcactact 2160
ctgtgctatg gtgttcaatg cttttcaaga tacccggatc atatgaaacg gcatgacttt 2220
ttcaagagtg ccatgcccga aggttatgta caggaaagga ccatcttctt caaagatgac 2280
ggcaactaca agacacgtgc tgaagtcaag tttgaaggtg atacccttgt taatagaatc 2340
gagttaaaag gtattgactt caaggaagat ggcaacattc tgggacacaa attggaatac 2400
aactataact cacacaatgt atacatcatg gcagacaaac aaaagaatgg aatcaaagtg 2460
aacttcaaga cccgccacaa cattgaagat ggaagcgttc aactagcaga ccattatcaa 2520
caaaatactc caattggcga tggccctgtc cttttaccag acaaccatta cctgtccaca 2580
caatctgccc tttcgaaaga tcccaacgaa aagagagacc acatggtcct tcttgagttt 2640
gtaacagctg ctgggattac acatggcatg gatgaactgt acaactga 2688

<210> 161
<211> 3447
<212> DNA
<213> Human

<400> 161
   atgaaagttg gagtgctgtg gctcatttct ttcttcacct tcactgacgg ccacggtggc 60
   ttcctgggga aaaatgatgg catcaaaaca aaaaaagaac tcattgtgaa taagaaaaaa 120
   catctaggcc cagtcgaaga atatcagctg ctgcttcagg tgacctatag agattccaag 180
   gagaaaagag atttgagaaa ttttctgaag ctcttgaagc ctccattatt atggtcacat 240
   gggctaatta gaattatcag agcaaaggct accacagact gcaacagcct gaatggagtc 300
   ctgcagtgta cctgtgaaga cagctacacc tggtttcctc cctcatgcct tgatccccag 360
   aactgctacc ttcacacggc tggagcactc ccaagctgtg aatgtcatct caacaacctc 420
   agccagagtg tcaatttctg tgagagaaca aagatttggg gcactttcaa aattaatgaa 480
   aggtttacaa atgacctttt gaattcatct tctgctatat actccaaata tgcaaatgga 540
   attgaaattc aacttaaaaa agcatatgaa agaattcaag gttttgagtc ggttcaggtc 600
   acccaatttc gaaatggaag catcgttgct gggtatgaag ttgttggctc cagcagtgca 660
   tctgaactgc tgtcagccat tgaacatgtt gccgagaagg ctaagacagc ccttcacaag 720
   ctgtttccat tagaagacgg ctctttcaga gtgttcggaa aagcccagtg taatgacatt 780
   gtctttggat ttgggtccaa ggatgatgaa tataccctgc cctgcagcag tggctacagg 840
   ggaaacatca cagccaagtg tgagtcctct gggtggcagg tcatcaggga gacttgtgtg 900
   ctctctctgc ttgaagaact gaacaagaat ttcagtatga ttgtaggcaa tgccactgag 960
   gcagctgtgt catccttcgt gcaaaatctt tctgtcatca ttcggcaaaa cccatcaacc 1020
   acagtgggga atctggcttc ggtggtgtcg attctgagca atatttcatc tctgtcactg 1080
   gccagccatt tcagggtgtc caattcaaca atggaggatg tcatcagtat agctgacaat 1140
   atccttaatt cagcctcagt aaccaactgg acagtcttac tgcgggaaga aaagtatgcc 1200
   agctcacggt tactagagac attagaaaac atcagcactc tggtgcctcc gacagctctt 1260
   cctctgaatt tttctcggaa attcattgac tggaaaggga ttccagtgaa caaaagccaa 1320
   ctcaaaaggg gttacagcta tcagattaaa atgtgtcccc aaaatacatc tattcccatc 1380
   agaggccgtg tgttaattgg gtcagaccaa ttccagagat cccttccaga aactattatc 1440
   agcatggcct cgttgactct ggggaacatt ctacccgttt ccaaaaatgg aaatgctcag 1500
   gtcaatggac ctgtgatatc cacggttatt caaaactatt ccataaatga agttttccta 1560
   tttttttcca agatagagtc aaacctgagc cagcctcatt gtgtgttttg ggatttcagt 1620
   catttgcagt ggaacgatgc aggctgccac ctagtgaatg aaactcaaga catcgtgacg 1680
   tgccaatgta ctcacttgac ctccttctcc atattgatgt caccttttgt cccctctaca 1740
   atcttccccg ttgtaaaatg gatcacctat gtgggactgg gtatctccat tggaagtctc 1800
   attttatgcc tgatcatcga ggctttgttt tggaagcaga ttaaaaaaag ccaaacctct 1860
   cacacacgtc gtatttgcat ggtgaacata gccctgtccc tcttgattgc tgatgtctgg 1920
   tttattgttg gtgccacagt ggacaccacg gtgaaccctt ctggagtctg cacagctgct 1980
   gtgttcttta cacacttctt ctacctctct ttgttcttct ggatgctcat gcttggcatc 2040
   ctgctggctt accggatcat cctcgtgttc catcacatgg cccagcattt gatgatggct 2100
   gttggatttt gcctgggtta tgggtgccct ctcattatat ctgtcattac cattgctgtc 2160
   acgcaaccta gcaataccta caaaaggaaa gatgtgtgtt ggcttaactg gtccaatgga 2220
   agcaaaccac tcctggcttt tgttgtccct gcactggcta ttgtggctgt gaacttcgtt 2280
   gtggtgctgc tagttctcac aaagctctgg aggccgactg ttggggaaag actgagtcgg 2340
   gatgacaagg ccaccatcat ccgcgtgggg aagagcctcc tcattctgac ccctctgcta 2400
   gggctcacct ggggctttgg aataggaaca atagtggaca gccagaatct ggcttggcat 2460
   gttatttttg ctttactcaa tgcattccag ggatttttta tcttatgctt tggaatactc 2520
   ttggacagta agctgcgaca acttctgttc aacaagttgt ctgccttaag ttcttggaag 2580
   caaacagaaa agcaaaactc atcagattta tctgccaaac ccaaattctc aaagcctttc 2640
   aacccactgc aaaacaaagg ccattatgca ttttctcata ctggagattc ctccgacaac 2700
   atcatgctaa ctcagtttgt ctcaaatgaa gctagcaaag gagaagaact cttcactgga 2760
   gttgtcccaa ttcttgttga attagatggt gatgttaacg ggcacaagtt ctctgtcagt 2820
   ggagagggtg aaggtgatgc aacatacgga aaacttaccc tgaagttcat ctgcactact 2880
   ggcaaactgc ctgttccatg gccaacacta gtcactactc tgtgctatgg tgttcaatgc 2940
   ttttcaagat acccggatca tatgaaacgg catgactttt tcaagagtgc catgcccgaa 3000
   ggttatgtac aggaaaggac catcttcttc aaagatgacg gcaactacaa gacacgtgct 3060
   gaagtcaagt ttgaaggtga tacccttgtt aatagaatcg agttaaaagg tattgacttc 3120
   aaggaagatg gcaacattct gggacacaaa ttggaataca actataactc acacaatgta 3180
   tacatcatgg cagacaaaca aaagaatgga atcaaagtga acttcaagac ccgccacaac 3240
   attgaagatg gaagcgttca actagcagac cattatcaac aaaatactcc aattggcgat 3300
   ggccctgtcc ttttaccaga caaccattac ctgtccacac aatctgccct ttcgaaagat 3360
   cccaacgaaa agagagacca catggtcctt cttgagtttg taacagctgc tgggattaca 3420
   catggcatgg atgaactgta caactga 3447

<210> 162
<211> 1929
<212> DNA
<213> Human

<400> 162
atgtcagtga aacccagctg gggcctggc ccctcggagg gggtcaccgc agtgcctacc 60
agtgaccttg gagagatcca caactggacc gagctgcttg acctcttcaa ccacactttg 120
tctgagtgcc acgtggagct cagccagagc accaagcgcg tggtcctctt tgccctctac 180
ctggccatgt ttgtggttgg gctggtggag aacctcctgg tgatatgcgt caactggcgc 240
ggctcaggcc gggcagggct gatgaacctc tacatcctca acatggccat cgcggacctg 300
ggcattgtcc tgtctctgcc cgtgtggatg ctggaggtca cgctggacta cacctggctc 360
tggggcagct tctcctgccg cttcactcac tacttctact ttgtcaacat gtatagcagc 420
atcttcttcc tggtgtgcct cagtgtcgac cgctatgcta ccctcaccag cgcctccccc 480
tcctggcagc gttaccagca ccgagtgcgg cgggccatgt gtgcaggcat ctgggtcctc 540
tcggccatca tcccgctgcc tgaggtggtc cacatccagc tggtggaggg ccctgagccc 600
atgtgcctct tcatggcacc ttttgaaacg tacagcacct gggccctggc ggtggccctg 660
tccaccacca tcctgggctt cctgctgccc ttccctctca tcacagtctt caatgtgctg 720
acagcctgcc ggctgcggca gccaggacaa cccaagagcc ggcgccactg cctgctgctg 780
tgcgcctacg tggccgtctt tgtcatgtgc tggctgccct atcatgtgac cctgctgctg 840
ctcacactgc atgggaccca catctccctc cactgccacc tggtccacct gctctacttc 900
ttctatgatg tcattgactg cttctccatg ctgcactgtg tcatcaaccc catcctttac 960
aactttctca gcccacactt ccggggccgg ctcctgaatg ctgtagtcca ttaccttcct 1020
aaggaccaga ccaaggcggg cacatgcgcc tcctcttcct cctgttccac ccagcattcc 1080
atcatcatca ccaagggtga tagccagcct gctgcagcag ccccccaccc tgagccaagc 1140
ctgagctttc aggcacacca tttgcttcca aatacttccc ccatctctcc cactcagcct 1200
cttacaccca gcgctagcaa aggagaagaa ctcttcactg gagttgtccc aattcttgtt 1260
gaattagatg gtgatgttaa cggccacaag ttctctgtca gtggagaggg tgaaggtgat 1320
gcaacatacg gaaaacttac cctgaagttc atctgcacta ctggcaaact gcctgttcca 1380
tggccaacac tagtcactac tctgtgctat ggtgttcaat gcttttcaag atacccggat 1440
catatgaaac ggcatgactt tttcaagagt gccatgcccg aaggttatgt acaggaaagg 1500
accatcttct tcaaagatga cggcaactac aagacacgtg ctgaagtcaa gtttgaaggt 1560
gataccettg ttaatagaat cgagttaaaa ggtattgact tcaaggaaga tggcaacatt 1620
ctgggacaca aattggaata caactataac tcacacaatg tatacatcat ggcagacaaa 1680
caaaagaatg gaatcaaagt gaacttcaag acccgccaca acattgaaga tggaagcgtt 1740
caactagcag accattatca acaaaatact ccaattggcg atggccctgt ccttttacca 1800
gacaaccatt acctgtccac acaatctgcc ctttcgaaag atcccaacga aaagagagac 1860
cacatggtcc ttcttgagtt tgtaacagct gctgggatta cacatggcat ggatgaactg 1920
tacaactga 1929

<210> 163
<211> 1962
<212> DNA
<213> Human

<400> 163
atggtttttg ctcacagaat ggataacagc aagccacatt tgattattcc tacacttctg 60
gtgcccctcc aaaaccgcag ctgcactgaa acagccacac ctctgccaag ccaatacctg 120
atggaattaa gtgaggagca cagttggatg agcaaccaaa cagaccttca ctatgtgctg 180
aaacccgggg aagtggccac agccagcatc ttctttggga ttctgtggtt gttttctatc 240
ttcggcaatt ccctggtttg tttggtcatc cataggagta ggaggactca gtctaccacc 300
aactactttg tggtctccat ggcatgtgct gaccttctca tcagcgttgc cagcacgcct 360
ttcgtcctgc tccagttcac cactggaagg tggacgctgg gtagtgcaac gtgcaaggtt 420
gtgcgatatt ttcaatatct cactccaggt gtccagatct acgttctcct ctccatctgc 480
atagaccggt tctacaccat cgtctatcct ctgagcttca aggtgtccag agaaaaagcc 540
aagaaaatga ttgcggcatc gtggatcttt gatgcaggct ttgtgacccc tgtgctcttt 600
ttctatggct ccaactggga cagtcattgt aactatttcc tcccctcctc ttgggaaggc 660
actgcctaca ctgtcatcca cttcttggtg ggctttgtga ttccatctgt cctcataatt 720
ttattttacc aaaaggtcat aaaatatatt tggagaatag gcacagatgg ccgaacggtg 780
aggaggacaa tgaacattgt ccctcggaca aaagtgaaaa ctatcaagat gttcctcatt 840
ttaaatctgt tgtttttgct ctcctggctg ccttttcatg tagctcagct atggcacccc 900
catgaacaag actataagaa aagttccctt gttttcacag ctatcacatg gatatccttt 960
agttcttcag cctctaaacc tactctgtat tcaatttata atgccaattt tcggagaggg 1020
atgaaagaga ctttttgcat gtcctctatg aaatgttacc gaagcaatgc ctatactatc 1080
acaacaagtt caaggatggc caaaaaaaac tacgttggca tttcagaaat cccttccatg 1140

gccaaaacta ttaccaaaga ctcgatctat gactcatttg acagagaagc caaggaaaaa 1200
aagcttgctt ggcccattaa ctcaaatcca ccaaatactt ttgtcgctag caaaggagaa 1260
gaactcttca ctggagttgt cccaattctt gttgaattag atggtgatgt taacggccac 1320
aagttctctg tcagtggaga gggtgaaggt gatgcaacat acggaaaact taccctgaag 1380
ttcatctgca ctactggcaa actgcctgtt ccatggccaa cactagtcac tactctgtgc 1440
tatggtgttc aatgcttttc aagatacccg gatcatatga aacggcatga ctttttcaag 1500
agtgccatgc ccgaaggtta tgtacaggaa aggaccatct tcttcaaaga tgacggcaac 1560
tacaagacac gtgctgaagt caagtttgaa ggtgataccc ttgttaatag aatcgagtta 1620
aaaggtattg acttcaagga agatggcaac attctgggac acaaattgga atacaactat 1680
aactcacaca atgtatacat catggcagac aaacaaaaga atggaatcaa agtgaacttc 1740
aagacccgcc acaacattga agatggaagc gttcaactag cagaccatta tcaacaaaat 1800
actccaattg gcgatggccc tgtcctttta ccagacaacc attacctgtc cacacaatct 1860
gccctttcga aagatcccaa cgaaaagaga gaccacatgg tccttcttga gtttgtaaca 1920
gctgctggga ttacacatgg catggatgaa ctgtacaact ga 1962

<210> 164
<211> 1833
<212> DNA
<213> Human

<400> 164
atggcctgca acagcacgtc ccttgaggct tacacatacc tgctgctgaa caccagcaac 60
gcctcagact cggggtccac ccagttgccc gcacccctca ggatctcctt ggccatagtg 120
atgctgctga tgaccgtggt ggggttcctg ggcaacactg tggtctgcat catcgtgtac 180
cagaggccgg ctatgcgctc ggccatcaac ctgctgctgg ccaccctggc cttctccgac 240
atcatgctgt ccctctgctg catgcccttc accgccgtca ccctcatcac cgtgcgctgg 300
cactttgggg accacttctg ccgcctctca gccacgctct actggttttt tgtcctggag 360
ggcgtggcca tcctgctcat catcagcgtg gaccgcttcc tcatcatcgt ccagcgccag 420
gacaagctga acccgcgcag ggccaaggtg atcatcgcgg tctcctgggt gctgtccttc 480
tgcatcgcgg ggccctcgct cacgggctgg acgctggtgg aggtgccggc gcgggcccca 540
cagtgcgtgc tgggctacac ggagctcccc gctgaccgcg cctacgtggt caccttggtg 600
gtggccgtgt tcttcgcgcc ctttggcgtc atgctgtgcg cctacatgtg catcctcaac 660
acggtccgca agaacgccgt gcgcgtgcac aaccagtcgg acagcctgga cctgcggcag 720
ctcaccaggg cgggcctgcg gcgcctgcag cggcagcaac aggtcagcgt ggacttgagc 780
ttcaagacca aggccttcac caccatcctg atcctcttcg tgggcttctc cctctgctgg 840
ctgccccact ccgtctacag cctcctgtct gtgtttagcc agcgctttta ctgcggttcc 900
tccttctacg ccaccagcac ctgcgtcctg tggctcagtt acctcaagtc cgtcttcaac 960
cccatcgtct actgctggag aatcaaaaaa ttccgcgagg cctgcataga gttgctgccc 1020
cagaccttcc aaatcctccc caaagtgcct gagcggatcc gaaggagaat ccagccaagc 1080
acagtctacg tgtgcaatga aaaccagtct gcggttgcta gcaaaggaga agaactcttc 1140
actggagttg tcccaattct tgttgaatta gatggtgatg ttaacggcca caagttctct 1200
gtcagtggag agggtgaagg tgatgcaaca tacggaaaac ttaccctgaa gttcatctgc 1260
actactggca aactgcctgt tccatggcca acactagtca ctactctgtg ctatggtgtt 1320
caatgctttt caagataccc ggatcatatg aaacggcatg actttttcaa gagtgccatg 1380
cccgaaggtt atgtacagga aaggaccatc ttcttcaaag atgacggcaa ctacaagaca 1440
cgtgctgaag tcaagtttga aggtgatacc cttgttaata gaatcgagtt aaaaggtatt 1500
gacttcaagg aagatggcaa cattctggga cacaaattgg aatacaacta taactcacac 1560
aatgtataca tcatggcaga caaacaaaag aatggaatca aagtgaactt caagacccgc 1620
cacaacattg aagatggaag cgttcaacta gcagaccatt atcaacaaaa tactccaatt 1680
ggcgatggcc ctgtcctttt accagacaac cattacctgt ccacacaatc tgccctttcg 1740
aaagatccca acgaaaagag agaccacatg gtccttcttg agtttgtaac agctgctggg 1800
attacacatg gcatggatga actgtacaac tga 1833

<210> 165
<211> 1752
<212> DNA
<213> Human

<400> 165
atgtacaagg actgcatcga gtccactgga gactattttc ttctctgtga cgccgagggg 60
ccatggggca tcattctgga gtccctggcc atacttggca tcgtggtcac aattctgcta 120
ctcttagcat ttctcttcct catgcgaaag atccaagact gcagccagtg gaatgtcctc 180
cccacccagc tcctcttcct cctgagtgtc ctggggctct tcggactcgc ttttgccttc 240
atcatcgagc tcaatcaaca aactgccccc gtacgctact ttctctttgg ggttctcttt 300
gctctctgtt tctcatgcct cttagctcat gcctccaatc tagtgaagct ggttcggggt 360
tgtgtctcct tctcctggac gacaattctg tgcattgcta ttggttgcag tctgttgcaa 420
atcattattg ccactgagta tgtgactctc atcatgacca gaggtatgat gtttgtgaat 480
atgacaccct gccagctcaa tgtggacttt gttgtactcc tggtctatgt cctcttcctg 540
atggccctca cattcttcgt ctccaaagcc accttctgtg gcccgtgtga gaactggaag 600
cagcatggaa ggctcatctt tatcactgtg ctcttctcca tcatcatctg ggtggtgtgg 660
atctccatgc tcctgagagg caaccccgag ttccagcgac agccccagtg ggatgacccg 720
gtcgtctgca ttgctctggt caccaacgca tgggttttcc tgctgctgta catcgtccct 780
gagctctgca ttctctacag atcgtgtaga caggagtgcc ctttacaagg caatgcctgc 840
cccgtcacag cctaccaaca cagcttccaa gtggagaacc aggagctctc cagagcccga 900
gacagtgatg gagctgagga ggatgtagca ttaacttcat atggtactcc cattcagccg 960

cagactgttg atcccacaca agagtgtttc atcccacagg ctaaactaag cccccagcaa 1020
gatgcaggag gagtagctag caaaggagaa gaactcttca ctggagttgt cccaattctt 1080
gttgaattag atggtgatgt taacggccac aagttctctg tcagtggaga gggtgaaggt 1140
gatgcaacat acggaaaact taccctgaag ttcatctgca ctactggcaa actgcctgtt 1200
ccatggccaa cactagtcac tactctgtgc tatggtgttc aatgcttttc aagatacccg 1260
gatcatatga aacggcatga ctttttcaag agtgccatgc ccgaaggtta tgtacaggaa 1320
aggaccatct tcttcaaaga tgacggcaac tacaagacac gtgctgaagt caagtttgaa 1380
ggtgataccc ttgttaatag aatcgagtta aaaggtattg acttcaagga agatggcaac 1440
attctgggac acaaattgga atacaactat aactcacaca atgtatacat catggcagac 1500
aaacaaaaga atggaatcaa agtgaacttc aagacccgcc acaacattga agatggaagc 1560
gttcaactag cagaccatta tcaacaaaat actccaattg gcgatggccc tgtcctttta 1620
ccagacaacc attacctgtc cacacaatct gccctttcga aagatcccaa cgaaaagaga 1680
gaccacatgg tccttcttga gtttgtaaca gctgctggga ttacacatgg catggatgaa 1740
ctgtacaact ga 1752

<210> 166
<211> 3618
<212> DNA
<213> Human

<400> 166
atgcccagcc cgccggggct ccgggcgcta tggctttgcg ccgcgctgtg cgcttcccgg 60
agggccggcg gcgcccccca gccccggcccg gggcccaccg cctgccccggc cccctgccac 120
tgccaggagg acggcatcat gctgtctgcc gactgctctg agctcgggct gtccgccgtt 180
ccgggggacc tggacccccct gacggcttac ctggacctca gcatgaacaa cctcacagag 240
cttcagcctg gcctcttcca ccacctgcgc ttcttggagg agctgcgtct ctctgggaac 300
catctctcac acatcccagg acaagcattc tctggtctct acagcctgaa aatcctgatg 360
ctgcagaaca atcagctggg aggaatcccc gcagaggcgc tgtgggagct gccgagcctg 420
cagtcgctgc gcctagatgc caacctcatc tccctggtcc cggagaggag ctttgagggg 480
ctgtcctccc tccgccacct ctggctggac gacaatgcac tcacggagat ccctgtcagg 540
gccctcaaca acctccctgc cctgcaggcc atgaccctgg ccctcaaccg catcagccac 600
atccccgact acgcgttcca gaatctcacc agccttgtgg tgctgcattt gcataacaac 660
cgcatccagc atctggggac ccacagcttc gaggggctgc acaatctgga gacactagac 720
ctgaattata acaagctgca ggagttccct gtggccatcc ggaccctggg cagactgcag 780
gaactggggt tccataacaa caacatcaag gccatcccag aaaaggcctt catggggaac 840
cctctgctac agacgataca cttttatgat aacccaatcc agtttgtggg aagatcggca 900
ttccagtacc tgcctaaact ccacacacta tctctgaatg gtgccatgga catccaggag 960
tttccagatc tcaaaggcac caccagcctg gagatcctga ccctgacccg cgcaggcatc 1020
cggctgctcc catcggggat gtgccaacag ctgcccaggc tccgagtcct ggaactgtct 1080
cacaatcaaa ttgaggagct gcccagcctg cacaggtgtc agaaattgga ggaaatcggc 1140
ctccaacaca accgcatctg ggaaattgga gctgacacct tcagccagct gagctccctg 1200
caagccctgg atcttagctg gaacgccatc cggtccatcc accccgaggc cttctccacc 1260
ctgcactccc tggtcaagct ggacctgaca gacaaccagc tgaccacact gcccctggct 1320
ggacttgggg gcttgatgca tctgaagctc aaagggaacc ttgctctctc ccaggccttc 1380
tccaaggaca gtttcccaaa actgaggatc ctggaggtgc cttatgccta ccagtgctgt 1440
ccctatggga tgtgtgccag cttcttcaag gcctctgggc agtgggaggc tgaagacctt 1500
caccttgatg atgaggagtc ttcaaaaagg cccctgggcc tccttgccag acaagcagag 1560
aaccactatg accaggacct ggatgagctc cagctggaga tggaggactc aaagccacac 1620
cccagtgtcc agtgtagccc tactccaggc cccttcaagc cctgtgagta cctctttgaa 1680
agctggggca tccgcctggc cgtgtgggcc atcgtgttgc tctccgtgct ctgcaatgga 1740
ctggtgctgc tgaccgtgtt cgctggcggg cctgtccccc tgccccggt caagtttgtg 1800
gtaggtgcga ttgcaggcgc caacaccttg actggcattt cctgtggcct tctagcctca 1860
gtcgatgccc tgacctttgg tcagttctct gagtacggag cccgctggga gacggggcta 1920
ggctgccggg ccactggctt cctggcagta cttgggtcgg aggcatcggt gctgctgctc 1980
actctggccg cagtgcagtg cagcgtctcc gtctcctgtg tccgggccta tgggaagtcc 2040
ccctccctgg gcagcgttcg agcaggggtc ctaggctgcc tggcactggc agggctggcc 2100
gccgcgctgc ccctggcctc agtgggagaa tacggggcct ccccactctg cctgccctac 2160
gcgccacctg agggtcagcc agcagccctg ggcttcaccg tggccctggt gatgatgaac 2220
tccttctgtt tcctggtcgt ggccggtgcc tacatcaaac tgtactgtga cctgccgcgg 2280
ggcgactttg aggccgtgtg ggactgcgcc atggtgaggc acgtggcctg gctcatcttc 2340
gcagacgggc tcctctactg tcccgtggcc ttcctcagct ttgcctccat gctgggcctc 2400
ttccctgtca cgcccgaggc cgtcaagtct gtcctgctgg tggtgctgcc cctgcctgcc 2460
tgcctcaacc cactgctgta cctgctcttc aacccccact tccgggatga ccttcggcgg 2520
cttcggcccc gcgcagggga ctcagggccc ctagcctatg ctgcggccgg ggagctggag 2580
aagagctcct gtgattctac ccaggccctg gtagccttct ctgatgtgga tctcattctg 2640
gaagcttctg aagctgggcg gccccctggg ctggagacct atggcttccc ctcagtgacc 2700
ctcatctcct gtcagcagcc aggggccccc aggctggagg gcagccattg tgtagagcca 2760
gaggggaacc actttgggaa cccccaaccc tccatggatg gagaactgct gctgagggca 2820
gagggatcta cgccagcagg tggaggcttg tcagggggtg gcggctttca gccctctggc 2880
ttggcctttg cttcacacgt ggctagcaaa ggagaagaac tcttcactgg agttgtccca 2940
attcttgttg aattagatgg tgatgttaac ggccacaagt tctctgtcag tggagagggt 3000
gaaggtgatg caacatacgg aaaacttacc ctgaagttca tctgcactac tggcaaactg 3060
cctgttccat ggccaacact agtcactact ctgtgctatg gtgttcaatg cttttcaaga 3120
tacccggatc atatgaaacg gcatgacttt ttcaagagtg ccatgcccga aggttatgta 3180
caggaaagga ccatcttctt caaagatgac ggcaactaca agacacgtgc tgaagtcaag 3240

tttgaaggtg atacccttgt taatagaatc gagttaaaag gtattgactt caaggaagat 3300
ggcaacattc tgggacacaa attggaatac aactataact cacacaatgt atacatcatg 3360
gcagacaaac aaaagaatgg aatcaaagtg aacttcaaga cccgccacaa cattgaagat 3420
ggaagcgttc aactagcaga ccattatcaa caaaatactc caattggcga tggccctgtc 3480
cttttaccag acaaccatta cctgtccaca caatctgccc tttcgaaaga tcccaacgaa 3540
aagagagacc acatggtcct tcttgagttt gtaacagctg ctgggattac acatggcatg 3600
gatgaactgt acaactga 3618

<210> 167
<211> 1722
<212> DNA
<213> Human

<400> 167
atggaatcat ctttctcatt tggagtgatc cttgctgtcc tggcctccct catcattgct 60
actaacacac tagtggctgt ggctgtgctg ctgttgatcc acaagaatga tggtgtcagt 120
ctctgcttca ccttgaatct ggctgtggct gacaccttga ttggtgtggc catctctggc 180
ctactcacag accagctctc cagcccttct cggcccacac agaagaccct gtgcagcctg 240
cggatggcat ttgtcacttc ctccgcagct gcctctgtcc tcacggtcat gctgatcacc 300
tttgacaggt accttgccat caagcagccc ttccgctact tgaagatcat gagtgggttc 360
gtggccgggg cctgcattgc cgggctgtgg ttagtgtctt acctcattgg cttcctccca 420
ctcggaatcc ccatgttcca gcagactgcc tacaaagggc agtgcagctt ctttgctgta 480
tttcaccctc acttcgtgct gaccctctcc tgcgttggct tcttcccagc catgctcctc 540
tttgtcttct tctactgcga catgctcaag attgcctcca tgcacagcca gcagattcga 600
aagatggaac atgcaggagc catggctgga ggttatcgat ccccacggac tcccagcgac 660
ttcaaagctc tccgtactgt gtctgttctc attgggagct ttgctctatc ctggaccccc 720
ttccttatca ctggcattgt gcaggtggcc tgccaggagt gtcacctcta cctagtgctg 780
gaacggtacc tgtggctgct cggcgtgggc aactccctgc tcaacccact catctatgcc 840
tattggcaga aggaggtgcg actgcagctc taccacatgg ccctaggagt gaagaaggtg 900
ctcacctcat tcctcctctt tctctcggcc aggaattgtg gcccagagag gcccagggaa 960
agttcctgtc acatcgtcac tatctccagc tcagagtttg atggcgctag caaaggagaa 1020
gaactcttca ctggagttgt cccaattctt gttgaattag atggtgatgt taacggccac 1080
aagttctctg tcagtggaga gggtgaaggt gatgcaacat acggaaaact taccctgaag 1140
ttcatctgca ctactggcaa actgcctgtt ccatggccaa cactagtcac tactctgtgc 1200
tatggtgttc aatgcttttc aagatacccg gatcatatga aacggcatga ctttttcaag 1260
agtgccatgc ccgaaggtta tgtacaggaa aggaccatct tcttcaaaga tgacggcaac 1320
tacaagacac gtgctgaagt caagtttgaa ggtgataccc ttgttaatag aatcgagtta 1380
aaaggtattg acttcaagga agatggcaac attctgggac acaaattgga atacaactat 1440
aactcacaca atgtatacat catggcagac aaacaaaaga atggaatcaa agtgaacttc 1500
aagacccgcc acaacattga agatggaagc gttcaactag cagaccatta tcaacaaaat 1560
actccaattg gcgatggccc tgtcctttta ccagacaacc attacctgtc cacacaatct 1620
gccctttcga aagatcccaa cgaaaagaga gaccacatgg tccttcttga gtttgtaaca 1680
gctgctggga ttacacatgg catggatgaa ctgtacaact ga 1722

<210> 168
<211> 1839
<212> DNA
<213> Human

<400> 168
atgctgacag ggagctgcgg ggaccctcag aaaaagccac aggtgaccca ggactcaggg 60
ccccagagca tggggcttga gggacgagag acagctggcc agccacgagt gaccctgctg 120
cccacgccca acgtcagcgg gctgagccag gagtttgaaa gccactggcc agagatcgca 180
gagaggtccc cgtgtgtggc tggcgtcatc cctgtcatct actacagtgt cctgctgggc 240
ttggggctgc ctgtcagcct cctgaccgca gtggccctgg cgcgccttgc caccaggacc 300
aggaggccct cctactacta ccttctggcg ctcacagcct cggatatcat catccaggtg 360
gtcatcgtgt tcgcgggctt cctcctgcag ggagcagtgc tggcccgcca ggtgccccag 420
gctgtggtgc gcacggccaa catcctggag tttgctgcca accacgcctc agtctggatc 480
gccatcctgc tcacggttga ccgctacact gccctgtgcc accccctgca ccatcgggcc 540
gcctcgtccc caggccggac ccgccgggcc attgctgctg tcctgagtgc tgccctgttg 600
accggcatcc ccttctactg gtggctggac atgtggagag acaccgactc acccagaaca 660
ctggacgagg tcctcaagtg ggctcactgt ctcactgtct atttcatccc ttgtggcgtg 720
ttcctggtca ccaactcggc catcatccac cggctacgga ggagggccgg gagtgggctg 780
cagccccggg tgggcaagag cacagccatc ctcctgggca tcaccacact gttcaccctc 840
ctgtgggcgc cccgggtctt cgtcatgctc taccacatgt acgtggcccc tgtccaccgg 900
gactggaggg tccacctggc cttggatgtg gccaatatgg tggccatgct ccacacggca 960
gccaacttcg gcctctactg ctttgtcagc aagactttcc gggccactgt ccgacaggtc 1020
atccacgatg cctacctgcc ctgcactttg gcatcacagc cagagggcat ggcggcgaag 1080
cctgtgatgg agcctccggg actccccaca ggggcagaag tggctagcaa aggagaagaa 1140
ctcttcactg gagttgtccc aattcttgtt gaattagatg gtgatgttaa cggccacaag 1200
ttctctgtca gtggagaggg tgaaggtgat gcaacatacg gaaaacttac cctgaagttc 1260
atctgcacta ctggcaaact gcctgttcca tggccaacac tagtcactac tctgtgctat 1320
ggtgttcaat gcttttcaag atacccggat catatgaaac ggcatgactt tttcaagagt 1380
gccatgcccg aaggttatgt acaggaaagg accatcttct tcaaagatga cggcaactac 1440
aagacacgtg ctgaagtcaa gtttgaaggt gatacccttg ttaatagaat cgagttaaaa 1500

ggtattgact tcaaggaaga tggcaacatt ctgggacaca aattggaata caactataac 1560
tcacacaatg tatacatcat ggcagacaaa caaaagaatg gaatcaaagt gaacttcaag 1620
acccgccaca acattgaaga tggaagcgtt caactagcag accattatca acaaaatact 1680
ccaattggcg atggccctgt ccttttacca gacaaccatt acctgtccac acaatctgcc 1740
ctttcgaaag atcccaacga aaagagagac cacatggtcc ttcttgagtt tgtaacagct 1800
gctgggatta cacatggcat ggatgaactg tacaactga 1839

<210> 169
<211> 1836
<212> DNA
<213> Human

<400> 169
atggacaccc tggaggaggt gacttgggcc aatgggagca cagcgctacc cccacccctg 60
gcaccaaaca tcagtgtgcc tcatcgctgc ctgctgctgc tctacgaaga cattggcacc 120
tccagggtcc ggtactggga cctcttgctg ctcatcccca atgtgctctt cctcatcttc 180
ctgctctgga agcttccatc tgctcgggcg aagatccgca tcacctccag ccccattttt 240
atcaccttct acatcctggt gtttgtggtg gcgctggtgg gcattgcccg ggccgtggta 300
tccatgacgg tgagcacctc gaacgctgca actgttgctg ataagatcct gtgggagatc 360
acccgcttct tcctgctggc catcgagctg agtgtgatca tcctgggcct ggcctttggc 420
cacctggaga gtaagtccag catcaagcgg gtgctggcca tcaccacagt gctgtccctg 480
gcctactctg tcacccaggg gaccctggag atcctgtacc ctgatgccca tctctcagct 540
gaggacttta atatctatgg ccatgggggc cgccagttct ggctggtcag ctcctgcttc 600
ttcttcctgg tctactctct ggtggtcatc cttcccaaga ccccgctgaa ggagcgcatc 660
tccctgcctt ctcggaggag cttctacgtg tatgcgggca tcctggcact gctcaaccta 720
ctgcaggggc tggggagtgt gctgctgtgc ttcgacatca tcgaggggct ctgctgtgta 780
gatgccacaa ccttcctgta cttcagcttc ttcgctccgc tcatctacgt ggctttcctc 840
cggggcttct tcggctcgga gcccaagatc ctcttctcct acaaatgcca agtggacgag 900
acagaggagc cagatgtaca cctaccccag ccctacgctg tggcccggcg ggagggcctg 960
gaggctgcag ggctgctggg ggcctcagct gccagctact cgagcacgca gttcgactct 1020
gccggcgggg tggcctacct ggatgacatc gcttccatgc cctgccacac tggcagcatc 1080
aacagcacag acagcgagcg ctggaaggcc atcaatgccg ctagcaaagg agaagaactc 1140
ttcactggag ttgtcccaat tcttgttgaa ttagatggtg atgttaacgg ccacaagttc 1200
tctgtcagtg gagagggtga aggtgatgca acatacggaa aacttaccct gaagttcatc 1260
tgcactactg gcaaactgcc tgttccatgg ccaacactag tcactactct gtgctatggt 1320
gttcaatgct tttcaagata cccggatcat atgaaacggc atgacttttt caagagtgcc 1380
atgcccgaag gttatgtaca ggaaaggacc atcttcttca aagatgacgg caactacaag 1440
acacgtgctg aagtcaagtt tgaaggtgat acccttgtta atagaatcga gttaaaaggt 1500
attgacttca aggaagatgg caacattctg ggacacaaat tggaatacaa ctataactca 1560
cacaatgtat acatcatggc agacaaacaa aagaatggaa tcaaagtgaa cttcaagacc 1620
cgccacaaca ttgaagatgg aagcgttcaa ctagcagacc attatcaaca aaatactcca 1680
attggcgatg gccctgtcct tttaccagac aaccattacc tgtccacaca atctgccctt 1740
tcgaaagatc ccaacgaaaa gagagaccac atggtccttc ttgagtttgt aacagctgct 1800
gggattacac atggcatgga tgaactgtac aactga 1836

<210> 170
<211> 2016
<212> DNA
<213> Human

<400> 170
atgtgttttt ctcccattct ggaaatcaac atgcagtctg aatctaacat tacagtgcga 60
gatgacattg atgacatcaa caccaatatg taccaaccac tatcatatcc gttaagcttt 120
caagtgtctc tcaccggatt tcttatgtta gaaattgtgt tgggacttgg cagcaacctc 180
actgtattgg tactttactg catgaaatcc aacttaatca actctgtcag taacattatt 240
acaatgaatc ttcatgtact tgatgtaata atttgtgtgg gatgtattcc tctaactata 300
gttatccttc tgctttcact ggagagtaac actgctctca tttgctgttt ccatgaggct 360
tgtgtatctt ttgcaagtgt ctcaacagca atcaacgttt ttgctatcac tttggacaga 420
tatgacatct ctgtaaaacc tgcaaaccga attctgacaa tgggcagagc tgtaatgtta 480
atgatatcca tttggatttt ttcttttttc tctttcctga ttccttttat tgaggtaaat 540
tttttcagtc ttcaaagtgg aaatacctgg gaaaacaaga cacttttatg tgtcagtaca 600
aatgaatact acactgaact gggaatgtat tatcacctgt tagtacagat cccaatattc 660
tttttcactg ttgtagtaat gttaatcaca tacaccaaaa tacttcaggc tcttaatatt 720
cgaataggca caagattttc aacagggcag aagaagaaag caagaaagaa aaagacaatt 780
tctctaacca cacaacatga ggctacagac atgtcacaaa gcagtggtgg gagaaatgta 840
gtctttggtg taagaacttc agtttctgta ataattgccc tccggcgagc tgtgaaacga 900
caccgtgaac gacgagaaag acaaaagaga gtcttcagga tgtctttatt gattatttct 960
acatttcttc tctgctggac accaatttct gttttaaata ccaccatttt atgtttaggc 1020
ccaagtgacc ttttagtaaa attaagattg tgttttttag tcatggctta tggaacaact 1080
atatttcacc ctctattata tgcattcact agacaaaaat ttcaaaaggt cttgaaaagt 1140
aaaatgaaaa agcgagttgt ttctatagta gaagctgatc ccctgcctaa taatgctgta 1200
atacacaact cttggataga tcctaaaaga aacaaaaaaa ttacctttga agatagtgaa 1260
ataagagaaa aatgtttagt gcctcaggtt gtcacagacg ctagcaaagg agaagaactc 1320
ttcactggag ttgtcccaat tcttgttgaa ttagatggtg atgttaacgg ccacaagttc 1380
tctgtcagtg gagagggtga aggtgatgca acatacggaa aacttaccct gaagttcatc 1440

tgcactactg gcaaactgcc tgttccatgg ccaacactag tcactactct gtgctatggt 1500
gttcaatgct tttcaagata cccggatcat atgaaacggc atgacttttt caagagtgcc 1560
atgcccgaag gttatgtaca ggaaaggacc atcttcttca aagatgacgg caactacaag 1620
acacgtgctg aagtcaagtt tgaaggtgat acccttgtta atagaatcga gttaaaaggt 1680
attgacttca aggaagatgg caacattctg ggacacaaat tggaatacaa ctataactca 1740
cacaatgtat acatcatggc agacaaacaa aagaatggaa tcaaagtgaa cttcaagacc 1800
cgccacaaca ttgaagatgg aagcgttcaa ctagcagacc attatcaaca aaatactcca 1860
attggcgatg gccctgtcct tttaccagac aaccattacc tgtccacaca atctgccctt 1920
tcgaaagatc ccaacgaaaa gagagaccac atggtccttc ttgagtttgt aacagctgct 1980
gggattacac atggcatgga tgaactgtac aactga 2016

<210> 171
<211> 1800
<212> DNA
<213> Human

<400> 171
atgaatgaat ccaggtggac tgaatggagg atcctgaaca tgagcagtgg cattgtgaat 60
gtgtccgagc gtcactcctg cccacttgga tttggccact acagtgtggt ggatgtctgc 120
atcttcgaga cagtggttat tgtgttgctg acatttctga tcattgctgg gaatctaaca 180
gttatctttg tctttcattg tgctccactg ttacatcatt atactaccag ctatttcatt 240
cagacgatgg catatgctga tcttttcgtt ggagttagct gcttggttcc tactctgtca 300
cttctccact actccacagg tgtccacgag tcattgactt gccaggtttt tggatatatc 360
atctcagttc taaaaagtgt ttctatggca tgtcttgctt gcatcagtgt ggatcgttat 420
cttgcaataa ccaagcctct ttcctacaat caactggtca ccccttgtcg cttgagaatt 480
tgcattattt tgatctggat ctactcctgc ctaattttct tgccttcctt ttttggctgg 540
gggaaacctg gttaccatgg tgacattttt gaatggtgtg ccacgtcttg gctcaccagt 600
gcctatttta ctggctttat tgtttgttta ctttatgctc ctgctgcctt tgttgtctgc 660
ttcacttact tccacatttt caaaatttgc cgtcagcaca ccaaagagat aaatgaccga 720
agagcccgat tccctagtca tgaggtagat tcttccagag agactggaca cagccctgac 780
cgtcgctacg ccatggtttt gtttaggata accagtgtat tttatatgct gtggctcccc 840
tatataattt actttcttct agaaagctcc cgggtcttgg acaatccaac tctgtccttc 900
ttaacaacct ggcttgcaat aagtaatagt ttttgtaact gtgtaatata cagcctctcc 960
aacagcgttt tccggctagg cctccgaaga ctgtctgaga caatgtgcac atcctgtatg 1020
tgtgtgaagg atcaggaagc acaagaaccc aaacctagga aacgggctaa ttcttgctcc 1080
attgctagca aaggagaaga actcttcact ggagttgtcc caattcttgt tgaattagat 1140
ggtgatgtta acggccacaa gttctctgtc agtggagagg gtgaaggtga tgcaacatac 1200
ggaaaactta ccctgaagtt catctgcact actggcaaac tgcctgttcc atggccaaca 1260
ctagtcacta ctctgtgcta tggtgttcaa tgcttttcaa gatacccgga tcatatgaaa 1320
cggcatgact ttttcaagag tgccatgccc gaaggttatg tacaggaaag gaccatcttc 1380
ttcaaagatg acggcaacta caagacacgt gctgaagtca agtttgaagg tgataccctt 1440
gttaatagaa tcgagttaaa aggtattgac ttcaaggaag atggcaacat tctgggacac 1500
aaattggaat acaactataa ctcacacaat gtatacatca tggcagacaa acaaaagaat 1560
ggaatcaaag tgaacttcaa gacccgccac aacattgaag atggaagcgt tcaactagca 1620
gaccattatc aacaaaatac tccaattggc gatggccctg tccttttacc agacaaccat 1680
tacctgtcca cacaatctgc cctttcgaaa gatcccaacg aaaagagaga ccacatggtc 1740
cttcttgagt ttgtaacagc tgctgggatt acacatggca tggatgaact gtacaactga 1800

<210> 172
<211> 1728
<212> DNA
<213> Human

<400> 172
atgaactcgt gggacgcggg cctggcgggg ctactggtgg gcacgatggg cgtctcgctg 60
ctgtccaacg cgctggtgct gctctgcctg ctgcacagcg cggacatccg ccgccaggcg 120
ccggcgctct tcaccctgaa cctcacgtgc gggaacttgc tgtgcaccgt ggtcaacatg 180
ccgctcacgc tggccggcgt cgtggcgcag cggcagccgg cgggcgaccg cctgtgccgc 240
ctggctgcct tcctcgacac cttcctggct gccaactcca tgctcagcat ggccgcgctc 300
agcatcgacc gctgggtggc cgtggtcttc ccgctgagct accgggccaa gatgcgcctc 360
cgcgacgcgg cgctcatggt ggcctacacg tggctgcacg cgctcacctt cccagccgcc 420
gcgctcgccc tgtcctggct cggcttccac cagctgtacg cctcgtgcac gctgtgcagc 480
cggcggccgg acgagcgcct gcgcttcgcc gtcttcactg gcgccttcca cgctctcagc 540
ttcctgctct ccttcgtcgt gctctgctgc acgtacctca aggtgctcaa ggtggcccgc 600
ttccattgca agcgcatcga cgtgatcacc atgcagacgc tcgtgctgct ggtggacctg 660
caccccagtg tgcgggaacg ctgtctggag gagcagaagc ggaggcgaca gcgagccacc 720
aagaagatca gcaccttcat agggaccttc cttgtgtgct tcgcgcccta tgtgatcacc 780
aggctagtgg agctcttctc cacggtgccc atcggctccc actggggggt gctgtccaag 840
tgcttggcgt acagcaaggc cgcatccgac ccctttgtgt actccttact gcgacaccag 900
taccgcaaaa gctgcaagga gattctgaac aggctcctgc acagacgctc catccactcc 960
tctggcctca caggcgactc tcacagccag aacattctgc cggtgtctga ggctagcaaa 1020
ggagaagaac tcttcactgg agttgtccca attcttgttg aattagatgg tgatgttaac 1080
ggccacaagt tctctgtcag tggagagggt gaaggtgatg caacatacgg aaaacttacc 1140
ctgaagttca tctgcactac tggcaaactg cctgttccat ggccaacact agtcactact 1200
ctgtgctatg gtgttcaatg cttttcaaga tacccggatc atatgaaacg gcatgacttt 1260

ttcaagagtg ccatgccoga aggttatgta caggaaagga ccatcttctt caaagatgac 1320
ggcaactaca agacacgtgc tgaagtcaag tttgaaggtg ataccottgt taatagaatc 1380
gagttaaaag gtattgactt caaggaagat ggcaacattc tgggacacaa attggaatac 1440
aactataact cacacaatgt atacatcatg gcagacaaac aaaagaatgg aatcaaagtg 1500
aacttcaaga cccgccacaa cattgaagat ggaagcgttc aactagcaga ccattatcaa 1560
caaaatactc caattggcga tggccctgtc cttttaccag acaaccatta cctgtccaca 1620
caatctgccc tttcgaaaga tcccaacgaa aagagagacc acatggtcct tcttgagttt 1680
gtaacagctg ctgggattac acatggcatg gatgaactgt acaactga 1728

<210> 173
<211> 2184
<212> DNA
<213> Human

<400> 173
   atgaaccctc cttcggggcc aagagtcctg cccagcccaa cccaagagcc cagctgcatg 60
   gccaccccag caccacccag ctggtgggac agctcccaga gcagcatctc cagcctgggc 120
   cggcttccat ccatcagtcc cacagcacct gggacttggg ctgctgcctg ggtccccctc 180
   cccacggttg atgttccgga ccatgcccac tataccctgg gcacagtgat cttgctggtg 240
   ggactcacgg ggatgctggg caacctgacg gtcatctata ccttctgcag agctgtgctt 300
   cgtggagtca ctgtgatgat gcagagcaga agcctccgga cacctgccaa catgttcatt 360
   atcaacctcg cggtcagcga cttcctcatg tccttcaccc aggcccctgt cttcttcacc 420
   agtagcctct ataagcagtg gctctttggg gagacaggct gcgagttcta tgccttctgt 480
   ggagctctct ttggcatttc ctccatgatc accctgacgg ccatcgccct ggaccgctac 540
   ctggtaatca cacgcccgct ggccaccttt ggtgtggcgt ccaagaggcg tgcggcattt 600
   gtcctgctgg gcgtttggct ctatgccctg gcctggagtc tgccaccctt cttcggctgg 660
   agcgcctacg tgcccgaggg gttgctgaca tcctgctcct gggactacat gagcttcacg 720
   ccggccgtgc gtgcctacac catgcttctc tgctgcttcg tgttcttcct ccctctgctt 780
   atcatcatct actgctacat cttcatcttc agggccatcc gggagacagg acgggctctc 840
   cagaccttcg gggcctgcaa gggcaatggc gagtccctgt ggcagcggca gcggctgcag 900
   agcgagtgca agatggccaa gatcatgctg ctggtcatcc tcctcttcgt gctctcctgg 960
   gctccctatt ccgctgtggc cctggtggcc tttgctgggt acgcacacgt cctgacaccc 1020
   tacatgagct cggtgccagc cgtcatcgcc aaggcctctg caatccacaa ccccatcatt 1080
   tacgccatca cccaccccaa gtacagggtg gccattgccc agcacctgcc ctgcctgggg 1140
   gtgctgctgg gtgtatcacg ccggcacagt cgcccctacc ccagctaccg ctccacccac 1200
   cgctccacgc tgatcagcca cacctccaac ctcagctgga tctccatacg gaggcgccag 1260
   gagtccctgg gctcggagag tgaggtgggc tggacacaca tggaggcagc agctgtgtgg 1320
   ggagctgccc agcaagcaaa tgggcggtcc ctctacggtc agggtctgga ggacttggaa 1380
   gccaaggcac cccccagacc ccagggacac gaagcagaga ctccagggaa gaccaagggg 1440
   ctgatcccca gccaggaccc caggatggct agcaaaggag aagaactctt cactggagtt 1500
   gtcccaattc ttgttgaatt agatggtgat gttaacggcc acaagttctc tgtcagtgga 1560
   gagggtgaag gtgatgcaac atacggaaaa cttaccctga agttcatctg cactactggc 1620
   aaactgcctg ttccatggcc aacactagtc actactctgt gctatggtgt tcaatgcttt 1680
   tcaagatacc cggatcatat gaaacggcat gactttttca agagtgccat gcccgaaggt 1740
   tatgtacagg aaaggaccat cttcttcaaa gatgacggca actacaagac acgtgctgaa 1800
   gtcaagtttg aaggtgatac ccttgttaat agaatcgagt taaaaggtat tgacttcaag 1860
   gaagatggca acattctggg acacaaattg gaatacaact ataactcaca caatgtatac 1920
   atcatggcag acaaacaaaa gaatggaatc aaagtgaact tcaagacccg ccacaacatt 1980
   gaagatggaa gcgttcaact agcagaccat tatcaacaaa atactccaat tggcgatggc 2040
   cctgtccttt taccagacaa ccattacctg tccacacaat ctgccctttc gaaagatccc 2100
   aacgaaaaga gagaccacat ggtccttctt gagtttgtaa cagctgctgg gattacacat 2160
   ggcatggatg aactgtacaa ctga 2184

<210> 174
<211> 1851
<212> DNA
<213> Human

<400> 174
atggtctggg ggaaaatttg ctggttcagc cagagggctg gatggacagt gtttgctgag 60
tcacagatat ctctctcatg tagcctttgt ctccacagtg gtgaccagga ggcacagaac 120
ccaaacctgg tatctcagct ctgtggcgtc tttcttcaaa atgagacgaa tgaaaccata 180
catatgcaga tgagcatggc agtgggacag caggccctgc ccttgaatat cattgccccc 240
aaggctgtgc tggtctccct ctgtggggtc ttattgaatg gcactgtctt ctggctgctt 300
tgctgtgggg ccacgaatcc ctacatggta tacatcctcc acctggtcgc tgctgacgtg 360
atctatcttt gctgctcggc agtggggttc ttacaggtga ctctgctaac ttatcatgga 420
gtcgtgtttt ttatccctga tttcctggcc atattgtctc ccttctcctt tgaggtgtgt 480
ctctgtctcc tggtggccat cagcacagag cggtgtgtgt gtgtcctctt ccccatctgg 540
tacagatgcc accgcccaaa atacacatct aatgttgtct gcaccctcat ctgggggctg 600
cctttttgca tcaacatagt aaaatcactt ttcctaactt actggaaaca tgtaaaggca 660
tgtgtcatat ttctaaagct ttctgggctc ttccatgcta tcctttcact tgtgatgtgt 720
gtgtcgagtc tgactctact cattagattc ctgtgctgct cccagcagca aaaggccacc 780
agggtctatg cggtggtgca gatctcggcc cccatgttcc tactctgggc cctacccctg 840
agcgtggcac ccctcataac agatttcaaa atgtttgtca ccacctccta tttaatttcc 900
ttgttcctca ttataaacag cagcgccaac cctatcattt atttctttgt ggggagcctc 960

agaaagaaaa ggctgaagga atctctcaga gtgattctcc aacgggcgtt agcagataag 1020
ccagaggtgg ggaggaacaa aaaggcagct ggcatcgacc caatggagca accacactct 1080
actcagcatg tggagaacct tcttcccagg gagcacaggg tcgatgtgga aacagctagc 1140
aaaggagaag aactcttcac tggagttgtc ccaattcttg ttgaattaga tggtgatgtt 1200
aacggccaca agttctctgt cagtggagag ggtgaaggtg atgcaacata cggaaaactt 1260
accctgaagt tcatctgcac tactggcaaa ctgcctgttc catggccaac actagtcact 1320
actctgtgct atggtgttca atgcttttca agatacccgg atcatatgaa acggcatgac 1380
tttttcaaga gtgccatgcc cgaaggttat gtacaggaaa ggaccatctt cttcaaagat 1440
gacggcaact acaagacacg tgctgaagtc aagtttgaag gtgataccct tgttaataga 1500
atcgagttaa aaggtattga cttcaaggaa gatggcaaca ttctgggaca caaattggaa 1560
tacaactata actcacacaa tgtatacatc atggcagaca aacaaaagaa tggaatcaaa 1620
gtgaacttca agacccgcca caacattgaa gatggaagcg ttcaactagc agaccattat 1680
caacaaaata ctccaattgg cgatggccct gtccttttac cagacaacca ttacctgtcc 1740
acacaatctg ccctttcgaa agatcccaac gaaaagagag accacatggt ccttcttgag 1800
tttgtaacag ctgctgggat tacacatggc atggatgaac tgtacaactg a 1851

<210> 175
<211> 1827
<212> DNA
<213> Human

<400> 175
atggcgaact atagccatgc agctgacaac attttgcaaa atctctcgcc tctaacagcc 60
tttctgaaac tgacttcctt gggtttcata ataggagtca gcgtggtggg caacctcctg 120
atctccattt tgctagtgaa agataagacc ttgcatagag caccttacta cttcctgttg 180
gatctttgct gttcagatat cctcagatct gcaatttgtt tcccatttgt gttcaactct 240
gtcaaaaatg gctctacctg gacttatggg actctgactt gcaaagtgat tgcctttctg 300
gggtttttgt cctgtttcca cactgctttc atgctcttct gcatcagtgt caccagatac 360
ttagctatcg cccatcaccg cttctataca aagaggctga ccttttggac gtgtctggct 420
gtgatctgta tggtgtggac tctgtctgtg gccatggcat ttcccccggt tttagacgtg 480
ggcacttact cattcattag ggaggaagat caatgcacct tccaacaccg ctccttcagg 540
gctaatgatt ccttaggatt tatgctgctt cttgctctca tcctcctagc cacacagctt 600
gtctacctca agctgatatt tttcgtccac gatcgaagaa aaatgaagcc agtccagttt 660
gtagcagcag tcagccagaa ctggactttt catggtcctg gagccagtgg ccaggcagct 720
gccaattggc tagcaggatt tggaaggggt cccacaccac ccaccttgct gggcatcagg 780
caaaatgcaa acaccacagg cagaagaagg ctattggtct tagacgagtt caaaatggag 840
aaaagaatca gcagaatgtt ctatataatg acttttctgt ttctaacctt gtggggcccc 900
tacctggtgg cctgttattg gagagttttt gcaagagggc ctgtagtacc agggggattt 960
ctaacagctg ctgtctggat gagttttgcc caagcaggaa tcaatccttt tgtctgcatt 1020
ttctcaaaca gggagctgag gcgctgtttc agcacaaccc ttctttactg cagaaaatcc 1080
aggttaccaa gggaacctta ctgtgttata tctagaaaag gagaagaact cttcactgga 1140
gttgtcccaa ttcttgttga attagatggt gatgttaacg gccacaagtt ctctgtcagt 1200
ggagagggtg aaggtgatgc aacatacgga aaacttaccc tgaagttcat ctgcactact 1260
ggcaaactgc ctgttccatg gccaacacta gtcactactc tgtgctatgg tgttcaatgc 1320
ttttcaagat acccggatca tatgaaacgg catgactttt tcaagagtgc catgcccgaa 1380
ggttatgtac aggaaaggac catcttcttc aaagatgacg gcaactacaa gacacgtgct 1440
gaagtcaagt ttgaaggtga tacccttgtt aatagaatcg agttaaaagg tattgacttc 1500
aaggaagatg gcaacattct gggacacaaa ttggaataca actataactc acacaatgta 1560
tacatcatgg cagacaaaca aaagaatgga atcaaagtga acttcaagac ccgccacaac 1620
attgaagatg gaagcgttca actagcagac cattatcaac aaaatactcc aattggcgat 1680
ggccctgtcc ttttaccaga caaccattac ctgtccacac aatctgccct ttcgaaagat 1740
cccaacgaaa agagagacca catggtcctt cttgagtttg taacagctgc tgggattaca 1800
catggcatgg atgaactgta caactga 1827

<210> 176
<211> 1719
<212> DNA
<213> Human

<400> 176
   atgaatgaag acctgaaggt caatttaagc gggctgcctc gggattattt agatgccgct 60
   gctgcggaga acatctcggc tgctgtctcc tcccgggttc ctgccgtaga gccagagcct 120
   gagctcgtag tcaacccctg ggacattgtc ttgtgtacct cgggaaccct catctcctgt 180
   gaaaatgcca ttgtggtcct tatcatcttc cacaacccca gcctgcgagc acccatgttc 240
   ctgctaatag gcagcctggc tcttgcagac ctgctggccg gcattggact catcaccaat 300
   tttgtttttg cctacctgct tcagtcagaa gccaccaagc tggtcacgat cggcctcatt 360
   gtcgcctctt tctctgcctc tgtctgcagc ttgctggcta tcactgttga ccgctacctc 420
   tcactgtact acgctctgac gtaccattcg gagaggacgg tcacgtttac ctatgtcatg 480
   ctcgtcatgc tctgggggac ctccatctgc ctggggctgc tgcccgtcat gggctggaac 540
   tgcctccgag acgagtccac ctgcagcgtg gtcagaccgc tcaccaagaa caacgcggcc 600
   atcctctcgg tgtccttcct cttcatgttt gcgctcatgc ttcagctcta catccagatc 660
   tgtaagattg tgatgaggca cgcccatcag atagccctgc agcaccactt cctggccacg 720
   tcgcactatg tgaccacccg gaaaggggtc tccaccctgg ctatcatcct ggggacgttt 780
   gctgcttgct ggatgccttt caccctctat tccttgatag cggattacac ctacccctcc 840
   atctatacct acgccaccct cctgcccgcc acctacaatt ccatcatcaa ccctgtcata 900
   tatgctttca gaaaccaaga gatccagaaa gcgctctgtc tcatttgctg cggctgcatc 960
   ccgtccagtc tcgcccagag agcgcgctcg cccagtgatg tggctagcaa aggagaagaa 1020
   ctcttcactg gagttgtccc aattcttgtt gaattagatg gtgatgttaa cggccacaag 1080
   ttctctgtca gtggagaggg tgaaggtgat gcaacatacg gaaaacttac cctgaagttc 1140
   atctgcacta ctggcaaact gcctgttcca tggccaacac tagtcactac tctgtgctat 1200
   ggtgttcaat gcttttcaag atacccggat catatgaaac ggcatgactt tttcaagagt 1260
   gccatgcccg aaggttatgt acaggaaagg accatcttct tcaaagatga cggcaactac 1320
   aagacacgtg ctgaagtcaa gtttgaaggt gatacccttg ttaatagaat cgagttaaaa 1380
   ggtattgact tcaaggaaga tggcaacatt ctgggacaca aattggaata caactataac 1440
   tcacacaatg tatacatcat ggcagacaaa caaaagaatg gaatcaaagt gaacttcaag 1500
   acccgccaca acattgaaga tggaagcgtt caactagcag accattatca acaaaatact 1560
   ccaattggcg atggccctgt ccttttacca gacaaccatt acctgtccac acaatctgcc 1620
   ctttcgaaag atcccaacga aaagagagac cacatggtcc ttcttgagtt tgtaacagct 1680
   gctgggatta cacatggcat ggatgaactg tacaactga 1719

<210> 177
<211> 1842
<212> DNA
<213> Human

<400> 177
atggatgtga cttcccaagc ccggggcgtg ggcctggaga tgtacctagg caccgcgcag 60
cctgcggccc ccaacaccac ctcccccgag ctcaacctgt cccacccgct cctgggcacc 120
gccctggcca atgggacagg tgagctctcg gagcaccagc agtacgtgat cggcctgttc 180
ctctcgtgcc tctacaccat cttcctcttc cccatcggct ttgtgggcaa catcctgatc 240
ctggtggtga acatcagctt ccgcgagaag atgaccatcc ccgacctgta cttcatcaac 300
ctggcggtgg cggacctcat cctggtggcc gactccctca ttgaggtgtt caacctgcac 360
gagcggtact acgacatcgc cgtcctgtgc accttcatgt cgctcttcct gcaggtcaac 420
atgtacagca gcgtcttctt cctcacctgg atgagcttcg accgctacat cgccctggcc 480
agggccatgc gctgcagcct gttccgcacc aagcaccacg cccggctgag ctgtggcctc 540
atctggatgg catccgtgtc agccacgctg gtgcccttca ccgccgtgca cctgcagcac 600
accgacgagg cctgcttctg tttcgcggat gtccgggagg tgcagtggct cgaggtcacg 660
ctgggcttca tcgtgccctt cgccatcatc ggcctgtgct actccctcat tgtccgggtg 720
ctggtcaggg cgcaccggca ccgtgggctg cggccccggc ggcagaaggc gctccgcatg 780
atcctcgcgg tggtgctggt cttcttcgtc tgctggctgc cggagaacgt cttcatcagc 840
gtgcacctcc tgcagcggac gcagcctggg gccgctccct gcaagcagtc tttccgccat 900
gcccaccccc tcacgggcca cattgtcaac ctcgccgcct tctccaacag ctgcctaaac 960
cccctcatct acagctttct cggggagacc ttcagggaca agctgaggct gtacattgag 1020
cagaaaacaa atttgccggc cctgaaccgc ttctgtcacg ctgccctgaa ggccgtcatt 1080
ccagacagca ctgagcagtc ggatgtgagg ttcagcagtg ccgtggctag caaaggagaa 1140
gaactcttca ctggagttgt cccaattctt gttgaattag atggtgatgt taacggccac 1200
aagttctctg tcagtggaga gggtgaaggt gatgcaacat acggaaaact taccctgaag 1260
ttcatctgca ctactggcaa actgcctgtt ccatggccaa cactagtcac tactctgtgc 1320
tatggtgttc aatgcttttc aagatacccg gatcatatga aacggcatga ctttttcaag 1380
agtgccatgc ccgaaggtta tgtacaggaa aggaccatct tcttcaaaga tgacggcaac 1440
tacaagacac gtgctgaagt caagtttgaa ggtgataccc ttgttaatag aatcgagtta 1500
aaaggtattg acttcaagga agatggcaac attctgggac acaaattgga atacaactat 1560
aactcacaca atgtatacat catggcagac aaacaaaaga atggaatcaa agtgaacttc 1620
aagacccgcc acaacattga agatggaagc gttcaactag cagaccatta tcaacaaaat 1680
actccaattg gcgatggccc tgtcctttta ccagacaacc attacctgtc cacacaatct 1740
gccctttcga aagatcccaa cgaaaagaga gaccacatgg tccttcttga gtttgtaaca 1800
gctgctggga ttacacatgg catggatgaa ctgtacaact ga 1842

<210> 178
<211> 1725
<212> DNA
<213> Human

<400> 178
atgaacaaca atacaacatg tattcaacca tctatgatct cttccatggc tttaccaatc 60
atttacatcc tcctttgtat tgttggtgtt tttggaaaca ctctctctca atggatattt 120
ttaacaaaaa taggtaaaaa aacatcaacg cacatctacc tgtcacacct tgtgactgca 180
aacttacttg tgtgcagtgc catgcctttc atgagtatct atttcctgaa aggtttccaa 240
tgggaatatc aatctgctca atgcagagtg gtcaattttc tgggaactct atccatgcat 300
gcaagtatgt ttgtcagtct cttaatttta agttggattg ccataagccg ctatgctacc 360
ttaatgcaaa aggattcctc gcaagagact acttcatgct atgagaaaat attttatggc 420
catttactga aaaaatttcg ccagcccaac tttgctagaa aactatgcat ttacatatgg 480
ggagttgtac tgggcataat cattccagtt accgtatact actcagtcat agaggctaca 540
gaaggagaag agagcctatg ctacaatcgg cagatggaac taggagccat gatctctcag 600
attgcaggtc tcattggaac cacatttatt ggattttcct ttttagtagt actaacatca 660
tactactctt ttgtaagcca tctgagaaaa ataagaacct gtacgtccat tatggagaaa 720
gatttgactt acagttctgt gaaaagacat cttttggtca tccagattct actaatagtt 780
tgcttccttc cttatagtat ttttaaaccc attttttatg ttctacacca aagagataac 840
tgtcagcaat tgaattattt aatagaaaca aaaaacattc tcacctgtct tgcttcggcc 900
agaagtagca cagaccccat tatatttctt ttattagaca aaacattcaa gaagacacta 960

tataatctct ttacaaagtc taattcagca catatgcaat catatggtgc tagcaaagga 1020
gaagaactct tcactggagt tgtcccaatt cttgttgaat tagatggtga tgttaacggc 1080
cacaagttct ctgtcagtgg agagggtgaa ggtgatgcaa catacggaaa acttaccctg 1140
aagttcatct gcactactgg caaactgcct gttccatggc caacactagt cactactctg 1200
tgctatggtg ttcaatgctt ttcaagatac ccggatcata tgaaacggca tgactttttc 1260
aagagtgcca tgcccgaagg ttatgtacag gaaaggacca tcttcttcaa agatgacggc 1320
aactacaaga cacgtgctga agtcaagttt gaaggtgata cccttgttaa tagaatcgag 1380
ttaaaaggta ttgacttcaa ggaagatggc aacattctgg gacacaaatt ggaatacaac 1440
tataactcac acaatgtata catcatggca gacaaacaaa agaatggaat caaagtgaac 1500
ttcaagaccc gccacaacat tgaagatgga agcgttcaac tagcagacca ttatcaacaa 1560
aatactccaa ttggcgatgg ccctgtcctt ttaccagaca accattacct gtccacacaa 1620
tctgcccttt cgaaagatcc caacgaaaag agagaccaca tggtccttct tgagtttgta 1680
acagctgctg ggattacaca tggcatggat gaactgtaca actga 1725

<210> 179
<211> 3186
<212> DNA
<213> Human

<400> 179
   atgggaggcc gcgtctttct cgtctttctc gcattctgtg tctggctgac tctgccggga 60
   gctgaaaccc aggactccag ggctgtgccc cggtggtgcc ctcaggactc ctcgtgtgtc 120
   aatgccaccg cctgtcgctg caatccaggg ttcagctctt tttctgagat catcaccacc 180
   cccatggaga cttgtgacga catcaacgag tgtgcaacac tgtcgaaagt gtcatgcgga 240
   aaattctcgg actgctggaa cacagagggg agctacgact gcgtgtgcag cccaggatat 300
   gagcctgttt ctggggcaaa aacattcaag aatgagagcg agaacacgtg tcaagatgtg 360
   gacgaatgtc agcagaaccc aaggctctgt aaaagctacg gcacctgcgt caacacccct 420
   ggcagctata cctgccagtg cctgcctggc ttcaagttca tacctgagga tccgaaggtc 480
   tgcacagatg tgaatgaatg cacctccgga caaaacccat gccacagctc cacccactgc 540
   ctcaacaacg tgggcagcta tcagtgccgc tgccgcccgg gctggcaacc gattccgggg 600
   tcccccaatg gccccaacaa taccgtctgt gaagatgtgg acgagtgcag ctccgggcag 660
   catcagtgtg acagctccac cgtctgcttc aacaccgtgg gttcatacag ctgccgctgc 720
   cgcccaggct ggaagcccag acacggaatc ccgaataacc aaaaggacac tgtctgtgaa 780
   gatatgactt tctccacctg gaccccgccc cctggagtcc acagccagac gctttcccga 840
   ttcttcgaca aagtccagga cctgggcaga gactacaagc caggcttggc caataacacc 900
   atccagagca tcttacaggc gctggatgag ctgctggagg tccctgggga cctggagacc 960
   ctgccccgct tacagcagca ctgtgtggcc agtcacctgc tggatggcct agaggatgtc 1020
   ctcagaggcc tgagcaagaa cctttccaat gggctgttga acttcagtta tcctgcaggc 1080
   acagaattgt ccctggaggt gcagaagcaa gtagacagga gtgtcacctt gagacagaat 1140
   caggcagtga tgcagctcga ctggaatcag gcacagaaat ctggtgaccc aggcccttct 1200
   gtggtgggcc ttgtctccat tccagggatg ggcaagttgc tggctgaggc ccctctggtc 1260
   ctggaacctg agaagcagat gcttctgcat gagacacacc agggcttgct gcaggacggc 1320
   tcccccatcc tgctctcaga tgtgatctct gcctttctga gcaacaacga cacccaaaac 1380
   ctcagctccc cagttacctt caccttctcc caccgttcag tgatcccgag acagaaggtg 1440
   ctctgtgtct tctgggagca tggccagaat ggatgtggtc actgggccac cacaggctgc 1500
   agcacaatag gcaccagaga caccagcacc atctgccgtt gcacccacct gagcagcttt 1560
   gccgtcctca tggcccacta cgatgtgcag gaggaggatc ccgtgctgac tgtcatcacc 1620
   tacatggggc tgagcgtctc tctgctgtgc ctcctcctgg cggccctcac ttttctcctg 1680
   tgtaaagcca tccagaacac cagcacctca ctgcatctgc agctctcgct ctgcctcttc 1740
   ctggcccacc tcctcttcct cgtggcaatt gatcaaaccg gacacaaggt gctgtgctcc 1800
   atcatcgccg gtaccttgca ctatctctac ctggccacct tgacctggat gctgctggag 1860
   gccctgtacc tcttcctcac tgcacggaac ctgacggtgg tcaactactc aagcatcaac 1920
   agattcatga agaagctcat gttccctgtg ggctacggag tcccagctgt gacagtggcc 1980
   atttctgcag cctccaggcc tcacctttat ggaacacctt cccgctgctg gctccaacca 2040
   gaaaagggat ttatatgggg cttccttgga cctgtctgcg ccatcttctc tgtgaattta 2100
   gttctctttc tggtgactct ctggattttg aaaaacagac tctcctccct caatagtgaa 2160
   gtgtccaccc tccggaacac aaggatgctg gcatttaaag cgacagctca gctgttcatc 2220
   ctgggctgca cgtggtgtct gggcatcttg caggtgggtc cggctgcccg ggtcatggcc 2280
   tacctcttca ccatcatcaa cagcctgcag ggtgtcttca tcttcctggt gtactgcctc 2340
   ctcagccagc aggtccggga gcaatatggg aaatggtcca aagggatcag gaaattgaaa 2400
   actgagtctg agatgcacac actctccagc agtgctaagg ctgacacctc caaacccagc 2460
   acggttaacg ctagcaaagg agaagaactc ttcactggag ttgtcccaat tcttgttgaa 2520
   ttagatggtg atgttaacgg ccacaagttc tctgtcagtg gagagggtga aggtgatgca 2580
   acatacggaa aacttaccct gaagttcatc tgcactactg gcaaactgcc tgttccatgg 2640
   ccaacactag tcactactct gtgctatggt gttcaatgct tttcaagata cccggatcat 2700
   atgaaacggc atgacttttt caagagtgcc atgcccgaag gttatgtaca ggaaaggacc 2760
   atcttcttca aagatgacgg caactacaag acacgtgctg aagtcaagtt tgaaggtgat 2820
   acccttgtta atagaatcga gttaaaaggt attgacttca aggaagatgg caacattctg 2880
   ggacacaaat tggaatacaa ctataactca cacaatgtat acatcatggc agacaaacaa 2940
   aagaatggaa tcaaagtgaa cttcaagacc cgccacaaca ttgaagatgg aagcgttcaa 3000
   ctagcagacc attatcaaca aaatactcca attggcgatg gccctgtcct tttaccagac 3060
   aaccattacc tgtccacaca atctgccctt tcgaaagatc ccaacgaaaa gagagaccac 3120
   atggtccttc ttgagtttgt aacagctgct gggattacac atggcatgga tgaactgtac 3180
   aactga 3186

<210> 180
<211> 2262
<212> DNA
<213> Human

<400> 180
atgggacata acgggagctg gatctctcca aatgccagcg agccgcacaa cgcgtccggc 60
gccgaggctg cgggtgtgaa ccgcagcgcg ctcggggagt tcggcgaggc gcagctgtac 120
cgccagttca ccaccaccgt gcaggtcgtc atcttcatag gctcgctgct cggaaacttc 180
atggtgttat ggtcaacttg ccgcacaacc gtgttcaaat ctgtcaccaa caggttcatt 240
aaaaacctgg cctgctcggg gatttgtgcc agcctggtct gtgtgccctt cgacatcatc 300
ctcagcacca gtcctcactg ttgctggtgg atctacacca tgctcttctg caaggtcgtc 360
aaatttttgc acaaagtatt ctgctctgtg accatcctca gcttccctgc tattgctttg 420
gacaggtact actcagtcct ctatccactg gagaggaaaa tatctgatgc caagtcccgt 480
gaactggtga tgtacatctg ggcccatgca gtggtggcca gtgtccctgt gtttgcagta 540
accaatgtgg ctgacatcta tgccacgtcc acctgcacgg aagtctggag caactccttg 600
ggccacctgg tgtacgttct ggtgtataac atcaccacgg tcattgtgcc tgtggtggtg 660
gtgttcctct tcttgatact gatccgacgg gccctgagtg ccagccagaa gaagaaggtc 720
atcatagcag cgctccggac cccacagaac accatctcta ttccctatgc ctcccagcgg 780
gaggccgagc tgcacgccac cctgctctcc atggtgatgg tcttcatctt gtgtagcgtg 840
ccctatgcca ccctggtcgt ctaccagact gtgctcaatg tccctgacac ttccgtcttc 900
ttgctgctca ctgctgtttg gctgcccaaa gtctccctgc tggcaaaccc tgttctcttt 960
cttactgtga acaaatctgt ccgcaagtgc ttgataggga ccctggtgca actacaccac 1020
cggtacagtc gccgtaatgt ggtcagtaca gggagtggca tggctgaggc cagcctggaa 1080
cccagcatac gctcgggtag ccagctcctg gagatgttcc acattgggca gcagcagatc 1140
tttaagccca cagaggatga ggaagagagt gaggccaagt acattggctc agctgacttc 1200
caggccaagg agatatttag cacctgcctg gagggagagc aggggccaca gtttgcgccc 1260
tctgccccac ccctgagcac agtggactct gtatcccagg tggcaccggc agcccctgtg 1320
gaacctgaaa cattccctga taagtattcc ctgcagtttg gctttgggcc ttttgagttg 1380
cctcctcagt ggctctcaga gacccgaaac agcaagaagc ggctgcttcc ccccttgggc 1440
aacaccccag aagagctgat ccagacaaag gtgcccaagg taggcagggt ggagcggaag 1500
atgagcagaa acaataaagt gagcattttt ccaaaggtgg attccgctag caaaggagaa 1560
gaactcttca ctggagttgt cccaattctt gttgaattag atggtgatgt taacggccac 1620
aagttctctg tcagtggaga gggtgaaggt gatgcaacat acggaaaact taccctgaag 1680
ttcatctgca ctactggcaa actgcctgtt ccatggccaa cactagtcac tactctgtgc 1740
tatggtgttc aatgcttttc aagatacccg gatcatatga aacggcatga ctttttcaag 1800
agtgccatgc ccgaaggtta tgtacaggaa aggaccatct tcttcaaaga tgacggcaac 1860
tacaagacac gtgctgaagt caagtttgaa ggtgataccc ttgttaatag aatcgagtta 1920
aaaggtattg acttcaagga agatggcaac attctgggac acaaattgga atacaactat 1980
aactcacaca atgtatacat catggcagac aaacaaaaga atggaatcaa agtgaacttc 2040
aagacccgcc acaacattga agatggaagc gttcaactag cagaccatta tcaacaaaat 2100
actccaattg gcgatggccc tgtcctttta ccagacaacc attacctgtc cacacaatct 2160
gccctttcga aagatcccaa cgaaaagaga gaccacatgg tccttcttga gtttgtaaca 2220
gctgctggga ttacacatgg catggatgaa ctgtacaact ga 2262

<210> 181
<211> 1803
<212> DNA
<213> Human

<400> 181
atggatctgc acctcttcga ctacgccgag ccagggaact tctcggacat cagctggcca 60
tgcaacagca gcgactgcat cgtggtggac acggtgatgt gtcccaacat gcccaacaaa 120
agcgtcctgc tctacacgct ctccttcatt tacattttca tcttcgtcat cggcatgatt 180
gccaactccg tggtggtctg ggtgaatatc caggccaaga ccacaggcta tgacacgcac 240
tgctacatct tgaacctggc cattgccgac ctgtgggttg tcctcaccat cccagtctgg 300
gtggtcagtc tcgtgcagca caaccagtgg cccatgggcg agctcacgtg caaagtcaca 360
cacctcatct tctccatcaa cctcttcggc agcattttct tcctcacgtg catgagcgtg 420
gaccgctacc tctccatcac ctacttcacc aacaccccca gcagcaggaa gaagatggta 480
cgccgtgtcg tctgcatcct ggtgtggctg ctggccttct gcgtgtctct gcctgacacc 540
tactacctga agaccgtcac gtctgcgtcc aacaatgaga cctactgccg gtccttctac 600
cccgagcaca gcatcaagga gtggctgatc ggcatggagc tggtctccgt tgtcttgggc 660
tttgccgttc ccttctccat tatcgctgtc ttctacttcc tgctggccag agccatctcg 720
gcgtccagtg accaggagaa gcacagcagc cggaagatca tcttctccta cgtggtggtc 780
ttccttgtct gctggctgcc ctaccacgtg gcggtgctgc tggacatctt ctccatcctg 840
cactacatcc ctttcacctg ccggctggag cacgccctct tcacggccct gcatgtcaca 900
cagtgcctgt cgctggtgca ctgctgcgtc aaccctgtcc tctacagctt catcaatcgc 960
aactacaggt acgagctgat gaaggccttc atcttcaagt actcggccaa aacagggctc 1020
accaagctca tcgatgcctc cagagtctca gagacggagt actctgcctt ggagcagagc 1080
accaaagcta gcaaaggaga agaactcttc actggagttg tcccaattct tgttgaatta 1140
gatggtgatg ttaacggcca caagttctct gtcagtggag agggtgaagg tgatgcaaca 1200
tacggaaaac ttaccctgaa gttcatctgc actactggca aactgcctgt tccatggcca 1260
acactagtca ctactctgtg ctatggtgtt caatgctttt caagataccc ggatcatatg 1320
aaacggcatg actttttcaa gagtgccatg cccgaaggtt atgtacagga aaggaccatc 1380
ttcttcaaag atgacggcaa ctacaagaca cgtgctgaag tcaagtttga aggtgatacc 1440

cttgttaata gaatcgagtt aaaaggtatt gacttcaagg aagatggcaa cattctggga 1500
cacaaattgg aatacaacta taactcacac aatgtataca tcatggcaga caaacaaaag 1560
aatggaatca aagtgaactt caagacccgc cacaacattg aagatggaag cgttcaacta 1620
gcagaccatt atcaacaaaa tactccaatt ggcgatggcc ctgtcctttt accagacaac 1680
cattacctgt ccacacaatc tgccctttcg aaagatccca acgaaaagag agaccacatg 1740
gtccttcttg agtttgtaac agctgctggg attacacatg gcatggatga actgtacaac 1800
tga 1803

<210> 182
<211> 2019
<212> DNA
<213> Human

<400> 182
   atggaggatc tctttagccc ctcaattctg ccgccggcgc ccaacatttc cgtgcccatc 60
   ttgctgggct ggggtctcaa cctgaccttg gggcaaggag cccctgcctc tggaccgccc 120
   agccgccgcg tccgcctggt gttcctgggg gtcatcctgg tggtggcggt ggcaggcaac 180
   accacagtgc tgtgccgcct gtgcggcggc ggcgggccct gggcgggccc caagcgtcgc 240
   aagatggact tcctgctggt gcagctggcc ctggcggacc tgtacgcgtg cgggggcacg 300
   gcgctgtcac agctggcctg ggaactgctg ggcgagcccc gcgcggccac gggggacctg 360
   gcgtgccgct tcctgcagct gctacaggca tccgggcggg gcgcctcggc ccacctcgtg 420
   gtgctcatcg ccctcgagcg ccggcgcgcg gtgcgtcttc cgcacggccg gccgctgccc 480
   gcgcgtgccc tcgccgccct gggctggctg ctggcactgc tgctggcgct gcccccggcc 540
   ttcgtggtgc gcggggactc cccctcgccg ctgccgccgc cgccgccgcc aacgtccctg 600
   cagccaggcg cgcccccggc cgcccgcgcc tggccggggc agcgtcgctg ccacggcatc 660
   ttcgcgcccc tgccgcgctg gcacctgcag gtctacgcgt tctacgaggc cgtcgcgggc 720
   ttcgtcgcgc ctgttacggt cctgggcgtc gcttgcggcc acctactctc cgtctggtgg 780
   cggcaccggc cgcaggcccc cgcggctgca gcgccctggt cggcgagccc aggtcgagcc 840
   cctgcgccca gcgcgctgcc ccgcgccaag gtgcagagcc tgaagatgag cctgctgctg 900
   gcgctgctgt tcgtgggctg cgagctgccc tactttgccg cccggctggc ggccgcgtgg 960
   tcgtccgggc ccgcgggaga ctgggaggga gagggcctgt cggcggcgct gcgcgtggtg 1020
   gcgatggcca acagcgctct caatcccttc gtctacctct tcttccaggc gggcgactgc 1080
   cggctccggc gacagctgcg gaagcggctg ggctctctgt gctgcgcgcc gcagggaggc 1140
   gcggaggacg aggagggccc ccggggccac caggcgctct accgccaacg ctggccccac 1200
   cctcattatc accatgctcg gcgggaaccg ctggacgagg gcggcttgcg cccaccccct 1260
   ccgcgcccca gacccctgcc ttgctcctgc gaaagtgcct tcgctagcaa aggagaagaa 1320
   ctcttcactg gagttgtccc aattcttgtt gaattagatg gtgatgttaa cggccacaag 1380
   ttctctgtca gtggagaggg tgaaggtgat gcaacatacg gaaaacttac cctgaagttc 1440
   atctgcacta ctggcaaact gcctgttcca tggccaacac tagtcactac tctgtgctat 1500
   ggtgttcaat gcttttcaag atacccggat catatgaaac ggcatgactt tttcaagagt 1560
   gccatgcccg aaggttatgt acaggaaagg accatcttct tcaaagatga cggcaactac 1620
   aagacacgtg ctgaagtcaa gtttgaaggt gatacccttg ttaatagaat cgagttaaaa 1680
   ggtattgact tcaaggaaga tggcaacatt ctgggacaca aattggaata caactataac 1740
   tcacacaatg tatacatcat ggcagacaaa caaaagaatg gaatcaaagt gaacttcaag 1800
   acccgccaca acattgaaga tggaagcgtt caactagcag accattatca acaaaatact 1860
   ccaattggcg atggccctgt ccttttacca gacaaccatt acctgtccac acaatctgcc 1920
   ctttcgaaag atcccaacga aaagagagac cacatggtcc ttcttgagtt tgtaacagct 1980
   gctgggatta cacatggcat ggatgaactg tacaactga 2019

<210> 183
<211> 1629
<212> DNA
<213> Human

<400> 183
   atgctgagca ctggggtggt gagcttcttc tccctcaagt cggactcggc gcccccctgg 60
   atggtgctgg ctgtgctgtg gtgctccatg gcacagacgc tgctgctgcc ctccttcatc 120
   tggtcctgcg agcgctaccg cgccgacgtg cgcacagtgt gggagcaatg cgtggccatc 180
   atgtctgagg aggatggaga tgacgatggg ggctgtgacg actatgcaga gggccgagtt 240
   tgcaaagttc gctttgatgc taacggagcc acaggaccag ggagccggga ccccgcccag 300
   gtgaagctgc tgcctggaag gcacatgctc ttccctcctc ttgagagagt ccactactta 360
   caggtccccc tatcccggcg tctgtcccat gatgagacaa acatcttctc tacccctcgg 420
   gaaccaggct ccttcctgca caagtggtca tcctctgatg acatccgggt cctcccagcc 480
   cagagccggg ccctcggggg tcctcctgag tacctgggac aaagacacag gttggaggac 540
   gaggaggacg aggaagaggc tgaaggtggg ggctggccca gccttcgcca attcttggag 600
   agtggggttc tggggtcagg tgggggaccc ccacggggtc ctggcttctt ccgggaggag 660
   atcaccacct tcatcgatga gacacctctg ccttctccga ctgcctcacc agggcactct 720
   cctcgtcggc cccggccact gggcctctca ccccgccgac tctcccttgg gtcccctgag 780
   agcagagccg ttggacttcc tttgggacta agcgcaggga gacgctgctc cctgacgggg 840
   ggtgaagaaa gtgcaagggc ttggggagga tcctgggccc caggcaaccc catctttccc 900
   cagctgaccc tggctagcaa aggagaagaa ctcttcactg gagttgtccc aattcttgtt 960
   gaattagatg gtgatgttaa cggccacaag ttctctgtca gtggagaggg tgaaggtgat 1020
   gcaacatacg gaaaacttac cctgaagttc atctgcacta ctggcaaact gcctgttcca 1080
   tggccaacac tagtcactac tctgtgctat ggtgttcaat gcttttcaag atacccggat 1140
   catatgaaac ggcatgactt tttcaagagt gccatgcccg aaggttatgt acaggaaagg 1200
   accatcttct tcaaagatga cggcaactac aagacacgtg ctgaagtcaa gtttgaaggt 1260
   gatacccttg ttaatagaat cgagttaaaa ggtattgact tcaaggaaga tggcaacatt 1320
   ctgggacaca aattggaata caactataac tcacacaatg tatacatcat ggcagacaaa 1380
   caaaagaatg gaatcaaagt gaacttcaag acccgccaca acattgaaga tggaagcgtt 1440
   caactagcag accattatca acaaaatact ccaattggcg atggccctgt ccttttacca 1500
   gacaaccatt acctgtccac acaatctgcc ctttcgaaag atcccaacga aaagagagac 1560
   cacatggtcc ttcttgagtt tgtaacagct gctgggatta cacatggcat ggatgaactg 1620
   tacaactga 1629

<210> 184
<211> 1716
<212> DNA
<213> Human

<400> 184
atgcctgcta attacacgtg taccaggcca gatggagaca atacagattt tcgatacttt 60
atttatgcag tgacatacac tgtcattctt gtgccaggtc tcatagggaa tatattagcc 120
ctgtgggtat tctatggtta tatgaaagaa acaaaacgag ctgtgatatt tatgataaac 180
ttagccattg ctgacttact acaagttctt tccttgccac tgaggatctt ctactacttg 240
aatcatgact ggccatttgg gcctggtctc tgcatgttct gtttctacct gaagtatgtc 300
aacatgtatg caagcatcta cttcttggtc tgcatcagtg tgcgacgatt ttggtttctc 360
atgtacccct ttcgcttcca tgactgcaaa cagaaatatg acctgtacat cagcattgct 420
ggctggctga tcatctgcct tgcctgtgta ctctttccac tcctcagaac cagtgatgat 480
acctctggca ataggaccaa atgctttgtg gatcttccta ccaggaatgt caacctggcc 540
cagtccgttg ttatgatgac cattggcgag ttgattgggt ttgtaactcc gcttctgatt 600
gtcctatatt gtacctggaa gacggtttta tcactgcaag ataaatatcc catggcccaa 660
gatcttggag agaaacagaa agccttgaag atgattctaa cctgtgcagg ggtattccta 720
atttgctttg caccttatca tttcagtttt cctttagatt tcctggtgaa gtccaatgaa 780
attaaaagct gcctagccag aagggtgatt ctaatatttc attctgtggc attgtgtctt 840
gctagtctga attcatgtct tgacccagtc atatactact tttccactaa tgagttccga 900
agacggcttt caagacaaga tttgcatgac agcatccaac tccatgcaaa atcctttgtg 960
agtaaccata cagcttccac catgacacct gaattatgcg ctagcaaagg agaagaactc 1020
ttcactggag ttgtcccaat tcttgttgaa ttagatggtg atgttaacgg ccacaagttc 1080
tctgtcagtg gagagggtga aggtgatgca acatacggaa aacttaccct gaagttcatc 1140
tgcactactg gcaaactgcc tgttccatgg ccaacactag tcactactct gtgctatggt 1200
gttcaatgct tttcaagata cccggatcat atgaaacggc atgacttttt caagagtgcc 1260
atgcccgaag gttatgtaca ggaaaggacc atcttcttca aagatgacgg caactacaag 1320
acacgtgctg aagtcaagtt tgaaggtgat acccttgtta atagaatcga gttaaaaggt 1380
attgacttca aggaagatgg caacattctg ggacacaaat tggaatacaa ctataactca 1440
cacaatgtat acatcatggc agacaaacaa aagaatggaa tcaaagtgaa cttcaagacc 1500
cgccacaaca ttgaagatgg aagcgttcaa ctagcagacc attatcaaca aaatactcca 1560
attggcgatg gccctgtcct tttaccagac aaccattacc tgtccacaca atctgccctt 1620
tcgaaagatc ccaacgaaaa gagagaccac atggtccttc ttgagtttgt aacagctgct 1680
gggattacac atggcatgga tgaactgtac aactga 1716

<210> 185
<211> 1734
<212> DNA
<213> Human

<220>
<221> misc feature
<222> (330)...(330)
<223> n stands for any base

<400> 185
atgaatggcc ttgaagtggc tcccccaggt ctgatcacca acttctccct ggccacggca 60
gagcaatgtg gccaggagac gccactggag aacatgctgt tcgcctcctt ctaccttctg 120
gattttatcc tggctttagt tggcaatacc ctggctctgt ggcttttcat ccgagaccac 180
aagtccggga ccccggccaa cgtgttcctg atgcatctgg ccgtggccga cttgtcgtgc 240
gtgctggtcc tgcccacccg cctggtctac cacttctctg ggaaccactg gccatttggg 300
gaaatcgcat gccgtctcac cggcttcctn ttctacctca acatgtacgc cagcatctac 360
ttcctcacct gcatcagcgc cgaccgtttc ctggccattg tgcacccggt caagtccctc 420
aagctccgca ggcccctcta cgcacacctg gcctgtgcct tcctgtgggt ggtggtggct 480
gtggccatgg ccccgctgct ggtgagccca cagaccgtgc agaccaacca cacggtggtc 540
tgcctgcagc tgtaccggga gaaggcctcc caccatgccc tggtgtccct ggcagtggcc 600
ttcaccttcc cgttcatcac cacggtcacc tgctacctgc tgatcatccg cagcctgcgg 660
cagggcctgc gtgtggagaa gcgcctcaag accaaggcag tgcgcatgat cgccatagtg 720
ctggccatct tcctggtctg cttcgtgccc taccacgtca accgctccgt ctacgtgctg 780
cactaccgca gccatggggc ctcctgcgcc acccagcgca tcctggccct ggcaaaccgc 840
atcacctcct gcctcaccag cctcaacggg gcactcgacc ccatcatgta tttcttcgtg 900
gctgagaagt tccgccacgc cctgtgcaac ttgctctgtg gcaaaaggct caagggcccg 960
ccccccagct tcgaagggaa aaccaacgag agctcgctga gtgccaagtc agagctggct 1020
agcaaaggag aagaactctt cactggagtt gtcccaattc ttgttgaatt agatggtgat 1080
gttaacggcc acaagttctc tgtcagtgga gagggtgaag gtgatgcaac atacggaaaa 1140

cttaccctga agttcatctg cactactggc aaactgcctg ttccatggcc aacactagtc 1200
actactctgt gctatggtgt tcaatgcttt tcaagatacc cggatcatat gaaacggcat 1260
gactttttca agagtgccat gcccgaaggt tatgtacagg aaaggaccat cttcttcaaa 1320
gatgacggca actacaagac acgtgctgaa gtcaagtttg aaggtgatac ccttgttaat 1380
agaatcgagt taaaaggtat tgacttcaag gaagatggca acattctggg acacaaattg 1440
gaatacaact ataactcaca caatgtatac atcatggcag acaaacaaaa gaatggaatc 1500
aaagtgaact tcaagacccg ccacaacatt gaagatggaa gcgttcaact agcagaccat 1560
tatcaacaaa atactccaat tggcgatggc cctgtccttt taccagacaa ccattacctg 1620
tccacacaat ctgccctttc gaaagatccc aacgaaaaga gagaccacat ggtccttctt 1680
gagtttgtaa cagctgctgg gattacacat ggcatggatg aactgtacaa ctga 1734

<210> 186
<211> 1644
<212> DNA
<213> Human

<400> 186
atgaatggca cctacaacac ctgtggctcc agcgacctca cctggccccc agcgatcaag 60
ctgggcttct acgcctactt gggcgtcctg ctggtgctag gcctgctgct caacagcctg 120
gcgctctggg tgttctgctg ccgcatgcag cagtggacgg agacccgcat ctacatgacc 180
aacctggcgg tggccgacct ctgcctgctg tgcaccttgc ccttcgtgct gcactccctg 240
cgagacacct cagacacgcc gctgtgccag ctctcccagg gcatctacct gaccaacagg 300
tacatgagca tcagcctggt cacggccatc gccgtggacc gctatgtggc cgtgcggcac 360
ccgctgcgtg cccgcgggct gcggtccccc aggcaggctg cggccgtgtg cgcggtcctc 420
tgggtgctgg tcatcggctc cctggtggct cgctggctcc tggggattca ggagggcggc 480
ttctgcttca ggagcacccg gcacaatttc aactccatgg cgttcccgct gctgggattc 540
tacctgcccc tggccgtggt ggtcttctgc tccctgaagg tggtgactgc cctggcccag 600
aggccaccca ccgacgtggg gcaggcagag gccacccgca aggctgcccg catggtctgg 660
gccaacctcc tggtgttcgt ggtctgcttc ctgcccctgc acgtggggct gacagtgcgc 720
ctcgcagtgg gctggaacgc ctgtgccctc ctggagacga tccgtcgcgc cctgtacata 780
accagcaagc tctcagatgc caactgctgc ctggacgcca tctgctacta ctacatggcc 840
aaggagttcc aggaggcgtc tgcactggcc gtggctcccc gtgctaaggc ccacaaaagc 900
caggactctc tgtgcgtgac cctcgccgct agcaaggag aagaactctt cactggagtt 960
gtcccaattc ttgttgaatt agatggtgat gttaacggcc acaagttctc tgtcagtgga 1020
gagggtgaag gtgatgcaac atacggaaaa cttaccctga agttcatctg cactactggc 1080
aaactgcctg ttccatggcc aacactagtc actactctgt gctatggtgt tcaatgcttt 1140
tcaagatacc cggatcatat gaaacggcat gactttttca agagtgccat gcccgaaggt 1200
tatgtacagg aaaggaccat cttcttcaaa gatgacggca actacaagac acgtgctgaa 1260
gtcaagtttg aaggtgatac ccttgttaat agaatcgagt taaaaggtat tgacttcaag 1320
gaagatggca acattctggg acacaaattg gaatacaact ataactcaca caatgtatac 1380
atcatggcag acaaacaaaa gaatggaatc aaagtgaact tcaagacccg ccacaacatt 1440
gaagatggaa gcgttcaact agcagaccat tatcaacaaa atactccaat tggcgatggc 1500
cctgtccttt taccagacaa ccattacctg tccacacaat ctgccctttc gaaagatccc 1560
aacgaaaaga gagaccacat ggtccttctt gagtttgtaa cagctgctgg gattacacat 1620
ggcatggatg aactgtacaa ctga 1644

<210> 187
<211> 2040
<212> DNA
<213> Human

<400> 187
atggccatcc acaaagcctt ggtgatgtgc ctgggactgc ctctcttcct gttcccaggg 60
gcctgggccc agggccatgt cccacccggc tgcagccaag gcctcaaccc cctgtactac 120
aacctgtgtg accgctctgg ggcgtggggc atcgtcctgg aggccgtggc tggggcgggc 180
attgtcacca cgtttgtgct caccatcatc ctggtggcca gcctcccctt tgtgcaggac 240
accaagaaac ggagcctgct ggggacccag gtattcttcc ttctggggac cctgggcctc 300
ttctgcctcg tgtttgcctg tgtggtgaag cccgacttct ccacctgtgc ctctcggcgc 360
ttcctctttg ggttctgttt cgccatctgc ttctcttgtc tggcggctca cgtctttgcc 420
ctcaacttcc tggcccggaa gaaccacggg ccccggggct gggtgatctt cactgtggct 480
ctgctgctga ccctggtaga ggtcatcatc aatacagagt ggctgatcat caccctggtt 540
cggggcagtg gcgagggcgg ccctcagggc aacagcagcg caggctgggc cgtggcctcc 600
ccctgtgcca tcgccaacat ggactttgtc atggcactca tctacgtcat gctgctgctg 660
ctgggtgcct tcctgggggc ctggcccgcc ctgtgtggcc gctacaagcg ctggcgtaag 720
catggggtct ttgtgctcct caccacagcc acctccgttg ccatatgggt ggtgtggatc 780
gtcatgtata cttacggcaa caagcagcac aacagtccca cctgggatga ccccacgctg 840
gccatcgccc tcgccgccaa tgcctgggcc ttcgtcctct tctacgtcat ccccgaggtc 900
tcccaggtga ccaagtccag cccagagcaa agctaccagg gggacatgta ccccacccgg 960
ggcgtgggct atgagaccat cctgaaagag cagaagggtc agagcatgtt cgtggagaac 1020
aaggcctttt ccatggatga gccggttgca gctaagaggc cggtgtcacc atacagcggg 1080
tacaatgggc agctgctgac cagtgtgtac cagcccactg agatggccct gatgcacaaa 1140
gttccgtccg aaggagctta cgacatcatc ctcccacggg ccaccgccaa cagccaggtg 1200
atgggcagtg ccaactcgac cctgcgggct gaagacatgt actcggccca gagccaccag 1260
gcggccacac cgccgaaaga cggcaagaac tctcaggtct ttagaaaccc ctacgtgtgg 1320
gacgctagca aaggagaaga actcttcact ggagttgtcc caattcttgt tgaattagat 1380

ggtgatgtta acggccacaa gttctctgtc agtggagagg gtgaaggtga tgcaacatac 1440
ggaaaactta ccctgaagtt catctgcact actggcaaac tgcctgttcc atggccaaca 1500
ctagtcacta ctctgtgcta tggtgttcaa tgcttttcaa gatacccgga tcatatgaaa 1560
cggcatgact ttttcaagag tgccatgccc gaaggttatg tacaggaaag gaccatcttc 1620
ttcaaagatg acggcaacta caagacacgt gctgaagtca agtttgaagg tgataccctt 1680
gttaatagaa tcgagttaaa aggtattgac ttcaaggaag atggcaacat tctgggacac 1740
aaattggaat acaactataa ctcacacaat gtatacatca tggcagacaa acaaaagaat 1800
ggaatcaaag tgaacttcaa gacccgccac aacattgaag atggaagcgt tcaactagca 1860
gaccattatc aacaaaatac tccaattggc gatggccctg tccttttacc agacaaccat 1920
tacctgtcca cacaatctgc cctttcgaaa gatcccaacg aaaagagaga ccacatggtc 1980
cttcttgagt ttgtaacagc tgctgggatt acacatggca tggatgaact gtacaactga 2040

<210> 188
<211> 1878
<212> DNA
<213> Human

<400> 188
atgaatcggc accatctgca ggatcacttt ctggaaatag acaagaagaa ctgctgtgtg 60
ttccgagatg acttcattgc caaggtgttg ccgccggtgt tggggctgga gtttatcttt 120
gggcttctgg gcaatggcct tgccctgtgg attttctgtt tccacctcaa gtcctggaaa 180
tccagccgga ttttcctgtt caacctggca gtagctgact ttctactgat catctgcctg 240
ccgttcgtga tggactacta tgtgcggcgt tcagactgga agtttgggga catcccttgc 300
cggctggtgc tcttcatgtt tgccatgaac cgccagggca gcatcatctt cctcacggtg 360
gtggcggtag acaggtattt ccgggtggtc catccccacc acgccctgaa caagatctcc 420
aattggacag cagccatcat ctcttgcctt ctgtggggca tcactgttgg cctaacagtc 480
cacctcctga agaagaagtt gctgatccag aatggccctg caaatgtgtg catcagcttc 540
agcatctgcc ataccttccg gtggcacgaa gctatgttcc tcctggagtt cctcctgccc 600
ctgggcatca tcctgttctg ctcagccaga attatctgga gcctgcggca gagacaaatg 660
gaccggcatg ccaagatcaa gagagccatc accttcatca tggtggtggc catcgtcttt 720
gtcatctgct tccttcccag cgtggttgtg cggatccgca tcttctggct cctgcacact 780
tcgggcacgc agaattgtga agtgtaccgc tcggtggacc tggcgttctt tatcactctc 840
agcttcacct acatgaacag catgctggac cccgtggtgt actacttctc cagcccatcc 900
tttcccaact tcttctccac tttgatcaac cgctgcctcc agaggaagat gacaggtgag 960
ccagataata accgcagcac gagcgtcgag ctcacagggg accccaacaa aaccagaggc 1020
gctccagagg cgttaatggc caactccggt gagccatgga gcccctctta tctgggccca 1080
acctcaaata accattccaa gaagggacat tgtcaccaag aaccagcatc tctggagaaa 1140
cagttgggct gttgcatcga ggctagcaaa ggagaagaac tcttcactgg agttgtccca 1200
attcttgttg aattagatgg tgatgttaac ggccacaagt tctctgtcag tggagagggt 1260
gaaggtgatg caacatacgg aaaacttacc ctgaagttca tctgcactac tggcaaactg 1320
cctgttccat ggccaacact agtcactact ctgtgctatg gtgttcaatg cttttcaaga 1380
tacccggatc atatgaaacg gcatgacttt ttcaagagtg ccatgcccga aggttatgta 1440
caggaaagga ccatcttctt caaagatgac ggcaactaca agacacgtgc tgaagtcaag 1500
tttgaaggtg atacccttgt taatagaatc gagttaaaag gtattgactt caaggaagat 1560
ggcaacattc tgggacacaa attggaatac aactataact cacacaatgt atacatcatg 1620
gcagacaaac aaaagaatgg aatcaaagtg aacttcaaga cccgccacaa cattgaagat 1680
ggaagcgttc aactagcaga ccattatcaa caaaatactc caattggcga tggccctgtc 1740
cttttaccag acaaccatta cctgtccaca caatctgccc tttcgaaaga tcccaacgaa 1800
aagagagacc acatggtcct tcttgagttt gtaacagctg ctgggattac acatggcatg 1860
gatgaactgt acaactga 1878

<210> 189
<211> 2337
<212> DNA
<213> Human

<400> 189
atgaactcaa caggccacct tcaggatgcc cccaatgcca cctcgctcca tgtgcctcac 60
tcacaggaag gaaacagcac ctctctccag gagggtcttc aggatctcat ccacacagcc 120
accttggtga cctgtacttt tctactggcg gtcatcttct gcctgggttc ctatggcaac 180
ttcattgtct tcttgtcctt cttcgatcca gccttcagga aattcagaac caactttgat 240
ttcatgatcc tgaacctgtc cttctgtgac ctcttcattt gtggagtgac agcccccatg 300
ttcacctttg tgttattctt cagctcagcc agtagtatcc cggatgcttt ctgcttcact 360
ttccatctca ccagttcagg cttcatcatc atgtctctga agacagtggc agtgatcgcc 420
ctgcaccggc tccggatggt gttggggaaa cagcctaatc gcacggcctc ctttccctgc 480
accgtactcc tcaccctgct tctctgggcc accagtttca cccttgccac cttggctacc 540
ttgaaaacca gcaagtccca cctctgtctt cccatgtcca gtctgattgc tggaaaaggg 600
aaagccattt tgtctctcta tgtggtcgac ttcaccttct gtgttgctgt ggtctctgtc 660
tcttacatca tgattgctca gaccctgcgg aagaacgctc aagtcagaaa gtgccccctt 720
gtaatcacag tcgatgcttc cagaccacag cctttcatgg gggtccctgt gcagggaggt 780
ggagatccca tccagtgtgc catgccggct ctgtatagga accagaatta caacaaactg 840
cagcacgttc agacccgtgg atataccaag agtcccaacc aactggtcac ccctgcagca 900
agccgactcc agctcgtatc agccatcaac ctctccactg ccaaggattc caaagccgtg 960
gtcacctgtg tgatcattgt gctgtcagtc ctggtgtgct gtcttccact ggggatttcc 1020
ttggtacagg tggttctctc cagcaatggg agcttcattc tttaccagtt tgaattgttt 1080

ggatttactc ttatattttt caagtcagga ttaaaccctt ttatatattc tcggaacagt 1140
gcagggctga gaaggaaagt gctctggtgc ctccaataca taggcctggg ttttttctgc 1200
tgcaaacaaa agactcgact tcgagccatg ggaaaaggga acctcgaagt caacagaaac 1260
aaatcctccc atcatgaaac aaactctgcc tacatgttat ctccaaagcc acagaagaaa 1320
tttgtggacc aggcttgtgg cccaagtcat tcaaaagaaa gtatggtgag tcccaagatc 1380
tctgctggac atcaacactg tggtcagagc agctcgaccc ccatcaacac tcggattgaa 1440
ccttactaca gcatctataa cagcagccct tcccaggagg agagcagccc atgtaactta 1500
cagccagtaa actcttttgg atttgccaat tcatatattg ccatgcatta tcacaccact 1560
aatgacttag tgcaggaata tgacagcact tcagccaagc agattccagt cccctccgtt 1620
gctagcaaag gagaagaact cttcactgga gttgtcccaa ttcttgttga attagatggt 1680
gatgttaacg gccacaagtt ctctgtcagt ggagagggtg aaggtgatgc aacatacgga 1740
aaacttaccc tgaagttcat ctgcactact ggcaaactgc ctgttccatg gccaacacta 1800
gtcactactc tgtgctatgg tgttcaatgc ttttcaagat acccggatca tatgaaacgg 1860
catgactttt tcaagagtgc catgcccgaa ggttatgtac aggaaaggac catcttcttc 1920
aaagatgacg gcaactacaa gacacgtgct gaagtcaagt ttgaaggtga tacccttgtt 1980
aatagaatcg agttaaaagg tattgacttc aaggaagatg gcaacattct gggacacaaa 2040
ttggaataca actataactc acacaatgta tacatcatgg cagacaaaca aaagaatgga 2100
atcaaagtga acttcaagac ccgccacaac attgaagatg gaagcgttca actagcagac 2160
cattatcaac aaaatactcc aattggcgat ggccctgtcc ttttaccaga caaccattac 2220
ctgtccacac aatctgccct ttcgaaagat cccaacgaaa agagagacca catggtcctt 2280
cttgagtttg taacagctgc tgggattaca catggcatgg atgaactgta caactga 2337

<210> 190
<211> 1755
<212> DNA
<213> Human

<400> 190
   atgtacaacg ggtcgtgctg ccgcatcgag ggggacacca tctcccaggt gatgccgccg 60
   ctgctcattg tggcctttgt gctgggcgca ctaggcaatg gggtcgccct gtgtggtttc 120
   tgcttccaca tgaagacctg gaagcccagc actgtttacc ttttcaattt ggccgtggct 180
   gatttcctcc ttatgatctg cctgcctttt cggacagact attacctcag acgtagacac 240
   tgggcttttg gggacattcc ctgccgagtg gggctcttca cgttggccat gaacagggcc 300
   gggagcatcg tgttccttac ggtggtggct gcggacaggt atttcaaagt ggtccacccc 360
   caccacgcgg tgaacactat ctccacccgg gtggcggctg gcatcgtctg caccctgtgg 420
   gccctggtca tcctgggaac agtgtatctt ttgctggaga accatctctg cgtgcaagag 480
   acggccgtct cctgtgagag cttcatcatg gagtcggcca atggctggca tgacatcatg 540
   ttccagctgg agttctttat gcccctcggc atcatcttat tttgctcctt caagattgtt 600
   tggagcctga ggcggaggca gcagctggcc agacaggctc ggatgaagaa ggcgacccgg 660
   ttcatcatgg tggtggcaat tgtgttcatc acatgctacc tgcccagcgt gtctgctaga 720
   ctctatttcc tctggacggt gccctcgagt gcctgcgatc cctctgtcca tggggccctg 780
   cacataaccc tcagcttcac ctacatgaac agcatgctgg atcccctggt gtattatttt 840
   tcaagcccct cctttcccaa attctacaac aagctcaaaa tctgcagtct gaaacccaag 900
   cagccaggac actcaaaaac acaaaggccg gaagagatgc caatttcgaa cctcggtcgc 960
   aggagttgca tcagtgtggc aaatagtttc caaagccagt ctgatgggca atgggatccc 1020
   cacattgttg agtggcacgc tagcaaagga gaagaactct tcactggagt tgtcccaatt 1080
   cttgttgaat tagatggtga tgttaacggc cacaagttct ctgtcagtgg agagggtgaa 1140
   ggtgatgcaa catacggaaa acttaccctg aagttcatct gcactactgg caaactgcct 1200
   gttccatggc caacactagt cactactctg tgctatggtg ttcaatgctt ttcaagatac 1260
   ccggatcata tgaaacggca tgactttttc aagagtgcca tgcccgaagg ttatgtacag 1320
   gaaaggacca tcttcttcaa agatgacggc aactacaaga cacgtgctga agtcaagttt 1380
   gaaggtgata cccttgttaa tagaatcgag ttaaaaggta ttgacttcaa ggaagatggc 1440
   aacattctgg gacacaaatt ggaatacaac tataactcac acaatgtata catcatggca 1500
   gacaaacaaa agaatggaat caaagtgaac ttcaagaccc gccacaacat tgaagatgga 1560
   agcgttcaac tagcagacca ttatcaacaa aatactccaa ttggcgatgg ccctgtcctt 1620
   ttaccagaca accattacct gtccacacaa tctgcccttt cgaaagatcc caacgaaaag 1680
   agagaccaca tggtccttct tgagtttgta acagctgctg ggattacaca tggcatggat 1740
   gaactgtaca actga 1755

<210> 191
<211> 1758
<212> DNA
<213> Human

<400> 191
atgggggatg agctggcacc ttgccctgtg ggcactacag cttggccggc cctgatccag 60
ctcatcagca agacaccctg catgccccaa gcagccagca acacttcctt gggcctgggg 120
gacctcaggg tgcccagctc catgctgtac tggcttttcc ttccctcaag cctgctggct 180
gcagccacac tggctgtcag cccccctgctg ctggtgacca tcctgcggaa ccaacggctg 240
cgacaggagc cccactacct gctcccggct aacatcctgc tctcagacct ggcctacatt 300
ctcctccaca tgctcatctc ctccagcagc ctgggtggct gggagctggg ccgcatggcc 360
tgtggcattc tcactgatgc tgtcttcgcc gcctgcacca gcaccatcct gtccttcacc 420
gccattgtgc tgcacaccta cctggcagtc atccatccac tgcgctacct ctccttcatg 480
tcccatgggg ctgcctggaa ggcagtggcc ctcatctggc tggtggcctg ctgcttcccc 540
acattcctta tttggctcag caagtggcag gatgcccagc tggaggagca aggagcttca 600

tacatcctac caccaagcat gggcacccag ccgggatgtg gcctcctggt cattgttacc 660
tacacctcca ttctgtgcgt tctgttcctc tgcacagctc tcattgccaa ctgtttctgg 720
aggatctatg cagaggccaa gacttcaggc atctgggggc agggctattc ccgggccagg 780
ggcaccctgc tgatccactc agtgctgatc acattgtacg tgagcacagg ggtggtgttc 840
tccctggaca tggtgctgac caggtaccac cacattgact ctgggactca cacatggctc 900
ctggcagcta acagtgaggt actcatgatg cttccccgtg ccatgctccc atacctgtac 960
ctgctccgct accggcagct gttgggcatg gtccggggcc acctcccatc caggaggcac 1020
caggccatct ttaccatttc cgctagcaaa ggagaagaac tcttcactgg agttgtccca 1080
attcttgttg aattagatgg tgatgttaac ggccacaagt tctctgtcag tggagagggt 1140
gaaggtgatg caacatacgg aaaacttacc ctgaagttca tctgcactac tggcaaactg 1200
cctgttccat ggccaacact agtcactact ctgtgctatg gtgttcaatg cttttcaaga 1260
tacccggatc atatgaaacg gcatgacttt ttcaagagtg ccatgcccga aggttatgta 1320
caggaaagga ccatcttctt caaagatgac ggcaactaca agacacgtgc tgaagtcaag 1380
tttgaaggtg atacccttgt taatagaatc gagttaaaag gtattgactt caaggaagat 1440
ggcaacattc tgggacacaa attggaatac aactataact cacacaatgt atacatcatg 1500
gcagacaaac aaaagaatgg aatcaaagtg aacttcaaga cccgccacaa cattgaagat 1560
ggaagcgttc aactagcaga ccattatcaa caaaatactc caattggcga tggccctgtc 1620
cttttaccag acaaccatta cctgtccaca caatctgccc tttcgaaaga tcccaacgaa 1680
aagagagacc acatggtcct tcttgagttt gtaacagctg ctgggattac acatggcatg 1740
gatgaactgt acaactga 1758

<210> 192
<211> 1836
<212> DNA
<213> Human

<400> 192
atgagaatgg aggatgaaga ttacaacact tccatcagtt acggtgatga ataccctgat 60
tatttagact ccattgtggt tttggaggac ttatccccct tggaagccag ggtgaccagg 120
atcttcctgg tggtggtcta cagcatcgtc tgcttcctcg ggattctggg caatggtctg 180
gtgatcatca ttgccacctt caagatgaag aagacagtga acatggtctg gttcctcaac 240
ctggcagtgg cagatttcct gttcaacgtc ttcctcccaa tccatatcac ctatgccgcc 300
atggactacc actgggtttt cgggacagcc atgtgcaaga tcagcaactt ccttctcatc 360
cacaacatgt tcaccagcgt cttcctgctg accatcatca gctctgaccg ctgcatctct 420
gtgctcctcc ctgtctggtc ccagaaccac cgcagcgttc gcctggctta catggcctgc 480
atggtcatct gggtcctggc tttcttcttg agttccccat ctctcgtctt ccgggacaca 540
gccaacctgc atgggaaaat atcctgcttc aacaacttca gcctgtccac acctgggtct 600
tcctcgtggc ccactcactc ccaaatggac cctgtggggt atagccggca catggtggtg 660
actgtcaccc gcttcctctg tggcttcctg gtcccagtcc tcatcatcac agcttgctac 720
ctcaccatcg tgtgcaaact gcagcgcaac cgcctggcca agaccaagaa gcccttcaag 780
attattgtga ccatcatcat taccttcttc ctctgctggt gcccctacca cacactcaac 840
ctcctagagc tccaccacac tgccatgcct ggctctgtct tcagcctggg tttgcccctg 900
gccactgccc ttgccattgc caacagctgc atgaacccca ttctgtatgt tttcatgggt 960
caggacttca agaagttcaa ggtggccctc ttctctcgcc tggtcaatgc tctaagtgaa 1020
gatacaggcc actcttccta ccccagccat agaagcttta ccaagatgtc atcaatgaat 1080
gagaggactt ctatgaatga gagggagacc ggcatgcttg ctagcaaagg agaagaactc 1140
ttcactggag ttgtcccaat tcttgttgaa ttagatggtg atgttaacgg ccacaagttc 1200
tctgtcagtg gagagggtga aggtgatgca acatacggaa aacttaccct gaagttcatc 1260
tgcactactg gcaaactgcc tgttccatgg ccaacactag tcactactct gtgctatggt 1320
gttcaatgct tttcaagata cccggatcat atgaaacggc atgacttttt caagagtgcc 1380
atgcccgaag gttatgtaca ggaaaggacc atcttcttca aagatgacgg caactacaag 1440
acacgtgctg aagtcaagtt tgaaggtgat acccttgtta atagaatcga gttaaaaggt 1500
attgacttca aggaagatgg caacattctg ggacacaaat tggaatacaa ctataactca 1560
cacaatgtat acatcatggc agacaaacaa aagaatggaa tcaaagtgaa cttcaagacc 1620
cgccacaaca ttgaagatgg aagcgttcaa ctagcagacc attatcaaca aaatactcca 1680
attggcgatg gccctgtcct tttaccagac aaccattacc tgtccacaca atctgccctt 1740
tcgaaagatc ccaacgaaaa gagagaccac atggtccttc ttgagtttgt aacagctgct 1800
gggattacac atggcatgga tgaactgtac aactga 1836

<210> 193
<211> 1776
<212> DNA
<213> Rat

<400> 193
atggaagtct caagggaaat gctatttgaa gaactggaca actactccta tgccttagaa 60
tattactccc aggaacctga cgcagaggag aatgtgtacc cgggaatcgt tcactggatc 120
tccctgctct tatatgccct tgcgtttgtt ctgggaattc cagggaatgc catcgtcatt 180
tggttcatgg gattcaagtg gaagaagacg gtcaccactc tttggtttct caatctggcc 240
attgcagact tcgtctttgt tctcttcctg cctctgtata tttcctatgt ggcactgagt 300
ttccactggc cctttggccg atggctctgc aagcttaatt ccttcattgc ccaactgaac 360
atgttttcca gtgtattctt cttgacagtg attagcctgg accgctacat tcacttgatc 420
caccctggct tgtctcatcc gcaccggacc ctgaagaact cactgcttgt tgttctattt 480
gtctggctgt tggcttctct gctcggaggt cctaccctgt acttccggga caccgtggag 540
gtcaacaacc gcattatttg ttataacaac ttccaggagt atgagctcac cctgatgaga 600

caccacgttc tgacctgggt gaagttcctt tttggctacc tcttgccttt gctgacaatg 660
agctcctgct acctgtgcct catcttcaag acgaagaagc aaaacattct gatatccagt 720
aagcatctct ggatgatcct gtctgtggtc atcgccttca tggtttgctg gactcctttt 780
cacctgttca gcatttggga actcagcatt catcacaaca gctctttcca gaacgtgctg 840
cagggcggaa tccctctctc tactggcttg gccttcctca acagctgctt gaaccccatc 900
ctttacgttc taataagcaa gaagtttcaa gctcgattca gggcctctgt tgccgaggta 960
ctaaagcggt cactgtggga ggccagttgc tctggtacag tgagtgaaca actcaggagt 1020
gctgaaacca agagcctgtc tctcctagaa actgcccaag ctagcaaagg agaagaactc 1080
ttcactggag ttgtcccaat tcttgttgaa ttagatggtg atgttaacgg ccacaagttc 1140
tctgtcagtg gagagggtga aggtgatgca acatacggaa aacttaccct gaagttcatc 1200
tgcactactg gcaaactgcc tgttccatgg ccaacactag tcactactct gtgctatggt 1260
gttcaatgct tttcaagata cccggatcat atgaaacggc atgacttttt caagagtgcc 1320
atgcccgaag gttatgtaca ggaaaggacc atcttcttca aagatgacgg caactacaag 1380
acacgtgctg aagtcaagtt tgaaggtgat acccttgtta atagaatcga gttaaaaggt 1440
attgacttca aggaagatgg caacattctg ggacacaaat tggaatacaa ctataactca 1500
cacaatgtat acatcatggc agacaaacaa aagaatggaa tcaaagtgaa cttcaagacc 1560
cgccacaaca ttgaagatgg aagcgttcaa ctagcagacc attatcaaca aaatactcca 1620
attggcgatg gccctgtcct tttaccagac aaccattacc tgtccacaca atctgccctt 1680
tcgaaagatc ccaacgaaaa gagagaccac atggtccttc ttgagtttgt aacagctgct 1740
gggattacac atggcatgga tgaactgtac aactga 1776

<210> 194
<211> 1707
<212> DNA
<213> Human

<400> 194
atgatgtggg gtgcaggcag ccctctggcc tggctctcag ctggctcagg caacgtgaat 60
gtaagcagcg tgggcccagc agaggggccc acaggtccag ccgcaccact gccctcgcct 120
aaggcctggg atgtggtgct ctgcatctca ggcaccctgg tgtcctgcga gaatgcgcta 180
gtggtggcca tcatcgtggg cactcctgcc ttccgtgccc ccatgttcct gctggtgggc 240
agcctggccg tggcagacct gctggcaggc ctgggcctgg tcctgcactt tgctgctgtc 300
ttctgcatcg gctcagcgga gatgagcctg gtgctggttg gcgtgctggc aatggccttt 360
accgccagca tcggcagtct actggccatc actgtcgacc gctacctttc tctgtacaat 420
gccctcacct actattcaga gacaacagtg acacggacct atgtgatgct ggccttagtg 480
tggggaggtg ccctgggcct ggggctgctg cctgtgctgg cctggaactg cctggatggc 540
ctgaccacat gtggcgtggt ttatccactc tccaagaacc atctggtagt tctggccatt 600
gccttcttca tggtgtttgg catcatgctg cagctctacg cccaaatctg ccgcatcgtc 660
tgccgccatg cccagcagat tgcccttcag cggcacctgc tgcctgcctc ccactatgtg 720
gccacccgca agggcattgc cacactggcc gtggtgcttg gagcctttgc cgcctgctgg 780
ttgcccttca ctgtctactg cctgctgggt gatgcccact ctccacctct ctacacctat 840
cttaccttgc tccctgccac ctacaactcc atgatcaacc ctatcatcta cgccttccgc 900
aaccaggatg tgcagaaagt gctgtgggct gtctgctgct gctgttcctc ttccaagatc 960
cccttccgat cccgctcccc cagtgatgtc gctagcaaag gagaagaact cttcactgga 1020
gttgtcccaa ttcttgttga attagatggt gatgttaacg gccacaagtt ctctgtcagt 1080
ggagagggtg aaggtgatgc aacatacgga aaacttaccc tgaagttcat ctgcactact 1140
ggcaaactgc ctgttccatg gccaacacta gtcactactc tgtgctatgg tgttcaatgc 1200
ttttcaagat acccggatca tatgaaacgg catgactttt tcaagagtgc catgcccgaa 1260
ggttatgtac aggaaaggac catcttcttc aaagatgacg gcaactacaa gacacgtgct 1320
gaagtcaagt ttgaaggtga tacccttgtt aatagaatcg agttaaaagg tattgacttc 1380
aaggaagatg gcaacattct gggacacaaa ttggaataca actataactc acacaatgta 1440
tacatcatgg cagacaaaca aaagaatgga atcaaagtga acttcaagac ccgccacaac 1500
attgaagatg gaagcgttca actagcagac cattatcaac aaaatactcc aattggcgat 1560
ggccctgtcc ttttaccaga caaccattac ctgtccacac aatctgccct ttcgaaagat 1620
cccaacgaaa agagagacca catggtcctt cttgagtttg taacagctgc tgggattaca 1680
catggcatgg atgaactgta caactga 1707

<210> 195
<211> 1803
<212> DNA
<213> Human

<400> 195
atgaacgcga gcgccgcctc gctcaacgac tcccaggtgg tggtagtggc ggccgaagga 60
gcggcggcgg cggccacagc agcagggggg ccggacacgg gcgaatgggg acccccctgct 120
gcggcggctc taggagccgg cggcggagct aatgggtctc tggagctgtc ctcgcagctg 180
tcggctgggc caccgggact cctgctgcca gcggtgaatc cgtgggacgt gctcctgtgc 240
gtgtcgggga cagtgatcgc tggagaaaac gcgctggtgg tggcgctcat cgcgtccact 300
ccggcgctac gcacgcccat gttcgtgctg gtaggcagcc tggccaccgc tgacctgttg 360
gcgggctgtg gcctcatctt gcactttgtg ttccagtact tggtgccctc ggagactgtg 420
agtctgctca cggtgggctt cctcgtggcc tccttcgccg cctctgtcag cagcctgctg 480
gccattacgg tggaccgcta cctgtccctg tataacgcgc tcacctatta ctcgcgccgg 540
accctgttgg gcgtgcacct cctgcttgcc gccacttgga ccgtgtccct aggcctgggg 600
ctgctgcccg tgctgggctg gaactgcctg gcagagcgcg ccgcctgcag cgtggtgcgc 660
ccgctggcgc gcagccacgt ggctctgctc tccgccgcct tcttcatggt cttcggcatc 720

atgctgcacc tgtacgtgcg catctgccag gtggtctggc gccacgcgca ccagatcgcg 780
ctgcagcagc actgcctggc gccaccccat ctcgctgcca ccagaaaggg tgtgggtaca 840
ctggctgtgg tgctgggcac tttcggcgcc agctggctgc ccttcgccat ctattgcgtg 900
gtgggcagcc atgaggaccc ggcggtctac acttacgcca ccctgctgcc cgccacctac 960
aactccatga tcaatcccat catctatgcc ttccgcaacc aggagatcca gcgcgccctg 1020
tggctcctgc tctgtggctg tttccagtcc aaagtgccct ttcgttccag gtctcccagc 1080
gaggtcgcta gcaaaggaga agaactcttc actggagttg tcccaattct tgttgaatta 1140
gatggtgatg ttaacggcca caagttctct gtcagtggag agggtgaagg tgatgcaaca 1200
tacggaaaac ttaccctgaa gttcatctgc actactggca aactgcctgt tccatggcca 1260
acactagtca ctactctgtg ctatggtgtt caatgctttt caagataccc ggatcatatg 1320
aaacggcatg actttttcaa gagtgccatg cccgaaggtt atgtacagga aaggaccatc 1380
ttcttcaaag atgacggcaa ctacaagaca cgtgctgaag tcaagtttga aggtgatacc 1440
cttgttaata gaatcgagtt aaaaggtatt gacttcaagg aagatggcaa cattctggga 1500
cacaaattgg aatacaacta taactcacac aatgtataca tcatggcaga caaacaaaag 1560
aatggaatca aagtgaactt caagacccgc cacaacattg aagatggaag cgttcaacta 1620
gcagaccatt atcaacaaaa tactccaatt ggcgatggcc ctgtcctttt accagacaac 1680
cattacctgt ccacacaatc tgccctttcg aaagatccca acgaaaagag agaccacatg 1740
gtccttcttg agtttgtaac agctgctggg attacacatg gcatggatga actgtacaac 1800
tga 1803

<210> 196
<211> 1788
<212> DNA
<213> Human

<400> 196
atggctacaa cagtccctga tggttgccgc aatggcctga aatccaagta ctacagactt 60
tgtgataagg ctgaagcttg gggcatcgtc ctagaaacgg tggccacagc tggggttgtg 120
acctcggtgg ccttcatgct cactctcccg atcctcgtct gcaaggtgca ggactccaac 180
aggcgaaaaa tgctgcctac tcagtttctc ttcctcctgg gtgtgttggg catctttggc 240
ctcaccttcg ccttcatcat cggactggac gggagcacag ggcccacacg cttcttcctc 300
tttgggatcc tcttttccat ctgcttctcc tgcctgctgg ctcatgctgt cagtctgacc 360
aagctcgtcc gggggaggaa gcccctttcc ctgttggtga ttctgggtct ggccgtgggc 420
ttcagcctag tccaggatgt tatcgctatt gaatatattg tcctgaccat gaataggacc 480
aacgtcaatg tcttttctga gctttccgct cctcgtcgca atgaagactt tgtcctcctg 540
ctcacctacg tcctcttctt gatggcgctg accttcctca tgtcctcctt caccttctgt 600
ggttccttca cgggctggaa gagacatggg gcccacatct acctcacgat gctcctctcc 660
attgccatct gggtggcctg gatcaccctg ctcatgcttc ctgactttga ccgcaggtgg 720
gatgacacca tcctcagctc cgccttggct gccaatggct gggtgttcct gttggcttat 780
gttagtcccg agttttggct gctcacaaag caacgaaacc ccatggatta tcctgttgag 840
gatgctttct gtaaacctca actcgtgaag aagagctatg gtgtggagaa cagagcctac 900
tctcaagagg aaatcactca aggttttgaa gagacagggg acacgctcta tgccccctat 960
tccacacatt ttcagctgca gaaccagcct ccccaaaagg aattctccat cccacgggcc 1020
cacgcttggc cgagccctta caaggactat gaagtaaaga aagagggcag cgctagcaaa 1080
ggagaagaac tcttcactgg agttgtccca attcttgttg aattagatgg tgatgttaac 1140
ggccacaagt tctctgtcag tggagagggt gaaggtgatg caacatacgg aaaacttacc 1200
ctgaagttca tctgcactac tggcaaactg cctgttccat ggccaacact agtcactact 1260
ctgtgctatg gtgttcaatg cttttcaaga tacccggatc atatgaaacg gcatgacttt 1320
ttcaagagtg ccatgcccga aggttatgta caggaaagga ccatcttctt caaagatgac 1380
ggcaactaca agacacgtgc tgaagtcaag tttgaaggtg atacccttgt taatagaatc 1440
gagttaaaag gtattgactt caaggaagat ggcaacattc tgggacacaa attggaatac 1500
aactataact cacacaatgt atacatcatg gcagacaaac aaaagaatgg aatcaaagtg 1560
aacttcaaga cccgccacaa cattgaagat ggaagcgttc aactagcaga ccattatcaa 1620
caaaatactc caattggcga tggccctgtc cttttaccag acaaccatta cctgtccaca 1680
caatctgccc tttcgaaaga tcccaacgaa aagagagacc acatggtcct tcttgagttt 1740
gtaacagctg ctgggattac acatggcatg gatgaactgt acaactga 1788

<210> 197
<211> 1974
<212> DNA
<213> Human

<400> 197
atgctggcag ctgcctttgc agactctaac tccagcagca tgaatgtgtc ctttgctcac 60
ctccactttg ccggagggta cctgccctct gattcccagg actggagaac catcatcccg 120
gctctcttgg tggctgtctg cctggtgggc ttcgtgggaa acctgtgtgt gattggcatc 180
ctccttcaca atgcttggaa aggaaagcca tccatgatcc actccctgat tctgaatctc 240
agcctggctg atctctccct cctgctgttt tctgcaccta tccgagctac ggcgtactcc 300
aaaagtgttt gggatctagg ctggtttgtc tgcaagtcct ctgactggtt tatccacaca 360
tgcatggcag ccaagagcct gacaatcgtt gtggtggcca aagtatgctt catgtatgca 420
agtgacccag ccaagcaagt gagtatccac aactacacca tctggtcagt gctggtggcc 480
atctggactg tggctagcct gttacccctg ccggaatggt tctttagcac catcaggcat 540
catgaaggtg tggaaatgtg cctcgtggat gtaccagctg tggctgaaga gtttatgtcg 600
atgtttggta agctctaccc actcctggca tttggccttc cattattttt tgccagcttt 660
tatttctgga gagcttatga ccaatgtaaa aaacgaggaa ctaagactca aaatcttaga 720

aaccagatac gctcaaagca agtcacagtg atgctgctga gcattgccat catctctgct 780
ctcttgtggc tccccgaatg ggtagcttgg ctgtgggtat ggcatctgaa ggctgcaggc 840
ccggccccac cacaaggttt catagccctg tctcaagtct tgatgttttc catctcttca 900
gcaaatcctc tcatttttct tgtgatgtcg gaagagttca gggaaggctt gaaaggtgta 960
tggaaatgga tgataaccaa aaaacctcca actgtctcag agtctcagga aacaccagct 1020
ggcaactcag agggtcttcc tgacaaggtt ccatctccag aatccccagc atccatacca 1080
gaaaaagaga aacccagctc tccctcctct ggcaaaggga aaactgagaa ggcagagatt 1140
cccatccttc ctgacgtaga gcagttttgg catgagaggg acacagtccc ttctgtacag 1200
gacaatgacc ctatcccctg ggaacatgaa gatcaagaga caggggaagg tgttaaaact 1260
agcaaaggag aagaactctt cactggagtt gtcccaattc ttgttgaatt agatggtgat 1320
gttaacggcc acaagttctc tgtcagtgga gagggtgaag gtgatgcaac atacggaaaa 1380
cttaccctga agttcatctg cactactggc aaactgcctg ttccatggcc aacactagtc 1440
actactctgt gctatggtgt tcaatgcttt tcaagatacc cggatcatat gaaacggcat 1500
gactttttca agagtgccat gcccgaaggt tatgtacagg aaaggaccat cttcttcaaa 1560
gatgacggca actacaagac acgtgctgaa gtcaagtttg aaggtgatac ccttgttaat 1620
agaatcgagt taaaaggtat tgacttcaag gaagatggca acattctggg acacaaattg 1680
gaatacaact ataactcaca caatgtatac atcatggcag acaaacaaaa gaatggaatc 1740
aaagtgaact tcaagacccg ccacaacatt gaagatggaa gcgttcaact agcagaccat 1800
tatcaacaaa atactccaat tggcgatggc cctgtccttt taccagacaa ccattacctg 1860
tccacacaat ctgccctttc gaaagatccc aacgaaaaga gagaccacat ggtccttctt 1920
gagtttgtaa cagctgctgg gattacacat ggcatggatg aactgtacaa ctga 1974

<210> 198
<211> 1974
<212> DNA
<213> Human

<400> 198
atgctggcag ctgcctttgc agactctaac tccagcagca tgaatgtgtc ctttgctcac 60
ctccactttg ccggagggta cctgccctct gattcccagg actggagaac catcatcccg 120
gctctcttgg tggctgtctg cctggtgggc ttcgtgggaa acctgtgtgt gattggcatc 180
ctccttcaca atgcttggaa aggaaagcca tccatgatcc actccctgat tctgaatctc 240
agcctggctg atctctccct cctgctgttt tctgcaccta tccgagctac ggcgtactcc 300
aaaagtgttt gggatctagg ctggtttgtc tgcaagtcct ctgactggtt tatccacaca 360
tgcatggcag ccaagagcct gacaatcgtt gtggtggcca aagtatgctt catgtatgca 420
agtgacccag ccaagcaagt gagtatccac aactacacca tctggtcagt gctggtggcc 480
atctggactg tggctagcct gttacccctg ccggaatggt tctttagcac catcaggcat 540
catgaaggtg tggaaatgtg cctcgtggat gtaccagctg tggctgaaga gtttatgtcg 600
atgtttggta agctctaccc actcctggca tttggccttc cattattttt tgccagcttt 660
tatttctgga gagcttatga ccaatgtaaa aaacgaggaa ctaagactca aaatcttaga 720
aaccagatac gctcaaagca agtcacagtg atgctgctga gcattgccat catctctgct 780
gtcttgtggc tccccgaatg ggtagcttgg ctgtgggtat ggcatctgaa ggctgcaggc 840
ccggccccac cacaaggttt catagccctg tctcaagtct tgatgttttc catctcttca 900
gcaaatcctc tcatttttct tgtgatgtcg gaagagttca gggaaggctt gaaaggtgta 960
tggaaatgga tgataaccaa aaaacctcca actgtctcag agtctcagga aacaccagct 1020
ggcaactcag agggtcttcc tgacaaggtt ccatctccag aatccccagc atccatacca 1080
gaaaaagaga aacccagctc tccctcctct ggcaaaggga aaactgagaa ggcagagatt 1140
cccatccttc ctgacgtaga gcagttttgg catgagaggg acacagtccc ttctgtacag 1200
gacaatgacc ctatcccctg ggaacatgaa gatcaagaga caggggaagg tgttaaaact 1260
agcaaaggag aagaactctt cactggagtt gtcccaattc ttgttgaatt agatggtgat 1320
gttaacggcc acaagttctc tgtcagtgga gagggtgaag gtgatgcaac atacggaaaa 1380
cttaccctga agttcatctg cactactggc aaactgcctg ttccatggcc aacactagtc 1440
actactctgt gctatggtgt tcaatgcttt tcaagatacc cggatcatat gaaacggcat 1500
gactttttca agagtgccat gcccgaaggt tatgtacagg aaaggaccat cttcttcaaa 1560
gatgacggca actacaagac acgtgctgaa gtcaagtttg aaggtgatac ccttgttaat 1620
agaatcgagt taaaaggtat tgacttcaag gaagatggca acattctggg acacaaattg 1680
gaatacaact ataactcaca caatgtatac atcatggcag acaaacaaaa gaatggaatc 1740
aaagtgaact tcaagacccg ccacaacatt gaagatggaa gcgttcaact agcagaccat 1800
tatcaacaaa atactccaat tggcgatggc cctgtccttt taccagacaa ccattacctg 1860
tccacacaat ctgccctttc gaaagatccc aacgaaaaga gagaccacat ggtccttctt 1920
gagtttgtaa cagctgctgg gattacacat ggcatggatg aactgtacaa ctga 1974

<210> 199
<211> 3699
<212> DNA
<213> Human

<400> 199
atggtctgtt cggctgcccc actgctgctc ctggccacaa ctcttcccct gctggggtca 60
ccagttgccc aagcatccca acctggacag agtcaggctg gaggggaatc tggatctggg 120
cagctcctgg accaagagaa tggagcaggg gaatcagcgc tggtctccgt ctatgtacat 180
ctggactttc cagataagac ctggccccct gaactctcca ggacactgac tctccctgct 240
gcctcagctt cctcttcccc aaggcctctt ctcactggcc tcagactcac aacagagtgt 300
aatgtcaacc acaaggggaa tttctattgt gcttgcctct ctggctacca gtggaacacc 360
agcatctgcc tccattaccc tccttgtcaa agcctccaca accaccagcc ttgtggctgc 420

cttgtcttca gccatcccga acccgggtac tgccagttgc tgccacctgt ccccgggatc 480
ctcaacctga actcccagct gcagatgcct ggtgacacgc tgagcctgac tctccatctg 540
agccaggagg ccaccaacct gagctggttc ctgaggcacc cagggagccc cagtcccatc 600
ctcctgcagc cagggacaca ggtgtctgtg acttccagcc acggccaggc tgccctcagc 660
gtctccaaca tgtcccatca ctgggcaggt gagtacatga gctgcttcga ggcccagggc 720
ttcaagtgga acctgtatga ggtggtgagg gtgcccttga aggcgacaga tgtggctcga 780
cttccatacc agctgtccat ctcctgtgcc acctcccctg gcttccagct gagctgctgc 840
atccccagca caaacctggc ctacaccgcg gcctggagcc ctggagaggg cagcaaagct 900
tcctccttca acgagtcagg ctctcagtgc tttgtgctgg ctgttcagcg ctgcccgatg 960
gctgacacca cgtacacttg tgacctgcag agcctgggcc tggctccact cagggtcccc 1020
atctccatca ccatcatcca ggatggagac atcacctgcc ctgaggacgc ctcggtgctc 1080
acctggaatg tcaccaaggc tggccacgtg gcacaggccc catgtcctga gagcaagagg 1140
ggcatagtga ggaggctctg tggggctgac ggagtctggg ggccggtcca cagcagctgc 1200
acagatgcga ggctcctggc cttgttcact agaaccaagc tgctgcaggc aggccagggc 1260
agtcctgctg aggaggtgcc acagatcctg gcacagctgc cagggcaggc ggcagaggca 1320
agttcaccct ccgacttact gaccctgctg agcaccatga aatacgtggc caaggtggtg 1380
gcagaggcca gaatacagct tgaccgcaga gccctgaaga atctcctgat tgccacagac 1440
aaggtcctag atatggacac caggtctctg tggaccctgg cccaagcccg gaagccctgg 1500
gcaggctcga ctctcctgct ggctgtggag accctggcat gcagcctgtg cccacaggac 1560
taccccttcg ccttcagctt acccaatgtg ctgctgcaga gccagctgtt tggacccacg 1620
tttcctgctg actacagcat ctccttccct actcggcccc cactgcaggc tcagattccc 1680
aggcactcac tggccccatt ggtccgtaat ggaactgaaa taagtattac tagcctggtg 1740
ctgcgaaaac tggaccacct tctgccctca aactatggac aagggctggg ggattccctc 1800
tatgccactc ctggcctggt ccttgtcatt tccatcatgg caggtgaccg ggccttcagc 1860
cagggagagg tcatcatgga ctttgggaac acagatggtt cccctcactg tgtcttctgg 1920
gatcacagtc tcttccaggg caggggggt tggtccaaag aagggtgcca ggcacaggtg 1980
gccagtgcca gccccactgc tcagtgcctc tgccagcacc tcactgcctt ctccgtcctc 2040
atgtccccac acactgttcc ggaagaaccc gctctggcgc tgctgactca agtgggcttg 2100
ggagcttcca tactggcgct gcttgtgtgc ctgggtgtgt actggctggt gtggagagtc 2160
gtggtgcgga acaagatctc ctatttccgc cacgccgccc tgctcaacat ggtgttctgc 2220
ttgctggccg cagacacttg cttcctgggc gccccattcc tctctccagg gccccgaagc 2280
ccgctctgcc ttgctgccgc cttcctctgt catttcctct acctggccac ctttttctgg 2340
atgctggcgc aggccctggt gttggcccac cagctgctct ttgtctttca ccagctggca 2400
aagcaccgag ttctccccct catggtgctc ctgggctacc tgtgcccact ggggttggca 2460
ggtgtcaccc tggggctcta cctacctcaa gggcaatacc tgagggaggg ggaatgctgg 2520
ttggatggga agggaggggc gttatacacc ttcgtggggc cagtgctggc catcataggc 2580
gtgaatgggc tggtactagc catggccatg ctgaagttgc tgagaccttc gctgtcagag 2640
ggacccccag cagagaagcg ccaagctctg ctgggggtga tcaaagccct gctcattctt 2700
acacccatct ttggcctcac ctgggggctg ggcctggcca ctctgttaga ggaagtctcc 2760
acggtccctc attacatctt caccattctc aacaccctcc agggcgtctt catcctattg 2820
tttggttgcc tcatggacag gaagatacaa gaagctttgc gcaaacgctt ctgccgcgcc 2880
caagccccca gctccaccat ctccctggcc acaaatgaag gctgcatctt ggaacacagc 2940
aaaggaggaa gcgacactgc caggaagaca gatgcttcag aggctagcaa aggagaagaa 3000
ctcttcactg gagttgtccc aattcttgtt gaattagatg gtgatgttaa cggccacaag 3060
ttctctgtca gtggagaggg tgaaggtgat gcaacatacg gaaaacttac cctgaagttc 3120
atctgcacta ctggcaaact gcctgttcca tggccaacac tagtcactac tctgtgctat 3180
ggtgttcaat gcttttcaag atacccggat catatgaaac ggcatgactt tttcaagagt 3240
gccatgcccg aaggttatgt acaggaaagg accatcttct tcaaagatga cggcaactac 3300
aagacacgtg ctgaagtcaa gtttgaaggt gataccttg ttaatagaat cgagttaaaa 3360
ggtattgact tcaaggaaga tggcaacatt ctgggacaca aattggaata caactataac 3420
tcacacaatg tatacatcat ggcagacaaa caaaagaatg gaatcaaagt gaacttcaag 3480
acccgccaca acattgaaga tggaagcgtt caactagcag accattatca acaaaatact 3540
ccaattggcg atggccctgt ccttttacca gacaaccatt acctgtccac acaatctgcc 3600
ctttcgaaag atcccaacga aaagagagac cacatggtcc ttcttgagtt tgtaacagct 3660
gctgggatta cacatggcat ggatgaactg tacaactga 3699

<210> 200
<211> 2796
<212> DNA
<213> Human

<400> 200
atgactcccc agtcgctgct gcagacgaca ctgttcctgc tgagtctgct cttcctggtc 60
caaggtgccc acggcagggg ccacagggaa gactttcgct tctgcagcca gcggaaccag 120
acacacagga gcagcctcca ctacaaaccc acaccagacc tgcgcatctc catcgagaac 180
tccgaagagg ccctcacagt ccatgcccct ttccctgcag cccaccctgc ttcccgatcc 240
ttccctgacc ccagggggct ctaccacttc tgcctctact ggaaccgaca tgctgggaga 300
ttacatcttc tctatggcaa gcgtgacttc ttgctgagtg acaaagcctc tagcctcctc 360
tgcttccagc accaggagga gagcctggct cagggccccc cgctgttagc cacttctgtc 420
acctcctggt ggagccctca gaacatcagc ctgcccagtg ccgccagctt caccttctcc 480
ttccacagtc ctccccacac ggccgctcac aatgcctcgg tggacatgtg cgagctcaaa 540
agggacctcc agctgctcag ccagttcctg aagcatcccc agaaggcctc aaggaggccc 600
tcggctgccc ccgccagcca gcagttgcag agcctggagt cgaaactgac ctctgtgaga 660
ttcatggggg acatggtgtc cttcgaggag gaccggatca acgccacggt gtggaagctc 720
cagcccacag ccggcctcca ggacctgcac atccactccc ggcaggagga ggagcagagc 780

gagatcatgg agtactcggt gctgctgcct cgaacactct tccagaggac gaaaggccgg 840
agcggggagg ctgagaagag actcctcctg gtggacttca gcagccaagc cctgttccag 900
gacaagaatt ccagccaagt cctgggtgag aaggtcttgg ggattgtggt acagaacacc 960
aaagtagcca acctcacgga gcccgtggtg ctcactttcc agcaccagct acagccgaag 1020
aatgtgactc tgcaatgtgt gttctgggtt gaagacccca cattgagcag ccccgggcat 1080
tggagcagtg ctgggtgtga gaccgtcagg agagaaaccc aaacatcctg cttctgcaac 1140
cacttgacct actttgcagt gctgatggtc tcctcggtgg aggtggacgc cgtgcacaag 1200
cactacctga gcctcctctc ctacgtgggc tgtgtcgtct ctgccctggc ctgccttgtc 1260
accattgccg cctacctctg ctccagggtg cccctgccgt gcaggaggaa acctcgggac 1320
tacaccatca aggtgcacat gaacctgctg ctggccgtct tcctgctgga cacgagcttc 1380
ctgctcagcg agccggtggc cctgacaggc tctgaggctg gctgccgagc cagtgccatc 1440
ttcctgcact tctccctgct cacctgcctt tcctggatgg gcctcgaggg gtacaacctc 1500
taccgactcg tggtggaggt ctttggcacc tatgtccctg gctacctact caagctgagc 1560
gccatgggct ggggcttccc catctttctg gtgacgctgg tggccctggt ggatgtggac 1620
aactatggcc ccatcatctt ggctgtgcat aggactccag agggcgtcat ctacccttcc 1680
atgtgctgga tccgggactc cctggtcagc tacatcacca acctgggcct cttcagcctg 1740
gtgtttctgt tcaacatggc catgctagcc accatggtgg tgcagatcct gcggctgcgc 1800
ccccacaccc aaaagtggtc acatgtgctg acactgctgg gcctcagcct ggtccttggc 1860
ctgccctggg ccttgatctt cttctccttt gcttctggca ccttccagct tgtcgtcctc 1920
taccttttca gcatcatcac ctccttccaa ggcttcctca tcttcatctg gtactggtcc 1980
atgcggctgc aggcccgggg tggcccctcc cctctgaaga gcaactcaga cagcgccagg 2040
ctccccatca gctcgggcag cacctcgtcc agccgcatct ctagcaaagg agaagaactc 2100
ttcactggag ttgtcccaat tcttgttgaa ttagatggtg atgttaacgg ccacaagttc 2160
tctgtcagtg gagagggtga aggtgatgca acatacggaa aacttaccct gaagttcatc 2220
tgcactactg gcaaactgcc tgttccatgg ccaacactag tcactactct gtgctatggt 2280
gttcaatgct tttcaagata cccggatcat atgaaacggc atgacttttt caagagtgcc 2340
atgcccgaag gttatgtaca ggaaaggacc atcttcttca aagatgacgg caactacaag 2400
acacgtgctg aagtcaagtt tgaaggtgat acccttgtta atagaatcga gttaaaaggt 2460
attgacttca aggaagatgg caacattctg ggacacaaat tggaatacaa ctataactca 2520
cacaatgtat acatcatggc agacaaacaa aagaatggaa tcaaagtgaa cttcaagacc 2580
cgccacaaca ttgaagatgg aagcgttcaa ctagcagacc attatcaaca aaatactcca 2640
attggcgatg gccctgtcct tttaccagac aaccattacc tgtccacaca atctgccctt 2700
tcgaaagatc ccaacgaaaa gagagaccac atggtccttc ttgagtttgt aacagctgct 2760
gggattacac atggcatgga tgaactgtac aactga 2796

<210> 201
<211> 4755
<212> DNA
<213> Human

<400> 201
atgaaatccc caaggagaac cactttgtgc ctcatgttta ttgtgattta ttcttccaaa 60
gctgcactga actggaatta cgagtctact attcatcctt tgagtcttca tgaacatgaa 120
ccagctggtg aagaggcact gaggcaaaaa cgagccgttg ccacaaaaag tcctacggct 180
gaagaataca ctgttaatat tgagatcagt tttgaaaatg catccttcct ggatcctatc 240
aaagcctact tgaacagcct cagttttcca attcatggga ataacactga ccaaattacc 300
gacattttga gcataaatgt gacaacagtc tgcagacctg ctggaaatga aatctggtgc 360
tcctgcgaga caggttatgg gtggcctcgg gaaaggtgtc ttcacaatct catttgtcaa 420
gagcgtgacg tcttcctccc agggcaccat tgcagttgcc ttaaagaact gcctcccaat 480
ggaccttttt gcctgcttca ggaagatgtt accctgaaca tgagagtcag actaaatgta 540
ggctttcaag aagacctcat gaacacttcc tccgccctct ataggtccta caagaccgac 600
ttggaaacag cgttccggaa gggttacgga attttaccag gcttcaaggg cgtgactgtg 660
acagggttca agtctggaag tgtggttgtg acatatgaag tcaagactac accaccatca 720
cttgagttaa tacataaagc caatgaacaa gttgtacaga gcctcaatca gacctacaaa 780
atggactaca actcctttca agcagttact atcaatgaaa gcaatttctt tgtcacacca 840
gaaatcatct ttgaagggga cacagtcagt ctggtgtgtg aaaaggaagt tttgtcctcc 900
aatgtgtctt ggcgctatga agaacagcag ttggaaatcc agaacagcag cagattctcg 960
atttacaccg cacttttcaa caacatgact tcggtgtcca agctcaccat ccacaacatc 1020
actccaggtg atgcaggtga atatgtttgc aaactgatat tagacatttt tgaatatgag 1080
tgcaagaaga aaatagatgt tatgcccatc caaattttgg caaatgaaga aatgaaggtg 1140
atgtgcgaca acaatcctgt atctttgaac tgctgcagtc agggtaatgt taattggagc 1200
aaagtagaat ggaagcagga aggaaaaata aatattccag gaacccctga gacagacata 1260
gattctagct gcagcagata caccctcaag gctgatggaa cccagtgccc aagcgggtcg 1320
tctggaacaa cagtcatcta cacttgtgag ttcatcagtg cctatggagc cagaggcagt 1380
gcaaacataa aagtgacatt catctctgtg gccaatctaa caataacccc ggacccaatt 1440
tctgtttctg agggacaaaa cttttctata aaatgcatca gtgatgtgag taactatgat 1500
gaggtttatt ggaacacttc tgctggaatt aaaatatacc aaagatttta taccacgagg 1560
aggtatcttg atggagcaga atcagtactg acagtcaaga cctcgaccag ggagtggaat 1620
ggaacctatc actgcatatt tagatataag aattcataca gtattgcaac caaagacgtc 1680
attgttcacc cgctgcctct aaagctgaac atcatggttg atcctttgga agctactgtt 1740
tcatgcagtg gttcccatca catcaagtgc tgcatagagg aggatggaga ctacaaagtt 1800
actttccata tgggttcctc atcccttcct gctgcaaaag aagttaacaa aaaacaagtg 1860
tgctacaaac acaatttcaa tgcaagctca gtttcctggt gttcaaaaac tgttgatgtg 1920
tgttgtcact ttaccaatgc tgctaataat tcagtttgga gcccatctat gaagctgaat 1980
ctggttcctg gggaaaacat cacatgccag gatcccgtaa taggtgtcgg agagccgggg 2040

aaagtcatcc agaagctatg ccggttctca aacgttccca gcagccctga gagtcccatt 2100
ggcgggacca tcacttacaa atgtgtaggc tcccagtggg aggagaagag aaatgactgc 2160
atctctgccc caataaacag tctgctccag atggctaagg ctttgatcaa gagcccctct 2220
caggatgaga tgctccctac atacctgaag gatctttcta ttagcataga caaagcggaa 2280
catgaaatca gctcttctcc tgggagtctg ggagccatta ttaacatcct tgatctgctc 2340
tcaacagttc caacccaagt aaattcagaa atgatgacgc acgtgctctc tacggttaat 2400
gtcatccttg gcaagcccgt cttgaacacc tggaaggttt tacaacagca atggaccaat 2460
cagagttcac agctactaca ttcagtggaa agattttccc aagcattaca gtcaggagat 2520
agccctcctt tgtccttctc ccaaactaat gtgcagatga gcagcacggt aatcaagtcc 2580
agccacccag aaacctatca acagaggttt gttttcccat actttgacct ctggggcaat 2640
gtggtcattg acaagagcta tctagaaaac ttgcagtcgg attcgtctat tgtcaccatg 2700
gctttcccaa ctctccaagc catccttgct caggatatcc aggaaaataa ctttgcagag 2760
agcttagtga tgacaaccac tgtcagccac aatacgacta tgccattcag gatttcaatg 2820
acttttaaga acaatagccc ttcaggcggc gaaacgaagt gtgtcttctg gaacttcagg 2880
cttgccaaca acacaggggg gtgggacagc agtgggtgct atgttgaaga aggtgatggg 2940
gacaatgtca cctgtatctg tgaccaccta acatcattct ccatcctcat gtcccctgac 3000
tccccagatc ctagttctct cctgggaata ctcctggata ttatttctta tgttggggtg 3060
ggcttttcca tcttgagctt ggcagcctgt ctagttgtgg aagctgtggt gtggaaatcg 3120
gtgaccaaga atcggacttc ttatatgcgc cacacctgca tagtgaatat cgctgcctcc 3180
cttctggtcg ccaacacctg gttcattgtg gtcgctgcca tccaggacaa tcgctacata 3240
ctctgcaaga cagcctgtgt ggctgccacc ttcttcatcc acttcttcta cctcagcgtc 3300
ttcttctgga tgctgacact gggcctcatg ctgttctatc gcctggtttt cattctgcat 3360
gaaacaagca ggtccactca gaaagccatt gccttctgtc ttggctatgg ctgcccactt 3420
gccatctcgg tcatcacgct gggagccacc cagccccggg aagtctatac gaggaagaat 3480
gtctgttggc tcaactggga ggacaccaag gccctgctgg ctttcgccat cccagcactg 3540
atcattgtgg tggtgaacat aaccatcact attgtggtca tcaccaagat cctgaggcct 3600
tccattggag acaagccatg caagcaggag aagagcagcc tgtttcagat cagcaagagc 3660
attggggtcc tcacaccact cttgggcctc acttggggtt ttggtctcac cactgtgttc 3720
ccagggacca accttgtgtt ccatatcata tttgccatcc tcaatgtctt ccagggatta 3780
ttcattttac tctttggatg cctctgggat ctgaaggtac aggaagcttt gctgaataag 3840
ttttcattgt cgagatggtc ttcacagcac tcaaagtcaa catccctggg ttcatccaca 3900
cctgtgtttt ctatgagttc tccaatatca aggagattta acaatttgtt tggtaaaaca 3960
ggaacgtata atgtttccac cccagaagca accagctcat ccctggaaaa ctcatccagt 4020
gcttcttcgt tgctcaacgc tagcaaagga gaagaactct tcactggagt tgtcccaatt 4080
cttgttgaat tagatggtga tgttaacggc cacaagttct ctgtcagtgg agagggtgaa 4140
ggtgatgcaa catacggaaa acttaccctg aagttcatct gcactactgg caaactgcct 4200
gttccatggc caacactagt cactactctg tgctatggtg ttcaatgctt ttcaagatac 4260
ccggatcata tgaaacggca tgactttttc aagagtgcca tgcccgaagg ttatgtacag 4320
gaaaggacca tcttcttcaa agatgacggc aactacaaga cacgtgctga agtcaagttt 4380
gaaggtgata cccttgttaa tagaatcgag ttaaaaggta ttgacttcaa ggaagatggc 4440
aacattctgg gacacaaatt ggaatacaac tataactcac acaatgtata catcatggca 4500
gacaaacaaa agaatggaat caaagtgaac ttcaagaccc gccacaacat tgaagatgga 4560
agcgttcaac tagcagacca ttatcaacaa aatactccaa ttggcgatgg ccctgtcctt 4620
ttaccagaca accattacct gtccacacaa tctgcccttt cgaaagatcc caacgaaaag 4680
agagaccaca tggtccttct tgagtttgta acagctgctg ggattacaca tggcatggat 4740
gaactgtaca actga 4755

<210> 202
<211> 1938
<212> DNA
<213> Human

<400> 202
   atgagcctca actcctccct cagctgcagg aaggagctga gtaatctcac tgaggaggag 60
   ggtggcgaag ggggcgtcat catcacccag ttcatcgcca tcattgtcat caccattttt 120
   gtctgcctgg gaaacctggt catcgtggtc accttgtaca agaagtccta cctcctcacc 180
   ctcagcaaca agttcgtctt cagcctgact ctgtccaact tcctgctgtc cgtgttggtg 240
   ctgccttttg tggtgacgag ctccatccgc agggaatgga tctttggtgt agtgtggtgc 300
   aacttctctg ccctcctcta cctgctgatc agctctgcca gcatgctaac cctcggggtc 360
   attgccatcg accgctacta tgctgtcctg taccccatgg tgtaccccat gaagatcaca 420
   gggaaccggg ctgtgatggc acttgtctac atctggcttc actcgctcat cggctgcctg 480
   ccacccctgt ttggttggtc atccgtggag tttgacgagt tcaaatggat gtgtgtggct 540
   gcttggcacc gggagcctgg ctacacggcc ttctggcaga tctggtgtgc cctcttcccc 600
   tttctggtca tgctggtgtg ctatggcttt atcttccgcg tggccagggt caaggcacgc 660
   aaggtgcact gtggcacagt cgtcatcgtg gaggaggatg ctcagaggac cgggaggaag 720
   aactccagca cctccacctc ctcttcaggc agcaggagga atgcctttca gggtgtggtc 780
   tactcggcca accagtgcaa agccctcatc accatcctgg tggtcctcgg tgccttcatg 840
   gtcacctggg gcccctacat ggttgtcatc gcctctgagg ccctctgggg gaaaagctcc 900
   gtctccccga gcctggagac ttgggccaca tggctgtcct ttgccagcgc tgtctgccac 960
   cccctgatct atggactctg gaacaagaca gttcgcaaag aactactggg catgtgcttt 1020
   ggggaccggt attatcggga accatttgtg caacgacaga ggacttccag gctcttcagc 1080
   atttccaaca ggatcacaga cctgggcctg tccccacacc tcactgcgct catggcaggt 1140
   ggacagcccc tggggcacag cagcagcacg ggggacactg gcttcagctg ctcccaggac 1200
   tcaggtaacc tgcgtgcttt agctagcaaa ggagaagaac tcttcactgg agttgtccca 1260
   attcttgttg aattagatgg tgatgttaac ggccacaagt tctctgtcag tggagagggt 1320
   gaaggtgatg caacatacgg aaaacttacc ctgaagttca tctgcactac tggcaaactg 1380
   cctgttccat ggccaacact agtcactact ctgtgctatg gtgttcaatg cttttcaaga 1440
   tacccggatc atatgaaacg gcatgacttt ttcaagagtg ccatgcccga aggttatgta 1500
   caggaaagga ccatcttctt caaagatgac ggcaactaca agacacgtgc tgaagtcaag 1560
   tttgaaggtg atacccttgt taatagaatc gagttaaaag gtattgactt caaggaagat 1620
   ggcaacattc tgggacacaa attggaatac aactataact cacacaatgt atacatcatg 1680
   gcagacaaac aaaagaatgg aatcaaagtg aacttcaaga cccgccacaa cattgaagat 1740
   ggaagcgttc aactagcaga ccattatcaa caaaatactc caattggcga tggccctgtc 1800
   cttttaccag acaaccatta cctgtccaca caatctgccc tttcgaaaga tcccaacgaa 1860
   aagagagacc acatggtcct tcttgagttt gtaacagctg ctgggattac acatggcatg 1920
   gatgaactgt acaactga 1938

<210> 203
<211> 1914
<212> DNA
<213> Human

<400> 203
atggctcaaa gggccttccc gaatccttat gctgattata acaaatccct ggccgaaggc 60
tactttgatg ctgccgggag gctgactcct gagttctcac aacgcttgac caataagatt 120
cgggagcttc ttcagcaaat ggagagaggc ctgaaatcag cagaccctcg ggatggcacc 180
ggttacactg gctgggcagg tattgctgtg ctttacttac atctttatga tgtatttggg 240
gaccctgcct acctacagtt agcacatggc tatgtaaagc aaagtctgaa ctgcttaacc 300
aagcgctcca tcaccttcct ttgtggggat gcaggccccc tggcagtggc cgctgtgcta 360
tatcacaaga tgaacaatga gaagcaggca gaagattgca tcacacggct aattcaccta 420
aataagattg atcctcatgc tccaaatgaa atgctctatg ggcgaatagg ctacatctat 480
gctcttcttt ttgtcaataa gaactttgga gtggaaaaga ttcctcaaag ccatattcag 540
cagatttgtg aaacaatttt aacctctgga gaaaacctag ctaggaagag aaacttcacg 600
gcaaagtctc cactgatgta tgaatggtac caggaatatt atgtaggggc tgctcatggc 660
ctggctggaa tttattacta cctgatgcag cccagccttc aagtgagcca agggaagtta 720
catagtttgg tcaagcccag tgtagactac gtctgccagc tgaaattccc ttctggcaat 780
taccctccat gtataggtga taatcgagat ctgcttgtcc attggtgcca tggcgcccct 840
ggggtaatct acatgctcat ccaggcctat aaggtattca gagaggaaaa gtatctctgt 900
gatgcctatc agtgtgctga tgtgatctgg caatatgggt tgctgaagaa gggatatggg 960
ctgtgccacg gttctgcagg gaatgcctat gccttcctga cactctacaa cctcacacag 1020
gacatgaagt acctgtatag ggcctgtaag tttgctgaat ggtgcttaga gtatggagaa 1080
catggatgca gaacaccaga cacccctttc tctctctttg aaggaatggc tggaacaata 1140
tatttcctgg ctgacctgct agtccccaca aaagccaggt tccctgcatt tgaactcgct 1200
agcaaaggag aagaactctt cactggagtt gtcccaattc ttgttgaatt agatggtgat 1260
gttaacggcc acaagttctc tgtcagtgga gagggtgaag gtgatgcaac atacggaaaa 1320
cttaccctga agttcatctg cactactggc aaactgcctg ttccatggcc aacactagtc 1380
actactctgt gctatggtgt tcaatgcttt tcaagatacc cggatcatat gaaacggcat 1440
gactttttca agagtgccat gcccgaaggt tatgtacagg aaaggaccat cttcttcaaa 1500
gatgacggca actacaagac acgtgctgaa gtcaagtttg aaggtgatac ccttgttaat 1560
agaatcgagt taaaaggtat tgacttcaag gaagatggca acattctggg acacaaattg 1620
gaatacaact ataactcaca caatgtatac atcatggcag acaaacaaaa gaatggaatc 1680
aaagtgaact tcaagacccg ccacaacatt gaagatggaa gcgttcaact agcagaccat 1740
tatcaacaaa atactccaat tggcgatggc cctgtccttt taccagacaa ccattacctg 1800
tccacacaat ctgccctttc gaaagatccc aacgaaaaga gagaccacat ggtccttctt 1860
gagtttgtaa cagctgctgg gattacacat ggcatggatg aactgtacaa ctga 1914

<210> 204
<211> 1842
<212> DNA
<213> Human

<400> 204
atggcgaacg cgagcgagcc gggtggcagc ggcggcggcg aggcggccgc cctgggcctc 60
aagctggcca cgctcagcct gctgctgtgc gtgagcctag cgggcaacgt gctgttcgcg 120
ctgctgatcg tgcgggagcg cagcctgcac cgcgccccgt actacctgct gctcgacctg 180
tgcctggccg acgggctgcg cgcgctcgcc tgcctcccgg ccgtcatgct ggcggcgcgg 240
cgtgcggcgg ccgcggcggg ggcgccgccg ggcgcgctgg gctgcaagct gctcgccttc 300
ctggccgcgc tcttctgctt ccacgccgcc ttcctgctgc tgggcgtggg cgtcacccgc 360
tacctggcca tcgcgcacca ccgcttctat gcagagcgcc tggccggctg gccgtgcgcc 420
gccatgctgg tgtgcgccgc ctgggcgctg gcgctggccg cggccttccc gccagtgctg 480
gacggcggtg gcgacgacga ggacgcgccg tgcgccctgg agcagcggcc cgacggcgcc 540
cccggcgcgc tgggcttcct gctgctgctg gccgtggtgg tgggcgccac gcacctcgtc 600
tacctccgcc tgctcttctt catccacgac cgccgcaaga tgcggcccgc gcgcctggtg 660
cccgccgtca gccacgactg gaccttccac ggcccgggcg ccaccggcca ggcggccgcc 720
aactggacgg cgggcttcgg ccgcgggccc acgccgcccg cgcttgtggg catccggccc 780
gcagggccgg gccgcggcgc gcgccgcctc ctcgtgctgg aagaattcaa gacggagaag 840
aggctgtgca agatgttcta cgccgtcacg ctgctcttcc tgctcctctg ggggccctac 900
gtcgtggcca gctacctgcg ggtcctggtg cggcccggcg ccgtccccca ggcctacctg 960
acggcctccg tgtggctgac cttcgcgcag gccggcatca accccgtcgt gtgcttcctc 1020
ttcaacaggg agctgaggga ctgcttcagg gcccagttcc cctgctgcca gagcccccgg 1080

accacccagg cgacccatcc ctgcgacctg aaaggcattg gtttagctag caaaggagaa 1140
gaactcttca ctggagttgt cccaattctt gttgaattag atggtgatgt taacggccac 1200
aagttctctg tcagtggaga gggtgaaggt gatgcaacat acggaaaact taccctgaag 1260
ttcatctgca ctactggcaa actgcctgtt ccatggccaa cactagtcac tactctgtgc 1320
tatggtgttc aatgcttttc aagatacccg gatcatatga aacggcatga ctttttcaag 1380
agtgccatgc ccgaaggtta tgtacaggaa aggaccatct tcttcaaaga tgacggcaac 1440
tacaagacac gtgctgaagt caagtttgaa ggtgataccc ttgttaatag aatcgagtta 1500
aaaggtattg acttcaagga agatggcaac attctgggac acaaattgga atacaactat 1560
aactcacaca atgtatacat catggcagac aaacaaaaga atggaatcaa agtgaacttc 1620
aagacccgcc acaacattga agatggaagc gttcaactag cagaccatta tcaacaaaat 1680
actccaattg gcgatggccc tgtcctttta ccagacaacc attacctgtc cacacaatct 1740
gccctttcga aagatcccaa cgaaaagaga gaccacatgg tccttcttga gtttgtaaca 1800
gctgctggga ttacacatgg catggatgaa ctgtacaact ga 1842

<210> 205
<211> 3438
<212> DNA
<213> Human

<400> 205
atggacacct cccggctcgg tgtgctcctg tccttgcctg tgctgctgca gctggcgacc 60
gggggcagct ctcccaggtc tggtgtgttg ctgaggggct gccccacaca ctgtcattgc 120
gagcccgacg gcaggatgtt gctcagggtg gactgctccg acctggggct ctcggagctg 180
ccttccaacc tcagcgtctt cacctcctac ctagacctca gtatgaacaa catcagtcag 240
ctgctcccga atcccctgcc cagtctccgc ttcctggagg agttacgtct tgcgggaaac 300
gctctgacat acgttcccaa gggagcattc actggccttt acagtcttaa agttcttatg 360
ctgcagaata atcagctaag acacgtaccc acagaagctc tgcagaattt gcgaagcctt 420
caatccctgc gtctggatgc taaccacatc agctatgtgc ccccaagctg tttcagtggc 480
ctgcattccc tgaggcacct gtggctggat gacaatgcgt taacagaaat ccccgtccag 540
gcttttagaa gtttatcggc attgcaagcc atgaccttgg ccctgaacaa aatacaccac 600
ataccagact atgcctttgg aaacctctcc agcttggtag ttctacatct ccataacaat 660
agaatccact ccctgggaaa gaaatgcttt gatgggctcc acagcctaga gactttagat 720
ttaaattaca ataaccttga tgaattcccc actgcaatta ggacactctc caaccttaaa 780
gaactaggat ttcatagcaa caatatcagg tcgatacctg agaaagcatt tgtaggcaac 840
ccttctctta ttacaataca tttctatgac aatcccatcc aatttgttgg gagatctgct 900
tttcaacatt tacctgaact aagaacactg actctgaatg gtgcctcaca aataactgaa 960
tttcctgatt taactggaac tgcaaacctg gagagtctga ctttaactgg agcacagatc 1020
tcatctcttc ctcaaaccgt ctgcaatcag ttacctaatc tccaagtgct agatctgtct 1080
tacaacctat tagaagattt acccagtttt tcagtctgcc aaaagcttca gaaaattgac 1140
ctaagacata atgaaatcta cgaaattaaa gttgacactt tccagcagtt gcttagcctc 1200
cgatcgctga atttggcttg gaacaaaatt gctattattc accccaatgc attttccact 1260
ttgccatccc taataaagct ggacctatcg tccaacctcc tgtcgtcttt tcctataact 1320
gggttacatg gtttaactca cttaaaatta acaggaaatc atgccttaca gagcttgata 1380
tcatctgaaa actttccaga actcaaggtt atagaaatgc cttatgctta ccagtgctgt 1440
gcatttggag tgtgtgagaa tgcctataag atttctaatc aatggaataa aggtgacaac 1500
agcagtatgg acgaccttca taagaaagat gctggaatgt ttcaggctca agatgaacgt 1560
gaccttgaag atttcctgct tgactttgag gaagacctga aagcccttca ttcagtgcag 1620
tgttcacctt ccccaggccc cttcaaaccc tgtgaacacc tgcttgatgg ctggctgatc 1680
agaattggag tgtggaccat agcagttctg gcacttactt gtaatgcttt ggtgacttca 1740
acagttttca gatcccctct gtacatttcc cccattaaac tgttaattgg ggtcatcgca 1800
gcagtgaaca tgctcacggg agtctccagt gccgtgctgg ctggtgtgga tgcgttcact 1860
tttggcagct ttgcacgaca tggtgcctgg tgggagaatg gggttggttg ccatgtcatt 1920
ggttttttgt ccatttttgc ttcagaatca tctgttttcc tgcttactct ggcagccctg 1980
gagcgtgggt tctctgtgaa atattctgca aaatttgaaa cgaaagctcc attttctagc 2040
ctgaaagtaa tcattttgct ctgtgccctg ctggccttga ccatggccgc agttcccctg 2100
ctgggtggca gcaagtatgg cgcctcccct ctctgcctgc ctttgccttt tggggagccc 2160
agcaccatgg gctacatggt cgctctcatc ttgctcaatt ccctttgctt cctcatgatg 2220
accattgcct acaccaagct ctactgcaat ttggacaagg gagacctgga gaatatttgg 2280
gactgctcta tggtaaaaca cattgccctg ttgctcttca ccaactgcat cctaaactgc 2340
cctgtggctt tcttgtcctt ctcctcttta ataaacctta catttatcag tcctgaagta 2400
attaagttta tccttctggt ggtagtccca cttcctgcat gtctcaatcc ccttctctac 2460
atcttgttca atcctcactt taaggaggat ctggtgagcc tgagaaagca aacctacgtc 2520
tggacaagat caaaacaccc aagcttgatg tcaattaact ctgatgatgt cgaaaaacag 2580
tcctgtgact caactcaagc cttggtaacc tttaccagct ccagcatcac ttatgacctg 2640
cctcccagtt ccgtgccatc accagcttat ccagtgactg agagctgcca tctttcctct 2700
gtggcatttg tcccatgtct cgctagcaaa ggagaagaac tcttcactgg agttgtccca 2760
attcttgttg aattagatgg tgatgttaac ggccacaagt tctctgtcag tggagagggt 2820
gaaggtgatg caacatacgg aaaacttacc ctgaagttca tctgcactac tggcaaactg 2880
cctgttccat ggccaacact agtcactact ctgtgctatg gtgttcaatg cttttcaaga 2940
tacccggatc atatgaaacg gcatgacttt ttcaagagtg ccatgcccga aggttatgta 3000
caggaaagga ccatcttctt caaagatgac ggcaactaca agacacgtgc tgaagtcaag 3060
tttgaaggtg atacccttgt taatagaatc gagttaaaag gtattgactt caaggaagat 3120
ggcaacattc tgggacacaa attggaatac aactataact cacacaatgt atacatcatg 3180
gcagacaaac aaaagaatgg aatcaaagtg aacttcaaga cccgccacaa cattgaagat 3240
ggaagcgttc aactagcaga ccattatcaa caaaatactc caattggcga tggccctgtc 3300

cttttaccag acaaccatta cctgtccaca caatctgccc tttcgaaaga tcccaacgaa 3360
aagagagacc acatggtcct tcttgagttt gtaacagctg ctgggattac acatggcatg 3420
gatgaactgt acaactga 3438

<210> 206
<211> 1683
<212> DNA
<213> Human

<400> 206
atggattcaa ccatcccagt cttgggtaca gaactgacac caatcaacgg acgtgaggag 60
actccttgct acaagcagac cctgagcttc acggggctga cgtgcatcgt ttcccttgtc 120
gcgctgacag gaaacgcggt tgtgctctgg ctcctgggct gccgcatgcg caggaacgct 180
gtctccatct acatcctcaa cctggtcgcg gccgacttcc tcttccttag cggccacatt 240
atatgttcgc cgttacgcct catcaatatc cgccatccca tctccaaaat cctcagtcct 300
gtgatgacct ttccctactt tataggccta agcatgctga gcgccatcag caccgagcgc 360
tgcctgtcca tcctgtggcc catctggtac cactgccgcc gccccagata cctgtcatcg 420
gtcatgtgtg tcctgctctg ggccctgtcc ctgctgcgga gtatcctgga gtggatgttc 480
tgtgacttcc tgtttagtgg tgctgattct gtttggtgtg aaacgtcaga tttcattaca 540
atcgcgtggc tggttttttt atgtgtggtt ctctgtgggt ccagcctggt cctgctggtc 600
aggattctct gtggatcccg gaagatgccg ctgaccaggc tgtacgtgac catcctcctc 660
acagtgctgg tcttcctcct ctgtggcctg ccctttggca ttcagtgggc cctgttttcc 720
aggatccacc tggattggaa agtcttattt tgtcatgtgc atctagtttc cattttcctg 780
tccgctctta acagcagtgc caaccccatc atttacttct tcgtgggctc ctttaggcag 840
cgtcaaaata ggcagaacct gaagctggtt ctccagaggg ctctgcagga cacgcctgag 900
gtggatgaag gtggagggtg gcttcctcag gaaaccctgg agctgtcggg aagcagattg 960
gagcaggcta gcaaaggaga agaactcttc actggagttg tcccaattct tgttgaatta 1020
gatggtgatg ttaacggcca caagttctct gtcagtggag agggtgaagg tgatgcaaca 1080
tacggaaaac ttaccctgaa gttcatctgc actactggca aactgcctgt tccatggcca 1140
acactagtca ctactctgtg ctatggtgtt caatgctttt caagataccc ggatcatatg 1200
aaacggcatg actttttcaa gagtgccatg cccgaaggtt atgtacagga aaggaccatc 1260
ttcttcaaag atgacggcaa ctacaagaca cgtgctgaag tcaagtttga aggtgatacc 1320
cttgttaata gaatcgagtt aaaaggtatt gacttcaagg aagatggcaa cattctggga 1380
cacaaattgg aatacaacta taactcacac aatgtataca tcatggcaga caaacaaaag 1440
aatggaatca aagtgaactt caagacccgc cacaacattg aagatggaag cgttcaacta 1500
gcagaccatt atcaacaaaa tactccaatt ggcgatggcc ctgtcctttt accagacaac 1560
cattacctgt ccacacaatc tgccctttcg aaagatccca acgaaaagag agaccacatg 1620
gtccttcttg agtttgtaac agctgctggg attacacatg gcatggatga actgtacaac 1680
tga 1683

<210> 207
<211> 1707
<212> DNA
<213> Human

<400> 207
atggatccaa ccaccccggc ctggggaaca gaaagtacaa cagtgaatgg aaatgaccaa 60
gcccttcttc tgctttgtgg caaggagacc ctgatcccgg tcttcctgat ccttttcatt 120
gccctggtcg ggctggtagg aaacgggttt gtgctctggc tcctgggctt ccgcatgcgc 180
aggaacgcct tctctgtcta cgtcctcagc ctggccgggg ccgacttcct cttcctctgc 240
ttccagatta taaattgcct ggtgtacctc agtaacttct tctgttccat ctccatcaat 300
ttccctagct tcttcaccac tgtgatgacc tgtgcctacc ttgcaggcct gagcatgctg 360
agcaccgtca gcaccgagcg ctgcctgtcc gtcctgtggc ccatctggta tcgctgccgc 420
cgccccagac acctgtcagc ggtcgtgtgt gtcctgctct gggccctgtc cctactgctg 480
agcatcttgg aagggaagtt ctgtggcttc ttatttagtg atggtgactc tggttggtgt 540
cagacatttg atttcatcac tgcagcgtgg ctgatttttt tattcatggt tctctgtggg 600
tccagtctgg ccctgctggt caggatcctc tgtggctcca ggggtctgcc actgaccagg 660
ctgtacctga ccatcctgct cacagtgctg gtgttcctcc tctgcggcct gccctttggc 720
attcagtggt tcctaatatt atggatctgg aaggattctg atgtcttatt ttgtcatatt 780
catccagttt cagttgtcct gtcatctctt aacagcagtg ccaaccccat catttacttc 840
ttcgtgggct cttttaggaa gcagtggcgg ctgcagcagc cgatcctcaa gctggctctc 900
cagagggctc tgcaggacat tgctgaggtg gatcacagtg aaggatgctt ccgtcagggc 960
accccggaga tgtcgagaag cagtctggtg gctagcaaag gagaagaact cttcactgga 1020
gttgtcccaa ttcttgttga attagatggt gatgttaacg gccacaagtt ctctgtcagt 1080
ggagagggtg aaggtgatgc aacatacgga aaacttaccc tgaagttcat ctgcactact 1140
ggcaaactgc ctgttccatg gccaacacta gtcactactc tgtgctatgg tgttcaatgc 1200
ttttcaagat acccggatca tatgaaacgg catgactttt tcaagagtgc catgcccgaa 1260
ggttatgtac aggaaaggac catcttcttc aaagatgacg gcaactacaa gacacgtgct 1320
gaagtcaagt ttgaaggtga tacccttgtt aatagaatcg agttaaaagg tattgacttc 1380
aaggaagatg gcaacattct gggacacaaa ttggaataca actataactc acacaatgta 1440
tacatcatgg cagacaaaca aaagaatgga atcaaagtga acttcaagac ccgccacaac 1500
attgaagatg gaagcgttca actagcagac cattatcaac aaaatactcc aattggcgat 1560
ggccctgtcc ttttaccaga caaccattac ctgtccacac aatctgccct ttcgaaagat 1620
cccaacgaaa agagagacca catggtcctt cttgagtttg taacagctgc tgggattaca 1680
catggcatgg atgaactgta caactga 1707

<210> 208
<211> 1653
<212> DNA
<213> Human

<400> 208
atgatggagc ccagagaagc tggacagcac gtgggggccg ccaacagcgc ccaggaggat 60
gtggccttca acctcatcat cctgtccctc accgaggggc tcggcctcgg tgggctgctg 120
gggaatgggg cagtcctctg gctgctcagc tccaatgtct acagaaaccc cttcgccatc 180
tacctcctgg acgtggcctg cgcggatctc atcttccttg gctgccacat ggtggccatc 240
gtccccgact tgctgcaagg ccggctggac ttcccgggct tcgtgcagac cagcctggca 300
acgctgcgct tcttctgcta catcgtgggc ctgagtctcc tggcggccgt cagcgtggag 360
cagtgcctgg ccgccctctt cccagcctgg tactcgtgcc gccgcccacg ccacctgacc 420
acctgtgtgt gcgccctcac ctgggccctc tgcctgctgc tgcacctgct gctcagcagc 480
gcctgcaccc agttcttcgg ggagcccagc cgccacttgt gccggacgct gtggctggtg 540
gcagcggtgc tgctggctct gctgtgttgc accatgtgtg ggccagcct tatgctgctg 600
ctgcgggtgg agcgaggccc ccagcggccc ccaccccggg gcttccctgg gctcatcctc 660
ctcaccgtcc tcctcttcct cttctgcggc ctgcccttcg gcatctactg gctgtcccgg 720
aacctgctct ggtacatccc ccactacttc taccacttca gcttcctcat ggccgccgtg 780
cactgcgcgg ccaagcccgt cgtctacttc tgcctgggca gtgcccaggg ccgcaggctg 840
cccctccggc tggtcctcca gcgagcgctg ggagacgagg ctgagctggg ggccgtcagg 900
gagacctccc gccggggcct ggtggacata gcagccgcta gcaaaggaga agaactcttc 960
actggagttg tcccaattct tgttgaatta gatggtgatg ttaacggcca caagttctct 1020
gtcagtggag agggtgaagg tgatgcaaca tacggaaaac ttaccctgaa gttcatctgc 1080
actactggca aactgcctgt tccatggcca acactagtca ctactctgtg ctatggtgtt 1140
caatgctttt caagataccc ggatcatatg aaacggcatg actttttcaa gagtgccatg 1200
cccgaaggtt atgtacagga aaggaccatc ttcttcaaag atgacggcaa ctacaagaca 1260
cgtgctgaag tcaagtttga aggtgatacc cttgttaata gaatcgagtt aaaaggtatt 1320
gacttcaagg aagatggcaa cattctggga cacaaattgg aatacaacta taactcacac 1380
aatgtataca tcatggcaga caaacaaaag aatggaatca aagtgaactt caagacccgc 1440
cacaacattg aagatggaag cgttcaacta gcagaccatt atcaacaaaa tactccaatt 1500
ggcgatggcc ctgtcctttt accagacaac cattacctgt ccacacaatc tgccctttcg 1560
aaagatccca acgaaaagag agaccacatg gtccttcttg agtttgtaac agctgctggg 1620
attacacatg gcatggatga actgtacaac tga 1653

<210> 209
<211> 1686
<212> DNA
<213> Human

<400> 209
atgccactcc ctgtgccccc agcgggggca caaaagactc ctgaggacca tgtctgtctc 60
catcttgcag gtccctcgcc agcaccctct gagccggcca ggatgtttgg gctgttcggc 120
ctctggagaa ccttcgacag tgtggtcttc tacctgacgc tgatcgtggg cctcggggga 180
ccggtaggta acgggctggt gctctggaac ctcggcttcc gcatcaagaa gggccccttc 240
tccatctacc tgctgcacct ggccgccgcc gacttcctgt tcctctcctg ccgtgtgggc 300
ttctccgtgg ctcaggctgc cctgggcgcc caggacacac tctacttcgt gctcaccttc 360
ctgtggttcg cggtggggct ctggctgctg gcggccttca gcgtggagcg ctgcctctcc 420
gacctcttcc ccgcctgcta ccagggctgc cggcccagac acgcctcggc cgtcctctgc 480
gccctggtgt ggacccccac cctgccggcc gtgccgctgc ccgccaacgc ctgcggcctg 540
ctgcgcaaca gcgcgtgccc cctggtctgc ccgcgctacc acgtggccag cgtcacctgg 600
ttcctggtgc tggcccgcgt cgcctggacg gctggcgtgg tcctctttgt ctgggtgacc 660
tgctgctcca ctcgcccgcg gcccaggctc tacggcatcg tcctgggcgc gctgctcctg 720
ctcttcttct gtggcctgcc ctcggtcttc tactggagcc tgcagcccct gctgaacttc 780
ctgctgcccg tgttttcccc gctggccacg ctgctggcct gcgtcaacag cagctccaag 840
cccctcatct actcggggtt gggccgacag cccgggaagc gggagccgct gaggtcggta 900
ctgcggaggg ccctggggga gggcgccgag ctgggtgcca ggggacagtc cctgcccatg 960
ggtctcctag ctagcaaagg agaagaactc ttcactggag ttgtcccaat tcttgttgaa 1020
ttagatggtg atgttaacgg ccacaagttc tctgtcagtg gagagggtga aggtgatgca 1080
acatacggaa aacttaccct gaagttcatc tgcactactg gcaaactgcc tgttccatgg 1140
ccaacactag tcactactct gtgctatggt gttcaatgct tttcaagata cccggatcat 1200
atgaaacggc atgacttttt caagagtgcc atgcccgaag gttatgtaca ggaaaggacc 1260
atcttcttca aagatgacgg caactacaag acacgtgctg aagtcaagtt tgaaggtgat 1320
acccttgtta atagaatcga gttaaaaggt attgacttca aggaagatgg caacattctg 1380
ggacacaaat tggaatacaa ctataactca cacaatgtat acatcatggc agacaaacaa 1440
aagaatggaa tcaaagtgaa cttcaagacc cgccacaaca ttgaagatgg aagcgttcaa 1500
ctagcagacc attatcaaca aaatactcca attggcgatg gccctgtcct tttaccagac 1560
aaccattacc tgtccacaca atctgccctt tcgaaagatc ccaacgaaaa gagagaccac 1620
atggtccttc ttgagtttgt aacagctgct gggattacac atggcatgga tgaactgtac 1680
aactga 1686

<210> 210
<211> 1776
<212> DNA

<213> Human

<400> 210
   atggagcaca cgcacgccca cctcgcagcc aacagctcgc tgtcttggtg gtcccccggc 60
   tcggcctgcg gcttgggttt cgtgcccgtg gtctactaca gcctcttgct gtgcctcggt 120
   ttaccagcaa atatcttgac agtgatcatc ctctcccagc tggtggcaag aagacagaag 180
   tcctcctaca actatctctt ggcactcgct gctgccgaca tcttggtcct ctttttcata 240
   gtgtttgtgg acttcctgtt ggaagatttc atcttgaaca tgcagatgcc tcaggtcccc 300
   gacaagatca tagaagtgct ggaattctca tccatccaca cctccatatg gattactgta 360
   ccgttaacca ttgacaggta tatcgctgtc tgccacccgc tcaagtacca cacggtctca 420
   tacccagccc gcacccggaa agtcattgta agtgtttaca tcacctgctt cctgaccagc 480
   atcccctatt actggtggcc caacatctgg actgaagact acatcagcac ctctgtgcat 540
   cacgtcctca tctggatcca ctgcttcacc gtctacctgg tgccctgctc catcttcttc 600
   atcttgaact caatcattgt gtacaagctc aggaggaaga gcaattttcg tctccgtggc 660
   tactccacgg ggaagaccac cgccatcttg ttcaccatta cctccatctt tgccacactt 720
   tgggcccccc gcatcatcat gattctttac cacctctatg gggcgcccat ccagaaccgc 780
   tggctggtgc acatcatgtc cgacattgcc aacatgctag cccttctgaa cacagccatc 840
   aacttcttcc tctactgctt catcagcaag cggttccgca ccatggcagc cgccacgctc 900
   aaggctttct tcaagtgcca gaagcaacct gtacagttct acaccaatca taacttttcc 960
   ataacaagta gcccctggat ctcgccggca aactcacact gcatcaagat gctggtgtac 1020
   cagtatgaca aaaatggaaa acctataaaa gtatccccgt ctagcaaagg agaagaactc 1080
   ttcactggag ttgtcccaat tcttgttgaa ttagatggtg atgttaacgg ccacaagttc 1140
   tctgtcagtg gagagggtga aggtgatgca acatacggaa aacttaccct gaagttcatc 1200
   tgcactactg gcaaactgcc tgttccatgg ccaacactag tcactactct gtgctatggt 1260
   gttcaatgct tttcaagata cccggatcat atgaaacggc atgacttttt caagagtgcc 1320
   atgcccgaag gttatgtaca ggaaaggacc atcttcttca aagatgacgg caactacaag 1380
   acacgtgctg aagtcaagtt tgaaggtgat acccttgtta atagaatcga gttaaaaggt 1440
   attgacttca aggaagatgg caacattctg ggacacaaat tggaatacaa ctataactca 1500
   cacaatgtat acatcatggc agacaaacaa aagaatggaa tcaaagtgaa cttcaagacc 1560
   cgccacaaca ttgaagatgg aagcgttcaa ctagcagacc attatcaaca aaatactcca 1620
   attggcgatg gccctgtcct tttaccagac aaccattacc tgtccacaca atctgccctt 1680
   tcgaaagatc ccaacgaaaa gagagaccac atggtccttc ttgagtttgt aacagctgct 1740
   gggattacac atggcatgga tgaactgtac aactga 1776

<210> 211
<211> 1791
<212> DNA
<213> Human

<400> 211
   atgccctctg tgtctccagc ggggccctcg gccggggcag tccccaatgc caccgcagtg 60
   acaacagtgc ggaccaatgc cagcgggctg gaggtgcccc tgttccacct gtttgcccgg 120
   ctggacgagg agctgcatgg caccttccca ggcctgtggc tggcgctgat ggcggtgcac 180
   ggagccatct tcctggcagg gctggtgctc aacgggctgg cgctgtacgt cttctgctgc 240
   cgcacccggg ccaagacacc ctcagtcatc tacaccatca acctggtggt gaccgatcta 300
   ctggtagggc tgtccctgcc cacgcgcttc gctgtgtact acggcgccag gggctgcctg 360
   cgctgtgcct tcccgcacgt cctcggttac ttcctcaaca tgcactgctc catcctcttc 420
   ctcacctgca tctgcgtgga ccgctacctg gccatcgtgc ggcctgaagg ctcccgccgc 480
   tgccgccagc ctgcctgtgc cagggccgtg tgcgccttcg tgtggctggc cgccggtgcc 540
   gtcaccctgt cggtgctggg cgtgacaggc agccggccct gctgccgtgt ctttgcgctg 600
   actgtcctgg agttcctgct gcctctgctg gtcatcagcg tgtttaccgg ccgcatcatg 660
   tgtgcactgt cgcggccggg tctgctccgc cagggtcgcc agcgccgcgt gcgggccatg 720
   cagctcctgc tcacggtgct catcatcttt ctcgtctgct tcacgccctt ccacgcccgc 780
   caagtggccg tggcgctgtg gcccgacatg ccacaccaca cgagcctcgt ggtctaccac 840
   gtggccgtga ccctcagcag cctcaacagc tgcatggacc ccatcgtcta ctgcttcgtc 900
   accagtggct tccaggccac cgtccgaggc ctcttcggcc agcacggaga gcgtgagccc 960
   agcagcggtg acgtggtcag catgcacagg agctccaagg gctcaggccg tcatcacatc 1020
   ctcagtgccg gccctcacgc cctcacccag gccctggcta atgggcccga ggctgctagc 1080
   aaaggagaag aactcttcac tggagttgtc ccaattcttg ttgaattaga tggtgatgtt 1140
   aacggccaca agttctctgt cagtggagag ggtgaaggtg atgcaacata cggaaaactt 1200
   accctgaagt tcatctgcac tactggcaaa ctgcctgttc catggccaac actagtcact 1260
   actctgtgct atggtgttca atgcttttca agatacccgg atcatatgaa acggcatgac 1320
   tttttcaaga gtgccatgcc cgaaggttat gtacaggaaa ggaccatctt cttcaaagat 1380
   gacggcaact acaagacacg tgctgaagtc aagtttgaag gtgataccct tgttaataga 1440
   atcgagttaa aaggtattga cttcaaggaa gatggcaaca ttctgggaca caaattggaa 1500
   tacaactata actcacacaa tgtatacatc atggcagaca aacaaaagaa tggaatcaaa 1560
   gtgaacttca agacccgcca caacattgaa gatggaagcg ttcaactagc agaccattat 1620
   caacaaaata ctccaattgg cgatggccct gtccttttac cagacaacca ttacctgtcc 1680
   acacaatctg ccctttcgaa agatcccaac gaaaagagag accacatggt ccttcttgag 1740
   tttgtaacag ctgctgggat tacacatggc atggatgaac tgtacaactg a 1791

<210> 212
<211> 1632
<212> DNA
<213> Human

<400> 212
atgcctggcc acaatacctc caggaattcc tcttgcgatc ctatagtgac accccactta 60
atcagcctct acttcatagt gcttattggc gggctggtgg gtgtcatttc cattcttttc 120
ctcctggtga aaatgaacac ccggtcagtg accaccatgg cggtcattaa cttggtggtg 180
gtccacagcg tttttctgct gacagtgcca tttcgcttga cctacctcat caagaagact 240
tggatgtttg ggctgccctt ctgcaaattt gtgagtgcca tgctgcacat ccacatgtac 300
ctcacgttcc tattctatgt ggtgatcctg gtcaccagat acctcatctt cttcaagtgc 360
aaagacaaag tggaattcta cagaaaactg catgctgtgg ctgccagtgc tggcatgtgg 420
acgctggtga ttgtcattgt ggtacccctg gttgtctccc ggtatggaat ccatgaggaa 480
tacaatgagg agcactgttt taaatttcac aaagagcttg cttacacata tgtgaaaatc 540
atcaactata tgatagtcat ttttgtcata gccgttgctg tgattctgtt ggtcttccag 600
gtcttcatca ttatgttgat ggtgcagaag ctacgccact ctttactatc ccaccaggag 660
ttctgggctc agctgaaaaa cctatttttt ataggggtca tccttgtttg tttccttccc 720
taccagttct ttaggatcta ttacttgaat gttgtgacgc attccaatgc ctgtaacagc 780
aaggttgcat tttataacga aatcttcttg agtgtaacag caattagctg ctatgatttg 840
cttctctttg tctttggggg aagccattgg tttaagcaaa agataattgg cttatggaat 900
tgtgttttgt gccgtgctag caaaggagaa gaactcttca ctggagttgt cccaattctt 960
gttgaattag atggtgatgt taacggccac aagttctctg tcagtggaga gggtgaaggt 1020
gatgcaacat acggaaaact taccctgaag ttcatctgca ctactggcaa actgcctgtt 1080
ccatggccaa cactagtcac tactctgtgc tatggtgttc aatgcttttc aagatacccg 1140
gatcatatga aacggcatga ctttttcaag agtgccatgc ccgaaggtta tgtacaggaa 1200
aggaccatct tcttcaaaga tgacggcaac tacaagacac gtgctgaagt caagtttgaa 1260
ggtgataccc ttgttaatag aatcgagtta aaaggtattg acttcaagga agatggcaac 1320
attctgggac acaaattgga atacaactat aactcacaca atgtatacat catggcagac 1380
aaacaaaaga atggaatcaa agtgaacttc aagacccgcc acaacattga agatggaagc 1440
gttcaactag cagaccatta tcaacaaaat actccaattg gcgatggccc tgtcctttta 1500
ccagacaacc attacctgtc cacacaatct gccctttcga aagatcccaa cgaaaagaga 1560
gaccacatgg tccttcttga gtttgtaaca gctgctggga ttacacatgg catggatgaa 1620
ctgtacaact ga 1632

<210> 213
<211> 1734
<212> DNA
<213> Human

<400> 213
atgaatgagc cactagacta tttagcaaat gcttctgatt tccccgatta tgcagctgct 60
tttggaaatt gcactgatga aaacatccca ctcaagatgc actacctccc tgttatttat 120
ggcattatct tcctcgtggg atttccaggc aatgcagtag tgatatccac ttacattttc 180
aaaatgagac cttggaagag cagcaccatc attatgctga acctggcctg cacagatctg 240
ctgtatctga ccagcctccc cttcctgatt cactactatg ccagtggcga aaactggatc 300
tttggagatt tcatgtgtaa gtttatccgc ttcagcttcc atttcaacct gtatagcagc 360
atcctcttcc tcacctgttt cagcatcttc cgctactgtg tgatcattca cccaatgagc 420
tgcttttcca ttcacaaaac tcgatgtgca gttgtagcct gtgctgtggt gtggatcatt 480
tcactggtag ctgtcattcc gatgaccttc ttgatcacat caaccaacag gaccaacaga 540
tcagcctgtc tcgacctcac cagttcggat gaactcaata ctattaagtg gtacaacctg 600
attttgactg caactacttt ctgcctcccc ttggtgatag tgacactttg ctataccacg 660
attatccaca ctctgaccca tggactgcaa actgacagct gccttaagca gaaagcacga 720
aggctaacca ttctgctact ccttgcattt tacgtatgtt ttttaccctt ccatatcttg 780
agggtcattc ggatcgaatc tcgcctgctt tcaatcagtt gttccattga gaatcagatc 840
catgaagctt acatcgtttc tagaccatta gctgctctga acacctttgg taacctgtta 900
ctatatgtgg tggtcagcga caactttcag caggctgtct gctcaacagt gagatgcaaa 960
gtaagcggga accttgagca agcaaagaaa attagttact caaacaaccc tgctagcgct 1020
agcaaaggag aagaactctt cactggagtt gtcccaattc ttgttgaatt agatggtgat 1080
gttaacggcc acaagttctc tgtcagtgga gagggtgaag gtgatgcaac atacggaaaa 1140
cttaccctga agttcatctg cactactggc aaactgcctg ttccatggcc aacactagtc 1200
actactctgt gctatggtgt tcaatgcttt tcaagatacc cggatcatat gaaacggcat 1260
gactttttca agagtgccat gcccgaaggt tatgtacagg aaaggaccat cttcttcaaa 1320
gatgacggca actacaagac acgtgctgaa gtcaagtttg aaggtgatac ccttgttaat 1380
agaatcgagt taaaaggtat tgacttcaag gaagatggca acattctggg acacaaattg 1440
gaatacaact ataactcaca caatgtatac atcatggcag acaaacaaaa gaatggaatc 1500
aaagtgaact tcaagacccg ccacaacatt gaagatggaa gcgttcaact agcagaccat 1560
tatcaacaaa atactccaat tggcgatggc cctgtccttt taccagacaa ccattacctg 1620
tccacacaat ctgccctttc gaaagatccc aacgaaaaga gagaccacat ggtccttctt 1680
gagtttgtaa cagctgctgg gattacacat ggcatggatg aactgtacaa ctga 1734

<210> 214
<211> 1710
<212> DNA
<213> Human

<400> 214
atgatcaccc tgaacaatca agatcaacct gtccctttta acagctcaca tccagatgaa 60
tacaaaattg cagcccttgt cttctatagc tgtatcttca taattggatt atttgttaac 120

atcactgcat tatgggtttt cagttgtacc accaagaaga gaaccacggt aaccatctat 180
atgatgaatg tggcattagt ggacttgata tttataatga ctttaccctt tcgaatgttt 240
tattatgcaa aagatgaatg gccatttgga gagtacttct gccagattct tggagctctc 300
acagtgtttt acccaagcat tgctttatgg cttcttgcct ttattagtgc tgacagatac 360
atggccattg tacagccgaa gtacgccaaa gaacttaaaa acacgtgcaa agccgtgctg 420
gcgtgtgtgg gagtctggat aatgaccctg accacgacca cccctctgct actgctctat 480
aaagacccag ataaagactc cactcccgcc acctgcctca agatttctga catcatctat 540
ctaaaagctg tgaacgtgct gaacctcact cgactgacat ttttttcttt gattcctttg 600
ttcatcatga ttgggtgcta cttggtcatt attcataatc tccttcacgg caggacgtct 660
aagctgaaac ccaaagtcaa ggagaagtcc ataaggatca tcatcacgct gctggtgcag 720
gtgctcgtct gctttatgcc cttccacatc tgtttcgctt tcctgatgct gggaacgggg 780
gagaacagtt acaatccctg ggagcccttt accaccttcc tcatgaacct cagcacgtgt 840
ctggatgtga ttctctacta catcgtttca aaacaatttc aggctcgagt cattagtgtc 900
atgctatacc gtaattacct tcgaagcatg cgcagaaaaa gtttccgatc tggtagtcta 960
cggtcactaa gcaatataaa cagtgaaatg ttagctagca aaggagaaga actcttcact 1020
ggagttgtcc caattcttgt tgaattagat ggtgatgtta acggccacaa gttctctgtc 1080
agtggagagg gtgaaggtga tgcaacatac ggaaaactta ccctgaagtt catctgcact 1140
actggcaaac tgcctgttcc atggccaaca ctagtcacta ctctgtgcta tggtgttcaa 1200
tgcttttcaa gatacccgga tcatatgaaa cggcatgact ttttcaagag tgccatgccc 1260
gaaggttatg tacaggaaag gaccatcttc ttcaaagatg acggcaacta caagacacgt 1320
gctgaagtca agtttgaagg tgataccctt gttaatagaa tcgagttaaa aggtattgac 1380
ttcaaggaag atggcaacat tctgggacac aaattggaat acaactataa ctcacacaat 1440
gtatacatca tggcagacaa acaaaagaat ggaatcaaag tgaacttcaa gacccgccac 1500
aacattgaag atggaagcgt tcaactagca gaccattatc aacaaaatac tccaattggc 1560
gatggccctg tccttttacc agacaaccat tacctgtcca cacaatctgc cctttcgaaa 1620
gatcccaacg aaaagagaga ccacatggtc cttcttgagt ttgtaacagc tgctgggatt 1680
acacatggca tggatgaact gtacaactga 1710

<210> 215
<211> 2364
<212> DNA
<213> Human

<400> 215
atggcgacgc ccaggggcct gggggccctg ctcctgctcc tcctgctccc gacctcaggt 60
caggaaaagc ccaccgaagg gccaagaaac acctgcctgg ggagcaacaa catgtacgac 120
atcttcaact tgaatgacaa ggctttgtgc ttcaccaagt gcaggcagtc gggcagcgac 180
tcctgcaatg tggaaaactt gcagagatac tggctaaact acgaggccca tctgatgaag 240
gaaggtttga cgcagaaggt gaacacgcct ttcctgaagg ctttggtcca gaacctcagc 300
accaacactg cagaagactt ctatttctct ctggagccct ctcaggttcc gaggcaggtg 360
atgaaggacg aggacaagcc ccctgacaga gtgcgacttc ccaagagcct tttcgatccc 420
ctgccaggca acaggtctgt ggtccgcttg gccgtcacca ttctggacat tggtccaggg 480
actctcttca agggcccccg gctcggcctg ggagatggca gcggcgtgtt gaacaatcgc 540
ctggtgggtt tgagtgtggg acaaatgcat gtcaccaagc tggctgagcc tctggagatc 600
gtcttctctc accagcgacc gccccctaac atgaccctca cctgtgtatt ctgggatgtg 660
actaaaggga ccactggaga ctggtcttct gagggctgct ccacggaggt cagacctgag 720
gggaccgtgt gctgctgtga ccacctgacc tttttcgccc tgctcctgag acccaccttg 780
gaccagtcca cggtgcatat cctcacacgc atctcccagg cgggctgtgg ggtctccatg 840
atcttcctgg ccttcaccat tattctttat gcctttctga ggctttcccg ggagaggttc 900
aagtcagaag atgcccccaa gatccacgtg gccctgggtg gcagcctgtt cctcctgaat 960
ctggccttct tggtcaatgt ggggagtggc tcaaaggggt ctgatgctgc ctgctgggcc 1020
cggggggctg tcttccacta cttcctgctc tgtgccttca cctggatggg ccttgaagcc 1080
ttccacctct acctgctcgc tgtcagggtc ttcaacacct acttcgggca ctacttcctg 1140
aagctgagcc tggtgggctg gggcctgccc gccctgatgg tcatcggcac tgggagtgcc 1200
aacagctacg gcctctacac catccgtgat agggagaacc gcacctctct ggagctatgc 1260
tggttccgtg aagggacaac catgtacgcc ctctatatca ccgtccacgg ctacttcctc 1320
atcaccttcc tctttggcat ggtggtcctg gccctggtgg tctggaagat cttcaccctg 1380
tcccgtgcta cagcggtcaa ggagcggggg aagaaccgga agaaggtgct caccctgctg 1440
ggcctctcga gcctggtggg tgtgacatgg gggttggcca tcttcacccc gttgggcctc 1500
tccaccgtct acatctttgc acttttcaac tccttgcaag gtgtcttcat ctgctgctgg 1560
ttcaccatcc tttacctccc aagtcagagc accacagtct cctcctctac tgcaagattg 1620
gaccaggccc actccgcatc tcaagaagct agcaaaggag aagaactctt cactggagtt 1680
gtcccaattc ttgttgaatt agatggtgat gttaacggcc acaagttctc tgtcagtgga 1740
gagggtgaag gtgatgcaac atacggaaaa cttaccctga agttcatctg cactactggc 1800
aaactgcctg ttccatggcc aacactagtc actactctgt gctatggtgt tcaatgcttt 1860
tcaagatacc cggatcatat gaaacggcat gactttttca agagtgccat gcccgaaggt 1920
tatgtacagg aaaggaccat cttcttcaaa gatgacggca actacaagac acgtgctgaa 1980
gtcaagtttg aaggtgatac ccttgttaat agaatcgagt taaaaggtat tgacttcaag 2040
gaagatggca acattctggg acacaaattg gaatacaact ataactcaca caatgtatac 2100
atcatggcag acaaacaaaa gaatggaatc aaagtgaact tcaagacccg ccacaacatt 2160
gaagatggaa gcgttcaact agcagaccat tatcaacaaa atactccaat tggcgatggc 2220
cctgtccttt taccagacaa ccattacctg tccacacaat ctgccctttc gaaagatccc 2280
aacgaaaaga gagaccacat ggtccttctt gagtttgtaa cagctgctgg gattacacat 2340
ggcatggatg aactgtacaa ctga 2364

<210> 216
<211> 1827
<212> DNA
<213> Human

<400> 216
atgggtgaca gaagattcat tgacttccaa ttccaagatt caaattcaag cctcagaccc 60
aggttgggca atgctactgc caataatact tgcattgttg atgattcctt caagtataat 120
ctcaatggtg ctgtctacag tgttgtattc atcttgggtc tgataaccaa cagtgtctct 180
ctgtttgtct tctgtttccg catgaaaatg agaagtgaga ctgctatttt tatcaccaat 240
ctagctgtct ctgatttgct ttttgtctgt acactacctt ttaaaatatt ttacaacttc 300
aaccgccact ggccttttgg tgacaccctc tgcaagatct ctggaactgc attccttacc 360
aacatctatg ggagcatgct ctttctcacc tgtattagtg tggatcgttt cctggccatt 420
gtctatcctt ttcgatctcg tactattagg actaggagga attctgccat tgtgtgtgct 480
ggtgtctgga tcctagtcct cagtggcggt atttcagcct ctttgttttc caccactaat 540
gtcaacaatg caaccaccac ctgctttgaa ggcttctcca aacgtgtctg gaagacttat 600
ttatccaaga tcacaatatt tattgaagtt gttgggttta tcattcctct aatattgaat 660
gtctcttgct cttctgtggt gctgagaact cttcgcaagc ctgctactct gtctcaaatt 720
gggaccaata agaaaaaagt actgaaaatg atcacagtac atatggcagt ctttgtggta 780
tgctttgtac cctacaactc tgtcctcttc ttgtatgccc tggtgcgctc ccaagctatt 840
actaattgct tttggaaag atttgcaaag atcatgtacc caatcacctt gtgccttgca 900
actctgaact gttgttttga ccctttcatc tattacttca cccttgaatc ctttcagaag 960
tccttctaca tcaatgccca catcagaatg gagtccctgt ttaagactga aacacctttg 1020
accacaaagc cttcccttcc agctattcaa gaggaagtga gtgatcaaac aacaaataat 1080
ggtggtgaat taatgctaga atccaccttt gctagcaaag gagaagaact cttcactgga 1140
gttgtcccaa ttcttgttga attagatggt gatgttaacg gccacaagtt ctctgtcagt 1200
ggagagggtg aaggtgatgc aacatacgga aaacttaccc tgaagttcat ctgcactact 1260
ggcaaactgc ctgttccatg gccaacacta gtcactactc tgtgctatgg tgttcaatgc 1320
ttttcaagat acccggatca tatgaaacgg catgactttt tcaagagtgc catgcccgaa 1380
ggttatgtac aggaaaggac catcttcttc aaagatgacg gcaactacaa gacacgtgct 1440
gaagtcaagt ttgaaggtga tacccttgtt aatagaatcg agttaaaagg tattgacttc 1500
aaggaagatg gcaacattct gggacacaaa ttggaataca actataactc acacaatgta 1560
tacatcatgg cagacaaaca aaagaatgga atcaaagtga acttcaagac ccgccacaac 1620
attgaagatg gaagcgttca actagcagac cattatcaac aaaatactcc aattggcgat 1680
ggccctgtcc ttttaccaga caaccattac ctgtccacac aatctgccct ttcgaaagat 1740
cccaacgaaa agagagacca catggtcctt cttgagtttg taacagctgc tgggattaca 1800
catggcatgg atgaactgta caactga 1827

<210> 217
<211> 1734
<212> DNA
<213> Human

<400> 217
atggctaacc ttgacaaata cactgaaaca ttcaagatgg gtagcaacag taccagcact 60
gctgagattt actgtaatgt cactaatgtg aaatttcaat actccctcta tgcaaccacc 120
tatatcctca tattcattcc tggtcttctg gctaacagtg cagccttgtg ggttctgtgc 180
cgcttcatca gcaagaaaaa taaagccatc attttcatga tcaacctctc tgtggctgac 240
cttgctcatg tattatcttt acccctccgg atttactatt acatcagcca ccactggcct 300
ttccagagag ccctttgcct gctctgcttc tacctgaagt atctcaacat gtatgccagc 360
atttgtttcc tgacgtgcat cagtcttcaa aggtgctttt ttctcctcaa gcccttcagg 420
gccagagact ggaagcgtag gtacgatgtg ggcatcagtg ctgccatctg gatcgttgtg 480
gggactgcct gtttgccatt tcccatcctg agaagcacag acttaaacaa caacaagtcc 540
tgctttgctg atcttggata caagcaaatg aatgcagttg cgttggtcgg gatgattaca 600
gttgctgagc ttgcaggatt tgtgatccca gtgatcatca tcgcatggtg tacctggaaa 660
actactatat ccttgagaca gccaccaatg gctttccaag ggatcagtga gaggcagaaa 720
gcactgcgga tggtgttcat gtgtgctgca gtcttcttca tctgcttcac tccctatcat 780
attaacttta ttttttacac catggtaaag gaaaccatca ttagcagttg tcccgttgtc 840
cgaatcgcac tgtatttcca ccctttttgc ctgtgccttg caagtctctg ctgccttttg 900
gatccaattc tttattactt tatggcttca gagtttcgtg accaactatc ccgccatggc 960
agttctgtga cccgctcccg cctcatgagc aaggagagtg gttcatcaat gattggctct 1020
agcaaaggag aagaactctt cactggagtt gtcccaattc ttgttgaatt agatggtgat 1080
gttaacggcc acaagttctc tgtcagtgga gagggtgaag gtgatgcaac atacggaaaa 1140
cttaccctga agttcatctg cactactggc aaactgcctg ttccatggcc aacactagtc 1200
actactctgt gctatggtgt tcaatgcttt tcaagatacc cggatcatat gaaacggcat 1260
gactttttca agagtgccat gcccgaaggt tatgtacagg aaaggaccat cttcttcaaa 1320
gatgacggca actacaagac acgtgctgaa gtcaagtttg aaggtgatac ccttgttaat 1380
agaatcgagt taaaaggtat tgacttcaag gaagatggca acattctggg acacaaattg 1440
gaatacaact ataactcaca caatgtatac atcatggcag acaaacaaaa gaatggaatc 1500
aaagtgaact tcaagacccg ccacaacatt gaagatggaa gcgttcaact agcagaccat 1560
tatcaacaaa atactccaat tggcgatggc cctgtccttt taccagacaa ccattacctg 1620
tccacacaat ctgccctttc gaaagatccc aacgaaaaga gagaccacat ggtccttctt 1680
gagtttgtaa cagctgctgg gattacacat ggcatggatg aactgtacaa ctga 1734

<210> 218

<211> 2556
<212> DNA
<213> Human

<400> 218
atgcgagccc cgggcgcgct tctcgcccgc atgtcgcggc tactgcttct gctactgctc 60
aaggtgtctg cctcttctgc cctcggggtc gcccctgcgt ccagaaacga aacttgtctg 120
gggagagagct gtgcacctac agtgatccag cgccgcggca gggacgcctg ggaccgggga 180
aattctgcaa gagacgttct gcgagcccga gcacccaggg aggagcaggg ggcagcgttt 240
cttgcgggac cctcctggga cctgccggcg gccccgggcc gtgacccggc tgcaggcaga 300
ggggcggagg cgtcggcagc cggacccccg ggacctccaa ccaggccacc tggcccctgg 360
aggtggaaag gtgctcgggg tcaggagcct tctgaaactt tggggagagg gaaccccacg 420
gccctccagc tcttccttca gatctcagag gaggaagaga agggtcccag aggcgctggc 480
atttccgggc gtagccagga gcagagtgtg aagacagtcc ccggagccag cgatcttttt 540
tactggccaa ggagagccgg gaaactccag ggttcccacc acaagcccct gtccaagacg 600
gccaatggac tggcggggca cgaagggtgg acaattgcac tcccgggccg ggcgctggcc 660
cagaatggat ccttgggtga aggaatccat gagcctgggg gtccccgccg gggaaacagc 720
acgaaccggc gtgtgagact gaagaacccc ttctacccgc tgacccagga gtcctatgga 780
gcctacgcgg tcatgtgtct gtccgtggtg atcttcggga ccggcatcat tggcaacctg 840
gcggtgatgt gcatcgtgtg ccacaactac tacatgcgga gcatctccaa ctccctcttg 900
gccaacctgg ccttctggga ctttctcatc atcttcttct gccttccgct ggtcatcttc 960
cacgagctga ccaagaagtg gctgctggag gacttctcct gcaagatcgt gccctatata 1020
gaggtcgctt ctctgggagt caccactttc accttatgtg ctctgtgcat agaccgcttc 1080
cgtgctgcca ccaacgtaca gatgtactac gaaatgatcg aaaactgttc ctcaacaact 1140
gccaaacttg ctgttatatg ggtgggagct ctattgttag cacttccaga agttgttctc 1200
cgccagctga gcaaggagga tttggggttt agtggccgag ctccggcaga aaggtgcatt 1260
attaagatct ctcctgattt accagacacc atctatgttc tagccctcac ctacgacagt 1320
gcgagactgt ggtggtattt tggctgttac ttttgtttgc ccacgctttt caccatcacc 1380
tgctctctag tgactgcgag gaaaatccgc aaagcagaga aagcctgtac ccgagggaat 1440
aaacggcaga ttcaactaga gagtcagatg aactgtacag tagtggcact gaccatttta 1500
tatggatttt gcattattcc tgaaaatatc tgcaacattg ttactgccta catggctaca 1560
ggggtttcac agcagacaat ggacctcctt aatatcatca gccagttcct tttgttcttt 1620
aagtcctgtg tcaccccagt cctccttttc tgtctctgca aacccttcag tcgggccttc 1680
atggagtgct gctgctgttg ctgtgaggaa tgcattcaga agtcttcaac ggtgaccagt 1740
gatgacaatg acaacgagta caccacggaa ctcgaactct cgcctttcag taccatacgc 1800
cgtgaaatgt ccacttttgc ttctgtcgga actcattgcg ctagcaaagg agaagaactc 1860
ttcactggag ttgtcccaat tcttgttgaa ttagatggtg atgttaacgg ccacaagttc 1920
tctgtcagtg gagagggtga aggtgatgca acatacggaa aacttaccct gaagttcatc 1980
tgcactactg gcaaactgcc tgttccatgg ccaacactag tcactactct gtgctatggt 2040
gttcaatgct tttcaagata cccggatcat atgaaacggc atgacttttt caagagtgcc 2100
atgcccgaag gttatgtaca ggaaaggacc atcttcttca aagatgacgg caactacaag 2160
acacgtgctg aagtcaagtt tgaaggtgat acccttgtta atagaatcga gttaaaaggt 2220
attgacttca aggaagatgg caacattctg ggacacaaat tggaatacaa ctataactca 2280
cacaatgtat acatcatggc agacaaacaa aagaatggaa tcaaagtgaa cttcaagacc 2340
cgccacaaca ttgaagatgg aagcgttcaa ctagcagacc attatcaaca aaatactcca 2400
attggcgatg gccctgtcct tttaccagac aaccattacc tgtccacaca atctgccctt 2460
tcgaaagatc ccaacgaaaa gagagaccac atggtccttc ttgagtttgt aacagctgct 2520
gggattacac atggcatgga tgaactgtac aactga 2556

<210> 219
<211> 2160
<212> DNA
<213> Human

<400> 219
atgcggtggc tgtggcccct ggctgtctct cttgctgtga ttttggctgt ggggctaagc 60
agggtctctg gggtgccccc cctgcacctg ggcaggcaca gagccgagac ccaggagcag 120
cagagccgat ccaagagggg caccgaggat gaggaggcca agggcgtgca gcagtatgtg 180
cctgaggagt gggcggagta cccccggccc attcaccctg ctggcctgca gccaaccaag 240
cccttggtgg ccaccagccc taaccccgac aaggatgggg gcaccccaga cagtgggcag 300
gaactgaggg gcaatctgac aggggcacca gggcagaggc tacagatcca gaaccccctg 360
tatccggtga ccgagagctc ctacagtgcc tatgccatca tgcttctggc gctggtggtg 420
tttgcggtgg gcattgtggg caacctgtcg gtcatgtgca tcgtgtggca cagctactac 480
ctgaagagcg cctggaactc catccttgcc agcctggccc tctgggattt tctggtcctc 540
tttttctgcc tccctattgt catcttcaac gagatcacca agcagaggct actgggtgac 600
gtttcttgtc gtgccgtgcc cttcatggag gtctcctctc tgggagtcac gactttcagc 660
ctctgtgccc tgggcattga ccgcttccac gtggccacca gcaccctgcc caaggtgagg 720
cccatcgagc ggtgccaatc catcctggcc aagttggctg tcatctgggt gggctccatg 780
acgctggctg tgcctgagct cctgctgtgg cagctggcac aggagcctgc ccccaccatg 840
ggcaccctgg actcatgcat catgaaaccc tcagccagcc tgcccgagtc cctgtattca 900
ctggtgatga cctaccagaa cgcccgcatg tggtggtact ttggctgcta cttctgcctg 960
cccatcctct tcacagtcac ctgccagctg gtgacatggc gggtgcgagg ccctccaggg 1020
aggaagtcag agtgcagggc cagcaagcac gagcagtgtg agagccagct caacagcacc 1080
gtggtgggcc tgaccgtggt ctacgccttc tgcaccctcc cagagaacgt ctgcaacatc 1140
gtggtggcct acctctccac cgagctgacc cgccagaccc tggacctcct gggcctcatc 1200

aaccagttct ccaccttctt caagggcgcc atcaccccag tgctgctcct ttgcatctgc 1260
aggccgctgg gccaggcctt cctggactgc tgctgctgct gctgctgtga ggagtgcggc 1320
ggggcttcgg aggcctctgc tgccaatggg tcggacaaca agctcaagac cgaggtgtcc 1380
tcttccatct acttccacaa gcccagggag tcacccccac tcctgcccct gggcacacct 1440
tgcgctagca aaggagaaga actcttcact ggagttgtcc caattcttgt tgaattagat 1500
ggtgatgtta acggccacaa gttctctgtc agtggagagg gtgaaggtga tgcaacatac 1560
ggaaaactta ccctgaagtt catctgcact actggcaaac tgcctgttcc atggccaaca 1620
ctagtcacta ctctgtgcta tggtgttcaa tgcttttcaa gatacccgga tcatatgaaa 1680
cggcatgact ttttcaagag tgccatgccc gaaggttatg tacaggaaag gaccatcttc 1740
ttcaaagatg acggcaacta caagacacgt gctgaagtca agtttgaagg tgataccctt 1800
gttaatagaa tcgagttaaa aggtattgac ttcaaggaag atggcaacat tctgggacac 1860
aaattggaat acaactataa ctcacacaat gtatacatca tggcagacaa acaaaagaat 1920
ggaatcaaag tgaacttcaa gacccgccac aacattgaag atggaagcgt tcaactagca 1980
gaccattatc aacaaaatac tccaattggc gatggccctg tccttttacc agacaaccat 2040
tacctgtcca cacaatctgc cctttcgaaa gatcccaacg aaaagagaga ccacatggtc 2100
cttcttgagt ttgtaacagc tgctgggatt acacatggca tggatgaact gtacaactga 2160

<210> 220
<211> 1776
<212> DNA
<213> Human

<400> 220
atggaaacca acttctccat tcctctgaat gaaactgagg aggtgctccc tgagcctgct 60
ggccacaccg ttctgtggat cttctcattg ctagtccacg gagtcacctt tgtcttcggg 120
gtcctgggca atgggcttgt gatctgggtg gctggattcc ggatgacacg cacagtcaac 180
accatctgtt acctgaacct ggccctagct gacttctctt tcagtgccat cctaccattc 240
cgaatggtct cagtcgccat gagagaaaaa tggccttttg gctcattcct atgtaagtta 300
gttcatgtta tgatagacat caacctgttt gtcagtgtct acctgatcac catcattgct 360
ctggaccgct gtatttgtgt cctgcatcca gcctgggccc agaaccatcg caccatgagt 420
ctggccaaga gggtgatgac gggactctgg attttcacca tagtccttac cttaccaaat 480
ttcatcttct ggactacaat aagtactacg aatggggaca catactgtat tttcaacttt 540
gcattctggg gtgacactgc tgtagagagg ttgaacgtgt tcattaccat ggccaaggtc 600
tttctgatcc tccacttcat tattggcttc agcgtgccta tgtccatcat cacagtctgc 660
tatgggatca tcgctgccaa aattcacaga aaccacatga ttaaatccag ccgtccctta 720
cgtgtcttcg ctgctgtggt ggcttctttc ttcatctgtt ggttccctta tgaactaatt 780
ggcattctaa tggcagtctg gctcaaagag atgttgttaa atggcaaata caaaatcatt 840
cttgtcctga ttaacccaac aagctccttg gcctttttta acagctgcct caacccaatt 900
ctctacgtct ttatgggtcg taacttccaa gaaagactga ttcgctcttt gcccactagt 960
ttggagaggg ccctgactga ggtccctgac tcagcccaga ccagcaacac agacaccact 1020
tctgcttcac ctcctgagga gacggagtta caagcaatgg ctagcaaagg agaagaactc 1080
ttcactggag ttgtcccaat tcttgttgaa ttagatggtg atgttaacgg ccacaagttc 1140
tctgtcagtg gagagggtga aggtgatgca acatacggaa aacttaccct gaagttcatc 1200
tgcactactg gcaaactgcc tgttccatgg ccaacactag tcactactct gtgctatggt 1260
gttcaatgct tttcaagata cccggatcat atgaaacggc atgacttttt caagagtgcc 1320
atgcccgaag gttatgtaca ggaaaggacc atcttcttca aagatgacgg caactacaag 1380
acacgtgctg aagtcaagtt tgaaggtgat acccttgtta atagaatcga gttaaaaggt 1440
attgacttca aggaagatgg caacattctg ggacacaaat tggaatacaa ctataactca 1500
cacaatgtat acatcatggc agacaaacaa aagaatggaa tcaaagtgaa cttcaagacc 1560
cgccacaaca ttgaagatgg aagcgttcaa ctagcagacc attatcaaca aaatactcca 1620
attggcgatg gccctgtcct tttaccagac aaccattacc tgtccacaca atctgccctt 1680
tcgaaagatc ccaacgaaaa gagagaccac atggtccttc ttgagtttgt aacagctgct 1740
gggattacac atggcatgga tgaactgtac aactga 1776

<210> 221
<211> 1785
<212> DNA
<213> Human

<400> 221
   atgaatgggg tctcggaggg gaccagaggc tgcagtgaca ggcaacctgg ggtcctgaca 60
   cgtgatcgct cttgttccag gaagatgaac tcttccggat gcctgtctga ggaggtgggg 120
   tccctccgcc cactgactgt ggttatcctg tctgcgtcca ttgtcgtcgg agtgctgggc 180
   aatgggctgg tgctgtggat gactgtcttc cgtatggcac gcacggtctc caccgtctgc 240
   ttcttccacc tggcccttgc cgatttcatg ctctcactgt ctctgcccat tgccatgtac 300
   tatattgtct ccaggcagtg gctcctcgga gagtgggcct gcaaactcta catcaccttt 360
   gtgttcctca gctactttgc cagtaactgc ctccttgtct tcatctctgt ggaccgttgc 420
   atctctgtcc tctacccggt ctgggccctg aaccaccgca ctgtgcagcg ggcgagctgg 480
   ctggcctttg ggtgtggctc cctggccgcc gccttgtgct ctgcgcacct gaaattccgg 540
   acaaccagaa aatggaatgg ctgtacgcac tgctacttgg cgttcaactc tgacaatgag 600
   actgcccaga tttggattga aggggtcgtg gagggacaca ttatagggac cattggccac 660
   ttcctgctgg gcttcctggg gcccttagca atcataggca cctgcgccca cctcatccgg 720
   gccaagctct tgcgggaggg ctgggtccat gccaaccggc ccaagaggct gctgctggtg 780
   ctggtgagcg ctttctttat cttctggtcc ccgtttaacg tggtgctgtt ggtccatctg 840
   tggcgacggg tgatgctcaa ggaaatctac cacccccgga tgctgctcat cctccaggct 900
   agctttgcct tgggctgtgt caacagcagc ctcaacccct tcctctacgt cttcgttggc 960
   agagatttcc aagaaaagtt tttccagtct ttgacttctg ccctggcgag ggcgtttgga 1020
   gaggaggagt ttctgtcatc ctgtccccgt ggcaacgccc cccgggaagc tagcaaagga 1080
   gaagaactct tcactggagt tgtcccaatt cttgttgaat tagatggtga tgttaacggc 1140
   cacaagttct ctgtcagtgg agagggtgaa ggtgatgcaa catacggaaa acttaccctg 1200
   aagttcatct gcactactgg caaactgcct gttccatggc caacactagt cactactctg 1260
   tgctatggtg ttcaatgctt ttcaagatac ccggatcata tgaaacggca tgactttttc 1320
   aagagtgcca tgcccgaagg ttatgtacag gaaaggacca tcttcttcaa agatgacggc 1380
   aactacaaga cacgtgctga agtcaagttt gaaggtgata cccttgttaa tagaatcgag 1440
   ttaaaaggta ttgacttcaa ggaagatggc aacattctgg gacacaaatt ggaatacaac 1500
   tataactcac acaatgtata catcatggca gacaaacaaa agaatggaat caaagtgaac 1560
   ttcaagaccc gccacaacat tgaagatgga agcgttcaac tagcagacca ttatcaacaa 1620
   aatactccaa ttggcgatgg ccctgtcctt ttaccagaca accattacct gtccacacaa 1680
   tctgcccttt cgaaagatcc caacgaaaag agagaccaca tggtccttct tgagtttgta 1740
   acagctgctg ggattacaca tggcatggat gaactgtaca actga 1785

<210> 222
<211> 4380
<212> DNA
<213> Human

<400> 222
atgatgtttc gctcagatcg aatgtggagc tgccattgga aatggaagcc cagtcctctc 60
ctgttcttat ttgctttata tatcatgtgt gttcctcact cagtgtgggg atgtgccaac 120
tgccgagtgg ttttgtccaa cccttctggg acctttactt ctccatgcta ccctaacgac 180
tacccaaaca gccaggcttg catgtggacg ctccgagccc ccaccggtta tatcattcag 240
ataacattta acgactttga cattgaagaa gctcccaatt gcatttatga ctcattatcc 300
cttgataatg gagagagcca gactaaattt tgtggagcaa ctgccaaagg cctatcattt 360
aactcaagtg cgaatgagat gcatgtgtcc ttttcaagtg actttagcat ccagaagaaa 420
ggtttcaatg ccagctacat cagagttgcc gtgtccttaa ggaatcaaaa ggtcatttta 480
ccccagacat cagatgctta ccaggtatct gttgcaaaaa gcatctctat tccagagctc 540
agtgctttca cactctgctt tgaagcaacc aaagttggcc atgaagacag tgattggaca 600
gctttctcct actcaaatgc atccttcaca caattgctca gttttggaaa ggccaagagt 660
ggctactttc tatccatttc tgattcaaaa tgtttgttga ataatgcatt acctgtcaaa 720
gaaaaagaag acatttttgc agaaagcttt gaacagctct gccttgtttg gaataattct 780
ttgggctcta ttggtgtaaa tttcaaaaga aactatgaaa cagttccatg tgattctacc 840
attagtaaag ttattcctgg gaatggaaaa ttgttgttgg gctccaatca aaatgaaatt 900
gtctctctaa aaggggacat ttataacttt cgactttgga attttaccat gaatgccaaa 960
atcctctcca acctcagctg taatgtgaaa gggaatgtag tcgactggca aaatgacttc 1020
tggaatatcc caaacctagc tctgaaagct gaaagcaacc taagctgtgg ttcctacctg 1080
atcccgctcc cagcagcaga actggccagc tgtgcagacc tggggaccct ctgtcaagct 1140
actgtaaact ctcctagtac tacaccaccc actgtcacca ctaacatgcc tgttactaac 1200
agaatcgata aacaaaggaa tgatggaatt atctatagaa tatccgtagt gattcagaac 1260
atccttcgtc accctgaggt aaaagtacag agcaaggtgg cagaatggct caattcaacc 1320
ttccaaaatt ggaactacac ggtttatgtc gttaatatca gttttcacct gagtgctgga 1380
gaggacaaga ttaaagtcaa gagaagcctt gaggatgagc caaggttggt gctttggccc 1440
cttctagttt acaatgctac caacaatact aatttagaag gaaaaatcat tcagcagaag 1500
ctcctaaaaa ataatgagtc cttggatgaa ggcttgaggc tacatacagt gaatgtgaga 1560
caactgggtc attgtcttgc catggaggaa cccaaaggct actactggcc atctatccaa 1620
ccttctgaat acgttcttcc ttgtccagac aagcctggct tttctgcttc tcggatatgt 1680
ttttacaatg ctaccaaccc attggtaacc tactggggac ctgttgatat ctccaactgt 1740
ttaaaagaag caaatgaagt tgctaaccag attttaaatt taactgctga tgggcagaac 1800
ttaacctcag ccaatattac caacattgtg gaacaggtca aaagaattgt gaataaagaa 1860
gaaaacattg atataacact tggctcaact ctaatgaata tattttctaa tatcttaagc 1920
agttcagaca gtgacttgct tgagtcatct tctgaagctt taaaaacaat tgatgaattg 1980
gccttcaaga tagacctaaa tagcacatca catgtgaata ttacaactcg gaacttggct 2040
ctcagcgtat catccctgtt accagggaca aatgcaattt caaattttag cattggtctt 2100
ccaagcaata atgaatcgta tttccagatg gattttgaga gtggacaagt ggatccactg 2160
gcatctgtaa ttttgcctcc aaacttactt gagaatttaa gtccagaaga ttctgtatta 2220
gttagaagag cacagtttac tttcttcaac aaaactggac ttttccagga tgtaggaccc 2280
caaagaaaaa ctttagtgag ttatgtgatg gcgtgcagta ttggaaacat tactatccag 2340
aatctgaagg atcctgttca aataaaaatc aaacatacaa gaactcagga agtgcatcat 2400
cccatctgtg ccttctggga tctgaacaaa aacaaaagtt ttggaggatg gaacacgtca 2460
ggatgtgttg cacacagaga ttcagatgca agtgagacag tctgcctgtg taaccacttc 2520
acacactttg gagttctgat ggaccttcca agaagtgcct cacagttaga tgcaagaaac 2580
actaaagtcc tcactttcat cagctatatt gggtgtggaa tatctgctat tttttcagca 2640
gcaactctcc tgacatatgt tgcttttgag aaattgcgaa gggattatcc ctccaaaatc 2700
ttgatgaacc tgagcacagc cctgctgttc ctgaatctcc tcttcctcct agatggctgg 2760
atcacctcct tcaatgtgga tggactttgc attgctgttg cagtcctgtt gcatttcttc 2820
cttctggcaa cctttacctg gatggggcta gaagcaattc acatgtacat tgctctagtt 2880
aaagtattta acacttacat tcgccgatac attctaaaat tctgcatcat tggctggggt 2940
ttgcctgcct tagtggtgtc agttgttcta gcgagcagaa acaacaatga agtctatgga 3000
aaagaaagtt atgggaaaga aaaaggtgat gaattctgtt ggattcaaga tccagtcata 3060
ttttatgtga cctgtgctgg gtattttgga gtcatgtttt ttctgaacat tgccatgttc 3120
attgtggtaa tggtgcagat ctgtgggagg aatggcaaga gaagcaaccg gaccctgaga 3180

gaagaagtgt taaggaacct gcgcagtgtg gttagcttga cctttctgtt gggcatgaca 3240
tggggttttg cattctttgc ctggggaccc ttaaatatcc ccttcatgta cctcttctcc 3300
atcttcaatt cattacaagg cttatttata ttcatcttcc actgtgctat gaaggagaat 3360
gttcagaaac agtggcggcg gcatctctgc tgtggtagat ttcggttagc agataactca 3420
gattggagta agacagctac caatatcatc aagaaaagtt ctgataatct aggaaaatct 3480
ttgtcttcaa gctccattgg ttccaactca acctatctta catccaaatc taaatccagc 3540
tctaccacct atttcaaaag gaatagccac acagataatg tctcctatga gcattccttc 3600
aacaaaagtg gatcactcag acagtgcttc catggacaag tccttgtcaa aactggccca 3660
tgctctagca aaggagaaga actcttcact ggagttgtcc caattcttgt tgaattagat 3720
ggtgatgtta acggccacaa gttctctgtc agtggagagg gtgaaggtga tgcaacatac 3780
ggaaaactta ccctgaagtt catctgcact actggcaaac tgcctgttcc atggccaaca 3840
ctagtcacta ctctgtgcta tggtgttcaa tgcttttcaa gatacccgga tcatatgaaa 3900
cggcatgact ttttcaagag tgccatgccc gaaggttatg tacaggaaag gaccatcttc 3960
ttcaaagatg acggcaacta caagacacgt gctgaagtca agtttgaagg tgataccctt 4020
gttaatagaa tcgagttaaa aggtattgac ttcaaggaag atggcaacat tctgggacac 4080
aaattggaat acaactataa ctcacacaat gtatacatca tggcagacaa acaaaagaat 4140
ggaatcaaag tgaacttcaa gacccgccac aacattgaag atggaagcgt tcaactagca 4200
gaccattatc aacaaaatac tccaattggc gatggccctg tccttttacc agacaaccat 4260
tacctgtcca cacaatctgc cctttcgaaa gatcccaacg aaaagagaga ccacatggtc 4320
cttcttgagt ttgtaacagc tgctgggatt acacatggca tggatgaact gtacaactga 4380

<210> 223
<211> 1914
<212> DNA
<213> Human

<400> 223
atggctcaaa ggcagcctca ctcacctaat cagactttaa tttcaatcac aaatgacaca 60
gaatcatcaa gctctgtggt ttctaacgat aacacaaata aaggatggag cggggacaac 120
tctccaggaa tagaagcatt gtgtgccatc tatattactt atgctgtgat catttcagtg 180
ggcatccttg gaaatgctat tctcatcaaa gtcttttca agaccaaatc catgcaaaca 240
gttccaaata ttttcatcac cagcctggct tttggagatc ttttacttct gctaacttgt 300
gtgccagtgg atgcaactca ctaccttgca gaaggatggc tgttcggaag aattggttgt 360
aaggtgctct ctttcatccg gctcacttct gttggtgtgt cagtgttcac attaacaatt 420
ctcagcgctg acagatacaa ggcagttgtg aagccacttg agcgacagcc ctccaatgcc 480
atcctgaaga cttgtgtaaa agctggctgc gtctggatcg tgtctatgat atttgctcta 540
cctgaggcta tattttcaaa tgtatacact tttcgagatc ccaataaaaa tatgacattt 600
gaatcatgta cctcttatcc tgtctctaag aagctcttgc aagaaataca ttctctgctg 660
tgcttcttag tgttctacat tattccactc tctattatct ctgtctacta ttccttgatt 720
gctaggaccc tttacaaaag caccctgaac atacctactg aggaacaaag ccatgcccgt 780
aagcagattg aatcccgaaa gagaattgcc agaacggtat tggtgttggt ggctctgttt 840
gccctctgct ggttgccaaa tcacctcctg tacctctacc attcattcac ttctcaaacc 900
tatgtagacc cctctgccat gcatttcatt ttcaccattt tctctcgggt tttggctttc 960
agcaattctt gcgtaaaccc ctttgctctc tactggctga gcaaaagctt ccagaagcat 1020
tttaaagctc agttgttctg ttgcaaggcg gagcggcctg agcctcctgt tgctgacacc 1080
tctcttacca ccctggctgt gatgggaacg gtcccgggca ctgggagcat acagatgtct 1140
gaaattagtg tgacctcgtt cactgggtgt agtgtgaagc aggcagagga cagattcgct 1200
agcaaaggag aagaactctt cactggagtt gtcccaattc ttgttgaatt agatggtgat 1260
gttaacggcc acaagttctc tgtcagtgga gagggtgaag gtgatgcaac atacggaaaa 1320
cttaccctga agttcatctg cactactggc aaactgcctg ttccatggcc aacactagtc 1380
actactctgt gctatggtgt tcaatgcttt tcaagatacc cggatcatat gaaacggcat 1440
gactttttca agagtgccat gcccgaaggt tatgtacagg aaaggaccat cttcttcaaa 1500
gatgacggca actacaagac acgtgctgaa gtcaagtttg aaggtgatac ccttgttaat 1560
agaatcgagt taaaaggtat tgacttcaag gaagatggca acattctggg acacaaattg 1620
gaatacaact ataactcaca caatgtatac atcatggcag acaaacaaaa gaatggaatc 1680
aaagtgaact tcaagacccg ccacaacatt gaagatggaa gcgttcaact agcagaccat 1740
tatcaacaaa atactccaat tggcgatggc cctgtccttt taccagacaa ccattacctg 1800
tccacacaat ctgccctttc gaaagatccc aacgaaaaga gagaccacat ggtccttctt 1860
gagtttgtaa cagctgctgg gattacacat ggcatggatg aactgtacaa ctga 1914

<210> 224
<211> 2076
<212> DNA
<213> Human

<400> 224
atggcttcac ccagcctccc gggcagtgac tgctcccaaa tcattgatca cagtcatgtc 60
cccgagtttg aggtggccac ctggatcaaa atcaccctta ttctggtgta cctgatcatc 120
ttcgtgatgg gccttctggg gaacagcgcc accattcggg tcacccaggt gctgcagaag 180
aaaggatact tgcagaagga ggtgacagac cacatggtga gtttggcttg ctcggacatc 240
ttggtgttcc tcatcggcat gcccatggag ttctacagca tcatctggaa tcccctgacc 300
acgtccagct acaccctgtc ctgcaagctg cacactttcc tcttcgaggc ctgcagctac 360
gctacgctgc tgcacgtgct gacactcagc tttgagcgct acatcgccat ctgtcacccc 420
ttcaggtaca aggctgtgtc gggaccttgc caggtgaagc tgctgattgg cttcgtctgg 480
gtcacctccg ccctggtggc actgcccttg ctgtttgcca tgggtactga gtaccccctg 540

gtgaacgtgc ccagccaccg gggtctcact tgcaaccgct ccagcacccg ccaccacgag 600
cagcccgaga cctccaatat gtccatctgt accaacctct ccagccgctg gaccgtgttc 660
cagtccagca tcttcggcgc cttcgtggtc tacctcgtgg tcctgctctc cgtagccttc 720
atgtgctgga acatgatgca ggtgctcatg aaaagccaga agggctcgct ggccgggggc 780
acgcggcctc cgcagctgag gaagtccgag agcgaagaga gcaggaccgc caggaggcag 840
accatcatct tcctgaggct gattgttgtg acattggccg tatgctggat gcccaaccag 900
attcggagga tcatggctgc ggccaaaccc aagcacgact ggacgaggtc ctacttccgg 960
gcgtacatga tcctcctccc cttctcggag acgtttttct acctcagctc ggtcatcaac 1020
ccgctcctgt acacggtgtc ctcgcagcag tttcggcggg tgttcgtgca ggtgctgtgc 1080
tgccgcctgt cgctgcagca cgccaaccac gagaagcgcc tgcgcgtaca tgcgcactcc 1140
accaccgaca gcgcccgctt tgtgcagcgc ccgttgctct tcgcgtcccg gcgccagtcc 1200
tctgcaagga gaactgagaa gatttcttta agcacttttc agagcgaggc cgagccccag 1260
tctaagtccc agtcattgag tctcgagtca ctagagccca actcaggcgc gaaaccagcc 1320
aattctgctg cagagaatgg ttttcaggag catgaagttg ctagcaaagg agaagaactc 1380
ttcactggag ttgtcccaat tcttgttgaa ttagatggtg atgttaacgg ccacaagttc 1440
tctgtcagtg gagagggtga aggtgatgca acatacggaa aacttaccct gaagttcatc 1500
tgcactactg gcaaactgcc tgttccatgg ccaacactag tcactactct gtgctatggt 1560
gttcaatgct tttcaagata cccggatcat atgaaacggc atgacttttt caagagtgcc 1620
atgcccgaag gttatgtaca ggaaaggacc atcttcttca aagatgacgg caactacaag 1680
acacgtgctg aagtcaagtt tgaaggtgat acccttgtta atagaatcga gttaaaaggt 1740
attgacttca aggaagatgg caacattctg ggacacaaat tggaatacaa ctataactca 1800
cacaatgtat acatcatggc agacaaacaa aagaatggaa tcaaagtgaa cttcaagacc 1860
cgccacaaca ttgaagatgg aagcgttcaa ctagcagacc attatcaaca aaatactcca 1920
attggcgatg gccctgtcct tttaccagac aaccattacc tgtccacaca atctgccctt 1980
tcgaaagatc ccaacgaaaa gagagaccac atggtccttc ttgagtttgt aacagctgct 2040
gggattacac atggcatgga tgaactgtac aactga 2076

<210> 225
<211> 1986
<212> DNA
<213> Human

<400> 225
atggtccctc acctcttgct gctctgtctc ctccccttgg tgcgagccac cgagccccac 60
gagggccggg ccgacgagca gagcgcggag gcggccctgg ccgtgcccaa tgcctcgcac 120
ttcttctctt ggaacaacta caccttctcc gactggcaga actttgtggg caggaggcgc 180
tacggcgctg agtcccagaa ccccacggtg aaagccctgc tcattgtggc ttactccttc 240
atcattgtct tctcactctt tggcaacgtc ctggtctgtc atgtcatctt caagaaccag 300
cgaatgcact cggccaccag cctcttcatc gtcaacctgg cagttgccga cataatgatc 360
acgctgctca acaccccctt cactttggtt cgctttgtga acagcacatg gatatttggg 420
aagggcatgt gccatgtcag ccgctttgcc cagtactgct cactgcacgt ctcagcactg 480
acactgacag ccattgcggt ggatcgccac caggtcatca tgcacccctt gaaaccccgg 540
atctcaatca caaagggtgt catctacatc gctgtcatct ggaccatggc tacgttcttt 600
tcactcccac atgctatctg ccagaaatta tttaccttca aatacagtga ggacattgtg 660
cgctccctct gcctgccaga cttccctgag ccagctgacc tcttctggaa gtacctggac 720
ttggccacct tcatcctgct ctacatcctg cccctcctca tcatctctgt ggcctacgct 780
cgtgtggcca agaaactgtg gctgtgtaat atgattggcg atgtgaccac agagcagtac 840
tttgccctgc ggcgcaaaaa gaagaagacc atcaagatgt tgatgctggt ggtagtcctc 900
tttgccctct gctggttccc cctcaactgc tacgtcctcc tcctgtccag caaggtcatc 960
cgcaccaaca atgccctcta ctttgccttc cactggtttg ccatgagcag cacctgctat 1020
aaccccttca tatactgctg gctgaacgag aacttcagga ttgagctaaa ggcattactg 1080
agcatgtgtc aaagacctcc caagcctcag gaggacaggc caccctcccc agttccttcc 1140
ttcagggtgg cctggacaga gaagaatgat ggccagaggg ctccccttgc caataacctc 1200
ctgcccacct cccaactcca gtctgggaag acagacctgt catctgtgga acccattgtg 1260
acgatgagtg ctagcaaagg agaagaactc ttcactggag ttgtcccaat tcttgttgaa 1320
ttagatggtg atgttaacgg ccacaagttc tctgtcagtg gagagggtga aggtgatgca 1380
acatacggaa aacttaccct gaagttcatc tgcactactg gcaaactgcc tgttccatgg 1440
ccaacactag tcactactct gtgctatggt gttcaatgct tttcaagata cccggatcat 1500
atgaaacggc atgacttttt caagagtgcc atgcccgaag gttatgtaca ggaaaggacc 1560
atcttcttca aagatgacgg caactacaag acacgtgctg aagtcaagtt tgaaggtgat 1620
acccttgtta atagaatcga gttaaaaggt attgacttca aggaagatgg caacattctg 1680
ggacacaaat tggaatacaa ctataactca cacaatgtat acatcatggc agacaaacaa 1740
aagaatggaa tcaaagtgaa cttcaagacc cgccacaaca ttgaagatgg aagcgttcaa 1800
ctagcagacc attatcaaca aaatactcca attggcgatg gccctgtcct tttaccagac 1860
aaccattacc tgtccacaca atctgccctt tcgaaagatc ccaacgaaaa gagagaccac 1920
atggtccttc ttgagtttgt aacagctgct gggattacac atggcatgga tgaactgtac 1980
aactga 1986

<210> 226
<211> 1905
<212> DNA
<213> Human

<400> 226
atgtggaaca gctctgacgc caacttctcc tgctaccatg agtctgtgct gggctatcgt 60

tatgttgcag ttagctgggg ggtggtggtg gctgtgacag gcaccgtggg caatgtgctc 120
accctactgg ccttggccat ccagcccaag ctccgtaccc gattcaacct gctcatagcc 180
aacctcacac tggctgatct cctctactgc acgctccttc agcccttctc tgtggacacc 240
tacctccacc tgcactggcg caccggtgcc accttctgca gggtatttgg gctcctcctt 300
tttgcctcca attctgtctc catcctgacc ctctgcctca tcgcactggg acgctacctc 360
ctcattgccc accctaagct tttcccccaa gttttcagtg ccaaggggat agtgctggca 420
ctggtgagca cctgggttgt gggcgtggcc agctttgctc ccctctggcc tatttatatc 480
ctggtacctg tagtctgcac ctgcagcttt gaccgcatcc gaggccggcc ttacaccacc 540
atcctcatgg gcatctactt tgtgcttggg ctcagcagtg ttggcatctt ctattgcctc 600
atccaccgcc aggtcaaacg agcagcacag gcactggacc aatacaagtt gcgacaggca 660
agcatccact ccaaccatgt ggccaggact gatgaggcca tgcctggtcg tttccaggag 720
ctggacagca ggttagcatc aggaggaccc agtgagggga tttcatctga gccagtcagt 780
gctgccacca cccagaccct ggaaggggac tcatcagaag tgggagacca gatcaacagc 840
aagagagcta agcagatggc agagaaaagc cctccagaag catctgccaa agcccagcca 900
attaaaggag ccagaagagc tccggattct tcatcggaat ttgggaaggt gactcgaatg 960
tgttttgctg tgttcctctg ctttgccctg agctacatcc ccttcttgct gctcaacatt 1020
ctggatgcca gagtccaggc tccccgggtg gtccacatgc ttgctgccaa cctcacctgg 1080
ctcaatggtt gcatcaaccc tgtgctctat gcagccatga accgccaatt ccgccaagca 1140
tatggctcca ttttaaaaag agggccccgg agtttccata ggctccatgc tagcaaagga 1200
gaagaactct tcactggagt tgtcccaatt cttgttgaat tagatggtga tgttaacggc 1260
cacaagttct ctgtcagtgg agagggtgaa ggtgatgcaa catacggaaa acttaccctg 1320
aagttcatct gcactactgg caaactgcct gttccatggc caacactagt cactactctg 1380
tgctatggtg ttcaatgctt ttcaagatac ccggatcata tgaaacggca tgactttttc 1440
aagagtgcca tgcccgaagg ttatgtacag gaaaggacca tcttcttcaa agatgacggc 1500
aactacaaga cacgtgctga agtcaagttt gaaggtgata cccttgttaa tagaatcgag 1560
ttaaaaggta ttgacttcaa ggaagatggc aacattctgg gacacaaatt ggaatacaac 1620
tataactcac acaatgtata catcatggca gacaaacaaa agaatggaat caaagtgaac 1680
ttcaagaccc gccacaacat tgaagatgga agcgttcaac tagcagacca ttatcaacaa 1740
aatactccaa ttggcgatgg ccctgtcctt ttaccagaca accattacct gtccacacaa 1800
tctgcccttt cgaaagatcc caacgaaaag agagaccaca tggtccttct tgagtttgta 1860
acagctgctg ggattacaca tggcatggat gaactgtaca actga 1905

<210> 227
<211> 1764
<212> DNA
<213> Human

<400> 227
atgaactcca ccttggatgg taatcagagc agccacccct tttgcctctt ggcatttggc 60
tatttggaaa ctgtcaattt ttgccttttg gaagtattga ttattgtctt tctaactgta 120
ttgattattt ctggcaacat cattgtgatt tttgtatttc actgtgcacc tttgttgaac 180
catcacacta caagttattt tatccagact atggcatatg ctgacctttt tgttggggtg 240
agctgcgtgg tcccttcttt atcactcctc catcaccccc ttccagtaga ggagtccttg 300
acttgccaga tatttggttt tgtagtatca gttctgaaga gcgtctccat ggcttctctg 360
gcctgtatca gcattgatag atacattgcc attactaaac ctttaaccta taatactctg 420
gttacaccct ggagactacg cctgtgtatt ttcctgattt ggctatactc gaccctggtc 480
ttcctgcctt cctttttcca ctggggcaaa cctggatatc atggagatgt gtttcagtgg 540
tgtgcggagt cctggcacac cgactcctac ttcaccctgt tcatcgtgat gatgttatat 600
gccccagcag cccttattgt ctgcttcacc tatttcaaca tcttccgcat ctgccaacag 660
cacacaaagg atatcagcga aaggcaagcc cgcttcagca gccagagtgg ggagactggg 720
gaagtgcagg cctgtcctga taagcgctat gccatggtcc tgtttcgaat cactagtgta 780
ttttacatcc tctggttgcc atatatcatc tacttcttgt tggaaagctc cactggccac 840
agcaaccgct tcgcatcctt cttgaccacc tggcttgcta ttagtaacag tttctgcaac 900
tgtgtaattt atagtctctc caacagtgta ttccaaagag gactaaagcg cctctcaggg 960
gctatgtgta cttcttgtgc aagtcagact acagccaacg acccttacac agttagaagc 1020
aaaggccctc ttaatggatg tcatatcgct agcaaaggag aagaactctt cactggagtt 1080
gtcccaattc ttgttgaatt agatggtgat gttaacggcc acaagttctc tgtcagtgga 1140
gagggtgaag gtgatgcaac atacggaaaa cttaccctga agttcatctg cactactggc 1200
aaactgcctg ttccatggcc aacactagtc actactctgt gctatggtgt tcaatgcttt 1260
tcaagatacc cggatcatat gaaacggcat gactttttca agagtgccat gcccgaaggt 1320
tatgtacagg aaaggaccat cttcttcaaa gatgacggca actacaagac acgtgctgaa 1380
gtcaagtttg aaggtgatac ccttgttaat agaatcgagt taaaaggtat tgacttcaag 1440
gaagatggca acattctggg acacaaattg gaatacaact ataactcaca caatgtatac 1500
atcatggcag acaaacaaaa gaatggaatc aaagtgaact tcaagacccg ccacaacatt 1560
gaagatggaa gcgttcaact agcagaccat tatcaacaaa atactccaat tggcgatggc 1620
cctgtccttt taccagacaa ccattacctg tccacacaat ctgccctttc gaaagatccc 1680
aacgaaaaga gagaccacat ggtccttctt gagtttgtaa cagctgctgg gattacacat 1740
ggcatggatg aactgtacaa ctga 1764

<210> 228
<211> 3570
<212> DNA
<213> Human

<400> 228

atgccgggcc cgctagggct gctctgcttc ctcgccctgg ggctgctcgg ctcggccggg 60
cccagcggcg cggcgccgcc tctctgcgcg gcgccctgca gctgcgacgg cgaccgtcgg 120
gtggactgct ccgggaaggg gctgacggcc gtgcccgagg ggctcagcgc cttcacccaa 180
gcgctggata tcagtatgaa caacattact cagttgccag aagatgcatt taagaacttt 240
ccttttctag aagagctaca attggcgggc aacgaccttt cttttatcca cccaaaggcc 300
ttgtctgggt tgaaagaact caaagttcta acgctccaga ataatcagtt gaaaacagta 360
cccagtgaag ccattcgagg gctgagtgct ttgcagtctt tgcgtttaga tgccaaccat 420
attacctcag tccccgagga cagttttgaa ggacttgttc agttacggca tctgtggctg 480
gatgacaaca gcttgacgga ggtgcctgtg caccccctca gcaatctgcc caccctacag 540
gcgctgaccc tggctctcaa caagatctca agcatccctg actttgcatt taccaacctt 600
tcaagcctgg tagttctgca tcttcataac aataaaatta gaagcctgag tcaacactgt 660
tttgatggac tagataacct ggagacctta gacttgaatt ataataactt gggggaattt 720
cctcaggcta ttaaagccct tcctagcctt aaagagctag gatttcatag taattctatt 780
tctgttatcc ctgatggagc atttgatggt aatccactct taagaactat acatttgtat 840
gataatcctc tgtcttttgt ggggaactca gcatttcaca atttatctga tcttcattcc 900
ctagtcattc gtggtgcaag catggtgcag cagttcccca atcttacagg aactgtccac 960
ctggaaagtc tgactttgac aggtacaaag ataagcagca tacctaataa tttgtgtcaa 1020
gaacaaaaga tgcttaggac tttggacttg tcttacaata atataagaga ccttccaagt 1080
tttaatggtt gccatgctct ggaagaaatt tctttacagc gtaatcaaat ctaccaaata 1140
aaggaaggca cctttcaagg cctgatatct ctaaggattc tagatctgag tagaaacctg 1200
atacatgaaa ttcacagtag agcttttgcc acacttgggc caataactaa cctagatgta 1260
agtttcaatg aattaacttc ctttcctacg gaaggcctga atgggctaaa tcaactgaaa 1320
cttgtgggca acttcaagct gaaagaagcc ttagcagcaa aagactttgt taacctcagg 1380
tctttatcag taccatatgc ttatcagtgc tgtgcatttt ggggttgtga ctcttatgca 1440
aatttaaaca cagaagataa cagcctccag gaccacagtg tggcacagga gaaaggtact 1500
gctgatgcag caaatgtcac aagcactctt gaaaatgaag aacatagtca aataattatc 1560
cattgtacac cttcaacagg tgcttttaag ccctgtgaat atttactggg aagctggatg 1620
attcgtctta ctgtgtggtt cattttcttg gttgcattat ttttcaacct gcttgttatt 1680
ttaacaacat ttgcatcttg tacatcactg ccttcgtcca aattgtttat aggcttgatt 1740
tctgtgtcta acttattcat gggaatctat actggcatcc taacttttct tgatgctgtg 1800
tcctggggca gattcgctga atttggcatt tggtggcaaa ctggcagtgg ctgcaaagta 1860
gctgggtttc ttgcagtttt ctcctcagaa agtgccatat ttttattaat gctagcaact 1920
gtcgaaagaa gcttatctgc aaaagatata atgaaaaatg ggaagagcaa tcatctcaaa 1980
cagttccggg ttgctgccct tttggctttc ctaggtgcta cagtagcagg ctgttttccc 2040
cttttccata gaggggaata ttctgcatca cccctttgtt tgccatttcc tacaggtgaa 2100
acgccatcat taggattcac tgtaacgtta gtgctattaa actcactagc atttttatta 2160
atggccgtta tctacactaa gctatactgc aacttggaaa aagaggacct ctcagaaaac 2220
tcacaatcta gcatgattaa gcatgtcgct tggctaatct tcaccaattg catctttttc 2280
tgccctgtgg cgtttttttc atttgcacca ttgatcactg caatctctat cagccccgaa 2340
ataatgaagt ctgttactct gatatttttt ccattgcctg cttgcctgaa tccagtcctg 2400
tatgttttct tcaacccaaa gtttaaagaa gactggaagt tactgaagcg acgtgttacc 2460
aagaaaagtg gatcagtttc agtttccatc agtagccaag gtggttgtct ggaacaggat 2520
ttctactacg actgtggcat gtactcacat ttgcagggca acctgactgt ttgcgactgc 2580
tgcgaatcgt ttcttttaac aaagccagta tcatgcaaac acttgataaa atcacacagc 2640
tgtcctgcat tggcagtggc ctcttgccaa agacctgagg gctactggtc cgactgtggc 2700
acacagtcgg cccactctga ttatgcagat gaagaagatt cctttgtctc agacagttct 2760
gaccaggtgc aggcctgtgg acgagcctgc ttctaccaga gtagaggatt ccctttggtg 2820
cgctatgctt acaatctacc aagagttaaa gacactagca aaggagaaga actcttcact 2880
ggagttgtcc caattcttgt tgaattagat ggtgatgtta acggccacaa gttctctgtc 2940
agtggagagg gtgaaggtga tgcaacatac ggaaaactta ccctgaagtt catctgcact 3000
actggcaaac tgcctgttcc atggccaaca ctagtcacta ctctgtgcta tggtgttcaa 3060
tgcttttcaa gatacccgga tcatatgaaa cggcatgact ttttcaagag tgccatgccc 3120
gaaggttatg tacaggaaag gaccatcttc ttcaaagatg acggcaacta caagacacgt 3180
gctgaagtca agtttgaagg tgataccctt gttaatagaa tcgagttaaa aggtattgac 3240
ttcaaggaag atggcaacat tctgggacac aaattggaat acaactataa ctcacacaat 3300
gtatacatca tggcagacaa acaaaagaat ggaatcaaag tgaacttcaa gacccgccac 3360
aacattgaag atggaagcgt tcaactagca gaccattatc aacaaaatac tccaattggc 3420
gatggccctg tccttttacc agacaaccat tacctgtcca cacaatctgc cctttcgaaa 3480
gatcccaacg aaaagagaga ccacatggtc cttcttgagt ttgtaacagc tgctgggatt 3540
acacatggca tggatgaact gtacaactga 3570

<210> 229
<211> 1761
<212> DNA
<213> Human

<400> 229
atggtgaata atttctccca agctgaggct gtggagctgt gttacaagaa cgtgaacgaa 60
tcctgcatta aaactcctta ctcgccaggt cctcgatcta tcctctacgc cgtccttggt 120
tttggggctg tgctggcagc gtttggaaac ttactggtca tgattgctat ccttcacttc 180
aaacaactgc acacacctac aaactttctg attgcgtcgc tggcctgtgc tgacttcttg 240
gtgggagtca ctgtgatgcc cttcagcaca gtgaggtctg tggagagctg ttggtacttt 300
ggggacagtt actgtaaatt ccatacatgt tttgacacat ccttctgttt tgcttcttta 360
tttcatttat gctgtatctc tgttgataga tacattgctg ttactgatcc tctgacctat 420
ccaaccaagt ttactgtgtc agtttcaggg atatgcattg ttctttcctg gttcttttct 480

gtcacataca gcttttcgat cttttacacg ggagccaacg aagaaggaat tgaggaatta 540
gtagttgctc taacctgtgt aggaggctgc caggctccac tgaatcaaaa ctgggtccta 600
ctttgttttc ttctattctt tatacccaat gtcgccatgg tgtttatata cagtaagata 660
tttttggtgg ccaagcatca ggctaggaag atagaaagta cagccagcca agctcagtcc 720
tcctcagaga gttacaagga aagagtagca aaaagagaga gaaaggctgc caaaaccttg 780
ggaattgcta tggcagcatt tcttgtctct tggctaccat acctcgttga tgcagtgatt 840
gatgcttata tgaattttat aactcctcct tatgtttatg agattttagt ttggtgtgtt 900
tattataatt cagctatgaa ccccttgatt tatgctttct tttaccaatg gtttgggaag 960
gcaataaaac ttattgtaag cggcaaggtc ttaaggactg attcgtcaac aactaattta 1020
ttttctgaag aagtagagac agatgctagc aaaggagaag aactcttcac tggagttgtc 1080
ccaattcttg ttgaattaga tggtgatgtt aacggccaca agttctctgt cagtggagag 1140
ggtgaaggtg atgcaacata cggaaaactt accctgaagt tcatctgcac tactggcaaa 1200
ctgcctgttc catggccaac actagtcact actctgtgct atggtgttca atgcttttca 1260
agatacccgg atcatatgaa acggcatgac tttttcaaga gtgccatgcc cgaaggttat 1320
gtacaggaaa ggaccatctt cttcaaagat gacggcaact acaagacacg tgctgaagtc 1380
aagtttgaag gtgataccct tgttaataga atcgagttaa aaggtattga cttcaaggaa 1440
gatggcaaca ttctgggaca caaattggaa tacaactata actcacacaa tgtatacatc 1500
atggcagaca aacaaaagaa tggaatcaaa gtgaacttca agacccgcca caacattgaa 1560
gatggaagcg ttcaactagc agaccattat caacaaaata ctccaattgg cgatggccct 1620
gtccttttac cagacaacca ttacctgtcc acacaatctg ccctttcgaa agatcccaac 1680
gaaaagagag accacatggt ccttcttgag tttgtaacag ctgctgggat tacacatggc 1740
atggatgaac tgtacaactg a 1761

<210> 230
<211> 5433
<212> DNA
<213> Human

<400> 230
atgaccccag cctgtcccct cttactgtct gtgattctgt ccctgcgcct ggccaccgcc 60
ttcgaccccg cccccagtgc ctgctctgcc ctggcctcgg gtgtgctcta cggggccttc 120
tcgctgcagg acctctttcc taccatcgcc tcgggctgct cctggaccct ggagaaccct 180
gaccccacca agtactccct ctacctgcgc ttcaaccgcc aggagcaggt gtgcgcacac 240
tttgcccccc gcctgctgcc cctggaccac tacctggtca actttacctg cctgcggcct 300
agccccgagg aggcggtggc ccaggcggag tcagaggtgg ggcggccaga agaggaggag 360
gcagaggcgg cagcggggtt ggagctgtgc agcggctcag gcccctttac cttcctgcac 420
ttcgacaaga acttcgtgca gctgtgcctg tcggctgagc cctccgaggc cccgcgcctg 480
ctggcgcccg ctgccctagc cttccgcttt gtcgaggtct tgctcatcaa caacaacaac 540
tctagccaat tcacctgtgg tgtgctctgc cgctggagtg aggagtgtgg ccgcgctgcc 600
ggcagggcct gcggctttgc tcagccaggc tgcagctgcc ctggagaggc gggggccggc 660
tccaccacca ccacatctcc aggccctcct gctgcccaca ccctgtccaa tgccctggtg 720
cccgggggcc cagccccacc tgctgaggcc gatttgcact cggggagcag caatgatctg 780
ttcacaaccg agatgagata tggtgaggag ccggaagagg aaccgaaagt gaaaacccag 840
tggccgaggt ctgcagatga gcctgggcta tacatggcgc agacaggcga ccccggcgct 900
gaggagtggt ccccgtggag cgtgtgttcc ctgacgtgtg ggcagggtct gcaggtgcgg 960
acccgctcct gtgtgtcctc cccctatggg accctgtgca gcgggcccct gcgggagacc 1020
aggccctgca acaattcagc cacctgccca gtgcacggcg tgtgggagga gtgggggtcc 1080
tggagcctgt gctcccgcag ctgcgggcgg gggtcccgga gccggatgcg gacctgcgtg 1140
cccccccagc acggcggcaa ggcctgcgag ggtcctgagc tgcagactaa gctctgcagt 1200
atggctgcct gcccggtgga aggccagtgg ttagaatggg gtccctgggg cccatgctcc 1260
acgtcctgtg ccaatgggac ccaacagcgc agccggaagt gcagcgtggc gggcccagcc 1320
tgggccacat gcacgggtgc cctcactgac acccgggagt gcagcaacct cgagtgcccg 1380
gccactgata gcaagtgggg gccatggaat gcgtggagcc tgtgctctaa gacgtgtgac 1440
acaggctggc agcgccgctt ccgcatgtgc caggccacgg gcacgcaggg ctacccctgc 1500
gagggcaccg gagaggaggt gaagccttgt agtgagaaga ggtgtccagc cttccatgag 1560
atgtgcaggg atgagtacgt gatgctgatg acgtggaaga aggcagctgc tggcgagatc 1620
atctacaaca agtgcccccc gaatgcctca gggtctgcca gccgccgctg cctcctcagt 1680
gcccaaggcg tggcgtactg gggcctgccc agctttgctc gctgcatctc ccatgagtac 1740
cgctacctgt atctgtcact tagggagcac ctggccaagg ggcagcgcat gctggcaggc 1800
gagggcatgt cgcaggtggt gcgcagcctg caggagctac tggcccggcg cacctactat 1860
agtggggacc tgctcttctc tgtggacatt ctgaggaatg tcactgacac ctttaagagg 1920
gccacctacg tgccctcggc tgatgatgtg cagcgcttct tccaggtggt gagcttcatg 1980
gtggatgcgg aaaacaagga gaagtgggac gatgctcagc aggtgtcccc tggctctgtg 2040
cacctgctcc gtgtcgtgga ggacttcatt cacctggtgg gcgatgctct caaggccttc 2100
cagagctctc tgattgtcac agataatcta gtgatcagca ttcagcgaga gcccgtctca 2160
gctgtgtcca gtgacatcac gttccccatg cggggccgcc ggggcatgaa ggactgggtg 2220
cggcactcag aggaccgcct cttcctgccc aaggaggtgc tcagcctctc ctccccaggg 2280
aagccagcca catctggggc agcaggcagc cctggcaggg ggaggggccc aggaacggtg 2340
cctcctggcc caggccactc ccaccagcgc ctcctcccag cagaccctga tgagtcctcc 2400
tactttgtga tcggtgctgt actctaccgc acccttggcc tcatcctgcc gcctcccagg 2460
cccccgctgg ccgtcacatc ccgggtgatg acagtgactg tgcgcccccc tacccagcct 2520
ccagctgagc ccctcatcac tgtggagctc tcctacatca tcaatgggac cacggatccc 2580
cattgcgcca gctgggacta ctccagagca gatgccagct caggagactg ggacactgaa 2640
aattgccaga ccctggagac ccaggcagct cacacccgct gccagtgcca gcacctgtcc 2700
acctttgctg tactagccca gccgcccaag gacctgaccc tggagctggc aggctccccc 2760

tcggtccccc tggtgatcgg ctgtgcagtg tcgtgcatgg cgctgcttac cctgctcgcc 2820
atctatgccg cctttttggag gttcataaaa tctgaacgct ccatcatctt gctgaacttc 2880
tgcctgtcca tcttggcatc caacatcctg atcctcgtgg gccagtcccg ggtgctgagc 2940
aagggcgtgt gcaccatgac ggctgccttc ctgcacttct tctttctctc ctcctttttgc 3000
tgggtgctta ccgaggcctg gcagtcctac ctggctgtca ttgggcggat gcgcacccgc 3060
ctcgttcgca agcgcttcct ctgcctgggc tggggtctgc ctgccctggt ggtggccgtg 3120
tctgttggct ttacccgaac gaaaggatac ggtacatcca gctactgctg gctctccctg 3180
gagggcggcc tgctctacgc ctttgtgggc cctgcagccg tcattgtcct ggtgaacatg 3240
ctcatcggaa tcatcgtctt caacaagctc atggcacgtg atggcatctc cgacaaatcc 3300
aagaagcaga gggccgggtc ggagcggtgc ccctgggcca gcctgctcct cccctgctca 3360
gcgtgtggag cggtccccag cccctgctc agctcagcct cggccaggaa cgccatggcc 3420
tcactctgga gctcctgcgt ggtgctgccc ctgctggcgc tcacctggat gtctgccgtc 3480
ctggctatga cagaccgccg ttccgtcctc ttccaggccc tctttgctgt cttcaactcc 3540
gcgcagggct ttgtcatcac tgctgtgcac tgcttcctgc gccgagaggt ccaggatgtg 3600
gtgaagtgcc agatgggggt gtgccgggct gatgagagcg aagactcccc tgactcgtgt 3660
aagaacgggc agctgcagat cctgtcagac tttgaaaagg atgtggatct ggcttgtcaa 3720
acagtgctgt tcaaggaggt caacacttgc aacccgtcca ccatcacggg cacactatcc 3780
cgcctgtccc tggatgagga tgaggagccc aagtcctgcc tcgtgggccc tgagggcagc 3840
ctcagcttct caccactgcc tgggaatatc ctggtgccca tggcagcctc accagggctg 3900
ggggagcctc cgcccccaca ggaggccaac cctgtttaca tgtgtgggga gggtggcctg 3960
cggcagctgg acctcacatg gctgcggccc actgagccag gctctgaggg agactacatg 4020
gtgctgcccc ggcggacttt gagcctgcag cctggcggtg gggtggagg tggtgaggat 4080
gcccccaggg cccggccgga ggggaccccc cggcgagctg ccaagacagt ggcccacact 4140
gaaggctacc ccagcttcct gtccgtggac cactcgggcc tggggctggg ccctgcctat 4200
ggatctctcc agaatcccta tggaatgacc ttccaaccgc caccgccgac acccagcgcc 4260
cgccaagtgc ccgagccagg ggagcgcagc cggaccatgc ctcgcaccgt gcccggctct 4320
accatgaaga tgggctccct ggagcgaaag aaattacggt attcagacct ggactttgag 4380
gtgatgcaca cccggaaacg gcattcagaa ctctaccacg agctcaacca gaagttccac 4440
actttcgacc gctaccgcag ccagtccacg gccaagaggg agaagcggtg gagtgtgtcc 4500
tcgggtgggg cggccgagcg gagcgtgtgc accgataagc ccagccctgg ggagcgcccc 4560
agcttgtccc aacatcggcg ccatcagagc tggagcacct tcaaatctat gacactgggc 4620
tcgctgcccc ccaagccccg agaacggctg actctgcacc gggcagcagc ctgggagccc 4680
acagaaccac cggatggtga cttccagaca gaggtggcta gcaaaggaga agaactcttc 4740
actggagttg tcccaattct tgttgaatta gatggtgatg ttaacggcca caagttctct 4800
gtcagtggag agggtgaagg tgatgcaaca tacggaaaac ttaccctgaa gttcatctgc 4860
actactggca aactgcctgt tccatggcca acactagtca ctactctgtg ctatggtgtt 4920
caatgctttt caagataccc ggatcatatg aaacggcatg actttttcaa gagtgccatg 4980
cccgaaggtt atgtacagga aaggaccatc ttcttcaaag atgacggcaa ctacaagaca 5040
cgtgctgaag tcaagtttga aggtgatacc cttgttaata gaatcgagtt aaaaggtatt 5100
gacttcaagg aagatggcaa cattctggga cacaaattgg aatacaacta taactcacac 5160
aatgtataca tcatggcaga caaacaaaag aatggaatca aagtgaactt caagacccgc 5220
cacaacattg aagatggaag cgttcaacta gcagaccatt atcaacaaaa tactccaatt 5280
ggcgatggcc ctgtcctttt accagacaac cattacctgt ccacacaatc tgccctttcg 5340
aaagatccca acgaaaagag agaccacatg gtccttcttg agtttgtaac agctgctggg 5400
attacacatg gcatggatga actgtacaac tga 5433

<210> 231
<211> 1692
<212> DNA
<213> Human

<400> 231
atggatgggt caaacgtgac atcatttgtt gttgaggaac ccacgaacat ctcaactggc 60
aggaacgcct cagtcgggaa tgcacatcgg caaatcccca tcgtgcactg ggtcattatg 120
agcatctccc cagtggggtt tgttgagaat gggattctcc tctggttcct gtgcttccgg 180
atgagaagaa atcccttcac tgtctacatc acccacctgt ctatcgcaga catctcactg 240
ctcttctgta ttttcatctt gtctatcgac tatgctttag attatgagct ttcttctggc 300
cattactaca caattgtcac attatcagtg acttttctgt ttggctacaa cacgggcctc 360
tatctgctga cggccattag tgtggagagg tgcctgtcag tcctttaccc catctggtac 420
cgatgccatc gccccaagta ccagtcggca ttggtctgtg cccttctgtg ggctctttct 480
tgcttggtga ccaccatgga gtatgtcatg tgcatcgaca gagaagaaga gagtcactct 540
cggaatgact gccgagcagt catcatcttt atagccatcc tgagcttcct ggtcttcacg 600
cccctcatgc tggtgtccag caccatcttg gtcgtgaaga tccggaagaa cacgtgggct 660
tcccattcct ccaagcttta catagtcatc atggtcacca tcattatatt cctcatcttc 720
gctatgccca tgagactcct ttacctgctg tactatgagt attggtcgac ctttgggaac 780
ctacaccaca tttccctgct cttctccaca atcaacagta gcgccaaccc tttcatttac 840
ttctttgtgg gaagcagtaa gaagaagaga ttcaaggagt ccttaaaagt tgttctgacc 900
agggctttca aagatgaaat gcaacctcgg cgccagaaag acaattgtaa tacggtcaca 960
gttgagactg tcgtcgctag caaaggagaa gaactcttca ctggagttgt cccaattctt 1020
gttgaattag atggtgatgt taacggccac aagttctctg tcagtggaga gggtgaaggt 1080
gatgcaacat acggaaaact taccctgaag ttcatctgca ctactggcaa actgcctgtt 1140
ccatggccaa cactagtcac tactctgtgc tatggtgttc aatgcttttc aagatacccg 1200
gatcatatga aacggcatga ctttttcaag agtgccatgc ccgaaggtta tgtacaggaa 1260
aggaccatct tcttcaaaga tgacggcaac tacaagacac gtgctgaagt caagtttgaa 1320
ggtgataccc ttgttaatag aatcgagtta aaaggtattg acttcaagga agatggcaac 1380

attctgggac acaaattgga atacaactat aactcacaca atgtatacat catggcagac 1440
aaacaaaaga atggaatcaa agtgaacttc aagacccgcc acaacattga agatggaagc 1500
gttcaactag cagaccatta tcaacaaaat actccaattg gcgatggccc tgtcctttta 1560
ccagacaacc attacctgtc cacacaatct gccctttcga aagatcccaa cgaaaagaga 1620
gaccacatgg tccttcttga gtttgtaaca gctgctggga ttacacatgg catggatgaa 1680
ctgtacaact ga 1692

<210> 232
<211> 2124
<212> DNA
<213> Human

<400> 232
   atgcagatgg ccgatgcagc cacgatagcc accatgaata aggcagcagg cggggacaag 60
   ctagcagaac tcttcagtct ggtcccggac cttctggagg cggccaacac gagtggtaac 120
   gcgtcgctgc agcttccgga cttgtggtgg gagctggggc tggagttgcc ggacggcgcg 180
   ccgccaggac atcccccggg cagcggcggg gcagagagcg cggacacaga ggcccgggtg 240
   cggattctca tcagcgtggt gtactgggtg gtgtgcgccc tggggttggc gggcaacctg 300
   ctggttctct acctgatgaa gagcatgcag ggctggcgca agtcctctat caacctcttc 360
   gtcaccaacc tggcgctgac ggactttcag tttgtgctca ccctgccctt ctgggcggtg 420
   gagaacgctc ttgacttcaa atggcccttc ggcaaggcca tgtgtaagat cgtgtccatg 480
   gtgacgtcca tgaacatgta cgccagcgtg ttcttcctca ctgccatgag tgtgacgcgc 540
   taccattcgg tggcctcggc tctgaagagc caccggaccc gaggacacgg ccggggcgac 600
   tgctgcggcc ggagcctggg ggacagctgc tgcttctcgg ccaaggcgct gtgtgtgtgg 660
   atctgggctt tggccgcgct ggcctcgctg cccagtgcca ttttctccac cacggtcaag 720
   gtgatgggcg aggagctgtg cctggtgcgt ttcccggaca agttgctggg ccgcgacagg 780
   cagttctggc tgggcctcta ccactcgcag aaggtgctgc tgggcttcgt gctgccgctg 840
   ggcatcatta tcttgtgcta cctgctgctg gtgcgcttca tcgccgaccg ccgcgcggcg 900
   gggaccaaag gaggggcggc ggtagccgga ggacgcccga ccggagccag cgcccggaga 960
   ctgtcgaagg tcaccaaatc agtgaccatc gttgtcctgt ccttcttcct gtgttggctg 1020
   cccaaccagg cgctcaccac ctggagcatc ctcatcaagt tcaacgcggt gcccttcagc 1080
   caggagtatt tcctgtgcca ggtatacgcg ttccctgtga gcgtgtgcct agcgcactcc 1140
   aacagctgcc tcaaccccgt cctctactgc ctcgtgcgcc gcgagttccg caaggcgctc 1200
   aagagcctgc tgtggcgcat cgcgtctcct tcgatcacca gcatgcgccc cttcaccgcc 1260
   actaccaagc cggagcacga ggatcagggg ctgcaggccc cggcgccgcc ccacgcggcc 1320
   gcggagccgg acctgctcta ctacccacct ggcgtcgtgg tctacagcgg ggggcgctac 1380
   gacctgctgc ccagcagctc tgcctacact agcaaaggag aagaactctt cactggagtt 1440
   gtcccaattc ttgttgaatt agatggtgat gttaacggcc acaagttctc tgtcagtgga 1500
   gagggtgaag gtgatgcaac atacggaaaa cttaccctga agttcatctg cactactggc 1560
   aaactgcctg ttccatggcc aacactagtc actactctgt gctatggtgt tcaatgcttt 1620
   tcaagatacc cggatcatat gaaacggcat gactttttca agagtgccat gcccgaaggt 1680
   tatgtacagg aaaggaccat cttcttcaaa gatgacggca actacaagac acgtgctgaa 1740
   gtcaagtttg aaggtgatac ccttgttaat agaatcgagt taaaaggtat tgacttcaag 1800
   gaagatggca acattctggg acacaaattg gaatacaact ataactcaca caatgtatac 1860
   atcatggcag acaaacaaaa gaatggaatc aaagtgaact tcaagacccg ccacaacatt 1920
   gaagatggaa gcgttcaact agcagaccat tatcaacaaa atactccaat tggcgatggc 1980
   cctgtccttt taccagacaa ccattacctg tccacacaat ctgccctttc gaaagatccc 2040
   aacgaaaaga gagaccacat ggtccttctt gagtttgtaa cagctgctgg gattacacat 2100
   ggcatggatg aactgtacaa ctga 2124

<210> 233
<211> 2241
<212> DNA
<213> Human

<400> 233
atgacgtcca cctgcaccaa cagcacgcgc gagagtaaca gcagccacac gtgcatgccc 60
ctctccaaaa tgcccatcag cctggcccac ggcatcatcc gctcaaccgt gctggttatc 120
ttcctcgccg cctctttcgt cggcaacata gtgctggcgc tagtgttgca gcgcaagccg 180
cagctgctgc aggtgaccaa ccgttttatc tttaacctcc tcgtcaccga cctgctgcag 240
atttcgctcg tggccccctg ggtggtggcc acctctgtgc ctctcttctg gcccctcaac 300
agccacttct gcacggccct ggttagcctc acccacctgt tcgccttcgc cagcgtcaac 360
accattgtct tggtgtcagt ggatcgctac ttgtccatca tccaccctct ctcctacccg 420
tccaagatga cccagcgccg cggttacctg ctcctctatg gcacctggat tgtggccatc 480
ctgcagagca ctcctccact ctacggctgg ggccaggctg cctttgatga gcgcaatgct 540
ctctgctcca tgatctgggg ggccagcccc agctacacta ttctcagcgt ggtgtccttc 600
atcgtcattc cactgattgt catgattgcc tgctactccg tggtgttctg tgcagcccgg 660
aggcagcatg ctctgctgta caatgtcaag agacacagct tggaagtgcg agtcaaggac 720
tgtgtggaga atgaggatga agagggagca gagaagaagg aggagttcca ggatgagagt 780
gagtttcgcc gccagcatga aggtgaggtc aaggccaagg agggcagaat ggaagccaag 840
gacggcagcc tgaaggccaa ggaaggaagc acggggacca gtgagagtag tgtagaggcc 900
aggggcagcg aggaggtcag agagagcagc acggtggcca gcgacggcag catggagggt 960
aaggaaggca gcaccaaagt tgaggagaac agcatgaagg cagacaaggg tcgcacagag 1020
gtcaaccagt gcagcattga cttgggtgaa gatgacatgg agtttggtga agacgacatc 1080
aatttcagtg aggatgacgt cgaggcagtg aacatcccgg agagcctccc acccagtcgt 1140

cgtaacagca acagcaaccc tcctctgccc aggtgctacc agtgcaaagc tgctaaagtg 1200
atcttcatca tcattttctc ctatgtgcta tccctggggc cctactgctt tttagcagtc 1260
ctggccgtgt gggtggatgt cgaaacccag gtaccccagt gggtgatcac cataatcatc 1320
tggcttttct tcctgcagtg ctgcatccac ccctatgtct atggctacat gcacaagacc 1380
attaagaagg aaatccagga catgctgaag aagttcttct gcaaggaaaa gcccccgaaa 1440
gaagatagcc acccagacct gcccggaaca gagggtggga ctgaaggcaa gattgtccct 1500
tcctacgatt ctgctacttt tcctgctagc aaaggagaag aactcttcac tggagttgtc 1560
ccaattcttg ttgaattaga tggtgatgtt aacggccaca agttctctgt cagtggagag 1620
ggtgaaggtg atgcaacata cggaaaactt accctgaagt tcatctgcac tactggcaaa 1680
ctgcctgttc catggccaac actagtcact actctgtgct atggtgttca atgcttttca 1740
agatacccgg atcatatgaa acggcatgac tttttcaaga gtgccatgcc cgaaggttat 1800
gtacaggaaa ggaccatctt cttcaaagat gacggcaact acaagacacg tgctgaagtc 1860
aagtttgaag gtgataccct tgttaataga atcgagttaa aaggtattga cttcaaggaa 1920
gatggcaaca ttctgggaca caaattggaa tacaactata actcacacaa tgtatacatc 1980
atggcagaca aacaaaagaa tggaatcaaa gtgaacttca agacccgcca caacattgaa 2040
gatggaagcg ttcaactagc agaccattat caacaaaata ctccaattgg cgatggccct 2100
gtccttttac cagacaacca ttacctgtcc acacaatctg ccctttcgaa agatcccaac 2160
gaaaagagag accacatggt ccttcttgag tttgtaacag ctgctgggat tacacatggc 2220
atggatgaac tgtacaactg a 2241